# EUROPEAN PATENT SPECIFICATION

(11) **EP 4 251 609 B1**
(45) Date of publication and mention of the grant of the patent: **23.04.2025**
(21) Application number: 21810637.5
(22) Date of filing: 24.11.2021
(51) Int. Cl.: C07D 207/16, C07D 305/06, C07D 307/16, C07D 307/28, C07D 309/28, C07D 317/32, C07D 327/04, C07D 493/18, A01N 43/08, A01N 43/16, A01N 43/20, A01N 43/28, A01N 43/36

(54) **HERBICIDAL MALONAMIDES**
HERBIZIDE MALONAMIDE
MALONAMIDES HERBICIDES

(30) Priority: 25.11.2020 EP 20209750; 01.09.2021 EP 21194334
(43) Date of publication of application: 04.10.2023
(73) Proprietor: BASF SE, 67056 Ludwigshafen am Rhein (DE)
(72) Inventor: ZIMMERMANN, Gunther, 67056 Ludwigshafen (DE); KORDES, Markus, 67056 Ludwigshafen (DE); SEISER, Tobias, 67056 Ludwigshafen (DE); HEINRICH, Marc, 67056 Ludwigshafen (DE); KRAEMER, Gerd, 67117 Limburgerhof (DE); SEITZ, Thomas, 67056 Ludwigshafen (DE); NEWTON, Trevor William, 67117 Limburgerhof (DE)
(74) Representative: BASF IP Association
(86) International application number: PCT/EP2021/082864
(87) International publication number: WO 2022/112351

(56) References cited:
- WO-A1-2012/130798
- SONG ET AL.: "One pot synthesis of [alpha],[alpha]-bis(N-arylamido) lactams via iodide-catalyzed rearrangement of [beta],[beta]-bis(N-arylamido) cyclic ketene-N,O-acetals", TETRAHEDRON LETT., vol. 52, no. 8, 2011, Amsterdam, NL, pages 853 - 858, XP055883410, ISSN: 0040-4039, DOI: 10.1016/j.tetlet.2010.11.148

## Description

The present invention relates to malonamide compounds and compositions comprising the same. The invention also relates to the use of the malonamide compounds or the corresponding compositions for controlling unwanted vegetation. Furthermore, the invention relates to methods of applying the malonamide compounds or the corresponding compositions.

For the purpose of controlling unwanted vegetation, especially in crops, there is an ongoing need for new herbicides that have high activity and selectivity together with a substantial lack of toxicity for humans and animals.

WO 2012/130798, WO 2014/04882, WO 2014/048882, WO 2018/228985, WO 2018/228986, WO 2019/034602, WO 2019/145245, WO 2020/114932, WO 2020/114934 and WO2020/182723 describe 3-phenylisoxazoline-5-carboxamides and their use as herbicides.

WO 87/05898 describes the use of malonic acid derivatives for retarding plant growth.

Malonic acid derivatives are also described in US3,072,473 as plant growth regulants.

WO 01/12183 describes the use of malonic acid derivatives, characterized by a monocyclic saturated heterocyclic ring having up to two heteroatoms, for the treatment of cell-adhesion mediated pathologies.

The compounds of the prior art often suffer from insufficient herbicidal activity, in particular at low application rates, and/or unsatisfactory selectivity resulting in a low compatibility with crop plants.

Accordingly, it is an object of the present invention to provide further malonamide compounds having a strong herbicidal activity, in particular even at low application rates, a sufficiently low toxicity for humans and animals and/or a high compatibility with crop plants. The malonamide compounds should also show a broad activity spectrum against a large number of different unwanted plants.

These and further objectives are achieved by the compounds of formula (I) defined below including their agriculturally acceptable salts.

Accordingly, the present invention provides compounds of formula (I) 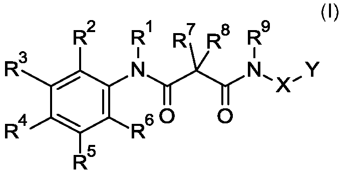
wherein the substituents have the following meanings:
   - R¹: is hydrogen;
   - R²: is hydrogen or halogen;
   - R³: is hydrogen, halogen, cyano, (C₁-C₃)-alkyl, (C₁-C₃)-haloalkyl, (C₁-C₃)-alkoxy or (C₁-C₃)-haloalkoxy;
   - R⁴: is hydrogen or halogen;
   - R⁵: is hydrogen, halogen, cyano, (C₁-C₃)-alkyl, (C₁-C₃)-haloalkyl, (C₁-C₃)-alkoxy or (C₁-C₃)-haloalkoxy;
   - R⁶: is hydrogen;
   - R⁷ and R⁸: form, together with the carbon atom to which they are bound, a saturated or partially unsaturated 3-, 4-, 5- or 6-membered monocyclic heterocyclic ring W or a 6-, 7- or 8-membered bicyclic heterocyclic ring W, the ring containing, in addition to said carbon atom, q carbon atoms, u oxygen atoms, v nitrogen atoms, w sulfur atoms and x elements selected from the group consisting of NR^{d} and NC(O)OR^{d}, where one carbon atom bears p oxo groups and where the ring is substituted by n radicals R^{g};
   - R⁹: is hydrogen or (C₁-C₄)-alkyl;
   - X: is a bond and Y is Z, where
   Z is a 3-, 4-, 5-, 6-, 7- or 8-membered saturated or partly unsaturated monocyclic carbocyclic ring substituted by a group CO₂R^{e}, by 0 or 1 fluorine atoms and by 0 or 1 (C₁-C₄)-alkyl groups; or
   Z is a 5-, 6-, 7- or 8-membered saturated or partly unsaturated bicyclic carbocyclic ring substituted by a group CO₂R^{e}, and by 0 or 1 (C₁-C₄)-alkyl groups; or
   Z is an 8-membered saturated polycyclic carbocyclic ring substituted by a group CO₂R^{e} and by 0 or 1 (C₁-C₄)-alkyl groups; or
   Z is a 5- or 6-membered saturated or partly unsaturated monocyclic heterocyclic ring containing one oxygen atom or one sulfur atom as ring member, where the heterocyclic ring is substituted by a group CO₂R^{e} and by 0 or 1 (C₁-C₄)-alkyl groups; or
   Z is a 5- or 6-membered heteroaromatic ring containing 1, 2, 3 or 4 heteroatoms selected from N, O and S as ring members, where the heteroaromatic ring is substituted by 0 or 1 (C₁-C₄)-alkyl groups; or
   - X: is a bond and Y is (C₁-C₆)-alkyl or (C₂-C₆)-alkenyl, where the two last-mentioned radicals are substituted by a group CO₂R^{e}, CONR^{b}R^{h} or CONR^{e}SO₂R^{a} and by 0 or 1 (C₁-C₄)-alkoxy groups; or
   - X: is a bond and Y is (C₁-C₆)-alkyl substituted by Z, where Z is a 3-, 4-, 5- or 6-membered saturated monocyclic carbocyclic ring substituted by a group CO₂R^{e} and by 0 or 1 (C₁-C₄)-alkyl groups or is a 5- or 6-membered saturated monocyclic heterocyclic ring containing 1 oxygen atom as ring member, where the heterocyclic ring is substituted by a group CO₂R^{e} and by 0 or 1 (C₁-C₄)-alkyl groups; or
   - X: is a bond and Y is (C₂-C₈)-alkynyl; or
   - X: is X⁶ 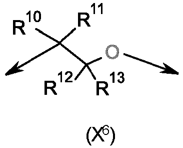 wherein R¹⁰ to R¹³ are independently hydrogen or methyl, preferably hydrogen; and Y is (C₁-C₆)-alkyl substituted by a group CO₂R^{e} and by 0 or 1 (C₁-C₄)-alkoxy groups;
   - R^{a}: is (C₁-C₆)-alkyl;
   - R^{b}: is hydrogen or (C₁-C₆)-alkyl;
   - each R^{d}: is independently hydrogen or (C₁-C₆)-alkyl;
   - each R^{e}: is independently hydrogen, (C₁-C₆)-alkyl which is unsubstituted or substituted by 1, 2 or 3 fluorine or chlorine atoms or by 1 radical selected from the group consisting of (C₁-C₂)-alkoxy, (C₁-C₃)-alkylsulfonyl, (C₁-C₃)-alkylthio, phenylthio, phenylsulfonyl and furanyl; or is (C₂-C₄)-alkynyl, (C₃-C₆)-cycloalkyl or (C₃-C₆)-cycloalkyl-(C₁-C₃)-alkyl;
   - R^{g}: is (C₁-C₃)-alkyl or (C₁-C₃)-haloalkyl, or two R^{g}, bound on the same carbon atom, form together a methylene group (=CH₂);
   - R^{h}: is hydrogen, (C₁-C₆)-alkyl substituted by 0 or 1 cyano groups; or (C₂-C₄)-alkynyl;
   n is 0, 1 or 2;
   p is 0 or 1;
   q is 1, 2, 3, 4, 5 or 6;
   u is 0, 1 or 2;
   v is 0 or 1;
   w is 0 or 1;
   x is 0 or 1;
with the proviso that the sum of u, v, w and x is 1 or 2
including their agriculturally acceptable salts, stereoisomers and tautomers.

The present invention also provides formulations comprising at least one compound of formula (I) and auxiliaries customary for formulating crop protection agents.

The present invention also provides combinations comprising at least one compound of formula (I) (component A) and at least one further compound selected from the herbicidal compounds B (component B) and safeners C (component C).

The present invention also provides the use of compounds of formula (I) as herbicides, i.e. for controlling undesired vegetation.

The present invention furthermore provides a method for controlling undesired vegetation where a herbicidal effective amount of at least one compound of formula (I) is allowed to act on plants, their seeds and/or their habitat.

If the compounds of formula (I), the herbicidal compounds B and/or the safeners C as described herein are capable of forming geometric isomers, for example E/Z isomers, it is possible to use both, the pure isomers and mixtures thereof, according to the invention.

If the compounds of formula (I), the herbicidal compounds B and/or the safeners C as described herein have one or more centres of chirality and, as a consequence, are present as enantiomers or diastereomers, it is possible to use both, the pure enantiomers and diastereomers and their mixtures, according to the invention. Just by way of example, a stereogenic center in compounds (I) is the C atom carrying R¹⁰ and R¹¹ in X⁶, provided of course that R¹⁰ and R¹¹ are different. Another example for a stereogenic center is the C atom carrying R⁷ and R⁸, provided the ring formed by these radicals and the carbon atom to which they are bound has not rotary mirror axis.

If the compounds of formula (I), the herbicidal compounds B and/or the safeners C as described herein have ionizable functional groups, they can also be employed in the form of their agriculturally acceptable salts. Suitable are, in general, the salts of those cations and the acid addition salts of those acids whose cations and anions, respectively, have no adverse effect on the activity of the active compounds. Just by way of example, a ionizable functional group is -CO₂R^{e}, wherein R^{e} is hydrogen. Compounds (I) containing such -C(O)OH groups can be used in form of their salts, i.e. in form of compounds containing one or more groups -C(O)O⁻M⁺, where M⁺ is a cation equivalent. Examples for agriculturally suitable cations are given below.

Preferred cations are the ions of the alkali metals, preferably of lithium, sodium and potassium, of the alkaline earth metals, preferably of calcium and magnesium, and of the transition metals, preferably of manganese, copper, zinc and iron, further ammonium and substituted ammonium in which one to four hydrogen atoms are replaced by C₁-C₄-alkyl, hydroxy-C₁-C₄-alkyl, C₁-C₄-alkoxy-C₁-C₄-alkyl, hydroxy-C₁-C₄-alkoxy-C₁-C₄-alkyl, phenyl or benzyl, preferably ammonium, methylammonium, isopropylammonium, dimethylammonium, diethylammonium, diisopropylammonium, trimethylammonium, triethylammonium, tris(isopropyl)ammonium, heptylammonium, dodecylammonium, tetradecylammonium, tetramethylammonium, tetraethylammonium, tetrabutylammonium, 2-hydroxyethylammonium (olamine salt), 2-(2-hydroxyeth-1-oxy)eth-1-ylammonium (diglycolamine salt), di(2-hydroxyeth-1-yl)ammonium (diolamine salt), tris(2-hydroxyethyl)ammonium (trolamine salt), tris(2-hydroxypropyl)ammonium, benzyltrimethylammonium, benzyltriethylammonium, N,N,N-trimethylethanolammonium (choline salt), furthermore phosphonium ions, sulfonium ions, preferably tri(C₁-C₄-alkyl)sulfonium, such as trimethylsulfonium, and sulfoxonium ions, preferably tri(C₁-C₄-alkyl)sulfoxonium, and finally the salts of polybasic amines such as N,N-bis-(3-aminopropyl)methylamine and diethylenetriamine.

Anions of useful acid addition salts are primarily chloride, bromide, fluoride, iodide, hydrogensulfate, methylsulfate, sulfate, dihydrogenphosphate, hydrogenphosphate, nitrate, bicarbonate, carbonate, hexafluorosilicate, hexafluorophosphate, benzoate and also the anions of C₁-C₄-alkanoic acids, preferably formate, acetate, propionate and butyrate.

The compounds (I) may be present in form of different tautomers. For instance, in the two mandatorily present amide groups of the malonamide moiety, the moiety -N(H)-C(=O)- can be in equilibrium with its tautomeric form -N=C(OH)-. To be more precise, in the two mandatorily present amide groups of the malonamide moiety -N(R¹)-C(=O)-C(R⁷)(R⁸)-C(=O)-N(R⁹)- if one or both of R¹ and R⁹ are hydrogen:
If only R¹ is hydrogen, the malonamide moiety can be present as -N(H)-C(=O)-C(R⁷)(R⁸)-C(=O)-N(R⁹)- or as -N=C(OH)-C(R⁷)(R⁸)-C(=O)-N(R⁹)- or as a mixture of the two forms;
If only R⁹ is hydrogen, the malonamide moiety can be present as -N(R¹)-C(=O)-C(R⁷)(R⁸)-C(=O)-N(H)- or as -N(R¹)-C(=O)-C(R⁷)(R⁸)-C(OH)=N- or as a mixture of the two forms;
If both of R¹ and R⁹ are hydrogen, the malonamide moiety can be present as -N(H)-C(=O)-C(R⁷)(R⁸)-C(=O)-N(H)- or as -N=C(OH)-C(R⁷)(R⁸)-C(=O)-N(H)- or as -N(H)-C(=O)-C(R⁷)(R⁸)-C(OH)=N- or as -N=C(OH)-C(R⁷)(R⁸)-C(OH)=N- or as mixture of two, three all four of the above forms.

The amount in which the one or other tautomeric form is present depends on the complete molecular structure and even stronger on the surrounding conditions (presence or absence of solvent, type of solvent, pH, temperature etc.).

Compounds of formula (I), herbicidal compounds B and/or safeners C as described herein having a carboxyl group can be employed in the form of the acid, in the form of an agriculturally suitable salt as mentioned above or else in the form of an agriculturally acceptable derivative, for example as amides, such as mono- and di-C₁-C₆-alkylamides or arylamides, as esters, for example as allyl esters, propargyl esters, C₁-C₁₀-alkyl esters, alkoxyalkyl esters, tefuryl ((tetrahydrofuran-2-yl)methyl) esters and also as thioesters, for example as C₁-C₁₀-alkylthio esters. Preferred mono- and di-C₁-C₆-alkylamides are the methyl and the dimethylamides. Preferred arylamides are, for example, the anilides and the 2-chloroanilides. Preferred alkyl esters are, for example, the methyl, ethyl, propyl, isopropyl, butyl, isobutyl, pentyl, mexyl (1-methylhexyl), meptyl (1-methylheptyl), heptyl, octyl or isooctyl (2-ethylhexyl) esters. Preferred C₁-C₄-alkoxy-C₁-C₄-alkyl esters are the straight-chain or branched C₁-C₄-alkoxy ethyl esters, for example the 2-methoxyethyl, 2-ethoxyethyl, 2-butoxyethyl (butotyl), 2-butoxypropyl or 3-butoxypropyl ester. An example of a straight-chain or branched C₁-C₁₀-alkylthio ester is the ethylthio ester.

The term "undesired vegetation" ("weeds") is understood to include any vegetation growing in non-crop-areas or at a crop plant site or locus of seeded and otherwise de-sired crop, where the vegetation is any plant species, including their germinant seeds, emerging seedlings and established vegetation, other than the seeded or desired crop (if any). Weeds, in the broadest sense, are plants considered undesirable in a particular location.

The terms used for organic groups in the definition of the variables are, for example the expression "halogen", collective terms which represent the individual members of these groups of organic units.

The prefix Cₓ-C_{y} denotes the number of possible carbon atoms in the particular case. All hydrocarbon chains can be straight-chain or branched.

Halogen: fluorine, chlorine, bromine, or iodine, especially fluorine, chlorine or bromine.

Alkyl and the alkyl moieties of composite groups such as, for example, alkoxy, alkylamino, alkoxycarbonyl: saturated straight-chain or branched hydrocarbon radicals having 1 to 12 carbon atoms (= C₁-C₁₂-alkyl) or 1 to 10 carbon atoms (C₁-C₁₀-akyl), frequently from 1 to 6 carbon atoms (= C₁-C₆-alkyl), in particular 1 to 4 carbon atoms (= C₁-C₄-alkyl) and especially from 1 to 3 carbon atoms (= C₁-C₃-alkyl) or 1 or 2 carbon atoms (= C₁-C₂-alkyl). C₁-C₂-Alkyl is methyl or ethyl. C₁-C₃-Alkyl is methyl, ethyl, n-propyl or iso-propyl. Examples of C₁-C₄-alkyl are methyl, ethyl, n-propyl, iso-propyl, n-butyl, 2-butyl (= sec-butyl), isobutyl and tert-butyl. Examples for C₁-C₆-alkyl are, in addition to those mentioned for C₁-C₄-alkyl, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl. Examples for C₁-C₈-alkyl are, in addition to those mentioned for C₁-C₆-alkyl, n-heptyl, 1-methylhexyl, 2-methylhexyl, 3-methylhexyl, 4-methylhexyl, 5-methylhexyl, 1-ethylpentyl, 2-ethylpentyl, 3-ethylpentyl, n-octyl, 1-methylheptyl, 2-methylheptyl, 1-ethylhexyl, 2-ethylhexyl, 1,2-dimethylhexyl, 1-propylpentyl and 2-propylpentyl. Examples for C₁-C₁₂-alkyl are, apart those mentioned for C₁-C₈-alkyl, nonyl, decyl, 2-propylheptyl, 3-propylheptyl, undecyl, dodecyl and positional isomers thereof.

Haloalkyl: straight-chain or branched alkyl groups having 1 to 10 carbon atoms (= C₁-C₁₀-haloalkyl), usually from 1 to 6 carbon atoms (= C₁-C₆-haloalkyl), more frequently 1 to 3 carbon atoms (= C₁-C₃-haloalkyl) (as mentioned above), where some or all of the hydrogen atoms in these groups are replaced by halogen atoms as mentioned above. In one embodiment, the alkyl groups are substituted at least once or completely by a particular halogen atom, preferably fluorine, chlorine or bromine. In a further embodiment, the alkyl groups are partially or fully halogenated by different halogen atoms; in the case of mixed halogen substitutions, the combination of chlorine and fluorine is preferred. Particular preference is given to (C₁-C₃)-haloalkyl, more preferably (C₁-C₂)-haloalkyl, such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl or pentafluoroethyl. Examples for C₁-C₃-haloalkyl are, in addition to those mentioned for C₁-C₂-haloalkyl, 1-fluoropropyl, 2-fluoropropyl, 3-fluoropropyl, 3,3-difluoropropyl, 3,3,3-trifluoropropyl, heptafluoropropyl, 1,1,1-trifluoroprop-2-yl, 3-chloropropyl.

Hydroxyalkyl denotes a straight-chain or branched alkyl group having usually from 1 to 6 carbon atoms (= C₁-C₆-hydroxyalkyl), more frequently 1 to 3 carbon atoms (= C₁-C₃-hydroxyalkyl), as defined above, wherein one hydrogen atom of this group is replaced with a hydroxyl group. Examples are hydroxymethyl, 1-hydroxyethyl, 2-hydroxyethyl, 1-hydroxypropyl, 2-hydroxypropyl, 3-hydroxypropyl, 1-hydroxy-2-propyl, 2-hydroxy-2-propyl and the like.

Alkenyl and also the alkenyl moieties in composite groups, such as alkenyloxy: unsaturated straight-chain or branched hydrocarbon radicals having 2 to 12 carbon atoms (= C₂-C₁₂-alkenyl) or 2 to 10 carbon atoms (= C₂-C₁₀-alkenyl), e.g. 2 to 8 carbon atoms (= C₂-C₈-alkenyl) or 2 to 6 carbon atoms (= C₂-C₆-alkenyl), in particular 2 to 4 carbon atoms (= C₂-C₄-alkenyl) or 2 or 3 carbon atoms (= C₂-C₃-alkenyl); and one double bond in any position. According to the invention, it may be preferred to use small alkenyl groups, such as (C₂-C₄)-alkenyl or C₂-C₃-alkenyl; on the other hand, it may also be preferred to employ larger alkenyl groups, such as (C₅-C₈)-alkenyl. Examples of C₂-C₃-alkenyl groups are ethenyl, 1-propenyl, 2-propenyl, and 1-methylethenyl; examples of C₂-C₄-alkenyl groups are, in addition to those mentioned for C₂-C₃-alkenyl, 1-butenyl, 2-butenyl, 3-butenyl, 1-methyl-1-propenyl, 2-methyl-1-propenyl, 1-methyl-2-propenyl, and 2-methyl-2-propenyl; examples of C₂-C₆-alkenyl groups are, in addition to those mentioned for C₂-C₄-alkenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl; examples of C₂-C₁₂-alkenyl groups are, in addition to those mentioned for C₂-C₆-alkenyl, 1-heptenyl, 2-heptenyl, 3-heptenyl, 1-octenyl, 2-octenyl, 3-octenyl, 4-octenyl, the nonenyls, decenyls, undecenyls, dodecenyls and the positional isomers thereof. Examples for C₃-C₆-alkenyl are those mentioned above for C₂-C₆-alkenyl, except for ethenyl.

Haloalkenyl: alkenyl groups as mentioned above which are partially or fully substituted by fluorine, chlorine, bromine and/or iodine, for example 2-chloroprop-2-en-1-yl, 3-chloroprop-2-en-1-yl, 2,3-dichloroprop-2-en-1-yl, 3,3-dichloroprop-2-en-1-yl, 2,3,3-trichloro-2-en-1-yl, 2,3-dichlorobut-2-en-1-yl, 2-bromoprop-2-en-1-yl, 3-bromoprop-2-en-1-yl, 2,3-dibromoprop-2-en-1-yl, 3,3-dibromoprop-2-en-1-yl, 2,3,3-tribromo-2-en-1-yl or 2,3-dibromobut-2-en-1-yl.

Alkynyl and the alkynyl moieties in composite groups, such as alkynyloxy: straight-chain or branched hydrocarbon groups having 2 to 12 carbon atoms (= C₂-C₁₂-alkynyl) or 2 to 10 carbon atoms (= C₂-C₁₀-alkynyl), e.g. 2 to 8 carbon atoms (= C₂-C₈-alkynyl) or 2 to 6 carbon atoms (= C₂-C₆-alkynyl), in particular 2 to 4 carbon atoms (= C₂-C₄-alkynyl) or 2 or 3 carbon atoms (= C₂-C₃-alkynyl); and one or two triple bonds in any position. Examples of C₂-C₃-alkynyl groups are ethynyl, 1-propynyl and 2-propynyl; examples of C₂-C₄-alkynyl groups are, in addition to those mentioned for C₂-C₃-alkynyl, 1-butynyl, 2-butynyl, 3-butynyl, and 1-methyl-2-propynyl; examples of C₂-C₆-alkynyl groups are, in addition to those mentioned for C₂-C₃-alkynyl, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-ethyl-2-propynyl, 1-hexynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl and 1-ethyl-1-methyl-2-propynyl.

Haloalkynyl: alkynyl groups as mentioned above which are partially or fully substituted by fluorine, chlorine, bromine and/or iodine, for example 1,1-difluoroprop-2-yn-1-yl, 3-chloroprop-2-yn-1-yl, 3-bromoprop-2-yn-1-yl, 3-iodoprop-2-yn-1-yl, 4-fluorobut-2-yn-1-yl, 4-chlorobut-2-yn-1-yl, 1,1-difluorobut-2-yn-1-yl, 4-iodobut-3-yn-1-yl, 5-fluoropent-3-yn-1-yl, 5-iodopent-4-yn-1-yl, 6-fluorohex-4-yn-1-yl or 6-iodohex-5-yn-1-yl

Cycloalkyl and also the cycloalkyl moieties in composite groups: mono- or bicyclic saturated hydrocarbon groups having 3 to 10 (= C₃-C₁₀ -cycloalkyl) or 3 to 8 (= C₃-C₈-cycloalkyl), in particular 3 to 6 (= C₃-C₆-cycloalkyl), 3 to 5 (= C₃-C₅-cycloalkyl) or 3 to 4 (= C₃-C₄-cycloalkyl) carbon atoms as (only) ring members. Examples of monocyclic saturated cycloaliphatic radicals having 3 or 4 carbon atoms are cyclopropyl and cyclobutyl. Examples of monocyclic saturated cycloaliphatic radicals having 3 to 5 carbon atoms are cyclopropyl, cyclobutyl and cyclopentyl. Examples of monocyclic saturated cycloaliphatic radicals having 3 to 6 carbon atoms are cyclopropyl, cyclobutyl, cyclopentyl and cyclohexyl. Examples of monocyclic saturated cycloaliphatic radicals having 3 to 8 carbon atoms are cyclopropyl, cyclobutyl, cyclopentyl, cyclohexyl, cycloheptyl or cyclooctyl. Examples of bicyclic radicals comprise bicyclo[1.1.1]pentyl, bicyclo[2.1.1]hexyl, bicyclo[2.2.1]heptyl, bicyclo[3.1.1]heptyl, bicyclo[2.2.2]octyl and bicyclo[3.2.1]octyl.

Halocycloalkyl and the halocycloalkyl moieties in halocycloalkoxy, halocycloalkylcarbonyl and the like: mono- or bicyclic saturated hydrocarbon groups having 3 to 10 (C₃-C₁₀-halocycloalkyl) or 3 to 8 (C₃-C₈-halocycloalkyl), preferably 3 to 5 (C₃-C₅-halocycloalkyl) or 3 to 4 (C₃-C₄-halocycloalkyl) carbon ring members (as mentioned above), in which some or all of the hydrogen atoms may be replaced by halogen atoms as mentioned above, in particular fluorine, chlorine and bromine. Examples are 1- and 2- fluorocyclopropyl, 1,2-, 2,2- and 2,3-difluorocyclopropyl, 1,2,2-trifluorocyclopropyl, 2,2,3,3-tetrafluorocyclpropyl, 1- and 2-chlorocyclopropyl, 1,2-, 2,2- and 2,3-dichlorocyclopropyl, 1,2,2-trichlorocyclopropyl, 2,2,3,3-tetrachlorocyclpropyl, 1-,2- and 3-fluorocyclopentyl, 1,2-, 2,2-, 2,3-, 3,3-, 3,4-, 2,5-difluorocyclopentyl, 1-,2- and 3-chlorocyclopentyl, 1,2-, 2,2-, 2,3-, 3,3-, 3,4-, 2,5-dichlorocyclopentyl and the like.

Hydroxycycloalkyl: a mono- or bicyclic cycloaliphatic radical having usually from 3 to 6 carbon atoms ("C₃-C₆-hydroxycycloalkyl"), preferably 3 to 5 carbon atoms ("C₃-C₅-hydroxycycloalkyl"), wherein at least one, e.g. 1, 2, 3, 4 or 5 of the hydrogen atoms are replaced by a hydroxyl group. Examples are 1-hydroxycyclopropyl, 2-hydroxycyclopropyl, 1,2-dihydroxycyclopropyl, 2,3-dihydroxycyclopropyl, 1-hydroxycyclobutyl, 2-hydroxycyclobutyl, 3-hydroxycyclobutyl, 1,2-dihydroxycyclobutyl, 1,3-dihydroxycyclobutyl, 2,3-dihydroxycyclobutyl, 1-hydroxycyclopentyl, 2-hydroxycyclopentyl, 3-hydroxycyclopentyl, 1,2-dihydroxycyclopentyl, 1,3-dihydroxycyclopentyl, 2,3-dihydroxycyclopentyl and the like.

Cycloalkyl-alkyl: alkyl group, as defined above, where one hydrogen atom is replaced by a cycloalkyl group, as defined above. The term "C₃-C₆-cycloalkyl-C₁-C₃-alkyl" as used herein, refers to an alkyl group having 1 to 3 carbon atoms, as defined above, where one hydrogen atom is replaced by a C₃-C₆-cycloalkyl group, as defined above. Examples are cyclopropylmethyl, cyclopropyl-1-ethyl, cyclopropyl-2-ethyl, cyclopropyl-1-propyl, cyclopropyl-2-propyl, cyclopropyl-3-propyl, cyclobutylmethyl, cyclobutyl-1-ethyl, cyclobutyl-2-ethyl, cyclobutyl-1-propyl, cyclobutyl-2-propyl, cyclobutyl-3-propyl, cyclopentylmethyl, cyclopentyl-1-ethyl, cyclopentyl-2-ethyl, cyclopentyl-1-propyl, cyclopentyl-2-propyl, cyclopentyl-3-propyl, cyclohexylmethyl, cyclohexyl-1-ethyl, cyclohexyl-2-ethyl, cyclohexyl-1-propyl, cyclohexyl-2-propyl, cyclohexyl-3-propyl and the like.

Alkoxy and also the alkoxy moieties in composite groups, such as alkoxyalkyl: an alkyl group as defined above which is attached via an oxygen atom to the remainder of the molecule, generally having 1 to 10 (C₁-C₁₀-alkoxy), preferably 1 to 6 (C₁-C₆-alkoxy), 1 to 4 (C₁-C₄-alkoxy), 1 to 3 (C₁-C₃-alkoxy) or 1 to 2 (C₁-C₂-alkoxy) carbon atoms. Examples for C₁-C₂-alkoxy are methoxy and ethoxy; examples for C₁-C₃-alkoxy are, in addition to those mentioned for C₁-C₂-alkoxy, n-propoxy and 1-methylethoxy (isopropoxy); examples for C₁-C₄-alkoxy are, in addition to those mentioned for C₁-C₃-alkoxy, butoxy, 1-methylpropoxy, 2-methylpropoxy or 1,1-di-methylethoxy; examples for C₁-C₆-alkoxy are, in addition to those mentioned for C₁-C₄-alkoxy, pentoxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy or 1-ethyl-2-methylpropoxy.

Haloalkoxy: alkoxy as defined above, where some or all of the hydrogen atoms in these groups are replaced by halogen atoms as described above under haloalkyl, in particular by fluorine, chlorine or bromine. Examples are OCH₂F, OCHF₂, OCF₃, OCH₂Cl, OCHCl₂, OCCl₃, chlor-ofluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy, OC₂F₅, 2-fluoropropoxy, 3-fluoropropoxy, 2,2-difluoropropoxy, 2,3-difluoropropoxy, 2-chloropropoxy, 3-chloropropoxy, 2,3-dichloropropoxy, 2-bromopropoxy, 3-bromopropoxy, 3,3,3-trifluoropropoxy, 3,3,3-trichloropropoxy, OCH₂-C₂F₅, OCF₂-C₂F₅, 1-(CH₂F)-2-fluoroethoxy, 1-(CH₂Cl)-2-chloroethoxy, 1-(CH₂Br)-2-bromoethoxy, 4-fluorobutoxy, 4-chlorobutoxy, 4-bromobutoxy or nonafluorobutoxy; and also 5-fluoropentoxy, 5-chloropentoxy, 5-bromopentoxy, 5-iodopentoxy, undecafluoropentoxy, 6-fluorohexoxy, 6-chlorohexoxy, 6-bromohexoxy, 6-iodohexoxy or dodecafluorohexoxy.

Alkenyloxy: alkenyl group, as defined above, attached via an oxygen atom to the remainder of the molecule. C₂-C₆-Alkenyloxy is a C₂-C₆-alkenyl group, as defined above, attached via an oxygen atom to the remainder of the molecule. C₃-C₆-Alkenyloxy is a C₃-C₆-alkenyl group, as defined above, attached via an oxygen atom to the remainder of the molecule.

Haloalkenyloxy: haloalkenyl group, as defined above, attached via an oxygen atom to the remainder of the molecule. C₂-C₆-Haloalkenyloxy is a C₂-C₆-haloalkenyl group, as defined above, attached via an oxygen atom to the remainder of the molecule. C₃-C₆-Haloalkenyloxy is a C₃-C₆-haloalkenyl group, as defined above, attached via an oxygen atom to the remainder of the molecule.

Alkynyloxy: alkynyl group, as defined above, attached via an oxygen atom to the remainder of the molecule. C₂-C₆-Alkynyloxy is a C₂-C₆-alkynyl group, as defined above, attached via an oxygen atom to the remainder of the molecule. C₃-C₆-Alkynyloxy is a C₃-C₆-alkynyl group, as defined above, attached via an oxygen atom to the remainder of the molecule.

Haloalkynyloxy: haloalkynyl group, as defined above, attached via an oxygen atom to the remainder of the molecule. C₂-C₆-Haloalkynyloxy is a C₂-C₆-haloalkynyl group, as defined above, attached via an oxygen atom to the remainder of the molecule. C₃-C₆-Haloalkynyloxy is a C₃-C₆-haloalkynyl group, as defined above, attached via an oxygen atom to the remainder of the molecule.

Cycloalkoxy: cycloalkyl group, as defined above, attached via an oxygen atom to the remainder of the molecule. C₃-C₆-Cycloalkoxy is a C₃-C₆-cycloalkyl group, as defined above, attached via an oxygen atom to the remainder of the molecule. Examples of C₃-C₆-cycloalkoxy comprise cyclopropoxy, cyclobutoxy, cyclopentoxy and cyclohexoxy.

Alkoxy-alkyl: alkyl group, as defined above, where one hydrogen atom is replaced by another alkoxy group, as defined above. The term "C₁-C₃-alkoxy-C₁-C₃-alkyl" as used herein, refers to an alkyl group having 1 to 3 carbon atoms, as defined above, where one hydrogen atom is replaced by a C₁-C₃-alkoxy group, as defined above. Examples are methoxymethyl, ethoxymethyl, propoxymethyl, isopropoxymethyl, 1-methoxyethyl, 1-ethoxyethyl, 1-propoxyethyl, 1-isopropoxyethyl, 2-methoxyethyl, 2-ethoxyethyl, 2-propoxyethyl, 2-isopropoxyethyl, 1-methoxypropyl, 1-ethoxypropyl, 1-propoxypropyl, 1-isopropoxypropyl, 2-methoxypropyl, 2-ethoxypropyl, 2-propoxypropyl, 2-isopropoxypropyl, 3-methoxypropyl, 3-ethoxypropyl, 3-propoxypropyl, 3-isopropoxypropyl, and the like.

Alkoxy-alkoxy: alkoxy group, as defined above, where one hydrogen atom is replaced by another alkoxy group, as defined above. The term "C₁-C₃-alkoxy-C₁-C₃-alkoxy" as used herein, refers to an alkoxy group having 1 to 3 carbon atoms, as defined above, where one hydrogen atom is replaced by a C₁-C₃-alkoxy group, as defined above. Examples are methoxymethoxy, ethoxymethoxy, propoxymethoxy, isopropoxymethoxy, 1-methoxyethoxy, 1-ethoxyethoxy, 1-propoxyethoxy, 1-isopropoxyethoxy, 2-methoxyethoxy, 2-ethoxyethoxy, 2-propoxyethoxy, 2-isopropoxyethoxy, 1-methoxypropoxy, 1-ethoxypropoxy, 1-propoxypropoxy, 1-isopropoxypropoxy, 2-methoxypropoxy, 2-ethoxypropoxy, 2-propoxypropoxy, 2-isopropoxypropoxy, 3-methoxypropoxy, 3-ethoxypropoxy, 3-propoxypropoxy, 3-isopropoxypropoxy, and the like.

Alkylthio: an alkyl group as defined above, which is attached via a sulfur atom to the remainder of the molecule, preferably having 1 to 6, more preferably 1 to 3, e.g. 1 or 2 carbon atoms. C₁-C₂-Alkylthio is methylthio or ethylthio. C₁-C₃-Alkylthio is additionally, for example, n-propylthio or 1-methylethylthio (isopropylthio). C₁-C₆-Alkylthio is additionally, for example, butylthio, 1-methylpropylthio (sec-butylthio), 2-methylpropylthio (isobutylthio), 1,1-dimethylethylthio (tert-butylthio), pentylthio, 1-methylbutylthio, 2-methylbutylthio, 3-methylbutylthio, 1,1-dimethylpropylthio, 1,2-dimethylpropylthio, 2,2-dimethylpropylthio, 1-ethylpropylthio, hexylthio, 1-methylpentylthio, 2-methylpentylthio, 3-methylpentylthio, 4-methylpentylthio, 1,1-dimethylbutylthio, 1,2-dimethylbutylthio, 1,3-dimethylbutylthio, 2,2-dimethylbutylthio, 2,3-dimethylbutylthio, 3,3-dimethylbutylthio, 1-ethylbutylthio, 2-ethylbutylthio, 1,1,2-trimethylpropylthio, 1,2,2-trimethylpropylthio, 1-ethyl-1-methylpropylthio or 1-ethyl-2-methylpropylthio.

Alkylsulfinyl: an alkyl group as defined above, which is attached via S(O) group to the remainder of the molecule, preferably having 1 to 6, more preferably 1 to 3, e.g. 1 or 2 carbon atoms. C₁-C₂-alkylsulfinyl is methylsulfinyl or ethylsulfinyl. C₁-C₃-alkylsulfinyl is additionally, for example, n-propylsulfinyl or 1-methylethylsulfinyl (isopropylsulfinyl). C₁-C₆-alkylsulfinyl is additionally, for example, butylsulfinyl, 1-methylpropylsulfinyl (sec-butylsulfinyl), 2-methylpropylsulfinyl (isobutylsulfinyl), 1,1-dimethylethylsulfinyl (tert-butylsulfinyl), pentylsulfinyl, 1-methylbutylsulfinyl, 2-methylbutylsulfinyl, 3-methylbutylsulfinyl, 1,1-dimethylpropylsulfinyl, 1,2-dimethylpropylsulfinyl, 2,2-dimethylpropylsulfinyl, 1-ethylpropylsulfinyl, hexylsulfinyl, 1-methylpentylsulfinyl, 2-methylpentylsulfinyl, 3-methylpentylsulfinyl, 4-methylpentylsulfinyl, 1,1-dimethylbutylsulfinyl, 1,2-dimethylbutylsulfinyl, 1,3-dimethylbutylsulfinyl, 2,2-dimethylbutylsulfinyl, 2,3-dimethylbutylsulfinyl, 3,3-dimethylbutylsulfinyl, 1-ethylbutylsulfinyl, 2-ethylbutylsulfinyl, 1,1,2-trimethylpropylsulfinyl, 1,2,2-trimethylpropylsulfinyl, 1-ethyl-1-methylpropylsulfinyl or 1-ethyl-2-methylpropylsulfinyl.

Alkysulfonyl: an alkyl group as defined above, which is attached via S(O)₂ group to the remainder of the molecule, preferably having 1 to 6, more preferably 1 to 3, e.g. 1 or 2 carbon atoms. C₁-C₂-alkylsulfonyl is methylsulfonyl or ethylsulfonyl. C₁-C₃-alkylsulfonyl is additionally, for example, n-propylsulfonyl or 1-methylethylsulfonyl (isopropylsulfonyl). C₁-C₆-alkylsulfonyl is additionally, for example, butylsulfonyl, 1-methylpropylsulfonyl (sec-butylsulfonyl), 2-methylpropylsulfonyl (isobutylsulfonyl), 1,1-dimethylethylsulfonyl (tert-butylsulfonyl), pentylsulfonyl, 1-methylbutylsulfonyl, 2-methylbutylsulfonyl, 3-methylbutylsulfonyl, 1,1-dimethylpropylsulfonyl, 1,2-dimethylpropylsulfonyl, 2,2-dimethylpropylsulfonyl, 1-ethylpropylsulfonyl, hexylsulfonyl, 1-methylpentylsulfonyl, 2-methylpentylsulfonyl, 3-methylpentylsulfonyl, 4-methylpentylsulfonyl, 1,1-dimethylbutylsulfonyl, 1,2-dimethylbutylsulfonyl, 1,3-dimethylbutylsulfonyl, 2,2-dimethylbutylsulfonyl, 2,3-dimethylbutylsulfonyl, 3,3-dimethylbutylsulfonyl, 1-ethylbutylsulfonyl, 2-ethylbutylsulfonyl, 1,1,2-trimethylpropylsulfonyl, 1,2,2-trimethylpropylsulfonyl, 1-ethyl-1-methylpropylsulfonyl or 1-ethyl-2-methylpropylsulfonyl.

The substituent "oxo" replaces a CH₂ group by a C(=O) group.

The suffix "-carbonyl" in a group denotes in each case that the group is bound to the remainder of the molecule via a carbonyl C=O group. This is the case e.g. in alkylcarbonyl, haloalkylcarbonyl, aminocarbonyl, alkylaminocarbonyl, dialkylaminocarbonyl, alkoxycarbonyl, haloalkoxycarbonyl.

Alkoxycarbonyl: alkoxy group, as defined above, attached via a carbonyl [C(=O)] group to the remainder of the molecule. C₁-C₃-Alkoxycarbonyl is a C₁-C₃-alkoxy group, as defined above, attached via a carbonyl [C(=O)] group to the remainder of the molecule. Examples for C₁-C₃-alkoxycarbonyl are methoxycarbonyl, ethoxycarbonyl, propoxycarbonyl and isopropoxycarbonyl. C₁-C₆-Alkoxycarbonyl is a C₁-C₆-alkoxy group, as defined above, attached via a carbonyl [C(=O)] group to the remainder of the molecule. Examples for C₁-C₆-alkoxycarbonyl are, in addition to those listed for C₁-C₃-alkoxycarbonyl, n-butoxycarbonyl, sec-butoxycarbonyl, isobutoxycarbonyl, tert-butoxycarbonyl, pentoxycarbonyl and hexoxycarbonyl.

Alkoxycarbonyl-alkyl: alkyl group, as defined above, in which one hydrogen atom is replaced by an alkoxycarbonyl group, as defined above. C₁-C₆-Alkoxycarbonyl-C₁-C₆-alkyl is a C₁-C₆-alkyl group, as defined above, in which one hydrogen atom is replaced by a C₁-C₆-alkoxycarbonyl group, as defined above.

Phenyl-C₁-C₃-alkyl is a C₁-C₃-alkyl group, as defined above, in which one hydrogen atom is replaced by a phenyl ring. Examples are benzyl, 1-phenylethyl, 2-phenylethyl, 1-phenylpropyl, 2-phenylpropyl, 3-phenylpropyl or 2-phenyl-2-propyl.

Phenylthio is a phenyl ring attached via an S atom to the remainder of the molecule.

Phenylsulfinyl is a phenyl ring attached via a S(O) group to the remainder of the molecule.

Phenylsulfonyl is a phenyl ring attached via a S(O)₂ group to the remainder of the molecule.
hydroxyl: OH group which is attached via an O atom;
cyano: CN group which is attached via an C atom;
nitro: NO₂ group which is attached via an N atom.

Bicyclic rings in terms of the present invention contain two rings which have at least one ring atom in common. The term comprises condensed (fused) ring systems, in which the two rings have two neighboring ring atoms in common, as well as spiro systems, in which the rings have only one ring atom in common, and bridged systems with at least three ring atoms in common. If not specified otherwise, the bicyclic rings can be carbocyclic, containing only carbon atoms as ring members, as well as heterocyclic, containing at least one heteroatom or heteroatom group generally selected from N, O S, S(O), and S(O)₂ as ring member(s). Further details are given below.

Polycyclic rings contain three or more rings, each of which having at least one ring atom in common with at least one of the other rings of the polycyclic system. The rings can be condensed, spiro-bound or bridged; mixed systems (e.g. one ring is spiro-bound to a condensed system, or a bridged system is condensed with another ring) are also possible. If not specified otherwise, the polycyclic rings can be carbocyclic, containing only carbon atoms as ring members, as well as heterocyclic, containing at least one heteroatom or heteroatom group generally selected from N, O S, S(O), and S(O)₂ as ring member(s). Further details are given below.
Z is a 3-, 4-, 5-, 6-, 7- or 8-membered saturated or partly unsaturated monocyclic carbocyclic ring substituted by a group CO₂R^{e}, by 0 or 1 fluorine atoms and by 0 or 1 (C₁-C₄)-alkyl groups; or
Z is a 5-, 6-, 7- or 8-membered saturated or partly unsaturated bicyclic carbocyclic ring substituted by a group CO₂R^{e}, and by 0 or 1 (C₁-C₄)-alkyl groups; or
Z is an 8-membered saturated polycyclic carbocyclic ring substituted by a group CO₂R^{e} and by 0 or 1 (C₁-C₄)-alkyl groups; or
Z is a 5- or 6-membered saturated or partly unsaturated monocyclic heterocyclic ring containing one oxygen atom or one sulfur atom as ring member, where the heterocyclic ring is substituted by a group CO₂R^{e} and by 0 or 1 (C₁-C₄)-alkyl groups; or
Z is a 5- or 6-membered heteroaromatic ring containing 1, 2, 3 or 4 heteroatoms selected from N, O and S as ring members, where the heteroaromatic ring is substituted by 0 or 1 (C₁-C₄)-alkyl groups

The ring Z can thus be carbocyclic (i.e. containing only carbon atoms as ring members) or heterocyclic (i.e. containing also at least one N, O and/or S atom as ring member(s)).

An unsaturated carbocycle contains at least one C-C double bond(s). An unsaturated heterocycle contains at least one C-C and/or C-N and/or N-N double bond(s). Partially unsaturated carbocyclic rings contain less than the maximum number of C-C double bond(s) allowed by the ring size. Partially unsaturated heterocyclic rings contain less than the maximum number of C-C and/or C-N and/or N-N double bond(s) allowed by the ring size. A fully (or maximally) unsaturated carbocyclic ring (not encompassed in the definition of Z) contains as many conjugated C-C double bonds as allowed by the size(s) of the ring(s). Not encompassed in the definition of Z is however phenyl. A fully (or maximally) unsaturated heterocycle contains as many conjugated C-C and/or C-N and/or N-N double bonds as allowed by the size(s) of the ring(s). Maximally unsaturated 5- or 6-membered heteromonocyclic rings are generally aromatic. Exceptions are maximally unsaturated 6-membered rings containing O, S, SO and/or SO₂ as ring members, such as pyran and thiopyran, which are not aromatic.

Examples for 3-, 4-, 5-, 6-, 7- or 8-membered saturated monocyclic carbocyclic rings Z are cyclopropyl, cyclobutyl, cyclopentyl, cyclohexyl, cycloheptyl and cyclooctyl.

Examples for 3-, 4-, 5-, 6-, 7- or 8-membered partly unsaturated or fully unsaturated monocyclic carbocyclic rings Z are cycloprop-1-enyl, cycloprop-2-enyl, cyclobut-1-enyl, cyclobut-2-enyl, cyclobutadienyl, cyclopent-1-enyl, cyclopent-2-enyl, cyclopent-3-enyl, cyclopenta-1,3-dienyl, cyclopenta-1,4-dienyl, cyclopenta-2,4-dienyl, cyclohex-1-enyl, cyclohex-2-enyl, cyclohex-3-enyl, cyclohexa-1,3-dienyl, cyclohexa-1,4-dienyl, cyclohexa-1,5-dienyl, cyclohexa-2,4-dienyl, cyclohexa-2,5-dienyl, cyclohept-1-enyl, cyclohept-2-enyl, cyclohept-3-enyl, cyclohept-4-enyl, cyclohepta-1,3-dienyl, cyclohepta-1,4-dienyl, cyclohepta-1,5-dienyl, cyclohepta-1,6-dienyl, cyclohepta-2,4-dienyl, cyclohepta-2,5-dienyl, cyclohepta-2,6-dienyl, cyclohepta-3,5-dienyl, cyclohepta-1,3,5-trienyl, cyclooct-1-enyl, cyclooct-2-enyl, cyclooct-3-enyl, cyclooct-4-enyl, cyclooct-5-enyl, cyclooct-6-enyl, cyclooct-7-enyl, cycloocta-1,3-dienyl, cycloocta-1,4-dienyl, cycloocta-1,5-dienyl, cycloocta-1,6-dienyl, cycloocta-1,7-dienyl, cycloocta-2,4-dienyl, cycloocta-2,5-dienyl, cycloocta-2,6-dienyl, cycloocta-2,7-dienyl, cycloocta-3,5-dienyl, cycloocta-3,6-dienyl, cycloocta-1,3,5-trienyl, cycloocta-1,3,7-trienyl, cycloocta-2,4,6-trienyl, cyclooctatetraenyl.

Examples for 3-, 4-, 5-, 6-, 7- or 8-membered saturated, partly unsaturated, fully unsaturated or aromatic heterocyclic rings Z are:
3-, 4-, 5-, 6-, 7- or 8-membered monocyclic saturated heterocycles: e.g. oxiran-2-yl, thiiran-2-yl, aziridin-1-yl, aziridin-2-yl, oxetan-2-yl, oxetan-3-yl, thietan-2-yl, thietan-3-yl, 1-oxothietan-2-yl, 1-oxothietan-3-yl, 1,1-dioxothietan-2-yl, 1,1-dioxothietan-3-yl, azetidin-1-yl, azetidin-2-yl, azetidin-3-yl, tetrahydrofuran-2-yl, tetrahydrofuran-3-yl, tetrahydrothien-2-yl, tetrahydrothien-3-yl, 1-oxotetrahydrothien-2-yl, 1,1-dioxotetrahydrothien-2-yl, 1-oxotetrahydrothien-3-yl, 1,1-dioxotetrahydrothien-3-yl, 1,3-dioxolan-2-yl, 1,3-dioxolan-4-yl, 1,3-ditholan-2-yl, 1,3-ditholan-4-yl, 1,3-oxathiolan-2-yl, 1,3-oxathiolan-4-yl, 1,3-oxathiolan-5-yl, pyrrolidin-1-yl, pyrrolidin-2-yl, pyrrolidin-3-yl, pyrazolidin-1-yl, pyrazolidin-3-yl, pyrazolidin-4-yl, pyrazolidin-5-yl, imidazolidin-1-yl, imidazolidin-2-yl, imidazolidin-4-yl, oxazolidin-2-yl, oxazolidin-3-yl, oxazolidin-4-yl, oxazolidin-5-yl, isoxazolidin-2-yl, isoxazolidin-3-yl, isoxazolidin-4-yl, isoxazolidin-5-yl, thiazolidin-2-yl, thiazolidin-3-yl, thiazolidin-4-yl, thiazolidin-5-yl, isothiazolidin-2-yl, isothiazolidin-3-yl, isothiazolidin-4-yl, isothiazolidin-5-yl, 1,2,4-oxadiazolidin-3-yl, 1,2,4-oxadiazolidin-5-yl, 1,2,4-thiadiazolidin-3-yl, 1,2,4-thiadiazolidin-5-yl, 1,3,4-oxadiazolidin-2-yl, 1,3,4-thiadiazolidin-2-yl, 1,2,4-triazolidin-1-yl, 1,2,4-triazolidin-3-yl, 1,2,4-triazolidin-4-yl, 2-tetrahydropyranyl, 3-tetrahydropyranyl, 4-tetrahydropyranyl, 1,3-dioxan-2-yl, 1,3-dioxan-4-yl, 1,3-dioxan-5-yl, 1,4-dioxan-2-yl, piperidin-1-yl, piperidin-2-yl, piperidin-3-yl, piperidin-4-yl, hexahydropyridazin-1-yl, hexahydropyridazin-3-yl, hexahydropyridazin-4-yl, hexahydropyrimidin-1-yl, hexahydropyrimidin-2-yl, hexahydropyrimidin-4-yl, hexahydropyrimidin-5-yl, piperazin-1-yl, piperazin-2-yl, 1,3,5-hexahydrotriazin-1-yl, 1,3,5-hexahydrotriazin-2-yl, 1,2,4-hexahydrotriazin-1-yl, 1,2,4-hexahydrotriazin-2-yl, 1,2,4-hexahydrotriazin-3-yl, 1,2,4-hexahydrotriazin-4-yl, 1,2,4-hexahydrotriazin-5-yl, 1,2,4-hexahydrotriazin-6-yl, morpholin-2-yl, morpholin-3-yl, morpholin-4-yl, thiomorpholin-2-yl, thiomorpholin-3-yl, thiomorpholin-4-yl, 1-oxothiomorpholin-2-yl, 1-oxothiomorpholin-3-yl, 1-oxothiomorpholin-4-yl, 1,1-dioxothiomorpholin-2-yl, 1,1-dioxothiomorpholin-3-yl, 1,1-dioxothiomorpholin-4-yl, azepan-1-, -2-, -3- or -4-yl, oxepan-2-, -3-, -4- or -5-yl, hexahydro-1,3-diazepinyl, hexahydro-1,4-diazepinyl, hexahydro-1,3-oxazepinyl, hexahydro-1,4-oxazepinyl, hexahydro-1,3-dioxepinyl, hexahydro-1,4-dioxepinyl, oxocane, thiocane, azocanyl, [1,3]diazocanyl, [1,4]diazocanyl, [1,5]diazocanyl, [1,5]oxazocanyl and the like;
3-, 4-, 5-, 6-, 7- or 8-membered partially unsaturated heteromonocyclic rings: 2,3-dihydrofuran-2-yl, 2,3-dihydrofuran-3-yl, 2,5-dihydrofuran-2-yl, 2,5-dihydrofuran-3-yl, 2,3-dihydrothien-2-yl, 2,3-dihydrothien-3-yl, 2,5-dihydrothien-2-yl, 2,5-dihydrothien-3-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl, 3,6-dihydro-2H-pyran-2-, -3-, -4-, -5- or 6-yl, 3,4-dihydro-2H-pyran-2-, -3-, -4-, -5- or 6-yl, 3,6-dihydro-2H-thiopyran-2-, -3-, -4-, -5- or 6-yl, 3,4-dihydro-2H-thiopyran-2-, -3-, -4-, -5- or 6-yl, 2-, 3-, 4-, 5- or 6-di- or tetrahydropyridinyl, 3-di- or tetrahydropyridazinyl, 4-di- or tetrahydropyridazinyl, 2-di- or tetrahydropyrimidinyl, 4-di- or tetrahydropyrimidinyl, 5-di- or tetrahydropyrimidinyl, di- or tetrahydropyrazinyl, 1,3,5-di- or tetrahydrotriazin-2-yl, 1,2,4-di- or tetrahydrotriazin-3-yl, 2,3,4,5-tetrahydro[1H]azepin-1-, -2-, -3-, -4-, -5-, -6- or -7-yl, 3,4,5,6-tetrahydro[2H]azepin-2-, -3-, -4-, -5-, -6- or -7-yl, 2,3,4,7-tetrahydro[1H]azepin-1-, -2-, -3-, -4-, -5-, -6- or -7-yl, 2,3,6,7-tetrahydro[1H]azepin-1-, -2-, -3-, -4-, -5-, -6- or -7-yl, tetrahydrooxepinyl, such as 2,3,4,5-tetrahydro[1H]oxepin-2-, -3-, -4-, -5-, -6- or -7-yl, 2,3,4,7-tetrahydro[1H]oxepin-2-, -3-, -4-, -5-, -6- or -7-yl, 2,3,6,7-tetrahydro[1H]oxepin-2-, -3-, -4-, -5-, -6- or -7-yl, tetrahydro-1,3-diazepinyl, tetrahydro-1,4-diazepinyl, tetrahydro-1,3-oxazepinyl, tetrahydro-1,4-oxazepinyl, tetrahydro-1,3-dioxepinyl, tetrahydro-1,4-dioxepinyl, 1,2,3,4,5,6-hexahydroazocine, 2,3,4,5,6,7-hexahydroazocine, 1,2,3,4,5,8-hexahydroazocine, 1,2,3,4,7,8-hexahydroazocine, 1,2,3,4,5,6-hexahydro-[1,5]diazocine,1,2,3,4,7,8-hexahydro-[1,5]diazocine and the like;
3-, 4-, 5-, 6-, 7- or 8-membered maximally unsaturated (but not aromatic) heteromonocyclic rings: pyran-2-yl, pyran-3-yl, pyran-4-yl, thiopryran-2-yl, thiopryran-3-yl, thiopryran-4-yl, 1-oxothiopryran-2-yl, 1-oxothiopryran-3-yl, 1-oxothiopryran-4-yl, 1,1-dioxothiopryran-2-yl, 1,1-dioxothiopryran-3-yl, 1,1-dioxothiopryran-4-yl, 2H-oxazin-2-yl, 2H-oxazin-3-yl, 2H-oxazin-4-yl, 2H-oxazin-5-yl, 2H-oxazin-6-yl, 4H-oxazin-3-yl, 4H-oxazin-4-yl, 4H-oxazin-5-yl, 4H-oxazin-6-yl, 6H-oxazin-3-yl, 6H-oxazin-4-yl, 7H-oxazin-5-yl, 8H-oxazin-6-yl, 2H-1,3-oxazin-2-yl, 2H-1,3-oxazin-4-yl, 2H-1,3-oxazin-5-yl, 2H-1,3-oxazin-6-yl, 4H-1,3-oxazin-2-yl, 4H-1,3-oxazin-4-yl, 4H-1,3-oxazin-5-yl, 4H-1,3-oxazin-6-yl, 6H-1,3-oxazin-2-yl, 6H-1,3-oxazin-4-yl, 6H-1,3-oxazin-5-yl, 6H-1,3-oxazin-6-yl, 2H-1,4-oxazin-2-yl, 2H-1,4-oxazin-3-yl, 2H-1,4-oxazin-5-yl, 2H-1,4-oxazin-6-yl, 4H-1,4-oxazin-2-yl, 4H-1,4-oxazin-3-yl, 4H-1,4-oxazin-4-yl, 4H-1,4-oxazin-5-yl, 4H-1,4-oxazin-6-yl, 6H-1,4-oxazin-2-yl, 6H-1,4-oxazin-3-yl, 6H-1,4-oxazin-5-yl, 6H-1,4-oxazin-6-yl, 1,4-dioxine-2-yl, 1,4-oxathiin-2-yl, 1H-azepine, 1H-[1,3]-diazepine, 1H-[1,4]-diazepine, [1,3]diazocine, [1,5]diazocine, [1,5]diazocine and the like;
5- or 6-membered monocyclic aromatic heterocyclic rings: e.g. 2-furyl, 3-furyl, 2 thienyl, 3-thienyl, 1-pyrrolyl, 2-pyrrolyl, 3-pyrrolyl, 1--pyrazolyl, 3--pyrazolyl, 4 pyrazolyl, 5-pyrazolyl, 1 imidazolyl, 2-imidazolyl, 4-imidazolyl, 5-imidazolyl, 2-oxazolyl, 4-oxazolyl, 5 oxazolyl, 3-isoxazolyl, 4-isoxazolyl, 5-isoxazolyl, 2-thiazolyl, 4-thiazolyl, 5 thiazolyl, 3-isothiazolyl, 4-isothiazolyl, 5-isothiazolyl, 1,3,4-triazol-1-yl, 1,3,4-triazol-2-yl, 1,3,4-triazol-3-yl, 1,2,3-triazol-1-yl, 1,2,3-triazol-2-yl, 1,2,3-triazol-4-yl, 1,2,5-oxadiazol-3-yl, 1,2,3-oxadiazol-4-yl, 1,2,3-oxadiazol-5-yl, 1,3,4-oxadiazol-2-yl, 1,2,5-thiadiazol-3-yl, 1,2,3-thiadiazol-4-yl, 1,2,3-thiadiazol-5-yl, 1,3,4-thiadiazol-2-yl, 1,2,3,4-tetrazol-1-yl, 1,2,3,4-tetrazol-2-yl, 1,2,3,4-[1H]-tetrazol-5-yl, 1,2,3,4-[2H]-tetrazol-5-yl, 2-pyridinyl, 3-pyridinyl, 4 pyridinyl, 1-oxopyridin-2-yl, 1-oxopyridin-3-yl, 1-oxopyridin-4-yl, 3-pyridazinyl, 4-pyridazinyl, 2-pyrimidinyl, 4 pyrimidinyl, 5-pyrimidinyl, 2-pyrazinyl, 1,3,5-triazin-2-yl, 1,2,4-triazin-3-yl, 1,2,4-triazin-5-yl, 1,2,3,4-tetrazin-1-yl, 1,2,3,4-tetrazin-2-yl, 1,2,3,4-tetrazin-5-yl and the like.

Bicyclic rings are 4- to 8-membered, preferably 5- to 8-membered.

Examples for 5- to 8-membered bicyclic spirocyclic saturated carbocyclic rings comprise spiro[2.2]pentyl, spiro[2.3]hexyl, spiro[2.4]heptyl, spiro[3.3]heptyl and the like.

Examples of 5- to 8-membered bicyclic condensed saturated carbocyclic rings comprise bicyclo[3.1.0]hexyl, bicyclo[3.2.0]heptyl, bicyclo[3.3.0]octyl, 1,2,3,3a,4,5,6,6a-octahydropentalenyl, bicyclo[4.2.0]octyl and the like.

Examples of 5- to 8-membered bicyclic bridged saturated carbocyclic rings comprise bicyclo[1.1.1]pentyl, bicyclo[2.1.1]hexyl, bicyclo[2.2.1]heptyl, bicyclo[3.1.1]heptyl, bicyclo[2.2.2]octyl, bicyclo[3.2.1]octyl and the like.

An example for a 5- to 8-membered polycyclic saturated carbocyclic is cubyl.

An example for a 5- to 8-membered partly unsaturated bicyclic bridged carbocyclic ring is bicyclo[2.2.2]oct-2-enyl.

Examples for saturated 5- to 8-membered bicyclic condensed heterocyclic rings are:

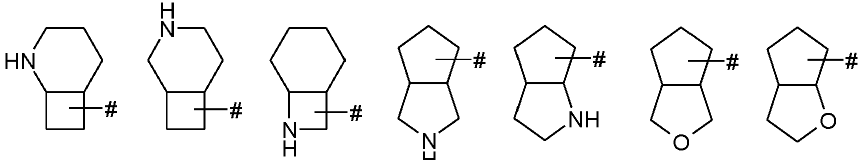

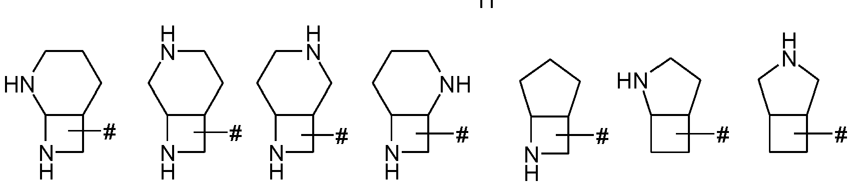

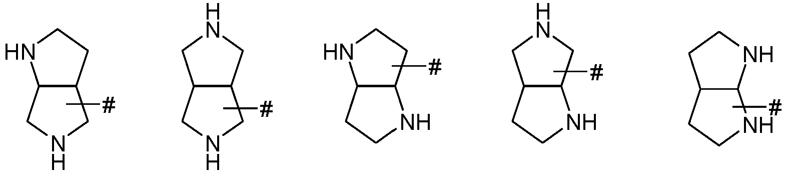

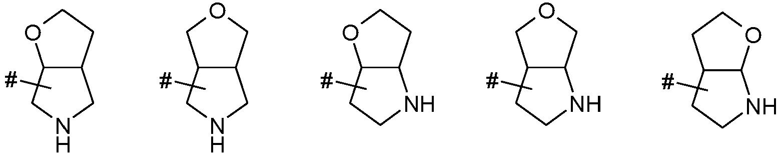

Examples for saturated 5- to 8-membered bicyclic spirocyclic heterocyclic rings are:

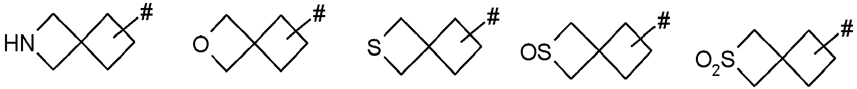

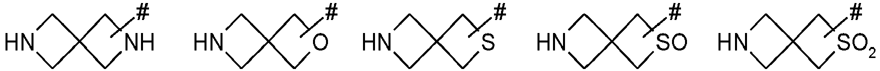

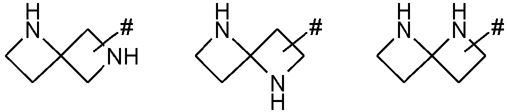

Examples for saturated 5- to 8-membered bicyclic bridged heterocyclic rings are:

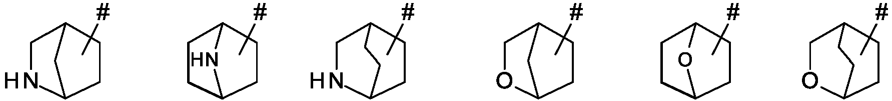

Examples for partly unsaturated 5- to 8-membered bicyclic bridged heterocyclic rings are:

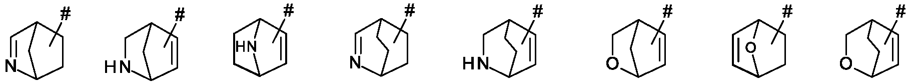

In the above structures # denotes the attachment point to the remainder of the molecule. The attachment point is not restricted to the ring on which this is shown, but can be on either of the two rings, and may be on a carbon or on a nitrogen ring atom. If the rings carry one or more substituents, these may be bound to carbon and/or to nitrogen ring atoms.

R⁷ and R⁸ form, together with the carbon atom to which they are bound, a saturated or partially unsaturated three- to six-membered monocyclic heterocyclic ring W or a six- to eight-membered bicyclic heterocyclic ring W, containing, in addition to this carbon atom, q carbon atoms, u oxygen atoms, v nitrogen atoms, w sulfur atoms, and x elements selected from the group consisting of NR^{d} and NC(O)OR^{d}, where one carbon atom bears p oxo groups.

Examples for saturated three- to six-membered monocyclic heterocyclic rings W are aziridin-2,2-diyl, oxiran-2,2-diyl, thiiran-2,2-diyl, 1-oxo-thiiran-2,2-diyl, 1,1-dioxothiiran-2,2-diyl, azetidin-2,2-diyl, azetidin-3,3-diyl, oxetan-2,2-diyl, oxetan-3,3,-diyl, thietan-2,2-diyl, thietan-3,3-diyl, 1-oxo-thietan-2,2-diyl, 1-oxothietan-3,3-diyl, 1,1-dioxo-thietan-2,2-diyl, 1,1-di-oxothietan-3,3-diyl, pyrrolidin-2,2-diyl, pyrrolidin-3,3-diyl, tetrahydrofuran-2,2-diyl, tetrahydrofuran-3,3-diyl, tetrahydrothiophen-2,2-diyl, tetrahydrothiophen-3,3-diyl, 1-oxo-tetrahydrothiophen-2,2-diyl, 1-oxotetrahydrothiophen-3,3-diyl, 1,1-dioxo-tetrahydrothiophen-2,2-diyl, 1,1-dioxotetrahydrothiophen-3,3-diyl, pyrazolidin-3,3-diyl, pyrazolidin-4,4-diyl, imidazolidin-2,2-diyl, imidazolidin-4,4-diyl, 1,3-dioxolan-2,2-diyl, 1,3-dioxolan-4,4-diyl, 1,3-dithiolan-2,2-diyl, 1,3-dithiolan-4,4-diyl, 1,3-oxathiolan-2,2-diyl, 1,3-oxathiolan-4,4-diyl, 1,3-oxathiolan-5,5-diyl, oxazolidin-2,2-diyl, oxazolidin-4,4-diyl, oxazolidin-5,5-diyl, isoxazolidin-3,3-diyl, isoxazolidin-4,4-diyl, isoxazolidin-5,5-diyl, thiazolidin-2,2-diyl, thiazolidin-4,4-diyl, thiazolidin-5,5-diyl, isothiazolidin-3,3-diyl, isothiazolidin-4,4-diyl, isothiazolidin-5,5-diyl, 1,2,4-oxadiazolidin-3,3-diyl, 1,2,4-oxadiazolidin-5,5-diyl, 1,2,4-thiadiazolidin-3,3-diyl, 1,2,4-thiadiazolidin-5,5-diyl, 1,3,4-oxadiazolidin-2,2-diyl, 1,3,4-thiadiazolidin-2,2-diyl, 1,2,4-triazolidin-3,3-diyl, 1,2,4-triazolidin-5,5-diyl, tetrahydropyran-2,2-diyl, tetrahydropyran-3,3-diyl, tetrahydropyran-4,4-diyl, tetrahydrothiopyran-2,2-diyl, tetrahydrothiopyran-3,3-diyl, tetrahydrothiopyran-4,4-diyl, 1-oxotetrahydrothiopyran-2,2-diyl, 1-oxotetrahydrothiopyran-3,3-diyl, 1-oxotetrahydrothiopyran-4,4-diyl, 1,1-dioxotetrahydrothiopyran-2,2-diyl, 1,1-dioxotetrahydrothiopyran-3,3-diyl, 1,1-dioxotetrahydrothiopyran-4,4-diyl, piperidin-2,2-diyl, piperidin-3,3-diyl, piperidin-4,4-diyl, 1,3-dioxan-2,2-diyl, 1,3-dioxan-4,4-diyl, 1,3-dioxan-5,5-diyl, 1,4-dioxan-2,2-diyl, piperazin-2,2-diyl, hexahydropyridazin-3,3-diyl, hexahydropyridazin-4,4-diyl, hexahydropyrimidin-2,2-diyl, hexahydropyrimidin-4,4-diyl, hexahydropyrimidin-5,5-diyl, morpholin-2,2-diyl, morpholin-3,3-diyl, thiomorpholin-2,2-diyl, thiomorpholin-3,3-diyl, 1-oxo-thiomorpholin-2,2-diyl, 1-oxo-thiomorpholin-3,3-diyl, 1,1-dioxo-thiomorpholin-2,2-diyl, 1,1-dioxo-thiomorpholin-3,3-diyl, and the like; and moreover the above rings which contain an N atom as ring member and which is substituted by R^{d} or C(O)OR^{d}.

Examples for partly unsaturated three- to six-membered monocyclic heterocyclic rings W are 2,3-dihydrofuran-2,2-diyl, 2,3-dihydrofuran-3,3-diyl, 2,5-dihydrofuran-2,2-diyl, 2,3-dihydrothien-2,2-diyl, 2,3-dihydrothien-3,3-diyl, 2,5-dihydrothien-2,2-diyl, 1-oxo-2,3-dihydrothien-2,2-diyl, 1-oxo-2,3-dihydrothien-3,3-diyl, 1-oxo-2,5-dihydrothien-2,2-diyl, 1,1-di-oxo-2,3-dihydrothien-2,2-diyl, 1,1-dioxo-2,3-dihydrothien-3,3-diyl, 1,1-dioxo-2,5-dihydrothien-2,2-diyl, 2,3-dihydro-1H-pyrrol-2,2-diyl, 2,3-dihydro-1H-pyrrol-3,3-diyl, 2,5-dihydro-1H-pyrrol-2,2-diyl, 1,3-dioxol-2,2-diyl, 1,3-dioxol-4,4-diyl, 1,3-dithiol-2,2-diyl, 1,3-dithiol-4,4-diyl, 1,3-oxathiol-2,2-diyl, 1,3-oxathiol-4,4-diyl, 1,3-oxathiol-5,5-diyl, 2,3-dihydro-1H-pyrazol-3,3-diyl, 2,5-dihydro-1H-imidazol-2,2-diyl, 2,5-dihydro-1H-imidazol-5,5-diyl, 2,3-dihydrooxazol-2,2-diyl, 2,5-dihydrooxazol-2,2-diyl, 2,5-dihydrooxazol-5,5-diyl, 4,5-dihydrooxazol-4,4-diyl, 4,5-dihydrooxazol-5,5-diyl, 2,3-dihydroisoxazol-3,3-diyl, 2,5-dihydroisoxazol-2,2-diyl, 4,5-dihydroisoxazol-4,4-diyl, 4,5-dihydroisoxazol-5,5-diyl, 2,3-dihydrothiazol-2,2-diyl, 2,5-dihydrothiazol-2,2-diyl, 2,5-dihydrothiazol-5,5-diyl, 4,5-dihydrothiazol-4,4-diyl, 4,5-dihydrothiazol-5,5-diyl, 2,3-dihydroisothiazol-3,3-diyl, 2,5-dihydroisothiazol-2,2-diyl, 4,5-dihydroisothiazol-4,4-diyl, 4,5-dihydroisothiazol-5,5-diyl, 3,6-dihydro-2H-pyran-2,2-diyl, 3,6-dihydro-2H-pyran-3,3-diyl, 3,6-dihydro-2H-pyran-6,6-diyl, 3,4-dihydro-2H-pyran-4,4-diyl, 3,4-dihydro-2H-pyran-5,5-diyl, 3,4-dihydro-2H-pyran-6,6-diyl, 3,6-dihydro-2H-thiopyran-2,2-diyl, 3,6-dihydro-2H-thiopyran-3,3-diyl, 3,6-dihydro-2H-thiopyran-6,6-diyl, 3,4-dihydro-2H-thiopyran-4,4-diyl, 3,4-dihydro-2H-thiopyran-5,5-diyl, 3,4-dihydro-2H-thiopyran-6,6-diyl, 1,2,3,4-tetrahydropyridin-2,2-diyl, 1,2,3,4-tetrahydropyridin-3,3-diyl, 1,2,3,4-tetrahydropyridin-4,4-diyl, 1,2,3,6-tetrahydropyridin-2,2-diyl, 1,2,3,6-tetrahydropyridin-3,3-diyl, 1,2,3,6-tetrahydropyridin-6,6-diyl, 1,2-dihydropyridin-2,2-diyl, 1,4-dihydropyridin-4,4-diyl and the like.

Examples for saturated and partily unsaturated six- to eight-membered bicyclic heterocyclic ring W are the above-shown saturated and partly unsaturated bicyclic heterocyclic rings, only that in case of the here-exemplified rings W there are two attachment points to the remainder of the molecule on the same carbon ring atom.

The preferred embodiments of the invention mentioned herein below have to be understood as being preferred either independently from each other or in combination with one another.

According to particular embodiments of the invention, preference is given to those compounds of formula (I) wherein the variables, either independently of one another or in combination with one another, have the following meanings:
According to the invention, R¹ is hydrogen.

According to the invention, R² is selected from the group consisting of hydrogen and halogen. Preferably, R² is selected from the group consisting of hydrogen, fluorine and chlorine, and specifically from H and F.

In particular, R² is hydrogen.

According to the invention, R³ is selected from the group consisting of hydrogen, halogen, cyano, (C₁-C₃)-alkyl, (C₁-C₃)-haloalkyl, (C₁-C₃)-alkoxy and (C₁-C₃)-haloalkoxy.

Preferably, R³ is selected from the group consisting of halogen and (C₁-C₃)-haloalkoxy.

Specifically, R³ is selected from the group consisting of halogen, cyano, methyl, ethyl, methoxy and halomethoxy, and very specifically from fluorine, chlorine, cyano and trifluoromethoxy.

In particular, R³ is hydrogen, halogen, cyano or (C₁-C₃)-haloalkoxy, more particularly halogen, very particularly chlorine or fluorine.

According to the invention, R⁴ is selected from the group consisting of hydrogen and halogen. Preferably, R⁴ is selected from the group consisting of hydrogen, fluorine, chlorine and bromine. In particular, R⁴ is hydrogen, fluorine or chlorine, very particularly hydrogen.

According to the invention, R⁵ is selected from the group consisting of hydrogen, halogen, cyano, (C₁-C₃)-alkyl, (C₁-C₃)-haloalkyl, (C₁-C₃)-alkoxy and (C₁-C₃)-haloalkoxy.

Preferably, R⁵ is selected from the group consisting of hydrogen, halogen and (C₁-C₃)-alkyl.

More preferably, R⁵ is selected from the group consisting of hydrogen and halogen.

Also preferred compounds according to the invention are compounds of formula (I), wherein R⁵ is selected from the group consisting of hydrogen, halogen and methyl, especially hydrogen, chlorine and fluorine.

In particular, R⁵ is hydrogen or halogen, very particular hydrogen, chlorine or fluorine.

According to the invention,, R⁶ is hydrogen.

Preferably, R⁹ is selected from the group consisting of hydrogen and (C₁-C₃)-alkyl. In particular, R⁹ is hydrogen, methyl or ethyl, very particular hydrogen.

In particular, R¹ and R⁹ are hydrogen.

In particular, R² and R⁶ are hydrogen.

A preferred embodiment of the invention relates to compounds of formula (I), wherein the substituents R³ and R⁵ have the following meanings:
- R³: is halogen, cyano, (C₁-C₃)-alkyl, (C₁-C₃)-haloalkyl, (C₁-C₃)-alkoxy or (C₁-C₃)-haloalkoxy; and
- R⁵: is hydrogen, halogen, or (C₁-C₃)-alkyl.

An more preferred embodiment of the invention relates to compounds of formula (I), wherein the substituents R³ and R⁵ have the following meanings:
- R³: is halogen, cyano or (C₁-C₃)-haloalkoxy, especially fluorine, chlorine, cyano or trifluoromethoxy; and
- R⁵: is hydrogen or halogen, especially hydrogen, chlorine or fluorine.

In rings formed by R⁷ and R⁸ together with the carbon atom to which they are bound, the sum of u+v+w+x is 1 or 2.

According to the invention, R⁷ and R⁸ form, together with the carbon atom to which they are bound, a saturated or partially unsaturated three-, four-, five- or six-membered monocyclic heterocyclic ring W or a six-, seven- or eight-membered bicyclic heterocyclic ring W, the ring containing, in addition to this carbon atom, q carbon atoms, u oxygen atoms, w sulfur atoms, v nitrogen atoms and x elements selected from the group consisting of NR^{d} and NC(O)OR^{d}, where the ring is substituted by n radicals R^{g} and where one carbon atom bears p oxo groups, and where R^{d}, R^{g}, q, u, w, v, x and p have the meanings defined herein, especially the ones mentioned herein as preferred.

Examples of the three- to six-membered monocyclic heterocyclic rings W or six- to eight-membered bicyclic rings W are the following rings, wherein nitrogen atoms which are ring members and not part of a double bond optionally carry a substituent R^{d} or C(O)OR^{d}, and wherein the rings may additionally be substituted with n radicals R^{g} and/or where 1 CH₂ ring member group may be replaced by p C=O groups; where n and R^{g} have the meanings defined herein, in particular the preferred ones:

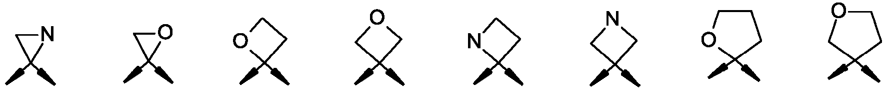

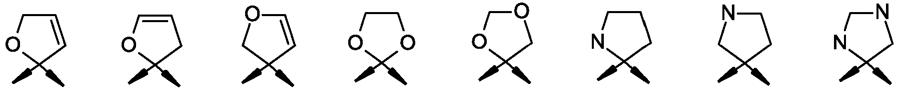

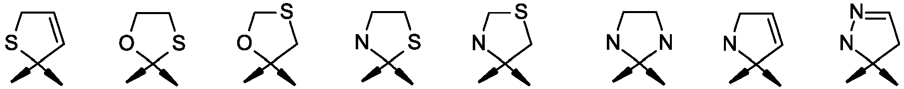

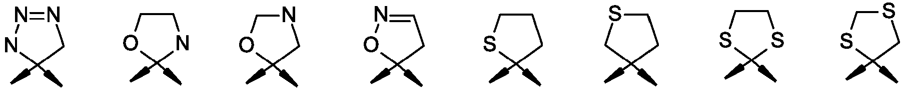

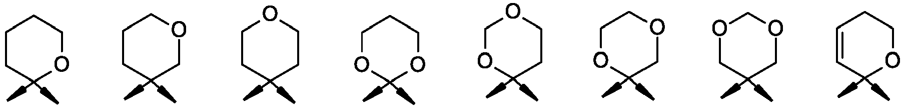

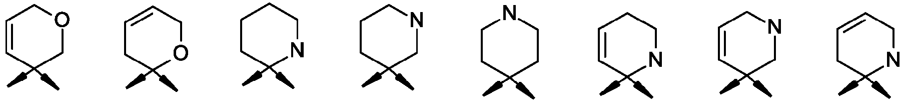

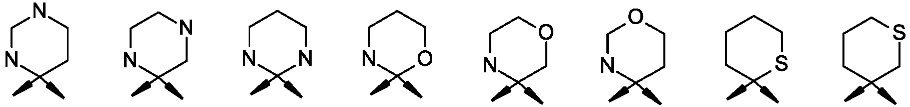

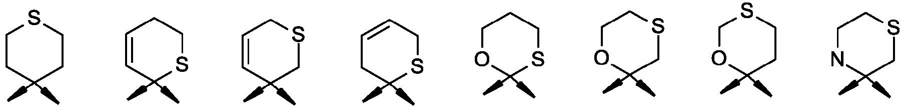

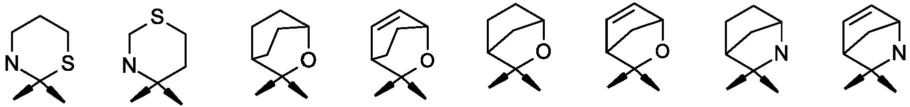

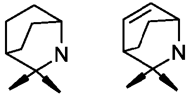

The arrows denote the bonds to the two C(O) groups.

A preferred embodiment W4 of the invention relates to the compounds of formula (I) defined in the preceding embodiments W1 to W3, wherein the variables u, v, w and x have the following meanings:
- u is 1 or 2, v is 0, w is 0 and x is 0; or alternatively
- u is 0 or 1, v is 1, w is 0 and x is 0; or alternatively
- u is 0 or 1, v is 0, w is 1 and x is 0; or alternatively
- u is 0, v is 0, w is 0 and x is 1.

A further preferred embodiment W5 of the invention relates to the compounds of formula (I) defined in the preceding embodiment W4, wherein the variables q, n and p have the following meanings:
- q: is 1, 2, 3, 4, 5, 6 or 7, preferably 1, 2, 3, 4, 5 or 6, more preferably 1, 2, 3 or 4, and especially 1, 2, or 3, and
- n: is 0, 1 or 2, and more preferably 0 or 1; and
- p: is 0 or 1, and preferably 0.

Preferred examples for the monocyclic or bicyclic rings W are the following rings, where q is 1, 2, 3, 5 or 6, u is 0, 1 or 2, v is 0 or 1, w is 0 or 1, x is 0 or 1, p is 0, n is 0, 1 or 2, and R^{d}, if present, is methyl or tert-butyl, and R^{g}, if present, is methyl, ethyl or chloromethyl, or two R^{g}, bound to the same carbon atom, form a methylene group (=CH₂), the arrows or # representating the bond to the carbon atoms of the adjacent carbonyl groups:

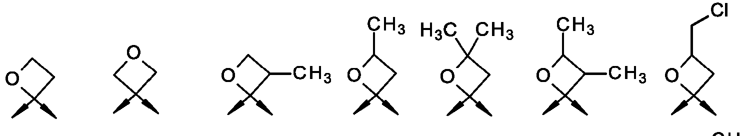

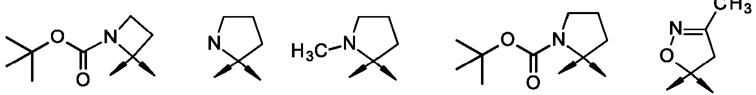

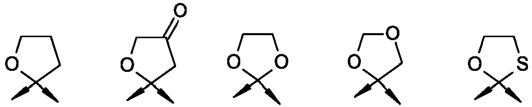

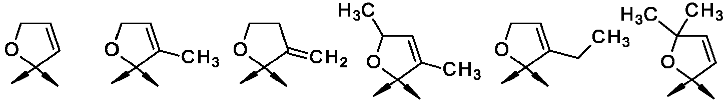

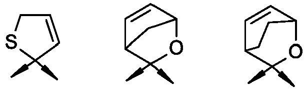

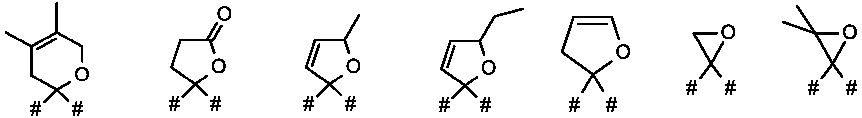

The arrows and # denote the bonds to the two C(O) groups.

Particularly preferred examples for the monocyclic or bicyclic rings W are the following rings, where q is 1, 2, 3, 5 or 6, u is 0, 1 or 2, v is 0 or 1, w is 0 or 1, x is 0 or 1, p is 0 or 1, n is 0, 1 or 2, and R^{d}, if present, is tert-butyl, and R^{g}, if present, is methyl, or two R^{g}, bound to the same carbon atom, form a methylene group (=CH₂), # representating the bond to the carbon atoms of the adjacent carbonyl groups:

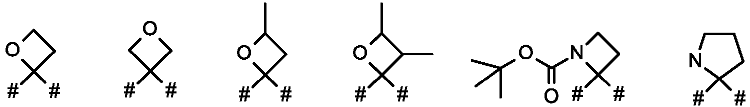

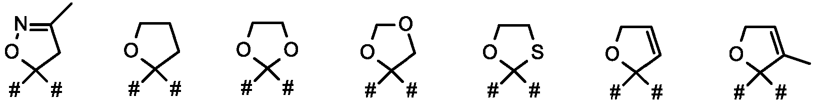

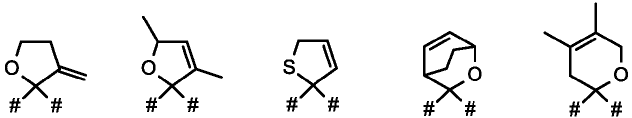

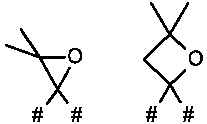

A further preferred embodiment W6 of the invention relates to compounds of formula (I), wherein R⁷ and R⁸ form, together with the carbon atom to which they are bound, a saturated or partially unsaturated three-, four-, five- or six-membered monocyclic heterocyclic ring W or a six-, seven- or eight-membered bicyclic heterocyclic ring W, the ring containing, in addition to this carbon atom, q carbon atoms, u oxygen atoms, w sulfur atoms, v nitrogen atoms and x elements selected from the group consisting of NR^{d} and NC(O)OR^{d}, where the ring is substituted by n radicals R^{g}, and where R^{d}, R^{g}, q, u, w, v, x and n have the meanings defined herein, especially the ones mentioned herein as preferred. In this embodiments q, u, w, v and x have in particular the following meanings:
- q: is 1, 2, 3, 4, 5 or 6, preferably 1, 2, 3, 4 or 5, more preferably 1, 2, 3 or 4, and especially 2, or 3,
- u: is 0, 1 or 2,
- w: is 0 or 1,
- v: is 0 or 1, and
- x: is 0 or 1, and
- n: is 0, 1 or 2, where
u+v+w+x = 1 or 2.

A further preferred embodiment W7 of the invention relates to compounds of formula (I), wherein R⁷ and R⁸ form, together with the carbon atom to which they are bound, a saturated or partially unsaturated three-, four-, five- or six-membered monocyclic heterocyclic ring W or a six- , seven- or eight-membered bicyclic heterocyclic ring W, the ring, containing, in addition to this carbon atom, q carbon atoms and u oxygen atoms, where the ring is substituted by n radicals R^{g}, and where
- q: is 1, 2, 3, 4, 5 or 6, and
- u: is 1 or 2, and where

R^{g} and n have the meanings defined herein, especially the ones mentioned herein as preferred.

A further preferred embodiment W8 of the invention relates to compounds of formula (I), wherein R⁷ and R⁸ form, together with the carbon atom to which they are bound, a saturated or partially unsaturated four- to five-membered heterocyclic ring W, containing, in addition to this carbon atom, q carbon atoms and u oxygen atoms, where the ring is substituted by n radicals R^{g}, and where
- q: is 2 or 3, and
- u: is 1 or 2, and where

R^{g} and n have the meanings defined herein, especially the ones mentioned herein as preferred.

A further preferred embodiment W9 of the invention relates to compounds of formula (I), wherein R⁷ and R⁸ form, together with the carbon atom to which they are bound, a saturated or partially unsaturated four- to five-membered heterocyclic ring W selected from following ring structures, which may additionally be substituted with n radicals R^{g}, where n is 0, 1 or 2, preferably 0 or 1 and especially 0, and where each R^{g} has one of the meanings defined herein, in particular one of the preferred meanings:

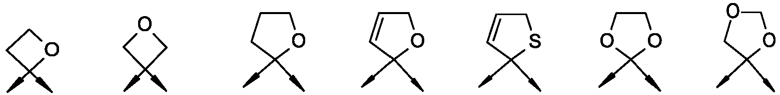

A further preferred embodiment W10 of the invention relates to compounds of formula (I), comprising a heterocyclic ring W which contains x elements selected from the group consisting of NR^{d} and NC(O)OR^{d}, where each substituent R^{d} is independently selected from hydrogen and (C₁-C₆)-alkyl, preferably from hydrogen and (C₁-C₄)-alkyl and especially from hydrogen and (C₁-C₃)-alkyl, e.g. selected from hydrogen, tert-butyl, isopropyl, ethyl and methyl.

A further preferred embodiment W11 of the invention relates to compounds of formula (I), wherein each substituent R^{g}, if present, is independently selected from (C₁-C₃)-alkyl and (C₁-C₃)-haloalkyl, especially from methyl, ethyl, chloromethyl and fluoromethyl, and is in particular methyl, or two R^{g}, bound to the same carbon atom, form together a methylene group (=CH₂).

The compounds of formula (I) of the present invention may comprise a stereogenic center (*) depending on the structure of the ring W (the ring must not contain a mirror axis for the carbon atom carrying R⁷ and R⁸ to be a stereogenic center), as exemplified below:

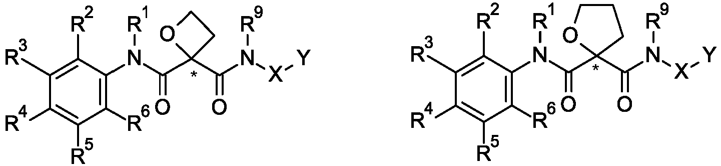

Accordingly, if the carbon atom to which R⁷ and R⁸ are bound to form a ring W is a stereogenic center, the compounds of formula (I) exist in two stereoisomeric forms (if no further stereogenic center is present; otherwise, the compounds of formula (I) exist in more than two stereoisomeric forms, of course; to be more precise in 2" stereoisomeric forms, n being the number of stereogenic centers in the molecule - provided no meso form is present). All stereoisomers as well as mxitures thereof are also part of the present invention.

In the compounds of formula (I), X is selected from the group consisting of a bond (X⁰) or a divalent unit (X⁶), wherein the orientation of (X⁶) within the molecule is as depicted, the left arrow representating the bond to the adjacent nitrogen, the right arrow representating the bond to the adjacent group Y.

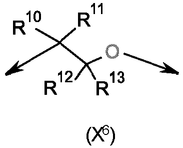

A preferred embodiment of the invention relates to compounds of formula (I.X⁰), wherein X is a bond (X⁰) and wherein Y is as defined above:

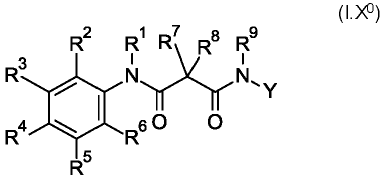

Another preferred embodiment of the invention relates to compounds of formula (I.X⁶), wherein X is (X⁶), and wherein the orientation of (X⁶) within the molecule is as depicted, the left arrow representating the bond to the adjacent nitrogen, the right arrow representating the bond to the adjacent group Y, wherein Y is as defined above:

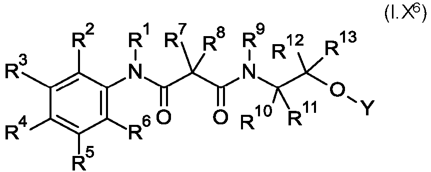

Further preferred compounds according to the invention are compounds of formula (I), wherein X is selected from the group consisting of a bond (X⁰) or one of the following divalent units of formula (X⁶): CH(CH₃)CH₂O, CH₂CH₂O.

The substituents R¹⁰- R¹³ independently of each other and independently of each occurrence are hydrogen or methyl.

A preferred embodiment Y1-2 of the invention relates to compounds according to the invention are compounds of formula (I), wherein X is a bond and Y is selected from the group consisting of (C₁-C₆)-alkyl and (C₂-C₆)-alkenyl, each substituted by one radical selected from the group consisting of CO₂R^{e}, CONR^{b}R^{h},and CONR^{e}SO₂R^{a}, and optionally by one (C₁-C₄)-alkoxy substituent, where R^{a}, R^{b}, R^{e}, R^{d} and R^{h} have the meanings defined herein, in particular the preferred meanings.

Preferred examples of moieties Y according to the embodiment Y1-2 are selected from the following structures, where the left dash represents the bond to the remainder of the molecule of compound (I) and the right dash respresents the bond to a substituent CO₂R^{e}, CONR^{b}R^{h} or CONR^{e}SO₂R^{a}, as defined herein, and where a substituent -OCH₃, if present, is an alkoxy substituent: -CH₂-, -CH₂CH₂-, -CH(CH₃)-, -CH₂CH₂CH₂-, -CH(CH₂CH₃)-, -CH(CH₃)CH₂-, - C(CH₃)₂-, -C(CH₃)₂CH₂-, -C(iPr)CH₃-, -CH(CH₂iPr)CH₂-, -CH₂CH=CH-, -C(CH₃)(CH₂OCH₃)-, -C(CH₃)CH₂CH₂-, -C(CH₃)₂CH=CH-, -CH₂CH₂CH₂CH₂-, -CH(CH₃)C(CH₃)₂-, -CH₂CH(CH₃)-, -CH₂CH(OCH₃)CH₂-, -CH₂CH(CH₃)CH₂- and -C(CH₃)(CH₂CH₃)-. iPr is isopropyl.

In the context of this embodiment Y1-2, particularly preferred examples of moieties Y are the following structures: -CH₂-, -CH₂CH₂-, -CH(CH₃)-, -CH₂CH₂CH₂-, -CH(CH₃)CH₂-, -C(CH₃)₂-, -C(CH₃)₂CH₂-, -C(CH₃)(CH₂OCH₃)-, -C(CH₃)CH₂CH₂-, -C(CH₃)₂CH=CH-, -CH₂CH₂CH₂CH₂-, -CH(CH₃)C(CH₃)₂-, -CH₂CH(CH₃)-, -CH₂CH(OCH₃)CH₂-, -CH₂CH(CH₃)CH₂- and -C(CH₃)(CH₂CH₃)-.

A further preferred embodiment Y1-3 of the invention relates to compounds according to the invention are compounds of formula (I), wherein X is a bond and Y is (C₂-C₈)-alkynyl, and in particular (C₂-C₄)-alkynyl.

A preferred example of moieties Y according to the embodiment Y1-3 is propargyl (-CH₂-C≡CH).

A further preferred embodiment of the present invention relates to compounds of the formula(I), where the group Y is Z.

Representative examples of Z are the following structures, which are substituted as herein defined, the arrow or # representing the bond to the remainder of the molecule of compound (I). n is 0. Saturated or partly unsaturated heterocyclic rings containing more than one heteroatom as ring member or containing a nitrogen atom as ring member are not according to the invention.

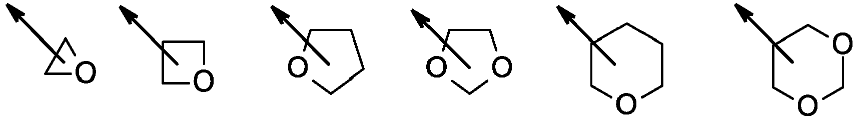

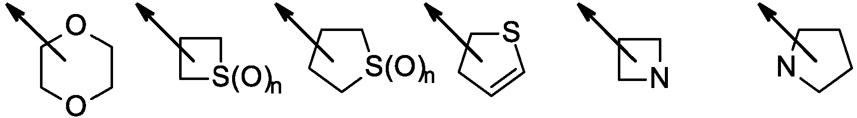

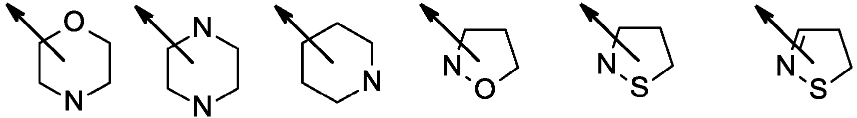

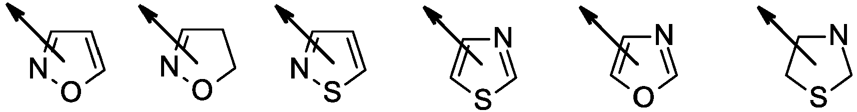

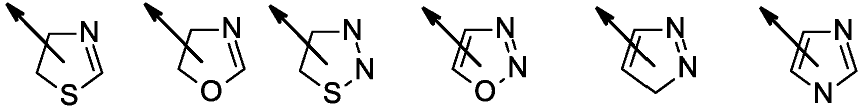

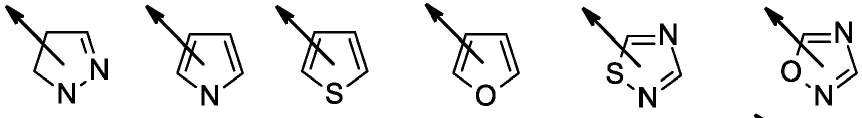

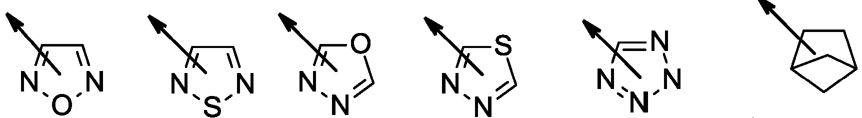

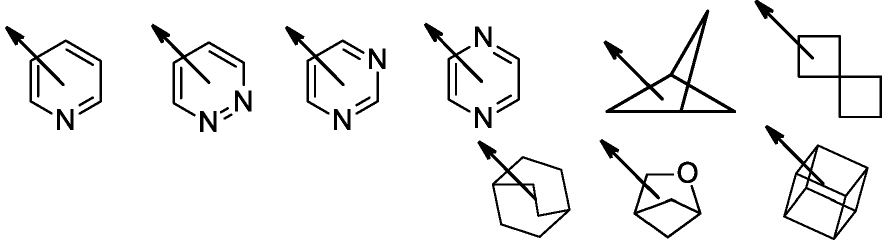

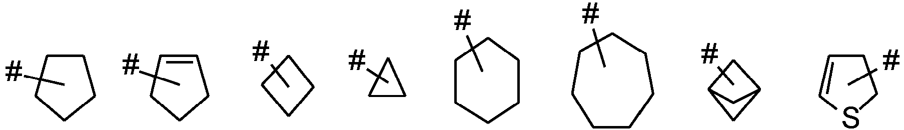

Also preferred compounds according to the invention are compounds of formula (I), wherein Z is selected from the group consisting of five-membered saturated or partly unsaturated rings, which are formed from 4 carbon atoms and 1 oxygen atom, each substituted by one radical CO₂R^{e} and by 0 or 1 (C₁-C₄)-alkyl groups.

Representative examples for the five-membered saturated or partly unsaturated rings Z, which are formed from 4 carbon atoms and 1 oxygen atom, each substituted by a radical CO₂R^{e} and by 0 or 1 (C₁-C₄)-alkyl groups , are the following structures, the wave line representing the bond to the remainder of the molecule of formula (I) (i.e. to X or to NR⁹ if X is a bond) and the arrow indicating the bond to any of the mentioned substituents. Those rings carrying a methoxy substituent or two methyl substituents are not according to the invention.

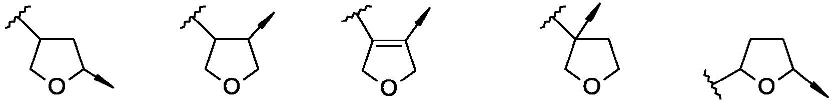

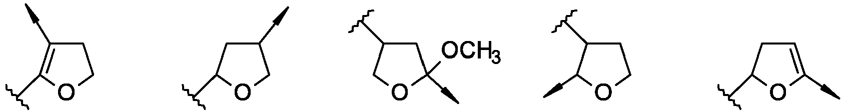

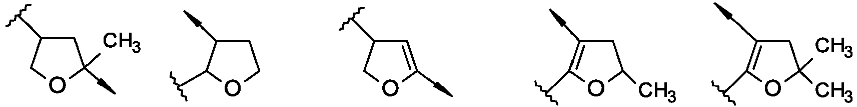

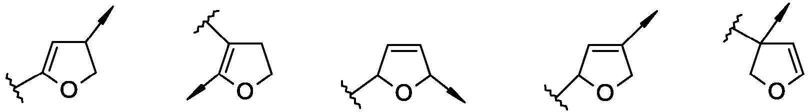

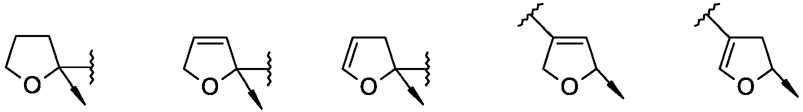

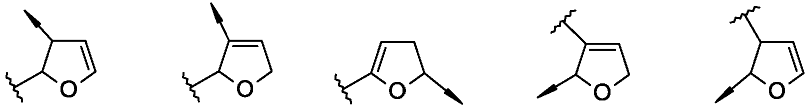

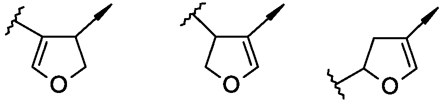

Preferred examples for the five-membered saturated or partly unsaturated rings Z, which are formed from 4 carbon atoms and 1 oxygen atom, each substituted by a radical CO₂R^{e} and by 0 or 1 (C₁-C₄)-alkyl groups are the following structures, the wave line representing the bond to the remainder of the molecule of formula (I) (i.e. to X or to NR⁹ if X is a bond) and the arrow or # indicating the bond to any of the mentioned substituents, preferably to CO₂R^{e}:

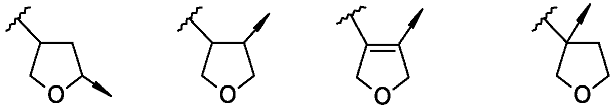

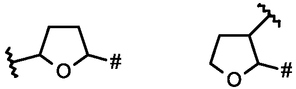

Also preferred compounds according to the invention are compounds of formula (I), wherein Z is selected from the group consisting of five-membered saturated or partly unsaturated rings, which are formed from 5 carbon atoms, each substituted by a radical CO₂R^{e}, by 0 or 1 fluorine atoms (if the ring is monocyclic) and by 0 or 1 (C₁-C₄)-alkyl groups.

Representative examples for the five-membered saturated or partly unsaturated rings, which are formed from 5 carbon atoms, each substituted by a radical CO₂R^{e}, by 0 or 1 fluorine atoms and by 0 or 1 (C₁-C₄)-alkyl groups, are the following structures, the wave line representing the bond to the remainder of the molecule of formula (I) (i.e. to X or to NR⁹ if X is a bond) and the arrow or # indicating the bond to any of the mentioned substituents. Those rings carrying a CF₃, methoxy or CN substituent or carrying 2 methyl groups are not according to the invention.

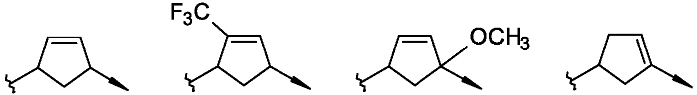

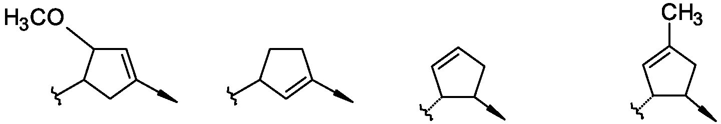

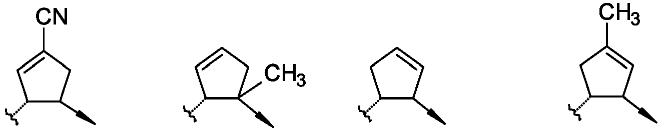

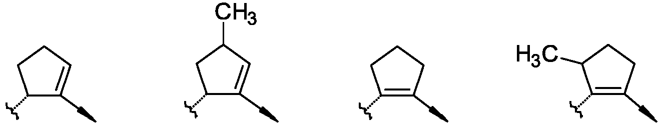

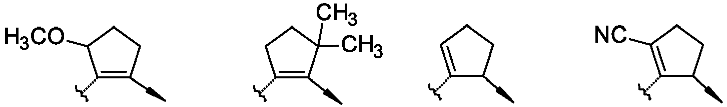

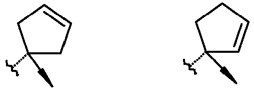

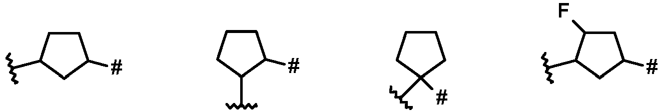

Preferred examples for the five-membered saturated or partly unsaturated rings, which are formed from 5 carbon atoms, each substituted by a radical CO₂R^{e}, by 0 or 1 fluorine atoms and by 0 or 1 (C₁-C₄)-alkyl groups, are the following structures, the the wave line representing the bond to the remainder of the molecule of formula (I) (i.e. to X or to NR⁹ if X is a bond) and the arrow or # indicating the bond to any of the mentioned substituents, preferably to CO₂R^{e}:

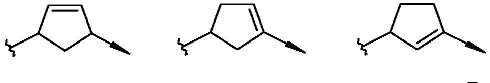

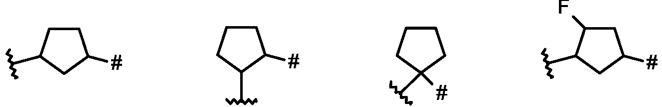

Preferred examples of the moiety Z of the compound (I) according to the present invention are the following structures Z.1 to Z.24, each substituted by 0 (only if Z is heteroaromatic) or 1 radical CO₂R^{e}, by 0 or 1 fluorine atoms and by 0 or 1 (C₁-C₄)-alkyl groups, where in each case the tilde (wave line) represents the bond to X or to NR⁹ if X is a bond, and #(1) and #(2) indicate the bonds to any of the mentioned substituents, i.e. to CO₂R^{e} (except for Z.17), F (if present) and (C₁-C₄)-alkyl (if present):

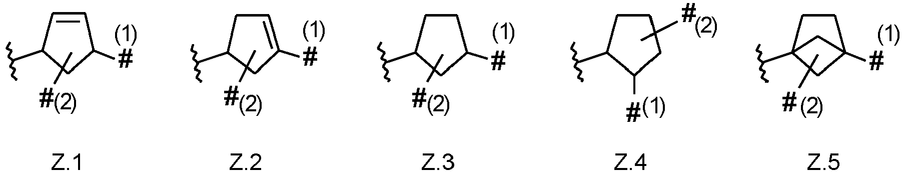

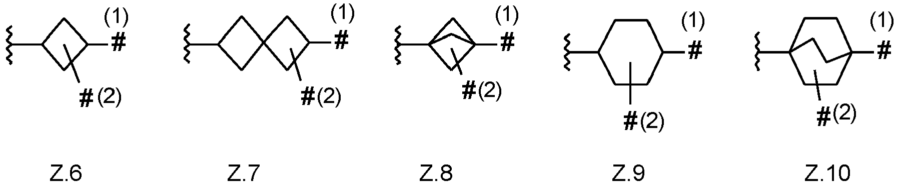

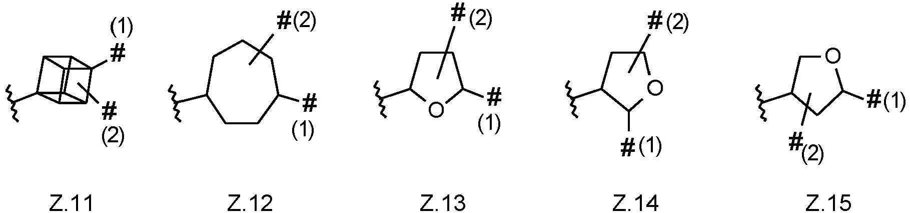

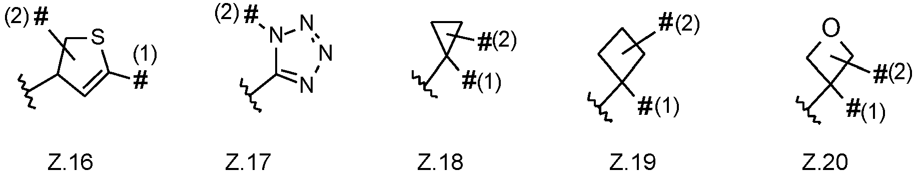

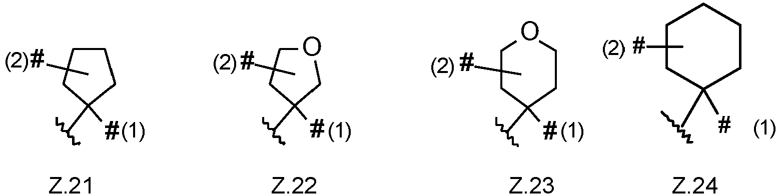

In particularly preferred structures Z.1 to Z.24 in each case #(1) indicates the bond to a substituent CO₂R^{e}, and #(2) indicates a bond to hydrogen, methyl or fluorine (the latter only if Z is a carbocyclic monocyclic ring and X is a bond).

Particularly preferred examples of the moiety Z of the compound (I) according to the present invention are the following structures Z.1 to Z.7, Z.9, Z.12 and Z.15 to Z.17, each substituted by 0 (only if Z is heteroaromatic) or 1 radicals CO₂R^{e}, by 0 or 1 fluorine atoms and by 0 or 1 (C₁-C₄)-alkyl groups, where in each case the tilde (wave line) represents the bond to X or to NR⁹ if X is a bond, and #(1) and #(2) indicate the bonds to any of the mentioned substituents, i.e. to CO₂R^{e} (except for Z.17), F (if present) and (C₁-C₄)-alkyl (if present):

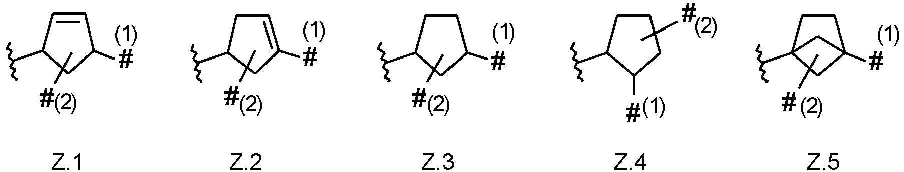

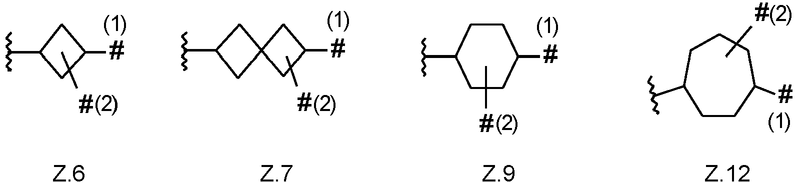

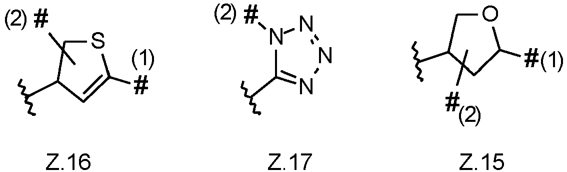

In particularly preferred structures Z.1 to Z.7, Z.9, Z.12 and Z.15 to Z.17 in each case #(1) indicates a bond to a substituent CO₂R^{e}, and #(2) indicates a bond to a substituent to hydrogen, methyl or fluorine (in case of Z.17 however to hydrogen or methyl).

Another preferred embodiment relates to compounds of the formula (I), where X is a bond and Y is (C₁-C₆)-alkyl or (C₂-C₆)-alkenyl, where the two last-mentioned radicals are substituted by a group CO₂R^{e}, CONR^{b}R^{h} or CONR^{e}SO₂R^{a} and by 0 or 1 (C₁-C₄)-alkoxy groups, and where R^{a}, R^{b}, R^{e} or R^{h} have the meanings defined herein, in particular the preferred meanings.

A more preferred embodiment relates to compounds of the formula (I), where X is a bond and Y is is (C₁-C₆)-alkyl substituted by a group CO₂R^{e}, CONR^{b}R^{h} or CONR^{e1}SO₂R^{a} and by 0 or 1 (C₁-C₄)-alkoxy groups, where R^{e1} is hydrogen or (C₁-C₄)-alkyl and R^{a}, R^{b}, R^{e} or R^{h} have the meanings defined herein, in particular the preferred meanings.

A further preferred embodiment relates to compounds of the formula (I), where X is a bond and Y is (C₁-C₆)-alkyl substituted by Z, where Z is a 3-, 4-, 5- or 6-membered saturated monocyclic carbocyclic ring substituted by a group CO₂R^{e} and by 0 or 1 (C₁-C₄)-alkyl groups or is a 5- or 6-membered saturated monocyclic heterocyclic ring containing 1 oxygen atom as ring member, where the heterocyclic ring is substituted by a group CO₂R^{e} and by 0 or 1 (C₁-C₄)-alkyl groups, and where R^{e} has one of the meanings defined herein, in particular one of the preferred meanings.

A further preferred embodiment relates to compounds of the formula (I), where X is a bond and Y is (C₂-C₈)-alkynyl.

Another preferred embodiment of the present invention relates to compounds of the formula (I), where X is X⁶, wherein R¹⁰ to R¹³ are independently hydrogen or methyl, preferably hydrogen, and Y is (C₁-C₆)-alkyl substituted by a group CO₂R^{e} and by 0 or 1 (C₁-C₄)-alkoxy groups, and where R^{e} has one of the meanings defined herein, in particular one of the preferred meanings.

A more preferred embodiment relates to compounds of the formula (I), where X is X⁶, wherein R¹⁰ to R¹³ are independently hydrogen, and Y is (C₁-C₄)-alkyl substituted by a group CO₂R^{e}, where R^{e} has one of the meanings defined herein, in particular one of the preferred meanings.

Another preferred embodiment relates to compounds of the formula (I), where X is a bond and Y is Z, which is a 3-, 4-, 5-, 6-, 7- or 8-membered saturated or partly unsaturated monocyclic carbocyclic ring substituted by a group CO₂R^{e}, by 0 or 1 fluorine atoms and by 0 or 1 (C₁-C₄)-alkyl groups, where R^{e} has one of the meanings defined herein, in particular one of the preferred meanings.

A more preferred embodiment relates to compounds of the formula (I), where X is a bond and Y is Z, which is a 4-, 5-, 6-, 7- or 8-membered saturated or partly unsaturated monocyclic carbocyclic ring substituted by a group CO₂R^{e} and by 0 or 1 fluorine atoms, where R^{e} has one of the meanings defined herein, in particular one of the preferred meanings. Specifically, Z is a 4-, 5-, 6- or 7-membered saturated or partly unsaturated monocyclic carbocyclic ring substituted by a group CO₂R^{e} and by 0 or 1 fluorine atoms, where R^{e} has one of the meanings defined herein, in particular one of the preferred meanings.

A further preferred embodiment relates to compounds of the formula (I), where X is a bond and Y is Z, which is a 5-, 6-, 7- or 8-membered saturated or partly unsaturated bicyclic carbocyclic ring substituted by a group CO₂R^{e}, and by 0 or 1 (C₁-C₄)-alkyl groups, where R^{e} has one of the meanings defined herein, in particular one of the preferred meanings.

A further more preferred embodiment relates to compounds of the formula (I), where X is a bond and Y is Z, which is a 6-, 7- or 8-membered saturated or partly unsaturated bicyclic carbocyclic ring substituted by a group CO₂R^{e}, and by 0 or 1 (C₁-C₄)-alkyl groups, where R^{e} has one of the meanings defined herein, in particular one of the preferred meanings. Specifically, Z is a 6- or 7-membered saturated bicyclic (spirocyclic or bridged) carbocyclic ring substituted by a group CO₂R^{e}, where R^{e} has one of the meanings defined herein, in particular one of the preferred meanings.

A further preferred embodiment relates to compounds of the formula (I), where X is a bond and Y is Z, which is an 8-membered saturated polycyclic carbocyclic ring substituted by a group CO₂R^{e} and by 0 or 1 (C₁-C₄)-alkyl groups, where R^{e} has one of the meanings defined herein, in particular one of the preferred meanings. Specifically, Z is cubyl substituted by a group CO₂R^{e}, where R^{e} has one of the meanings defined herein, in particular one of the preferred meanings.

A further preferred embodiment relates to compounds of the formula (I), where X is a bond and Y is Z, which is a 5- or 6-membered saturated or partly unsaturated monocyclic heterocyclic ring containing one oxygen atom or one sulfur atom as ring member, where the heterocyclic ring is substituted by a group CO₂R^{e} and by 0 or 1 (C₁-C₄)-alkyl groups, and where R^{e} has one of the meanings defined herein, in particular one of the preferred meanings.

A more preferred embodiment relates to compounds of the formula (I), where X is a bond and Y is Z, which is a 5- or 6-membered saturated or partly unsaturated monocyclic heterocyclic ring containing one oxygen atom or one sulfur atom as ring member, where the heterocyclic ring is substituted by a group CO₂R^{e}, and where R^{e} has one of the meanings defined herein, in particular one of the preferred meanings.

A further preferred embodiment relates to compounds of the formula (I), where X is a bond and Y is Z, which is a 5- or 6-membered heteroaromatic ring containing 1, 2, 3 or 4 heteroatoms selected from N, O and S as ring members, where the heteroaromatic ring is substituted by 0 or 1 (C₁-C₄)-alkyl groups.

A more preferred embodiment relates to compounds of the formula (I), where X is a bond and Y is Z, which a 5-membered heteroaromatic ring containing 1, 2, 3 or 4 nitrogen atoms as ring members, where the heteroaromatic ring is substituted by 0 or 1 (C₁-C₄)-alkyl groups.

R^{e} is hydrogen, (C₁-C₆)-alkyl which is unsubstituted or substituted by 1, 2 or 3 fluorine or chlorine atoms or by 1 radical selected from the group consisting of (C₁-C₂)-alkoxy, (C₁-C₃)-alkylsulfonyl, (C₁-C₃)-alkylthio, phenylthio, phenylsulfonyl and furanyl; or is (C₂-C₄)-alkynyl, (C₃-C₆)-cycloalkyl or (C₃-C₆)-cycloalkyl-(C₁-C₃)-alkyl. (C₁-C₃)-Alkylsulfonyl, (C₁-C₃)-alkylthio, phenylthio and phenylsulfonyl can be substituted by 0, 1, 2, 3, 4 or 5 fluorine, chlorine and/or bromine atoms. In particular, R^{e} is hydrogen, (C₁-C₆)-alkyl which is unsubstituted or substituted by 1, 2 or 3 fluorine or chlorine atoms or by 1 radical selected from the group consisting of (C₁-C₂)-alkoxy, (C₁-C₃)-alkylsulfonyl, (C₁-C₃)-alkylthio, phenylsulfonyl, phenylthio and furanyl; or is (C₂-C₄)-alkynyl or (C₃-C₆)-cycloalkyl-(C₁-C₃)-alkyl.

Accoding to the invention, in compounds of formula (I), the substituents have the following meanings:
- R¹: is hydrogen;
- R²: is hydrogen or halogen;
- R³: is hydrogen, halogen, cyano, (C₁-C₃)-alkyl, (C₁-C₃)-haloalkyl, (C₁-C₃)-alkoxy or (C₁-C₃)-haloalkoxy;
- R⁴: is hydrogen or halogen;
- R⁵: is hydrogen, halogen, cyano, (C₁-C₃)-alkyl or (C₁-C₃)-haloalkyl, (C₁-C₃)-alkoxy or (C₁-C₃)-haloalkoxy;
- R⁶: is hydrogen;
- R⁷ and R³: form, together with the carbon atom to which they are bound, a saturated or partially unsaturated 3-, 4-, 5- or 6-membered monocyclic heterocyclic ring W or a 6-, 7- or 8-membered bicyclic (preferably bridged) heterocyclic ring W, the ring containing, in addition to said carbon atom, q carbon atoms, u oxygen atoms, v nitrogen atoms, w sulfur atoms and x elements selected from the group consisting of NR^{d} and NC(O)OR^{d}, where one carbon atom bears p oxo groups and where the ring is substituted by n radicals R^{g};
- R⁹: is hydrogen or (C₁-C₄)-alkyl;
- X: is a bond and Y is Z, where
Z is a 3-, 4-, 5-, 6-, 7- or 8-membered saturated or partly unsaturated monocyclic carbocyclic ring substituted by a group CO₂R^{e}, by 0 or 1 fluorine atoms and by 0 or 1 (C₁-C₄)-alkyl groups; or
Z is a 6-, 7- or 8-membered saturated or partly unsaturated bicyclic carbocyclic ring substituted by a group CO₂R^{e}, and by 0 or 1 (C₁-C₄)-alkyl groups; or
Z is an 8-membered saturated polycyclic carbocyclic ring substituted by a group CO₂R^{e} and by 0 or 1 (C₁-C₄)-alkyl groups; or
Z is a 5- or 6-membered saturated or partly unsaturated monocyclic heterocyclic ring containing one oxygen atom or one sulfur atom as ring member, where the heterocyclic ring is substituted by a group CO₂R^{e} and by 0 or 1 (C₁-C₄)-alkyl groups; or
Z is a 5- or 6-membered heteroaromatic ring containing 1, 2, 3 or 4 heteroatoms selected from N, O and S as ring members, where the heteroaromatic ring is substituted by 0 or 1 (C₁-C₄)-alkyl groups; or
- X: is a bond and Y is (C₁-C₆)-alkyl or (C₂-C₆)-alkenyl, where the two last-mentioned radicals are substituted by a group CO₂R^{e}, CONR^{b}R^{h} or CONR^{e}SO₂R^{a} and by 0 or 1 (C₁-C₄)-alkoxy groups; or
- X: is a bond and Y is (C₁-C₆)-alkyl substituted by Z, where Z is a 3-, 4-, 5- or 6-membered saturated monocyclic carbocyclic ring substituted by a group CO₂R^{e} and by 0 or 1 (C₁-C₄)-alkyl groups or is a 5- or 6-membered saturated monocyclic heterocyclic ring containing 1 oxygen atom as ring member, where the heterocyclic ring is substituted by a group CO₂R^{e} and by 0 or 1 (C₁-C₄)-alkyl groups; or
- X: is a bond and Y is (C₂-C₈)-alkynyl; or
- X: is X⁶, wherein R¹⁰ to R¹³ are independently hydrogen or methyl, preferably hydrogen; and Y is (C₁-C₆)-alkyl substituted by a group CO₂R^{e} and by 0 or 1 (C₁-C₄)-alkoxy groups;
- R^{a}: is (C₁-C₆)-alkyl;
- R^{b}: is hydrogen or (C₁-C₆)-alkyl;
- each R^{d}: is independently hydrogen or (C₁-C₆)-alkyl;
- each R^{e}: is independently hydrogen, (C₁-C₆)-alkyl which is substituted by 0, 1, 2 or 3 fluorine or chlorine atoms or by 0 or 1 radicals selected from the group consisting of (C₁-C₂)-alkoxy, (C₁-C₃)-alkylsulfonyl, (C₁-C₃)-alkylthio, phenylthio, phenylsulfonyl and furanyl; or R^{e} is (C₂-C₄)-alkynyl, (C₃-C₆)-cycloalkyl or (C₃-C₆)-cycloalkyl-(C₁-C₃)-alkyl;
- R^{g}: is (C₁-C₃)-alkyl or (C₁-C₃)-haloalkyl, or two R^{g}, bound on the same carbon atom, form together a methylene group (=CH₂);
- R^{h}: is hydrogen, (C₁-C₆)-alkyl substituted by 0 or 1 cyano groups; or (C₂-C₄)-alkynyl;
- n: is 0, 1 or 2;
- p: is 0 or 1;
- q: is 1, 2, 3, 4, 5 or 6;
- u: is 0, 1 or 2;
- v: is 0 or 1;
- w: is 0 or 1;
- x: is 0 or 1;
with the proviso that the sum of u, v, w and x is 1 or 2.

In particular, in the compounds of formula (I),the substituents have the following meanings:
- R¹: is hydrogen;
- R²: is hydrogen;
- R³: is halogen, cyano or (C₁-C₃)-haloalkoxy;
- R⁴: is hydrogen or halogen;
- R⁵: is hydrogen or halogen;
- R⁶: is hydrogen;
- R⁷ and R⁸: form, together with the carbon atom to which they are bound, a saturated or partially unsaturated 3-, 4-, 5- or 6-membered monocyclic heterocyclic ring W or a 6-, 7- or 8-membered bicyclic (preferably bridged) heterocyclic ring W, the ring containing, in addition to said carbon atom, q carbon atoms, u oxygen atoms, v nitrogen atoms, w sulfur atoms and x elements selected from the group consisting of NR^{d} and NC(O)OR^{d}, where the ring is substituted by n radicals R^{g};
- R⁹: is hydrogen;
- X: is a bond and Y is Z, where
Z is a 4-, 5-, 6-, 7- or 8-membered saturated or partly unsaturated monocyclic carbocyclic ring substituted by a group CO₂R^{e} and by 0 or 1 fluorine atoms; or
Z is a 5-, 6-, 7- or 8-membered saturated or partly unsaturated bicyclic (preferably spirocyclic or bridged) carbocyclic ring substituted by a group CO₂R^{e} and by 0 or 1 (C₁-C₄)-alkyl groups; or
Z is a 5- or 6-membered saturated or partly unsaturated monocyclic heterocyclic ring containing 1 oxygen atom or one sulfur atom as ring member, where the heterocyclic ring is substituted by a group CO₂R^{e}; or
Z is a 5-membered heteroaromatic ring containing 1, 2, 3 or 4 nitrogen atoms as ring members, where the heteroaromatic ring is substituted by 0 or 1 (C₁-C₄)-alkyl groups; or
- X: is a bond and Y is (C₁-C₆)-alkyl substituted by a group CO₂R^{e}, CONR^{b}R^{h} or CONR^{e1}SO₂R^{a} and by 0 or 1 (C₁-C₄)-alkoxy groups; or
- X: is a bond and Y is (C₂-C₈)-alkynyl; or
- X: is X⁶, wherein R¹⁰ to R¹³ are independently hydrogen; and Y is (C₁-C₄)-alkyl substituted by a group CO₂R^{e};
- R^{a}: is (C₁-C₆)-alkyl;
- R^{b}: is hydrogen;
- each R^{d}: is independently hydrogen or (C₁-C₆)-alkyl;
- R^{e1}: is hydrogen or (C₁-C₄)-alkyl;
- each R^{e}: is independently hydrogen, (C₁-C₆)-alkyl which is substituted by 0, 1, 2 or 3 fluorine and/or chlorine atoms or by 0 or 1 radicals selected from the group consisting of (C₁-C₂)-alkoxy, (C₁-C₃)-alkylsulfonyl, (C₁-C₃)-alkylthio, phenylsulfonyl, phenylthio and furanyl, where the aliphatic or aromatic moieties in (C₁-C₂)-alkoxy, (C₁-C₃)-alkylsulfonyl, (C₁-C₃)-alkylthio, phenylsulfonyl and phenylthio may carry 0, 1, 2, 3, 4 or 5 fluorine, chlorine and/or bromine atoms (but are in particular unsubstituted); or is (C₂-C₄)-alkynyl or (C₃-C₆)-cycloalkyl-(C₁-C₃)-alkyl;
- R^{g}: is (C₁-C₃)-alkyl, or two R^{g}, bound on the same carbon atom, form together a methylene group (=CH₂);
- R^{h}: is (C₁-C₆)-alkyl substituted by 0 or 1 cyano groups; or (C₂-C₄)-alkynyl;
- n: is 0, 1 or 2;
- q: is 1, 2, 3, 4, 5 or 6;
- u: is 0, 1 or 2;
- v: is 0 or 1;
- w: is 0 or 1;
- x: is 0 or 1;
with the proviso that the sum of u, v, w and x is 1 or 2.

Specifically, in the compounds of formula (I),the substituents have the following meanings:
- R¹: is hydrogen;
- R²: is hydrogen;
- R³: is halogen, cyano or (C₁-C₃)-haloalkoxy;
- R⁴: is hydrogen or halogen;
- R⁵: is hydrogen or halogen;
- R⁶: is hydrogen;
- R⁷ and R⁸: form, together with the carbon atom to which they are bound, a saturated or partially unsaturated 3-, 4-, 5- or 6-membered monocyclic heterocyclic ring W or a 6-, 7- or 8-membered bicyclic (preferably bridged) heterocyclic ring W, the ring containing, in addition to said carbon atom, q carbon atoms, u oxygen atoms, v nitrogen atoms, w sulfur atoms and x elements selected from the group consisting of NR^{d} and NC(O)OR^{d}, where the ring is substituted by n radicals R^{g};
- R⁹: is hydrogen;
- X: is a bond and Y is Z, where
Z is a 4-, 5-, 6- or 7-membered saturated or partly unsaturated monocyclic carbocyclic ring substituted by a group CO₂R^{e} and by 0 or 1 fluorine atoms; or
Z is a 6- or 7-membered saturated bicyclic (preferably spirocyclic or bridged) carbocyclic ring substituted by a group CO₂R^{e}; or
Z is a 5-membered saturated or partly unsaturated monocyclic heterocyclic ring containing 1 oxygen atom or one sulfur atom as ring member, where the heterocyclic ring is substituted by a group CO₂R^{e}; or
Z is a 5-membered heteroaromatic ring containing 1, 2, 3 or 4 nitrogen atoms as ring members, where the heteroaromatic ring is substituted by 1 (C₁-C₄)-alkyl group; or
- X: is a bond and Y is (C₁-C₆)-alkyl substituted by a group CO₂R^{e}, CONR^{b}R^{h} or CONR^{e1}SO₂R^{a} and by 0 or 1 (C₁-C₄)-alkoxy groups; or
- X: is a bond and Y is (C₂-C₈)-alkynyl; or
- X: is X⁶, wherein R¹⁰ to R¹³ are independently hydrogen; and Y is (C₁-C₄)-alkyl substituted by a group CO₂R^{e};
- R^{a}: is (C₁-C₆)-alkyl;
- R^{b}: is hydrogen;
- each R^{d}: is independently hydrogen or (C₁-C₆)-alkyl;
- R^{e1}: is hydrogen or (C₁-C₄)-alkyl;
- each R^{e}: is independently hydrogen, (C₁-C₆)-alkyl which is substituted by 0, 1, 2 or 3 fluorine and/or chlorine atoms or by 0 or 1 radicals selected from the group consisting of (C₁-C₂)-alkoxy, (C₁-C₃)-alkylsulfonyl, (C₁-C₃)-alkylthio, phenylthio and furanyl; or is (C₂-C₄)-alkynyl or (C₃-C₆)-cycloalkyl-(C₁-C₃)-alkyl;
- R^{g}: is (C₁-C₃)-alkyl, or two R^{g}, bound on the same carbon atom, form together a methylene group (=CH₂);
- R^{h}: is (C₁-C₃)-alkyl substituted by 0 or 1 cyano groups; or (C₂-C₄)-alkynyl;
- n: is 0, 1 or 2;
- q: is 1, 2, 3, 4, 5 or 6;
- u: is 0, 1 or 2;
- v: is 0 or 1;
- w: is 0 or 1;
- x: is 0 or 1;
with the proviso that the sum of u, v, w and x is 1 or 2.

Further preferred embodiments (I.I to I.II) of compounds of formula (I) are compounds, wherein
(I.I): R¹, R⁹ is hydrogen: 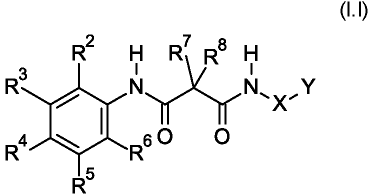
(I.II): R¹ is hydrogen, R⁹ is methyl:

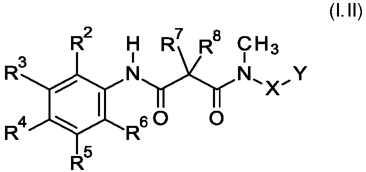

Compounds of formula (I.I.a,) wherein R¹, R², R⁶ and R⁹ are hydrogen are particularly preferred:

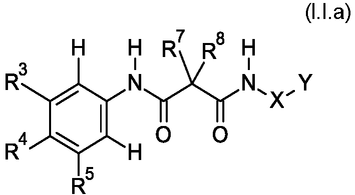

Compounds of formula (I.I.b,) wherein R¹, R², R⁴, R⁶ and R⁹ are hydrogen are also particularly preferred:

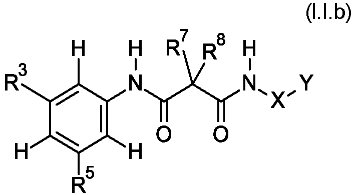

Compounds of formula (I.I.c,) wherein R¹, R², R⁶ and R⁹ are hydrogen, X is a bond (X⁰), and Y is Z are particularly preferred:

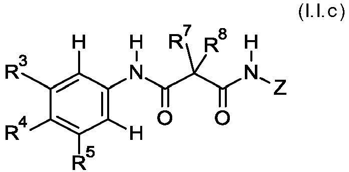

Compounds of formula (I.I.d,) wherein R¹, R², R⁴, R⁶ and R⁹ are hydrogen, X is a bond (X⁰), and Y is Z are also particularly preferred:

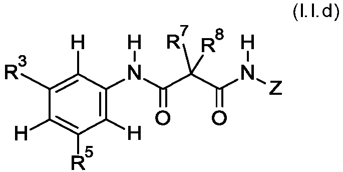

Compounds of formula (I.II.a,) wherein R¹, R², R⁶ are hydrogen and R⁹ is methyl are also particularly preferred:

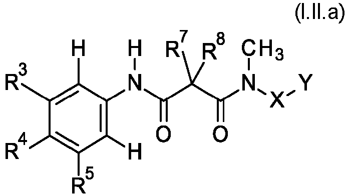

Compounds of formula (I.II.b,) wherein R¹, R², R⁴, R⁶ are hydrogen and R⁹ is methyl are also particularly preferred: and R⁹

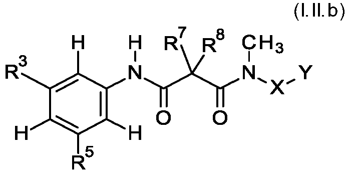

In the context of the present invention, compounds of the formula (I), wherein R¹, R², R⁶ and R⁹ are hydrogen and R³, R⁴, R⁵ and W (W is formed by R⁷ and R⁸ together with the carbon atom to which they are bound) have the meanings as defined lines in 1 to 2541 of Table 1 below, are particularly preferred. In case the carbon atom to which R⁷ and R⁸ are bound to form W is a center of chirality, both enantiomers are subject of this invention as well as mixtures thereof.

**Table 1:**

| Cpd. | R³ | R⁴ | R⁵ | W*⁾ |
|---|---|---|---|---|
| 1. | H | H | H | A.1 |
| 2. | F | H | H | A.1 |
| 3. | Cl | H | H | A.1 |
| 4. | Br | H | H | A.1 |
| 5. | I | H | H | A.1 |
| 6. | CN | H | H | A.1 |
| 7. | CH₃ | H | H | A.1 |
| 8. | CF₃ | H | H | A.1 |
| 9. | OCH₃ | H | H | A.1 |
| 10. | OCF₃ | H | H | A.1 |
| 11. | CH₂CH₃ | H | H | A.1 |
| 12. | H | F | H | A.1 |
| 13. | F | F | H | A.1 |
| 14. | Cl | F | H | A.1 |
| 15. | Br | F | H | A.1 |
| 16. | I | F | H | A.1 |
| 17. | CN | F | H | A.1 |
| 18. | CH₃ | F | H | A.1 |
| 19. | CF₃ | F | H | A.1 |
| 20. | OCH₃ | F | H | A.1 |
| 21. | OCF₃ | F | H | A.1 |
| 22. | CH₂CH₃ | F | H | A.1 |
| 23. | H | Cl | H | A.1 |
| 24. | F | Cl | H | A.1 |
| 25. | Cl | Cl | H | A.1 |
| 26. | Br | Cl | H | A.1 |
| 27. | I | Cl | H | A.1 |
| 28. | CN | Cl | H | A.1 |
| 29. | CH₃ | Cl | H | A.1 |
| 30. | CF₃ | Cl | H | A.1 |
| 31. | OCH₃ | Cl | H | A.1 |
| 32. | OCF₃ | Cl | H | A.1 |
| 33. | CH₂CH₃ | Cl | H | A.1 |
| 34. | H | H | F | A.1 |
| 35. | F | H | F | A.1 |
| 36. | Cl | H | F | A.1 |
| 37. | Br | H | F | A.1 |
| 38. | I | H | F | A.1 |
| 39. | CN | H | F | A.1 |
| 40. | CH₃ | H | F | A.1 |
| 41. | CF₃ | H | F | A.1 |
| 42. | OCH₃ | H | F | A.1 |
| 43. | OCF₃ | H | F | A.1 |
| 44. | CH₂CH₃ | H | F | A.1 |
| 45. | H | F | F | A.1 |
| 46. | F | F | F | A.1 |
| 47. | Cl | F | F | A.1 |
| 48. | Br | F | F | A.1 |
| 49. | I | F | F | A.1 |
| 50. | CN | F | F | A.1 |
| 51. | CH₃ | F | F | A.1 |
| 52. | CF₃ | F | F | A.1 |
| 53. | OCH₃ | F | F | A.1 |
| 54. | OCF₃ | F | F | A.1 |
| 55. | CH₂CH₃ | F | F | A.1 |
| 56. | H | Cl | F | A.1 |
| 57. | F | Cl | F | A.1 |
| 58. | Cl | Cl | F | A.1 |
| 59. | Br | Cl | F | A.1 |
| 60. | I | Cl | F | A.1 |
| 61. | CN | Cl | F | A.1 |
| 62. | CH₃ | Cl | F | A.1 |
| 63. | CF₃ | Cl | F | A.1 |
| 64. | OCH₃ | Cl | F | A.1 |
| 65. | OCF₃ | Cl | F | A.1 |
| 66. | CH₂CH₃ | Cl | F | A.1 |
| 67. | H | H | Cl | A.1 |
| 68. | F | H | Cl | A.1 |
| 69. | Cl | H | Cl | A.1 |
| 70. | Br | H | Cl | A.1 |
| 71. | I | H | Cl | A.1 |
| 72. | CN | H | Cl | A.1 |
| 73. | CH₃ | H | Cl | A.1 |
| 74. | CF₃ | H | Cl | A.1 |
| 75. | OCH₃ | H | Cl | A.1 |
| 76. | OCF₃ | H | Cl | A.1 |
| 77. | CH₂CH₃ | H | Cl | A.1 |
| 78. | H | F | Cl | A.1 |
| 79. | F | F | Cl | A.1 |
| 80. | Cl | F | Cl | A.1 |
| 81. | Br | F | Cl | A.1 |
| 82. | I | F | Cl | A.1 |
| 83. | CN | F | Cl | A.1 |
| 84. | CH₃ | F | Cl | A.1 |
| 85. | CF₃ | F | Cl | A.1 |
| 86. | OCH₃ | F | Cl | A.1 |
| 87. | OCF₃ | F | Cl | A.1 |
| 88. | CH₂CH₃ | F | Cl | A.1 |

| Cpd. | R³ | R⁴ | R⁵ | W *⁾ |
|---|---|---|---|---|
| 89. | H | Cl | Cl | A.1 |
| 90. | F | Cl | Cl | A.1 |
| 91. | Cl | Cl | Cl | A.1 |
| 92. | Br | Cl | Cl | A.1 |
| 93. | I | Cl | Cl | A.1 |
| 94. | CN | Cl | Cl | A.1 |
| 95. | CH₃ | Cl | Cl | A.1 |
| 96. | CF₃ | Cl | Cl | A.1 |
| 97. | OCH₃ | Cl | Cl | A.1 |
| 98. | OCF₃ | Cl | Cl | A.1 |
| 99. | CH₂CH₃ | Cl | Cl | A.1 |
| 100. | H | H | Br | A.1 |
| 101. | F | H | Br | A.1 |
| 102. | Cl | H | Br | A.1 |
| 103. | Br | H | Br | A.1 |
| 104. | I | H | Br | A.1 |
| 105. | CN | H | Br | A.1 |
| 106. | CH₃ | H | Br | A.1 |
| 107. | CF₃ | H | Br | A.1 |
| 108. | OCH₃ | H | Br | A.1 |
| 109. | OCF₃ | H | Br | A.1 |
| 110. | CH₂CH₃ | H | Br | A.1 |
| 111. | H | F | Br | A.1 |
| 112. | F | F | Br | A.1 |
| 113. | Cl | F | Br | A.1 |
| 114. | Br | F | Br | A.1 |
| 115. | I | F | Br | A.1 |
| 116. | CN | F | Br | A.1 |
| 117. | CH₃ | F | Br | A.1 |
| 118. | CF₃ | F | Br | A.1 |
| 119. | OCH₃ | F | Br | A.1 |
| 120. | OCF₃ | F | Br | A.1 |
| 121. | CH₂CH₃ | F | Br | A.1 |
| 122. | H | Cl | Br | A.1 |
| 123. | F | Cl | Br | A.1 |
| 124. | Cl | Cl | Br | A.1 |
| 125. | Br | Cl | Br | A.1 |
| 126. | I | Cl | Br | A.1 |
| 127. | CN | Cl | Br | A.1 |
| 128. | CH₃ | Cl | Br | A.1 |
| 129. | CF₃ | Cl | Br | A.1 |
| 130. | OCH₃ | Cl | Br | A.1 |
| 131. | OCF₃ | Cl | Br | A.1 |
| 132. | CH₂CH₃ | Cl | Br | A.1 |
| 133. | H | H | I | A.1 |
| 134. | F | H | I | A.1 |
| 135. | Cl | H | I | A.1 |
| 136. | Br | H | I | A.1 |
| 137. | I | H | I | A.1 |
| 138. | CN | H | I | A.1 |
| 139. | CH₃ | H | I | A.1 |
| 140. | CF₃ | H | I | A.1 |
| 141. | OCH₃ | H | I | A.1 |
| 142. | OCF₃ | H | I | A.1 |
| 143. | CH₂CH₃ | H | I | A.1 |
| 144. | H | F | I | A.1 |
| 145. | F | F | I | A.1 |
| 146. | Cl | F | I | A.1 |
| 147. | Br | F | I | A.1 |
| 148. | I | F | I | A.1 |
| 149. | CN | F | I | A.1 |
| 150. | CH₃ | F | I | A.1 |
| 151. | CF₃ | F | I | A.1 |
| 152. | OCH₃ | F | I | A.1 |
| 153. | OCF₃ | F | I | A.1 |
| 154. | CH₂CH₃ | F | I | A.1 |
| 155. | H | Cl | I | A.1 |
| 156. | F | Cl | I | A.1 |
| 157. | Cl | Cl | I | A.1 |
| 158. | Br | Cl | I | A.1 |
| 159. | I | Cl | I | A.1 |
| 160. | CN | Cl | I | A.1 |
| 161. | CH₃ | Cl | I | A.1 |
| 162. | CF₃ | Cl | I | A.1 |
| 163. | OCH₃ | Cl | I | A.1 |
| 164. | OCF₃ | Cl | I | A.1 |
| 165. | CH₂CH₃ | Cl | I | A.1 |
| 166. | H | H | CN | A.1 |
| 167. | F | H | CN | A.1 |
| 168. | Cl | H | CN | A.1 |
| 169. | Br | H | CN | A.1 |
| 170. | I | H | CN | A.1 |
| 171. | CN | H | CN | A.1 |
| 172. | CH₃ | H | CN | A.1 |
| 173. | CF₃ | H | CN | A.1 |
| 174. | OCH₃ | H | CN | A.1 |
| 175. | OCF₃ | H | CN | A.1 |
| 176. | CH₂CH₃ | H | CN | A.1 |
| 177. | H | F | CN | A.1 |
| 178. | F | F | CN | A.1 |
| 179. | Cl | F | CN | A.1 |
| 180. | Br | F | CN | A.1 |
| 181. | I | F | CN | A.1 |
| 182. | CN | F | CN | A.1 |
| 183. | CH₃ | F | CN | A.1 |
| 184. | CF₃ | F | CN | A.1 |
| 185. | OCH₃ | F | CN | A.1 |
| 186. | OCF₃ | F | CN | A.1 |
| 187. | CH₂CH₃ | F | CN | A.1 |
| 188. | H | Cl | CN | A.1 |
| 189. | F | Cl | CN | A.1 |
| 190. | Cl | Cl | CN | A.1 |
| 191. | Br | Cl | CN | A.1 |
| 192. | I | Cl | CN | A.1 |
| 193. | CN | Cl | CN | A.1 |
| 194. | CH₃ | Cl | CN | A.1 |
| 195. | CF₃ | Cl | CN | A.1 |
| 196. | OCH₃ | Cl | CN | A.1 |
| 197. | OCF₃ | Cl | CN | A.1 |
| 198. | CH₂CH₃ | Cl | CN | A.1 |
| 199. | H | H | CH₃ | A.1 |
| 200. | F | H | CH₃ | A.1 |
| 201. | Cl | H | CH₃ | A.1 |
| 202. | Br | H | CH₃ | A.1 |
| 203. | I | H | CH₃ | A.1 |
| 204. | CN | H | CH₃ | A.1 |
| 205. | CH₃ | H | CH₃ | A.1 |
| 206. | CF₃ | H | CH₃ | A.1 |
| 207. | OCH₃ | H | CH₃ | A.1 |
| 208. | OCF₃ | H | CH₃ | A.1 |
| 209. | CH₂CH₃ | H | CH₃ | A.1 |
| 210. | H | F | CH₃ | A.1 |
| 211. | F | F | CH₃ | A.1 |
| 212. | Cl | F | CH₃ | A.1 |
| 213. | Br | F | CH₃ | A.1 |
| 214. | I | F | CH₃ | A.1 |
| 215. | CN | F | CH₃ | A.1 |
| 216. | CH₃ | F | CH₃ | A.1 |
| 217. | CF₃ | F | CH₃ | A.1 |
| 218. | OCH₃ | F | CH₃ | A.1 |
| 219. | OCF₃ | F | CH₃ | A.1 |
| 220. | CH₂CH₃ | F | CH₃ | A.1 |
| 221. | H | Cl | CH₃ | A.1 |
| 222. | F | Cl | CH₃ | A.1 |
| 223. | Cl | Cl | CH₃ | A.1 |
| 224. | Br | Cl | CH₃ | A.1 |
| 225. | I | Cl | CH₃ | A.1 |
| 226. | CN | Cl | CH₃ | A.1 |
| 227. | CH₃ | Cl | CH₃ | A.1 |
| 228. | CF₃ | Cl | CH₃ | A.1 |
| 229. | OCH₃ | Cl | CH₃ | A.1 |
| 230. | OCF₃ | Cl | CH₃ | A.1 |
| 231. | CH₂CH₃ | Cl | CH₃ | A.1 |
| 232. | H | H | H | A.2 |
| 233. | F | H | H | A.2 |
| 234. | Cl | H | H | A.2 |
| 235. | Br | H | H | A.2 |
| 236. | I | H | H | A.2 |
| 237. | CN | H | H | A.2 |
| 238. | CH₃ | H | H | A.2 |
| 239. | CF₃ | H | H | A.2 |
| 240. | OCH₃ | H | H | A.2 |
| 241. | OCF₃ | H | H | A.2 |
| 242. | CH₂CH₃ | H | H | A.2 |
| 243. | H | F | H | A.2 |
| 244. | F | F | H | A.2 |
| 245. | Cl | F | H | A.2 |
| 246. | Br | F | H | A.2 |
| 247. | I | F | H | A.2 |
| 248. | CN | F | H | A.2 |
| 249. | CH₃ | F | H | A.2 |
| 250. | CF₃ | F | H | A.2 |
| 251. | OCH₃ | F | H | A.2 |
| 252. | OCF₃ | F | H | A.2 |
| 253. | CH₂CH₃ | F | H | A.2 |
| 254. | H | Cl | H | A.2 |
| 255. | F | Cl | H | A.2 |
| 256. | Cl | Cl | H | A.2 |
| 257. | Br | Cl | H | A.2 |
| 258. | I | Cl | H | A.2 |
| 259. | CN | Cl | H | A.2 |
| 260. | CH₃ | Cl | H | A.2 |
| 261. | CF₃ | Cl | H | A.2 |
| 262. | OCH₃ | Cl | H | A.2 |
| 263. | OCF₃ | Cl | H | A.2 |
| 264. | CH₂CH₃ | Cl | H | A.2 |
| 265. | H | H | F | A.2 |
| 266. | F | H | F | A.2 |
| 267. | Cl | H | F | A.2 |
| 268. | Br | H | F | A.2 |
| 269. | I | H | F | A.2 |
| 270. | CN | H | F | A.2 |
| 271. | CH₃ | H | F | A.2 |
| 272. | CF₃ | H | F | A.2 |
| 273. | OCH₃ | H | F | A.2 |
| 274. | OCF₃ | H | F | A.2 |
| 275. | CH₂CH₃ | H | F | A.2 |
| 276. | H | F | F | A.2 |
| 277. | F | F | F | A.2 |
| 278. | Cl | F | F | A.2 |
| 279. | Br | F | F | A.2 |
| 280. | I | F | F | A.2 |
| 281. | CN | F | F | A.2 |
| 282. | CH₃ | F | F | A.2 |
| 283. | CF₃ | F | F | A.2 |
| 284. | OCH₃ | F | F | A.2 |
| 285. | OCF₃ | F | F | A.2 |
| 286. | CH₂CH₃ | F | F | A.2 |
| 287. | H | Cl | F | A.2 |
| 288. | F | Cl | F | A.2 |
| 289. | Cl | Cl | F | A.2 |
| 290. | Br | Cl | F | A.2 |
| 291. | I | Cl | F | A.2 |
| 292. | CN | Cl | F | A.2 |
| 293. | CH₃ | Cl | F | A.2 |
| 294. | CF₃ | Cl | F | A.2 |
| 295. | OCH₃ | Cl | F | A.2 |
| 296. | OCF₃ | Cl | F | A.2 |
| 297. | CH₂CH₃ | Cl | F | A.2 |
| 298. | H | H | Cl | A.2 |
| 299. | F | H | Cl | A.2 |
| 300. | Cl | H | Cl | A.2 |
| 301. | Br | H | Cl | A.2 |
| 302. | I | H | Cl | A.2 |
| 303. | CN | H | Cl | A.2 |
| 304. | CH₃ | H | Cl | A.2 |
| 305. | CF₃ | H | Cl | A.2 |
| 306. | OCH₃ | H | Cl | A.2 |
| 307. | OCF₃ | H | Cl | A.2 |
| 308. | CH₂CH₃ | H | Cl | A.2 |
| 309. | H | F | Cl | A.2 |
| 310. | F | F | Cl | A.2 |
| 311. | Cl | F | Cl | A.2 |
| 312. | Br | F | Cl | A.2 |
| 313. | I | F | Cl | A.2 |
| 314. | CN | F | Cl | A.2 |
| 315. | CH₃ | F | Cl | A.2 |
| 316. | CF₃ | F | Cl | A.2 |
| 317. | OCH₃ | F | Cl | A.2 |
| 318. | OCF₃ | F | Cl | A.2 |
| 319. | CH₂CH₃ | F | Cl | A.2 |
| 320. | H | Cl | Cl | A.2 |
| 321. | F | Cl | Cl | A.2 |
| 322. | Cl | Cl | Cl | A.2 |
| 323. | Br | Cl | Cl | A.2 |
| 324. | I | Cl | Cl | A.2 |
| 325. | CN | Cl | Cl | A.2 |
| 326. | CH₃ | Cl | Cl | A.2 |
| 327. | CF₃ | Cl | Cl | A.2 |
| 328. | OCH₃ | Cl | Cl | A.2 |
| 329. | OCF₃ | Cl | Cl | A.2 |
| 330. | CH₂CH₃ | Cl | Cl | A.2 |
| 331. | H | H | Br | A.2 |
| 332. | F | H | Br | A.2 |
| 333. | Cl | H | Br | A.2 |
| 334. | Br | H | Br | A.2 |
| 335. | I | H | Br | A.2 |
| 336. | CN | H | Br | A.2 |
| 337. | CH₃ | H | Br | A.2 |
| 338. | CF₃ | H | Br | A.2 |
| 339. | OCH₃ | H | Br | A.2 |
| 340. | OCF₃ | H | Br | A.2 |
| 341. | CH₂CH₃ | H | Br | A.2 |
| 342. | H | F | Br | A.2 |
| 343. | F | F | Br | A.2 |
| 344. | Cl | F | Br | A.2 |
| 345. | Br | F | Br | A.2 |
| 346. | I | F | Br | A.2 |
| 347. | CN | F | Br | A.2 |
| 348. | CH₃ | F | Br | A.2 |
| 349. | CF₃ | F | Br | A.2 |
| 350. | OCH₃ | F | Br | A.2 |
| 351. | OCF₃ | F | Br | A.2 |
| 352. | CH₂CH₃ | F | Br | A.2 |
| 353. | H | Cl | Br | A.2 |
| 354. | F | Cl | Br | A.2 |
| 355. | Cl | Cl | Br | A.2 |
| 356. | Br | Cl | Br | A.2 |
| 357. | I | Cl | Br | A.2 |
| 358. | CN | Cl | Br | A.2 |
| 359. | CH₃ | Cl | Br | A.2 |
| 360. | CF₃ | Cl | Br | A.2 |
| 361. | OCH₃ | Cl | Br | A.2 |
| 362. | OCF₃ | Cl | Br | A.2 |
| 363. | CH₂CH₃ | Cl | Br | A.2 |
| 364. | H | H | I | A.2 |
| 365. | F | H | I | A.2 |
| 366. | Cl | H | I | A.2 |
| 367. | Br | H | I | A.2 |
| 368. | I | H | I | A.2 |
| 369. | CN | H | I | A.2 |
| 370. | CH₃ | H | I | A.2 |
| 371. | CF₃ | H | I | A.2 |
| 372. | OCH₃ | H | I | A.2 |
| 373. | OCF₃ | H | I | A.2 |
| 374. | CH₂CH₃ | H | I | A.2 |
| 375. | H | F | I | A.2 |
| 376. | F | F | I | A.2 |
| 377. | Cl | F | I | A.2 |
| 378. | Br | F | I | A.2 |
| 379. | I | F | I | A.2 |
| 380. | CN | F | I | A.2 |
| 381. | CH₃ | F | I | A.2 |
| 382. | CF₃ | F | I | A.2 |
| 383. | OCH₃ | F | I | A.2 |
| 384. | OCF₃ | F | I | A.2 |
| 385. | CH₂CH₃ | F | I | A.2 |
| 386. | H | Cl | I | A.2 |
| 387. | F | Cl | I | A.2 |
| 388. | Cl | Cl | I | A.2 |
| 389. | Br | Cl | I | A.2 |
| 390. | I | Cl | I | A.2 |
| 391. | CN | Cl | I | A.2 |
| 392. | CH₃ | Cl | I | A.2 |
| 393. | CF₃ | Cl | I | A.2 |
| 394. | OCH₃ | Cl | I | A.2 |
| 395. | OCF₃ | Cl | I | A.2 |
| 396. | CH₂CH₃ | Cl | I | A.2 |
| 397. | H | H | CN | A.2 |
| 398. | F | H | CN | A.2 |
| 399. | Cl | H | CN | A.2 |
| 400. | Br | H | CN | A.2 |
| 401. | I | H | CN | A.2 |
| 402. | CN | H | CN | A.2 |
| 403. | CH₃ | H | CN | A.2 |
| 404. | CF₃ | H | CN | A.2 |
| 405. | OCH₃ | H | CN | A.2 |
| 406. | OCF₃ | H | CN | A.2 |
| 407. | CH₂CH₃ | H | CN | A.2 |
| 408. | H | F | CN | A.2 |
| 409. | F | F | CN | A.2 |
| 410. | Cl | F | CN | A.2 |
| 411. | Br | F | CN | A.2 |
| 412. | I | F | CN | A.2 |
| 413. | CN | F | CN | A.2 |
| 414. | CH₃ | F | CN | A.2 |
| 415. | CF₃ | F | CN | A.2 |
| 416. | OCH₃ | F | CN | A.2 |
| 417. | OCF₃ | F | CN | A.2 |
| 418. | CH₂CH₃ | F | CN | A.2 |
| 419. | H | Cl | CN | A.2 |
| 420. | F | Cl | CN | A.2 |
| 421. | Cl | Cl | CN | A.2 |
| 422. | Br | Cl | CN | A.2 |
| 423. | I | Cl | CN | A.2 |
| 424. | CN | Cl | CN | A.2 |
| 425. | CH₃ | Cl | CN | A.2 |
| 426. | CF₃ | Cl | CN | A.2 |
| 427. | OCH₃ | Cl | CN | A.2 |
| 428. | OCF₃ | Cl | CN | A.2 |
| 429. | CH₂CH₃ | Cl | CN | A.2 |
| 430. | H | H | CH₃ | A.2 |
| 431. | F | H | CH₃ | A.2 |
| 432. | Cl | H | CH₃ | A.2 |
| 433. | Br | H | CH₃ | A.2 |
| 434. | I | H | CH₃ | A.2 |
| 435. | CN | H | CH₃ | A.2 |
| 436. | CH₃ | H | CH₃ | A.2 |
| 437. | CF₃ | H | CH₃ | A.2 |
| 438. | OCH₃ | H | CH₃ | A.2 |
| 439. | OCF₃ | H | CH₃ | A.2 |
| 440. | CH₂CH₃ | H | CH₃ | A.2 |
| 441. | H | F | CH₃ | A.2 |
| 442. | F | F | CH₃ | A.2 |
| 443. | Cl | F | CH₃ | A.2 |
| 444. | Br | F | CH₃ | A.2 |
| 445. | I | F | CH₃ | A.2 |
| 446. | CN | F | CH₃ | A.2 |
| 447. | CH₃ | F | CH₃ | A.2 |
| 448. | CF₃ | F | CH₃ | A.2 |
| 449. | OCH₃ | F | CH₃ | A.2 |
| 450. | OCF₃ | F | CH₃ | A.2 |
| 451. | CH₂CH₃ | F | CH₃ | A.2 |
| 452. | H | Cl | CH₃ | A.2 |
| 453. | F | Cl | CH₃ | A.2 |
| 454. | Cl | Cl | CH₃ | A.2 |
| 455. | Br | Cl | CH₃ | A.2 |
| 456. | I | Cl | CH₃ | A.2 |
| 457. | CN | Cl | CH₃ | A.2 |
| 458. | CH₃ | Cl | CH₃ | A.2 |
| 459. | CF₃ | Cl | CH₃ | A.2 |
| 460. | OCH₃ | Cl | CH₃ | A.2 |
| 461. | OCF₃ | Cl | CH₃ | A.2 |
| 462. | CH₂CH₃ | Cl | CH₃ | A.2 |
| 463. | H | H | H | A.3 |
| 464. | F | H | H | A.3 |
| 465. | Cl | H | H | A.3 |
| 466. | Br | H | H | A.3 |
| 467. | I | H | H | A.3 |
| 468. | CN | H | H | A.3 |
| 469. | CH₃ | H | H | A.3 |
| 470. | CF₃ | H | H | A.3 |
| 471. | OCH₃ | H | H | A.3 |
| 472. | OCF₃ | H | H | A.3 |
| 473. | CH₂CH₃ | H | H | A.3 |
| 474. | H | F | H | A.3 |
| 475. | F | F | H | A.3 |
| 476. | Cl | F | H | A.3 |
| 477. | Br | F | H | A.3 |
| 478. | I | F | H | A.3 |
| 479. | CN | F | H | A.3 |
| 480. | CH₃ | F | H | A.3 |
| 481. | CF₃ | F | H | A.3 |
| 482. | OCH₃ | F | H | A.3 |
| 483. | OCF₃ | F | H | A.3 |
| 484. | CH₂CH₃ | F | H | A.3 |
| 485. | H | Cl | H | A.3 |
| 486. | F | Cl | H | A.3 |
| 487. | Cl | Cl | H | A.3 |
| 488. | Br | Cl | H | A.3 |
| 489. | I | Cl | H | A.3 |
| 490. | CN | Cl | H | A.3 |
| 491. | CH₃ | Cl | H | A.3 |
| 492. | CF₃ | Cl | H | A.3 |
| 493. | OCH₃ | Cl | H | A.3 |
| 494. | OCF₃ | Cl | H | A.3 |
| 495. | CH₂CH₃ | Cl | H | A.3 |
| 496. | H | H | F | A.3 |
| 497. | F | H | F | A.3 |
| 498. | Cl | H | F | A.3 |
| 499. | Br | H | F | A.3 |
| 500. | I | H | F | A.3 |
| 501. | CN | H | F | A.3 |
| 502. | CH₃ | H | F | A.3 |
| 503. | CF₃ | H | F | A.3 |
| 504. | OCH₃ | H | F | A.3 |
| 505. | OCF₃ | H | F | A.3 |
| 506. | CH₂CH₃ | H | F | A.3 |
| 507. | H | F | F | A.3 |
| 508. | F | F | F | A.3 |
| 509. | Cl | F | F | A.3 |
| 510. | Br | F | F | A.3 |
| 511. | I | F | F | A.3 |
| 512. | CN | F | F | A.3 |
| 513. | CH₃ | F | F | A.3 |
| 514. | CF₃ | F | F | A.3 |
| 515. | OCH₃ | F | F | A.3 |
| 516. | OCF₃ | F | F | A.3 |
| 517. | CH₂CH₃ | F | F | A.3 |
| 518. | H | Cl | F | A.3 |
| 519. | F | Cl | F | A.3 |
| 520. | Cl | Cl | F | A.3 |
| 521. | Br | Cl | F | A.3 |
| 522. | I | Cl | F | A.3 |
| 523. | CN | Cl | F | A.3 |
| 524. | CH₃ | Cl | F | A.3 |
| 525. | CF₃ | Cl | F | A.3 |
| 526. | OCH₃ | Cl | F | A.3 |
| 527. | OCF₃ | Cl | F | A.3 |
| 528. | CH₂CH₃ | Cl | F | A.3 |
| 529. | H | H | Cl | A.3 |
| 530. | F | H | Cl | A.3 |
| 531. | Cl | H | Cl | A.3 |
| 532. | Br | H | Cl | A.3 |
| 533. | I | H | Cl | A.3 |
| 534. | CN | H | Cl | A.3 |
| 535. | CH₃ | H | Cl | A.3 |
| 536. | CF₃ | H | Cl | A.3 |
| 537. | OCH₃ | H | Cl | A.3 |
| 538. | OCF₃ | H | Cl | A.3 |
| 539. | CH₂CH₃ | H | Cl | A.3 |
| 540. | H | F | Cl | A.3 |
| 541. | F | F | Cl | A.3 |
| 542. | Cl | F | Cl | A.3 |
| 543. | Br | F | Cl | A.3 |
| 544. | I | F | Cl | A.3 |
| 545. | CN | F | Cl | A.3 |
| 546. | CH₃ | F | Cl | A.3 |
| 547. | CF₃ | F | Cl | A.3 |
| 548. | OCH₃ | F | Cl | A.3 |
| 549. | OCF₃ | F | Cl | A.3 |
| 550. | CH₂CH₃ | F | Cl | A.3 |
| 551. | H | Cl | Cl | A.3 |
| 552. | F | Cl | Cl | A.3 |
| 553. | Cl | Cl | Cl | A.3 |
| 554. | Br | Cl | Cl | A.3 |
| 555. | I | Cl | Cl | A.3 |
| 556. | CN | Cl | Cl | A.3 |
| 557. | CH₃ | Cl | Cl | A.3 |
| 558. | CF₃ | Cl | Cl | A.3 |
| 559. | OCH₃ | Cl | Cl | A.3 |
| 560. | OCF₃ | Cl | Cl | A.3 |
| 561. | CH₂CH₃ | Cl | Cl | A.3 |
| 562. | H | H | Br | A.3 |
| 563. | F | H | Br | A.3 |
| 564. | Cl | H | Br | A.3 |
| 565. | Br | H | Br | A.3 |
| 566. | I | H | Br | A.3 |
| 567. | CN | H | Br | A.3 |
| 568. | CH₃ | H | Br | A.3 |
| 569. | CF₃ | H | Br | A.3 |
| 570. | OCH₃ | H | Br | A.3 |
| 571. | OCF₃ | H | Br | A.3 |
| 572. | CH₂CH₃ | H | Br | A.3 |
| 573. | H | F | Br | A.3 |
| 574. | F | F | Br | A.3 |
| 575. | Cl | F | Br | A.3 |
| 576. | Br | F | Br | A.3 |
| 577. | I | F | Br | A.3 |
| 578. | CN | F | Br | A.3 |
| 579. | CH₃ | F | Br | A.3 |
| 580. | CF₃ | F | Br | A.3 |
| 581. | OCH₃ | F | Br | A.3 |
| 582. | OCF₃ | F | Br | A.3 |
| 583. | CH₂CH₃ | F | Br | A.3 |
| 584. | H | Cl | Br | A.3 |
| 585. | F | Cl | Br | A.3 |
| 586. | Cl | Cl | Br | A.3 |
| 587. | Br | Cl | Br | A.3 |
| 588. | I | Cl | Br | A.3 |
| 589. | CN | Cl | Br | A.3 |
| 590. | CH₃ | Cl | Br | A.3 |
| 591. | CF₃ | Cl | Br | A.3 |
| 592. | OCH₃ | Cl | Br | A.3 |
| 593. | OCF₃ | Cl | Br | A.3 |
| 594. | CH₂CH₃ | Cl | Br | A.3 |
| 595. | H | H | I | A.3 |
| 596. | F | H | I | A.3 |
| 597. | Cl | H | I | A.3 |
| 598. | Br | H | I | A.3 |
| 599. | I | H | I | A.3 |
| 600. | CN | H | I | A.3 |
| 601. | CH₃ | H | I | A.3 |
| 602. | CF₃ | H | I | A.3 |
| 603. | OCH₃ | H | I | A.3 |
| 604. | OCF₃ | H | I | A.3 |
| 605. | CH₂CH₃ | H | I | A.3 |
| 606. | H | F | I | A.3 |
| 607. | F | F | I | A.3 |
| 608. | Cl | F | I | A.3 |
| 609. | Br | F | I | A.3 |
| 610. | I | F | I | A.3 |
| 611. | CN | F | I | A.3 |
| 612. | CH₃ | F | I | A.3 |
| 613. | CF₃ | F | I | A.3 |
| 614. | OCH₃ | F | I | A.3 |
| 615. | OCF₃ | F | I | A.3 |
| 616. | CH₂CH₃ | F | I | A.3 |
| 617. | H | Cl | I | A.3 |
| 618. | F | Cl | I | A.3 |
| 619. | Cl | Cl | I | A.3 |
| 620. | Br | Cl | I | A.3 |
| 621. | I | Cl | I | A.3 |
| 622. | CN | Cl | I | A.3 |
| 623. | CH₃ | Cl | I | A.3 |
| 624. | CF₃ | Cl | I | A.3 |
| 625. | OCH₃ | Cl | I | A.3 |
| 626. | OCF₃ | Cl | I | A.3 |
| 627. | CH₂CH₃ | Cl | I | A.3 |
| 628. | H | H | CN | A.3 |
| 629. | F | H | CN | A.3 |
| 630. | CI | H | CN | A.3 |
| 631. | Br | H | CN | A.3 |
| 632. | I | H | CN | A.3 |
| 633. | CN | H | CN | A.3 |
| 634. | CH₃ | H | CN | A.3 |
| 635. | CF₃ | H | CN | A.3 |
| 636. | OCH₃ | H | CN | A.3 |
| 637. | OCF₃ | H | CN | A.3 |
| 638. | CH₂CH₃ | H | CN | A.3 |
| 639. | H | F | CN | A.3 |
| 640. | F | F | CN | A.3 |
| 641. | Cl | F | CN | A.3 |
| 642. | Br | F | CN | A.3 |
| 643. | I | F | CN | A.3 |
| 644. | CN | F | CN | A.3 |
| 645. | CH₃ | F | CN | A.3 |
| 646. | CF₃ | F | CN | A.3 |
| 647. | OCH₃ | F | CN | A.3 |
| 648. | OCF₃ | F | CN | A.3 |
| 649. | CH₂CH₃ | F | CN | A.3 |
| 650. | H | Cl | CN | A.3 |
| 651. | F | Cl | CN | A.3 |
| 652. | Cl | Cl | CN | A.3 |
| 653. | Br | Cl | CN | A.3 |
| 654. | I | Cl | CN | A.3 |
| 655. | CN | Cl | CN | A.3 |
| 656. | CH₃ | Cl | CN | A.3 |
| 657. | CF₃ | Cl | CN | A.3 |
| 658. | OCH₃ | Cl | CN | A.3 |
| 659. | OCF₃ | Cl | CN | A.3 |
| 660. | CH₂CH₃ | Cl | CN | A.3 |
| 661. | H | H | CH₃ | A.3 |
| 662. | F | H | CH₃ | A.3 |
| 663. | Cl | H | CH₃ | A.3 |
| 664. | Br | H | CH₃ | A.3 |
| 665. | I | H | CH₃ | A.3 |
| 666. | CN | H | CH₃ | A.3 |
| 667. | CH₃ | H | CH₃ | A.3 |
| 668. | CF₃ | H | CH₃ | A.3 |
| 669. | OCH₃ | H | CH₃ | A.3 |
| 670. | OCF₃ | H | CH₃ | A.3 |
| 671. | CH₂CH₃ | H | CH₃ | A.3 |
| 672. | H | F | CH₃ | A.3 |
| 673. | F | F | CH₃ | A.3 |
| 674. | Cl | F | CH₃ | A.3 |
| 675. | Br | F | CH₃ | A.3 |
| 676. | I | F | CH₃ | A.3 |
| 677. | CN | F | CH₃ | A.3 |
| 678. | CH₃ | F | CH₃ | A.3 |
| 679. | CF₃ | F | CH₃ | A.3 |
| 680. | OCH₃ | F | CH₃ | A.3 |
| 681. | OCF₃ | F | CH₃ | A.3 |
| 682. | CH₂CH₃ | F | CH₃ | A.3 |
| 683. | H | Cl | CH₃ | A.3 |
| 684. | F | Cl | CH₃ | A.3 |
| 685. | CI | Cl | CH₃ | A.3 |
| 686. | Br | Cl | CH₃ | A.3 |
| 687. | I | Cl | CH₃ | A.3 |
| 688. | CN | Cl | CH₃ | A.3 |
| 689. | CH₃ | Cl | CH₃ | A.3 |
| 690. | CF₃ | Cl | CH₃ | A.3 |
| 691. | OCH₃ | Cl | CH₃ | A.3 |
| 692. | OCF₃ | Cl | CH₃ | A.3 |
| 693. | CH₂CH₃ | Cl | CH₃ | A.3 |
| 694. | H | H | H | A.4 |
| 695. | F | H | H | A.4 |
| 696. | Cl | H | H | A.4 |
| 697. | Br | H | H | A.4 |
| 698. | I | H | H | A.4 |
| 699. | CN | H | H | A.4 |
| 700. | CH₃ | H | H | A.4 |
| 701. | CF₃ | H | H | A.4 |
| 702. | OCH₃ | H | H | A.4 |
| 703. | OCF₃ | H | H | A.4 |
| 704. | CH₂CH₃ | H | H | A.4 |
| 705. | H | F | H | A.4 |
| 706. | F | F | H | A.4 |
| 707. | Cl | F | H | A.4 |
| 708. | Br | F | H | A.4 |
| 709. | I | F | H | A.4 |
| 710. | CN | F | H | A.4 |
| 711. | CH₃ | F | H | A.4 |
| 712. | CF₃ | F | H | A.4 |
| 713. | OCH₃ | F | H | A.4 |
| 714. | OCF₃ | F | H | A.4 |
| 715. | CH₂CH₃ | F | H | A.4 |
| 716. | H | Cl | H | A.4 |
| 717. | F | Cl | H | A.4 |
| 718. | Cl | Cl | H | A.4 |
| 719. | Br | Cl | H | A.4 |
| 720. | I | Cl | H | A.4 |
| 721. | CN | Cl | H | A.4 |
| 722. | CH₃ | Cl | H | A.4 |
| 723. | CF₃ | Cl | H | A.4 |
| 724. | OCH₃ | Cl | H | A.4 |
| 725. | OCF₃ | Cl | H | A.4 |
| 726. | CH₂CH₃ | Cl | H | A.4 |
| 727. | H | H | F | A.4 |
| 728. | F | H | F | A.4 |
| 729. | Cl | H | F | A.4 |
| 730. | Br | H | F | A.4 |
| 731. | I | H | F | A.4 |
| 732. | CN | H | F | A.4 |
| 733. | CH₃ | H | F | A.4 |
| 734. | CF₃ | H | F | A.4 |
| 735. | OCH₃ | H | F | A.4 |
| 736. | OCF₃ | H | F | A.4 |
| 737. | CH₂CH₃ | H | F | A.4 |
| 738. | H | F | F | A.4 |
| 739. | F | F | F | A.4 |
| 740. | Cl | F | F | A.4 |
| 741. | Br | F | F | A.4 |
| 742. | I | F | F | A.4 |
| 743. | CN | F | F | A.4 |
| 744. | CH₃ | F | F | A.4 |
| 745. | CF₃ | F | F | A.4 |
| 746. | OCH₃ | F | F | A.4 |
| 747. | OCF₃ | Cl | F | A.4 |
| 748. | CH₂CH₃ | Cl | F | A.4 |
| 749. | H | Cl | F | A.4 |
| 750. | F | Cl | F | A.4 |
| 751. | Cl | Cl | F | A.4 |
| 752. | Br | Cl | F | A.4 |
| 753. | I | Cl | F | A.4 |
| 754. | CN | Cl | F | A.4 |
| 755. | CH₃ | Cl | F | A.4 |
| 756. | CF₃ | Cl | F | A.4 |
| 757. | OCH₃ | Cl | F | A.4 |
| 758. | OCF₃ | Cl | F | A.4 |
| 759. | CH₂CH₃ | Cl | F | A.4 |
| 760. | H | H | Cl | A.4 |
| 761. | F | H | Cl | A.4 |
| 762. | Cl | H | Cl | A.4 |
| 763. | Br | H | Cl | A.4 |
| 764. | I | H | Cl | A.4 |
| 765. | CN | H | Cl | A.4 |
| 766. | CH₃ | H | Cl | A.4 |
| 767. | CF₃ | H | Cl | A.4 |
| 768. | OCH₃ | H | Cl | A.4 |
| 769. | OCF₃ | H | Cl | A.4 |
| 770. | CH₂CH₃ | H | Cl | A.4 |
| 771. | H | F | Cl | A.4 |
| 772. | F | F | Cl | A.4 |
| 773. | Cl | F | Cl | A.4 |
| 774. | Br | F | Cl | A.4 |
| 775. | I | F | Cl | A.4 |
| 776. | CN | F | Cl | A.4 |
| 777. | CH₃ | F | Cl | A.4 |
| 778. | CF₃ | F | Cl | A.4 |
| 779. | OCH₃ | F | Cl | A.4 |
| 780. | OCF₃ | F | Cl | A.4 |
| 781. | CH₂CH₃ | F | Cl | A.4 |
| 782. | H | Cl | Cl | A.4 |
| 783. | F | Cl | Cl | A.4 |
| 784. | Cl | Cl | Cl | A.4 |
| 785. | Br | Cl | Cl | A.4 |
| 786. | I | Cl | Cl | A.4 |
| 787. | CN | Cl | Cl | A.4 |
| 788. | CH₃ | Cl | Cl | A.4 |
| 789. | CF₃ | Cl | Cl | A.4 |
| 790. | OCH₃ | Cl | Cl | A.4 |
| 791. | OCF₃ | Cl | Cl | A.4 |
| 792. | CH₂CH₃ | Cl | Cl | A.4 |
| 793. | H | H | Br | A.4 |
| 794. | F | H | Br | A.4 |
| 795. | Cl | H | Br | A.4 |
| 796. | Br | H | Br | A.4 |
| 797. | I | H | Br | A.4 |
| 798. | CN | H | Br | A.4 |
| 799. | CH₃ | H | Br | A.4 |
| 800. | CF₃ | H | Br | A.4 |
| 801. | OCH₃ | H | Br | A.4 |
| 802. | OCF₃ | H | Br | A.4 |
| 803. | CH₂CH₃ | H | Br | A.4 |
| 804. | H | F | Br | A.4 |
| 805. | F | F | Br | A.4 |
| 806. | Cl | F | Br | A.4 |
| 807. | Br | F | Br | A.4 |
| 808. | I | F | Br | A.4 |
| 809. | CN | F | Br | A.4 |
| 810. | CH₃ | F | Br | A.4 |
| 811. | CF₃ | F | Br | A.4 |
| 812. | OCH₃ | F | Br | A.4 |
| 813. | OCF₃ | F | Br | A.4 |
| 814. | CH₂CH₃ | F | Br | A.4 |
| 815. | H | Cl | Br | A.4 |
| 816. | F | Cl | Br | A.4 |
| 817. | Cl | Cl | Br | A.4 |
| 818. | Br | Cl | Br | A.4 |
| 819. | I | Cl | Br | A.4 |
| 820. | CN | Cl | Br | A.4 |
| 821. | CH₃ | Cl | Br | A.4 |
| 822. | CF₃ | Cl | Br | A.4 |
| 823. | OCH₃ | Cl | Br | A.4 |
| 824. | OCF₃ | Cl | Br | A.4 |
| 825. | CH₂CH₃ | Cl | Br | A.4 |
| 826. | H | H | I | A.4 |
| 827. | F | H | I | A.4 |
| 828. | Cl | H | I | A.4 |
| 829. | Br | H | I | A.4 |
| 830. | I | H | I | A.4 |
| 831. | CN | H | I | A.4 |
| 832. | CH₃ | H | I | A.4 |
| 833. | CF₃ | H | I | A.4 |
| 834. | OCH₃ | H | I | A.4 |
| 835. | OCF₃ | H | I | A.4 |
| 836. | CH₂CH₃ | H | I | A.4 |
| 837. | H | F | I | A.4 |
| 838. | F | F | I | A.4 |
| 839. | Cl | F | I | A.4 |
| 840. | Br | F | I | A.4 |
| 841. | I | F | I | A.4 |
| 842. | CN | F | I | A.4 |
| 843. | CH₃ | F | I | A.4 |
| 844. | CF₃ | F | I | A.4 |
| 845. | OCH₃ | F | I | A.4 |
| 846. | OCF₃ | F | I | A.4 |
| 847. | CH₂CH₃ | F | I | A.4 |
| 848. | H | Cl | I | A.4 |
| 849. | F | Cl | I | A.4 |
| 850. | Cl | Cl | I | A.4 |
| 851. | Br | Cl | I | A.4 |
| 852. | I | Cl | I | A.4 |
| 853. | CN | Cl | I | A.4 |
| 854. | CH₃ | Cl | I | A.4 |
| 855. | CF₃ | Cl | I | A.4 |
| 856. | OCH₃ | Cl | I | A.4 |
| 857. | OCF₃ | Cl | I | A.4 |
| 858. | CH₂CH₃ | Cl | I | A.4 |
| 859. | H | H | CN | A.4 |
| 860. | F | H | CN | A.4 |
| 861. | Cl | H | CN | A.4 |
| 862. | Br | H | CN | A.4 |
| 863. | I | H | CN | A.4 |
| 864. | CN | H | CN | A.4 |
| 865. | CH₃ | H | CN | A.4 |
| 866. | CF₃ | H | CN | A.4 |
| 867. | OCH₃ | H | CN | A.4 |
| 868. | OCF₃ | H | CN | A.4 |
| 869. | CH₂CH₃ | H | CN | A.4 |
| 870. | H | F | CN | A.4 |
| 871. | F | F | CN | A.4 |
| 872. | Cl | F | CN | A.4 |
| 873. | Br | F | CN | A.4 |
| 874. | I | F | CN | A.4 |
| 875. | CN | F | CN | A.4 |
| 876. | CH₃ | F | CN | A.4 |
| 877. | CF₃ | F | CN | A.4 |
| 878. | OCH₃ | F | CN | A.4 |
| 879. | OCF₃ | F | CN | A.4 |
| 880. | CH₂CH₃ | F | CN | A.4 |
| 881. | H | Cl | CN | A.4 |
| 882. | F | Cl | CN | A.4 |
| 883. | Cl | Cl | CN | A.4 |
| 884. | Br | Cl | CN | A.4 |
| 885. | I | Cl | CN | A.4 |
| 886. | CN | Cl | CN | A.4 |
| 887. | CH₃ | Cl | CN | A.4 |
| 888. | CF₃ | Cl | CN | A.4 |
| 889. | OCH₃ | Cl | CN | A.4 |
| 890. | OCF₃ | Cl | CN | A.4 |
| 891. | CH₂CH₃ | Cl | CN | A.4 |
| 892. | H | H | CH₃ | A.4 |
| 893. | F | H | CH₃ | A.4 |
| 894. | Cl | H | CH₃ | A.4 |
| 895. | Br | H | CH₃ | A.4 |
| 896. | I | H | CH₃ | A.4 |
| 897. | CN | H | CH₃ | A.4 |
| 898. | CH₃ | H | CH₃ | A.4 |
| 899. | CF₃ | H | CH₃ | A.4 |
| 900. | OCH₃ | H | CH₃ | A.4 |
| 901. | OCF₃ | H | CH₃ | A.4 |
| 902. | CH₂CH₃ | H | CH₃ | A.4 |
| 903. | H | F | CH₃ | A.4 |
| 904. | F | F | CH₃ | A.4 |
| 905. | Cl | F | CH₃ | A.4 |
| 906. | Br | F | CH₃ | A.4 |
| 907. | I | F | CH₃ | A.4 |
| 908. | CN | F | CH₃ | A.4 |
| 909. | CH₃ | F | CH₃ | A.4 |
| 910. | CF₃ | F | CH₃ | A.4 |
| 911. | OCH₃ | F | CH₃ | A.4 |
| 912. | OCF₃ | F | CH₃ | A.4 |
| 913. | CH₂CH₃ | F | CH₃ | A.4 |
| 914. | H | Cl | CH₃ | A.4 |
| 915. | F | Cl | CH₃ | A.4 |
| 916. | Cl | Cl | CH₃ | A.4 |
| 917. | Br | Cl | CH₃ | A.4 |
| 918. | I | Cl | CH₃ | A.4 |
| 919. | CN | Cl | CH₃ | A.4 |
| 920. | CH₃ | Cl | CH₃ | A.4 |
| 921. | CF₃ | Cl | CH₃ | A.4 |
| 922. | OCH₃ | Cl | CH₃ | A.4 |
| 923. | OCF₃ | Cl | CH₃ | A.4 |
| 924. | CH₂CH₃ | Cl | CH₃ | A.4 |
| 925. | H | H | H | A.5 |
| 926. | F | H | H | A.5 |
| 927. | Cl | H | H | A.5 |
| 928. | Br | H | H | A.5 |
| 929. | I | H | H | A.5 |
| 930. | CN | H | H | A.5 |
| 931. | CH₃ | H | H | A.5 |
| 932. | CF₃ | H | H | A.5 |
| 933. | OCH₃ | H | H | A.5 |
| 934. | OCF₃ | H | H | A.5 |
| 935. | CH₂CH₃ | H | H | A.5 |
| 936. | H | F | H | A.5 |
| 937. | F | F | H | A.5 |
| 938. | Cl | F | H | A.5 |
| 939. | Br | F | H | A.5 |
| 940. | I | F | H | A.5 |
| 941. | CN | F | H | A.5 |
| 942. | CH₃ | F | H | A.5 |
| 943. | CF₃ | F | H | A.5 |
| 944. | OCH₃ | F | H | A.5 |
| 945. | OCF₃ | F | H | A.5 |
| 946. | CH₂CH₃ | F | H | A.5 |
| 947. | H | Cl | H | A.5 |
| 948. | F | Cl | H | A.5 |
| 949. | Cl | Cl | H | A.5 |
| 950. | Br | Cl | H | A.5 |
| 951. | I | Cl | H | A.5 |
| 952. | CN | Cl | H | A.5 |
| 953. | CH₃ | Cl | H | A.5 |
| 954. | CF₃ | Cl | H | A.5 |
| 955. | OCH₃ | Cl | H | A.5 |
| 956. | OCF₃ | Cl | H | A.5 |
| 957. | CH₂CH₃ | Cl | H | A.5 |
| 958. | H | H | F | A.5 |
| 959. | F | H | F | A.5 |
| 960. | Cl | H | F | A.5 |
| 961. | Br | H | F | A.5 |
| 962. | I | H | F | A.5 |
| 963. | CN | H | F | A.5 |
| 964. | CH₃ | H | F | A.5 |
| 965. | CF₃ | H | F | A.5 |
| 966. | OCH₃ | H | F | A.5 |
| 967. | OCF₃ | H | F | A.5 |
| 968. | CH₂CH₃ | H | F | A.5 |
| 969. | H | F | F | A.5 |
| 970. | F | F | F | A.5 |
| 971. | Cl | F | F | A.5 |
| 972. | Br | F | F | A.5 |
| 973. | I | F | F | A.5 |
| 974. | CN | F | F | A.5 |
| 975. | CH₃ | F | F | A.5 |
| 976. | CF₃ | F | F | A.5 |
| 977. | OCH₃ | F | F | A.5 |
| 978. | OCF₃ | Cl | F | A.5 |
| 979. | CH₂CH₃ | Cl | F | A.5 |
| 980. | H | Cl | F | A.5 |
| 981. | F | Cl | F | A.5 |
| 982. | Cl | Cl | F | A.5 |
| 983. | Br | Cl | F | A.5 |
| 984. | I | Cl | F | A.5 |
| 985. | CN | Cl | F | A.5 |
| 986. | CH₃ | Cl | F | A.5 |
| 987. | CF₃ | Cl | F | A.5 |
| 988. | OCH₃ | Cl | F | A.5 |
| 989. | OCF₃ | Cl | F | A.5 |
| 990. | CH₂CH₃ | Cl | F | A.5 |
| 991. | H | H | Cl | A.5 |
| 992. | F | H | Cl | A.5 |
| 993. | Cl | H | Cl | A.5 |
| 994. | Br | H | Cl | A.5 |
| 995. | I | H | Cl | A.5 |
| 996. | CN | H | Cl | A.5 |
| 997. | CH₃ | H | Cl | A.5 |
| 998. | CF₃ | H | Cl | A.5 |
| 999. | OCH₃ | H | Cl | A.5 |
| 1000. | OCF₃ | H | Cl | A.5 |
| 1001. | CH₂CH₃ | H | Cl | A.5 |
| 1002. | H | F | Cl | A.5 |
| 1003. | F | F | Cl | A.5 |
| 1004. | Cl | F | Cl | A.5 |
| 1005. | Br | F | Cl | A.5 |
| 1006. | I | F | Cl | A.5 |
| 1007. | CN | F | Cl | A.5 |
| 1008. | CH₃ | F | Cl | A.5 |
| 1009. | CF₃ | F | Cl | A.5 |
| 1010. | OCH₃ | F | Cl | A.5 |
| 1011. | OCF₃ | F | Cl | A.5 |
| 1012. | CH₂CH₃ | F | Cl | A.5 |
| 1013. | H | Cl | Cl | A.5 |
| 1014. | F | Cl | Cl | A.5 |
| 1015. | Cl | Cl | Cl | A.5 |
| 1016. | Br | Cl | Cl | A.5 |
| 1017. | I | Cl | Cl | A.5 |
| 1018. | CN | Cl | Cl | A.5 |
| 1019. | CH₃ | Cl | Cl | A.5 |
| 1020. | CF₃ | Cl | Cl | A.5 |
| 1021. | OCH₃ | Cl | Cl | A.5 |
| 1022. | OCF₃ | Cl | Cl | A.5 |
| 1023. | CH₂CH₃ | Cl | Cl | A.5 |
| 1024. | H | H | Br | A.5 |
| 1025. | F | H | Br | A.5 |
| 1026. | Cl | H | Br | A.5 |
| 1027. | Br | H | Br | A.5 |
| 1028. | I | H | Br | A.5 |
| 1029. | CN | H | Br | A.5 |
| 1030. | CH₃ | H | Br | A.5 |
| 1031. | CF₃ | H | Br | A.5 |
| 1032. | OCH₃ | H | Br | A.5 |
| 1033. | OCF₃ | H | Br | A.5 |
| 1034. | CH₂CH₃ | H | Br | A.5 |
| 1035. | H | F | Br | A.5 |
| 1036. | F | F | Br | A.5 |
| 1037. | Cl | F | Br | A.5 |
| 1038. | Br | F | Br | A.5 |
| 1039. | I | F | Br | A.5 |
| 1040. | CN | F | Br | A.5 |
| 1041. | CH₃ | F | Br | A.5 |
| 1042. | CF₃ | F | Br | A.5 |
| 1043. | OCH₃ | F | Br | A.5 |
| 1044. | OCF₃ | F | Br | A.5 |
| 1045. | CH₂CH₃ | F | Br | A.5 |
| 1046. | H | Cl | Br | A.5 |
| 1047. | F | Cl | Br | A.5 |
| 1048. | Cl | Cl | Br | A.5 |
| 1049. | Br | Cl | Br | A.5 |
| 1050. | I | Cl | Br | A.5 |
| 1051. | CN | Cl | Br | A.5 |
| 1052. | CH₃ | Cl | Br | A.5 |
| 1053. | CF₃ | Cl | Br | A.5 |
| 1054. | OCH₃ | Cl | Br | A.5 |
| 1055. | OCF₃ | Cl | Br | A.5 |
| 1056. | CH₂CH₃ | Cl | Br | A.5 |
| 1057. | H | H | I | A.5 |
| 1058. | F | H | I | A.5 |
| 1059. | Cl | H | I | A.5 |
| 1060. | Br | H | I | A.5 |
| 1061. | I | H | I | A.5 |
| 1062. | CN | H | I | A.5 |
| 1063. | CH₃ | H | I | A.5 |
| 1064. | CF₃ | H | I | A.5 |
| 1065. | OCH₃ | H | I | A.5 |
| 1066. | OCF₃ | H | I | A.5 |
| 1067. | CH₂CH₃ | H | I | A.5 |
| 1068. | H | F | I | A.5 |
| 1069. | F | F | I | A.5 |
| 1070. | Cl | F | I | A.5 |
| 1071. | Br | F | I | A.5 |
| 1072. | I | F | I | A.5 |
| 1073. | CN | F | I | A.5 |
| 1074. | CH₃ | F | I | A.5 |
| 1075. | CF₃ | F | I | A.5 |
| 1076. | OCH₃ | F | I | A.5 |
| 1077. | OCF₃ | F | I | A.5 |
| 1078. | CH₂CH₃ | F | I | A.5 |
| 1079. | H | Cl | I | A.5 |
| 1080. | F | Cl | I | A.5 |
| 1081. | Cl | Cl | I | A.5 |
| 1082. | Br | Cl | I | A.5 |
| 1083. | I | Cl | I | A.5 |
| 1084. | CN | Cl | I | A.5 |
| 1085. | CH₃ | Cl | I | A.5 |
| 1086. | CF₃ | Cl | I | A.5 |
| 1087. | OCH₃ | Cl | I | A.5 |
| 1088. | OCF₃ | Cl | I | A.5 |
| 1089. | CH₂CH₃ | Cl | I | A.5 |
| 1090. | H | H | CN | A.5 |
| 1091. | F | H | CN | A.5 |
| 1092. | CI | H | CN | A.5 |
| 1093. | Br | H | CN | A.5 |
| 1094. | I | H | CN | A.5 |
| 1095. | CN | H | CN | A.5 |
| 1096. | CH₃ | H | CN | A.5 |
| 1097. | CF₃ | H | CN | A.5 |
| 1098. | OCH₃ | H | CN | A.5 |
| 1099. | OCF₃ | H | CN | A.5 |
| 1100. | CH₂CH₃ | H | CN | A.5 |
| 1101. | H | F | CN | A.5 |
| 1102. | F | F | CN | A.5 |
| 1103. | Cl | F | CN | A.5 |
| 1104. | Br | F | CN | A.5 |
| 1105. | I | F | CN | A.5 |
| 1106. | CN | F | CN | A.5 |
| 1107. | CH₃ | F | CN | A.5 |
| 1108. | CF₃ | F | CN | A.5 |
| 1109. | OCH₃ | F | CN | A.5 |
| 1110. | OCF₃ | F | CN | A.5 |
| 1111. | CH₂CH₃ | F | CN | A.5 |
| 1112. | H | Cl | CN | A.5 |
| 1113. | F | Cl | CN | A.5 |
| 1114. | Cl | Cl | CN | A.5 |
| 1115. | Br | Cl | CN | A.5 |
| 1116. | I | Cl | CN | A.5 |
| 1117. | CN | Cl | CN | A.5 |
| 1118. | CH₃ | Cl | CN | A.5 |
| 1119. | CF₃ | Cl | CN | A.5 |
| 1120. | OCH₃ | Cl | CN | A.5 |
| 1121. | OCF₃ | Cl | CN | A.5 |
| 1122. | CH₂CH₃ | Cl | CN | A.5 |
| 1123. | H | H | CH₃ | A.5 |
| 1124. | F | H | CH₃ | A.5 |
| 1125. | Cl | H | CH₃ | A.5 |
| 1126. | Br | H | CH₃ | A.5 |
| 1127. | I | H | CH₃ | A.5 |
| 1128. | CN | H | CH₃ | A.5 |
| 1129. | CH₃ | H | CH₃ | A.5 |
| 1130. | CF₃ | H | CH₃ | A.5 |
| 1131. | OCH₃ | H | CH₃ | A.5 |
| 1132. | OCF₃ | H | CH₃ | A.5 |
| 1133. | CH₂CH₃ | H | CH₃ | A.5 |
| 1134. | H | F | CH₃ | A.5 |
| 1135. | F | F | CH₃ | A.5 |
| 1136. | Cl | F | CH₃ | A.5 |
| 1137. | Br | F | CH₃ | A.5 |
| 1138. | I | F | CH₃ | A.5 |
| 1139. | CN | F | CH₃ | A.5 |
| 1140. | CH₃ | F | CH₃ | A.5 |
| 1141. | CF₃ | F | CH₃ | A.5 |
| 1142. | OCH₃ | F | CH₃ | A.5 |
| 1143. | OCF₃ | F | CH₃ | A.5 |
| 1144. | CH₂CH₃ | F | CH₃ | A.5 |
| 1145. | H | Cl | CH₃ | A.5 |
| 1146. | F | Cl | CH₃ | A.5 |
| 1147. | Cl | Cl | CH₃ | A.5 |
| 1148. | Br | Cl | CH₃ | A.5 |
| 1149. | I | Cl | CH₃ | A.5 |
| 1150. | CN | Cl | CH₃ | A.5 |
| 1151. | CH₃ | Cl | CH₃ | A.5 |
| 1152. | CF₃ | Cl | CH₃ | A.5 |
| 1153. | OCH₃ | Cl | CH₃ | A.5 |
| 1154. | OCF₃ | Cl | CH₃ | A.5 |
| 1155. | CH₂CH₃ | Cl | CH₃ | A.5 |
| 1156. | H | H | H | A.6 |
| 1157. | F | H | H | A.6 |
| 1158. | Cl | H | H | A.6 |
| 1159. | Br | H | H | A.6 |
| 1160. | I | H | H | A.6 |
| 1161. | CN | H | H | A.6 |
| 1162. | CH₃ | H | H | A.6 |
| 1163. | CF₃ | H | H | A.6 |
| 1164. | OCH₃ | H | H | A.6 |
| 1165. | OCF₃ | H | H | A.6 |
| 1166. | CH₂CH₃ | H | H | A.6 |
| 1167. | H | F | H | A.6 |
| 1168. | F | F | H | A.6 |
| 1169. | Cl | F | H | A.6 |
| 1170. | Br | F | H | A.6 |
| 1171. | I | F | H | A.6 |
| 1172. | CN | F | H | A.6 |
| 1173. | CH₃ | F | H | A.6 |
| 1174. | CF₃ | F | H | A.6 |
| 1175. | OCH₃ | F | H | A.6 |
| 1176. | OCF₃ | F | H | A.6 |
| 1177. | CH₂CH₃ | F | H | A.6 |
| 1178. | H | Cl | H | A.6 |
| 1179. | F | Cl | H | A.6 |
| 1180. | Cl | Cl | H | A.6 |
| 1181. | Br | Cl | H | A.6 |
| 1182. | I | Cl | H | A.6 |
| 1183. | CN | Cl | H | A.6 |
| 1184. | CH₃ | Cl | H | A.6 |
| 1185. | CF₃ | Cl | H | A.6 |
| 1186. | OCH₃ | Cl | H | A.6 |
| 1187. | OCF₃ | Cl | H | A.6 |
| 1188. | CH₂CH₃ | Cl | H | A.6 |
| 1189. | H | H | F | A.6 |
| 1190. | F | H | F | A.6 |
| 1191. | Cl | H | F | A.6 |
| 1192. | Br | H | F | A.6 |
| 1193. | I | H | F | A.6 |
| 1194. | CN | H | F | A.6 |
| 1195. | CH₃ | H | F | A.6 |
| 1196. | CF₃ | H | F | A.6 |
| 1197. | OCH₃ | H | F | A.6 |
| 1198. | OCF₃ | H | F | A.6 |
| 1199. | CH₂CH₃ | H | F | A.6 |
| 1200. | H | F | F | A.6 |
| 1201. | F | F | F | A.6 |
| 1202. | Cl | F | F | A.6 |
| 1203. | Br | F | F | A.6 |
| 1204. | I | F | F | A.6 |
| 1205. | CN | F | F | A.6 |
| 1206. | CH₃ | F | F | A.6 |
| 1207. | CF₃ | F | F | A.6 |
| 1208. | OCH₃ | F | F | A.6 |
| 1209. | OCF₃ | F | F | A.6 |
| 1210. | CH₂CH₃ | F | F | A.6 |
| 1211. | H | Cl | F | A.6 |
| 1212. | F | Cl | F | A.6 |
| 1213. | Cl | Cl | F | A.6 |
| 1214. | Br | Cl | F | A.6 |
| 1215. | I | Cl | F | A.6 |
| 1216. | CN | Cl | F | A.6 |
| 1217. | CH₃ | Cl | F | A.6 |
| 1218. | CF₃ | Cl | F | A.6 |
| 1219. | OCH₃ | Cl | F | A.6 |
| 1220. | OCF₃ | Cl | F | A.6 |
| 1221. | CH₂CH₃ | Cl | F | A.6 |
| 1222. | H | H | Cl | A.6 |
| 1223. | F | H | Cl | A.6 |
| 1224. | Cl | H | Cl | A.6 |
| 1225. | Br | H | Cl | A.6 |
| 1226. | I | H | Cl | A.6 |
| 1227. | CN | H | Cl | A.6 |
| 1228. | CH₃ | H | Cl | A.6 |
| 1229. | CF₃ | H | Cl | A.6 |
| 1230. | OCH₃ | H | Cl | A.6 |
| 1231. | OCF₃ | H | Cl | A.6 |
| 1232. | CH₂CH₃ | H | Cl | A.6 |
| 1233. | H | F | Cl | A.6 |
| 1234. | F | F | Cl | A.6 |
| 1235. | Cl | F | Cl | A.6 |
| 1236. | Br | F | Cl | A.6 |
| 1237. | I | F | Cl | A.6 |
| 1238. | CN | F | Cl | A.6 |
| 1239. | CH₃ | F | Cl | A.6 |
| 1240. | CF₃ | F | Cl | A.6 |
| 1241. | OCH₃ | F | Cl | A.6 |
| 1242. | OCF₃ | F | Cl | A.6 |
| 1243. | CH₂CH₃ | F | Cl | A.6 |
| 1244. | H | Cl | Cl | A.6 |
| 1245. | F | Cl | Cl | A.6 |
| 1246. | Cl | Cl | Cl | A.6 |
| 1247. | Br | Cl | Cl | A.6 |
| 1248. | I | Cl | Cl | A.6 |
| 1249. | CN | Cl | Cl | A.6 |
| 1250. | CH₃ | Cl | Cl | A.6 |
| 1251. | CF₃ | Cl | Cl | A.6 |
| 1252. | OCH₃ | Cl | Cl | A.6 |
| 1253. | OCF₃ | Cl | Cl | A.6 |
| 1254. | CH₂CH₃ | Cl | Cl | A.6 |
| 1255. | H | H | Br | A.6 |
| 1256. | F | H | Br | A.6 |
| 1257. | Cl | H | Br | A.6 |
| 1258. | Br | H | Br | A.6 |
| 1259. | I | H | Br | A.6 |
| 1260. | CN | H | Br | A.6 |
| 1261. | CH₃ | H | Br | A.6 |
| 1262. | CF₃ | H | Br | A.6 |
| 1263. | OCH₃ | H | Br | A.6 |
| 1264. | OCF₃ | H | Br | A.6 |
| 1265. | CH₂CH₃ | H | Br | A.6 |
| 1266. | H | F | Br | A.6 |
| 1267. | F | F | Br | A.6 |
| 1268. | Cl | F | Br | A.6 |
| 1269. | Br | F | Br | A.6 |
| 1270. | I | F | Br | A.6 |
| 1271. | CN | F | Br | A.6 |
| 1272. | CH₃ | F | Br | A.6 |
| 1273. | CF₃ | F | Br | A.6 |
| 1274. | OCH₃ | F | Br | A.6 |
| 1275. | OCF₃ | F | Br | A.6 |
| 1276. | CH₂CH₃ | F | Br | A.6 |
| 1277. | H | Cl | Br | A.6 |
| 1278. | F | Cl | Br | A.6 |
| 1279. | Cl | Cl | Br | A.6 |
| 1280. | Br | Cl | Br | A.6 |
| 1281. | I | Cl | Br | A.6 |
| 1282. | CN | Cl | Br | A.6 |
| 1283. | CH₃ | Cl | Br | A.6 |
| 1284. | CF₃ | Cl | Br | A.6 |
| 1285. | OCH₃ | Cl | Br | A.6 |
| 1286. | OCF₃ | Cl | Br | A.6 |
| 1287. | CH₂CH₃ | Cl | Br | A.6 |
| 1288. | H | H | I | A.6 |
| 1289. | F | H | I | A.6 |
| 1290. | Cl | H | I | A.6 |
| 1291. | Br | H | I | A.6 |
| 1292. | I | H | I | A.6 |
| 1293. | CN | H | I | A.6 |
| 1294. | CH₃ | H | I | A.6 |
| 1295. | CF₃ | H | I | A.6 |
| 1296. | OCH₃ | H | I | A.6 |
| 1297. | OCF₃ | H | I | A.6 |
| 1298. | CH₂CH₃ | H | I | A.6 |
| 1299. | H | F | I | A.6 |
| 1300. | F | F | I | A.6 |
| 1301. | Cl | F | I | A.6 |
| 1302. | Br | F | I | A.6 |
| 1303. | I | F | I | A.6 |
| 1304. | CN | F | I | A.6 |
| 1305. | CH₃ | F | I | A.6 |
| 1306. | CF₃ | F | I | A.6 |
| 1307. | OCH₃ | F | I | A.6 |
| 1308. | OCF₃ | F | I | A.6 |
| 1309. | CH₂CH₃ | F | I | A.6 |
| 1310. | H | Cl | I | A.6 |
| 1311. | F | Cl | I | A.6 |
| 1312. | Cl | Cl | I | A.6 |
| 1313. | Br | Cl | I | A.6 |
| 1314. | I | Cl | I | A.6 |
| 1315. | CN | Cl | I | A.6 |
| 1316. | CH₃ | Cl | I | A.6 |
| 1317. | CF₃ | Cl | I | A.6 |
| 1318. | OCH₃ | Cl | I | A.6 |
| 1319. | OCF₃ | Cl | I | A.6 |
| 1320. | CH₂CH₃ | Cl | I | A.6 |
| 1321. | H | H | CN | A.6 |
| 1322. | F | H | CN | A.6 |
| 1323. | Cl | H | CN | A.6 |
| 1324. | Br | H | CN | A.6 |
| 1325. | I | H | CN | A.6 |
| 1326. | CN | H | CN | A.6 |
| 1327. | CH₃ | H | CN | A.6 |
| 1328. | CF₃ | H | CN | A.6 |
| 1329. | OCH₃ | H | CN | A.6 |
| 1330. | OCF₃ | H | CN | A.6 |
| 1331. | CH₂CH₃ | H | CN | A.6 |
| 1332. | H | F | CN | A.6 |
| 1333. | F | F | CN | A.6 |
| 1334. | Cl | F | CN | A.6 |
| 1335. | Br | F | CN | A.6 |
| 1336. | I | F | CN | A.6 |
| 1337. | CN | F | CN | A.6 |
| 1338. | CH₃ | F | CN | A.6 |
| 1339. | CF₃ | F | CN | A.6 |
| 1340. | OCH₃ | F | CN | A.6 |
| 1341. | OCF₃ | F | CN | A.6 |
| 1342. | CH₂CH₃ | F | CN | A.6 |
| 1343. | H | Cl | CN | A.6 |
| 1344. | F | Cl | CN | A.6 |
| 1345. | Cl | Cl | CN | A.6 |
| 1346. | Br | Cl | CN | A.6 |
| 1347. | I | Cl | CN | A.6 |
| 1348. | CN | Cl | CN | A.6 |
| 1349. | CH₃ | Cl | CN | A.6 |
| 1350. | CF₃ | Cl | CN | A.6 |
| 1351. | OCH₃ | Cl | CN | A.6 |
| 1352. | OCF₃ | Cl | CN | A.6 |
| 1353. | CH₂CH₃ | Cl | CN | A.6 |
| 1354. | H | H | CH₃ | A.6 |
| 1355. | F | H | CH₃ | A.6 |
| 1356. | Cl | H | CH₃ | A.6 |
| 1357. | Br | H | CH₃ | A.6 |
| 1358. | I | H | CH₃ | A.6 |
| 1359. | CN | H | CH₃ | A.6 |
| 1360. | CH₃ | H | CH₃ | A.6 |
| 1361. | CF₃ | H | CH₃ | A.6 |
| 1362. | OCH₃ | H | CH₃ | A.6 |
| 1363. | OCF₃ | H | CH₃ | A.6 |
| 1364. | CH₂CH₃ | H | CH₃ | A.6 |
| 1365. | H | F | CH₃ | A.6 |
| 1366. | F | F | CH₃ | A.6 |
| 1367. | Cl | F | CH₃ | A.6 |
| 1368. | Br | F | CH₃ | A.6 |
| 1369. | I | F | CH₃ | A.6 |
| 1370. | CN | F | CH₃ | A.6 |
| 1371. | CH₃ | F | CH₃ | A.6 |
| 1372. | CF₃ | F | CH₃ | A.6 |
| 1373. | OCH₃ | F | CH₃ | A.6 |
| 1374. | OCF₃ | F | CH₃ | A.6 |
| 1375. | CH₂CH₃ | F | CH₃ | A.6 |
| 1376. | H | Cl | CH₃ | A.6 |
| 1377. | F | Cl | CH₃ | A.6 |
| 1378. | Cl | Cl | CH₃ | A.6 |
| 1379. | Br | Cl | CH₃ | A.6 |
| 1380. | I | Cl | CH₃ | A.6 |
| 1381. | CN | Cl | CH₃ | A.6 |
| 1382. | CH₃ | Cl | CH₃ | A.6 |
| 1383. | CF₃ | Cl | CH₃ | A.6 |
| 1384. | OCH₃ | Cl | CH₃ | A.6 |
| 1385. | OCF₃ | Cl | CH₃ | A.6 |
| 1386. | CH₂CH₃ | Cl | CH₃ | A.6 |
| 1387. | H | H | H | A.7 |
| 1388. | F | H | H | A.7 |
| 1389. | Cl | H | H | A.7 |
| 1390. | Br | H | H | A.7 |
| 1391. | I | H | H | A.7 |
| 1392. | CN | H | H | A.7 |
| 1393. | CH₃ | H | H | A.7 |
| 1394. | CF₃ | H | H | A.7 |
| 1395. | OCH₃ | H | H | A.7 |
| 1396. | OCF₃ | H | H | A.7 |
| 1397. | CH₂CH₃ | H | H | A.7 |
| 1398. | H | F | H | A.7 |
| 1399. | F | F | H | A.7 |
| 1400. | Cl | F | H | A.7 |
| 1401. | Br | F | H | A.7 |
| 1402. | I | F | H | A.7 |
| 1403. | CN | F | H | A.7 |
| 1404. | CH₃ | F | H | A.7 |
| 1405. | CF₃ | F | H | A.7 |
| 1406. | OCH₃ | F | H | A.7 |
| 1407. | OCF₃ | F | H | A.7 |
| 1408. | CH₂CH₃ | F | H | A.7 |
| 1409. | H | Cl | H | A.7 |
| 1410. | F | Cl | H | A.7 |
| 1411. | Cl | Cl | H | A.7 |
| 1412. | Br | Cl | H | A.7 |
| 1413. | I | Cl | H | A.7 |
| 1414. | CN | Cl | H | A.7 |
| 1415. | CH₃ | Cl | H | A.7 |
| 1416. | CF₃ | Cl | H | A.7 |
| 1417. | OCH₃ | Cl | H | A.7 |
| 1418. | OCF₃ | Cl | H | A.7 |
| 1419. | CH₂CH₃ | Cl | H | A.7 |
| 1420. | H | H | F | A.7 |
| 1421. | F | H | F | A.7 |
| 1422. | Cl | H | F | A.7 |
| 1423. | Br | H | F | A.7 |
| 1424. | I | H | F | A.7 |
| 1425. | CN | H | F | A.7 |
| 1426. | CH₃ | H | F | A.7 |
| 1427. | CF₃ | H | F | A.7 |
| 1428. | OCH₃ | H | F | A.7 |
| 1429. | OCF₃ | H | F | A.7 |
| 1430. | CH₂CH₃ | H | F | A.7 |
| 1431. | H | F | F | A.7 |
| 1432. | F | F | F | A.7 |
| 1433. | Cl | F | F | A.7 |
| 1434. | Br | F | F | A.7 |
| 1435. | I | F | F | A.7 |
| 1436. | CN | F | F | A.7 |
| 1437. | CH₃ | F | F | A.7 |
| 1438. | CF₃ | F | F | A.7 |
| 1439. | OCH₃ | F | F | A.7 |
| 1440. | OCF₃ | F | F | A.7 |
| 1441. | CH₂CH₃ | F | F | A.7 |
| 1442. | H | Cl | F | A.7 |
| 1443. | F | Cl | F | A.7 |
| 1444. | Cl | Cl | F | A.7 |
| 1445. | Br | Cl | F | A.7 |
| 1446. | I | Cl | F | A.7 |
| 1447. | CN | Cl | F | A.7 |
| 1448. | CH₃ | Cl | F | A.7 |
| 1449. | CF₃ | Cl | F | A.7 |
| 1450. | OCH₃ | Cl | F | A.7 |
| 1451. | OCF₃ | Cl | F | A.7 |
| 1452. | CH₂CH₃ | Cl | F | A.7 |
| 1453. | H | H | Cl | A.7 |
| 1454. | F | H | Cl | A.7 |
| 1455. | Cl | H | Cl | A.7 |
| 1456. | Br | H | Cl | A.7 |
| 1457. | I | H | Cl | A.7 |
| 1458. | CN | H | Cl | A.7 |
| 1459. | CH₃ | H | Cl | A.7 |
| 1460. | CF₃ | H | Cl | A.7 |
| 1461. | OCH₃ | H | Cl | A.7 |
| 1462. | OCF₃ | H | Cl | A.7 |
| 1463. | CH₂CH₃ | H | Cl | A.7 |
| 1464. | H | F | Cl | A.7 |
| 1465. | F | F | Cl | A.7 |
| 1466. | Cl | F | Cl | A.7 |
| 1467. | Br | F | Cl | A.7 |
| 1468. | I | F | Cl | A.7 |
| 1469. | CN | F | Cl | A.7 |
| 1470. | CH₃ | F | Cl | A.7 |
| 1471. | CF₃ | F | Cl | A.7 |
| 1472. | OCH₃ | F | Cl | A.7 |
| 1473. | OCF₃ | F | Cl | A.7 |
| 1474. | CH₂CH₃ | F | Cl | A.7 |
| 1475. | H | Cl | Cl | A.7 |
| 1476. | F | Cl | Cl | A.7 |
| 1477. | Cl | Cl | Cl | A.7 |
| 1478. | Br | Cl | Cl | A.7 |
| 1479. | I | Cl | Cl | A.7 |
| 1480. | CN | Cl | Cl | A.7 |
| 1481. | CH₃ | Cl | Cl | A.7 |
| 1482. | CF₃ | Cl | Cl | A.7 |
| 1483. | OCH₃ | Cl | Cl | A.7 |
| 1484. | OCF₃ | Cl | Cl | A.7 |
| 1485. | CH₂CH₃ | Cl | Cl | A.7 |
| 1486. | H | H | Br | A.7 |
| 1487. | F | H | Br | A.7 |
| 1488. | Cl | H | Br | A.7 |
| 1489. | Br | H | Br | A.7 |
| 1490. | I | H | Br | A.7 |
| 1491. | CN | H | Br | A.7 |
| 1492. | CH₃ | H | Br | A.7 |
| 1493. | CF₃ | H | Br | A.7 |
| 1494. | OCH₃ | H | Br | A.7 |
| 1495. | OCF₃ | H | Br | A.7 |
| 1496. | CH₂CH₃ | H | Br | A.7 |
| 1497. | H | F | Br | A.7 |
| 1498. | F | F | Br | A.7 |
| 1499. | Cl | F | Br | A.7 |
| 1500. | Br | F | Br | A.7 |
| 1501. | I | F | Br | A.7 |
| 1502. | CN | F | Br | A.7 |
| 1503. | CH₃ | F | Br | A.7 |
| 1504. | CF₃ | F | Br | A.7 |
| 1505. | OCH₃ | F | Br | A.7 |
| 1506. | OCF₃ | F | Br | A.7 |
| 1507. | CH₂CH₃ | F | Br | A.7 |
| 1508. | H | Cl | Br | A.7 |
| 1509. | F | Cl | Br | A.7 |
| 1510. | Cl | Cl | Br | A.7 |
| 1511. | Br | Cl | Br | A.7 |
| 1512. | I | Cl | Br | A.7 |
| 1513. | CN | Cl | Br | A.7 |
| 1514. | CH₃ | Cl | Br | A.7 |
| 1515. | CF₃ | Cl | Br | A.7 |
| 1516. | OCH₃ | Cl | Br | A.7 |
| 1517. | OCF₃ | Cl | Br | A.7 |
| 1518. | CH₂CH₃ | Cl | Br | A.7 |
| 1519. | H | H | I | A.7 |
| 1520. | F | H | I | A.7 |
| 1521. | Cl | H | I | A.7 |
| 1522. | Br | H | I | A.7 |
| 1523. | I | H | I | A.7 |
| 1524. | CN | H | I | A.7 |
| 1525. | CH₃ | H | I | A.7 |
| 1526. | CF₃ | H | I | A.7 |
| 1527. | OCH₃ | H | I | A.7 |
| 1528. | OCF₃ | H | I | A.7 |
| 1529. | CH₂CH₃ | H | I | A.7 |
| 1530. | H | F | I | A.7 |
| 1531. | F | F | I | A.7 |
| 1532. | Cl | F | I | A.7 |
| 1533. | Br | F | I | A.7 |
| 1534. | I | F | I | A.7 |
| 1535. | CN | F | I | A.7 |
| 1536. | CH₃ | F | I | A.7 |
| 1537. | CF₃ | F | I | A.7 |
| 1538. | OCH₃ | F | I | A.7 |
| 1539. | OCF₃ | F | I | A.7 |
| 1540. | CH₂CH₃ | F | I | A.7 |
| 1541. | H | Cl | I | A.7 |
| 1542. | F | Cl | I | A.7 |
| 1543. | Cl | Cl | I | A.7 |
| 1544. | Br | Cl | I | A.7 |
| 1545. | I | Cl | I | A.7 |
| 1546. | CN | Cl | I | A.7 |
| 1547. | CH₃ | Cl | I | A.7 |
| 1548. | CF₃ | Cl | I | A.7 |
| 1549. | OCH₃ | Cl | I | A.7 |
| 1550. | OCF₃ | Cl | I | A.7 |
| 1551. | CH₂CH₃ | Cl | I | A.7 |
| 1552. | H | H | CN | A.7 |
| 1553. | F | H | CN | A.7 |
| 1554. | Cl | H | CN | A.7 |
| 1555. | Br | H | CN | A.7 |
| 1556. | I | H | CN | A.7 |
| 1557. | CN | H | CN | A.7 |
| 1558. | CH₃ | H | CN | A.7 |
| 1559. | CF₃ | H | CN | A.7 |
| 1560. | OCH₃ | H | CN | A.7 |
| 1561. | OCF₃ | H | CN | A.7 |
| 1562. | CH₂CH₃ | H | CN | A.7 |
| 1563. | H | F | CN | A.7 |
| 1564. | F | F | CN | A.7 |
| 1565. | Cl | F | CN | A.7 |
| 1566. | Br | F | CN | A.7 |
| 1567. | I | F | CN | A.7 |
| 1568. | CN | F | CN | A.7 |
| 1569. | CH₃ | F | CN | A.7 |
| 1570. | CF₃ | F | CN | A.7 |
| 1571. | OCH₃ | F | CN | A.7 |
| 1572. | OCF₃ | F | CN | A.7 |
| 1573. | CH₂CH₃ | F | CN | A.7 |
| 1574. | H | Cl | CN | A.7 |
| 1575. | F | Cl | CN | A.7 |
| 1576. | Cl | Cl | CN | A.7 |
| 1577. | Br | Cl | CN | A.7 |
| 1578. | I | Cl | CN | A.7 |
| 1579. | CN | Cl | CN | A.7 |
| 1580. | CH₃ | Cl | CN | A.7 |
| 1581. | CF₃ | Cl | CN | A.7 |
| 1582. | OCH₃ | Cl | CN | A.7 |
| 1583. | OCF₃ | Cl | CN | A.7 |
| 1584. | CH₂CH₃ | Cl | CN | A.7 |
| 1585. | H | H | CH₃ | A.7 |
| 1586. | F | H | CH₃ | A.7 |
| 1587. | Cl | H | CH₃ | A.7 |
| 1588. | Br | H | CH₃ | A.7 |
| 1589. | I | H | CH₃ | A.7 |
| 1590. | CN | H | CH₃ | A.7 |
| 1591. | CH₃ | H | CH₃ | A.7 |
| 1592. | CF₃ | H | CH₃ | A.7 |
| 1593. | OCH₃ | H | CH₃ | A.7 |
| 1594. | OCF₃ | H | CH₃ | A.7 |
| 1595. | CH₂CH₃ | H | CH₃ | A.7 |
| 1596. | H | F | CH₃ | A.7 |
| 1597. | F | F | CH₃ | A.7 |
| 1598. | Cl | F | CH₃ | A.7 |
| 1599. | Br | F | CH₃ | A.7 |
| 1600. | I | F | CH₃ | A.7 |
| 1601. | CN | F | CH₃ | A.7 |
| 1602. | CH₃ | F | CH₃ | A.7 |
| 1603. | CF₃ | F | CH₃ | A.7 |
| 1604. | OCH₃ | F | CH₃ | A.7 |
| 1605. | OCF₃ | F | CH₃ | A.7 |
| 1606. | CH₂CH₃ | F | CH₃ | A.7 |
| 1607. | H | Cl | CH₃ | A.7 |
| 1608. | F | Cl | CH₃ | A.7 |
| 1609. | Cl | Cl | CH₃ | A.7 |
| 1610. | Br | Cl | CH₃ | A.7 |
| 1611. | I | Cl | CH₃ | A.7 |
| 1612. | CN | Cl | CH₃ | A.7 |
| 1613. | CH₃ | Cl | CH₃ | A.7 |
| 1614. | CF₃ | Cl | CH₃ | A.7 |
| 1615. | OCH₃ | Cl | CH₃ | A.7 |
| 1616. | OCF₃ | Cl | CH₃ | A.7 |
| 1617. | CH₂CH₃ | Cl | CH₃ | A.7 |
| 1618. | H | H | H | A.8 |
| 1619. | F | H | H | A.8 |
| 1620. | Cl | H | H | A.8 |
| 1621. | Br | H | H | A.8 |
| 1622. | I | H | H | A.8 |
| 1623. | CN | H | H | A.8 |
| 1624. | CH₃ | H | H | A.8 |
| 1625. | CF₃ | H | H | A.8 |
| 1626. | OCH₃ | H | H | A.8 |
| 1627. | OCF₃ | H | H | A.8 |
| 1628. | CH₂CH₃ | H | H | A.8 |
| 1629. | H | F | H | A.8 |
| 1630. | F | F | H | A.8 |
| 1631. | Cl | F | H | A.8 |
| 1632. | Br | F | H | A.8 |
| 1633. | I | F | H | A.8 |
| 1634. | CN | F | H | A.8 |
| 1635. | CH₃ | F | H | A.8 |
| 1636. | CF₃ | F | H | A.8 |
| 1637. | OCH₃ | F | H | A.8 |
| 1638. | OCF₃ | F | H | A.8 |
| 1639. | CH₂CH₃ | F | H | A.8 |
| 1640. | H | Cl | H | A.8 |
| 1641. | F | Cl | H | A.8 |
| 1642. | Cl | Cl | H | A.8 |
| 1643. | Br | Cl | H | A.8 |
| 1644. | I | Cl | H | A.8 |
| 1645. | CN | Cl | H | A.8 |
| 1646. | CH₃ | Cl | H | A.8 |
| 1647. | CF₃ | Cl | H | A.8 |
| 1648. | OCH₃ | Cl | H | A.8 |
| 1649. | OCF₃ | Cl | H | A.8 |
| 1650. | CH₂CH₃ | Cl | H | A.8 |
| 1651. | H | H | F | A.8 |
| 1652. | F | H | F | A.8 |
| 1653. | Cl | H | F | A.8 |
| 1654. | Br | H | F | A.8 |
| 1655. | I | H | F | A.8 |
| 1656. | CN | H | F | A.8 |
| 1657. | CH₃ | H | F | A.8 |
| 1658. | CF₃ | H | F | A.8 |
| 1659. | OCH₃ | H | F | A.8 |
| 1660. | OCF₃ | H | F | A.8 |
| 1661. | CH₂CH₃ | H | F | A.8 |
| 1662. | H | F | F | A.8 |
| 1663. | F | F | F | A.8 |
| 1664. | Cl | F | F | A.8 |
| 1665. | Br | F | F | A.8 |
| 1666. | I | F | F | A.8 |
| 1667. | CN | F | F | A.8 |
| 1668. | CH₃ | F | F | A.8 |
| 1669. | CF₃ | F | F | A.8 |
| 1670. | OCH₃ | F | F | A.8 |
| 1671. | OCF₃ | F | F | A.8 |
| 1672. | CH₂CH₃ | F | F | A.8 |
| 1673. | H | Cl | F | A.8 |
| 1674. | F | Cl | F | A.8 |
| 1675. | Cl | Cl | F | A.8 |
| 1676. | Br | Cl | F | A.8 |
| 1677. | I | Cl | F | A.8 |
| 1678. | CN | Cl | F | A.8 |
| 1679. | CH₃ | Cl | F | A.8 |
| 1680. | CF₃ | Cl | F | A.8 |
| 1681. | OCH₃ | Cl | F | A.8 |
| 1682. | OCF₃ | Cl | F | A.8 |
| 1683. | CH₂CH₃ | Cl | F | A.8 |
| 1684. | H | H | Cl | A.8 |
| 1685. | F | H | Cl | A.8 |
| 1686. | Cl | H | Cl | A.8 |
| 1687. | Br | H | Cl | A.8 |
| 1688. | I | H | Cl | A.8 |
| 1689. | CN | H | Cl | A.8 |
| 1690. | CH₃ | H | Cl | A.8 |
| 1691. | CF₃ | H | Cl | A.8 |
| 1692. | OCH₃ | H | Cl | A.8 |
| 1693. | OCF₃ | H | Cl | A.8 |
| 1694. | CH₂CH₃ | H | Cl | A.8 |
| 1695. | H | F | Cl | A.8 |
| 1696. | F | F | Cl | A.8 |
| 1697. | Cl | F | Cl | A.8 |
| 1698. | Br | F | Cl | A.8 |
| 1699. | I | F | Cl | A.8 |
| 1700. | CN | F | Cl | A.8 |
| 1701. | CH₃ | F | Cl | A.8 |
| 1702. | CF₃ | F | Cl | A.8 |
| 1703. | OCH₃ | F | Cl | A.8 |
| 1704. | OCF₃ | F | Cl | A.8 |
| 1705. | CH₂CH₃ | F | Cl | A.8 |
| 1706. | H | Cl | Cl | A.8 |
| 1707. | F | Cl | Cl | A.8 |
| 1708. | Cl | Cl | Cl | A.8 |
| 1709. | Br | Cl | Cl | A.8 |
| 1710. | I | Cl | Cl | A.8 |
| 1711. | CN | Cl | Cl | A.8 |
| 1712. | CH₃ | Cl | Cl | A.8 |
| 1713. | CF₃ | Cl | Cl | A.8 |
| 1714. | OCH₃ | Cl | Cl | A.8 |
| 1715. | OCF₃ | Cl | Cl | A.8 |
| 1716. | CH₂CH₃ | Cl | Cl | A.8 |
| 1717. | H | H | Br | A.8 |
| 1718. | F | H | Br | A.8 |
| 1719. | Cl | H | Br | A.8 |
| 1720. | Br | H | Br | A.8 |
| 1721. | I | H | Br | A.8 |
| 1722. | CN | H | Br | A.8 |
| 1723. | CH₃ | H | Br | A.8 |
| 1724. | CF₃ | H | Br | A.8 |
| 1725. | OCH₃ | H | Br | A.8 |
| 1726. | OCF₃ | H | Br | A.8 |
| 1727. | CH₂CH₃ | H | Br | A.8 |
| 1728. | H | F | Br | A.8 |
| 1729. | F | F | Br | A.8 |
| 1730. | Cl | F | Br | A.8 |
| 1731. | Br | F | Br | A.8 |
| 1732. | I | F | Br | A.8 |
| 1733. | CN | F | Br | A.8 |
| 1734. | CH₃ | F | Br | A.8 |
| 1735. | CF₃ | F | Br | A.8 |
| 1736. | OCH₃ | F | Br | A.8 |
| 1737. | OCF₃ | F | Br | A.8 |
| 1738. | CH₂CH₃ | F | Br | A.8 |
| 1739. | H | Cl | Br | A.8 |
| 1740. | F | Cl | Br | A.8 |
| 1741. | Cl | Cl | Br | A.8 |
| 1742. | Br | Cl | Br | A.8 |
| 1743. | I | Cl | Br | A.8 |
| 1744. | CN | Cl | Br | A.8 |
| 1745. | CH₃ | Cl | Br | A.8 |
| 1746. | CF₃ | Cl | Br | A.8 |
| 1747. | OCH₃ | Cl | Br | A.8 |
| 1748. | OCF₃ | Cl | Br | A.8 |
| 1749. | CH₂CH₃ | Cl | Br | A.8 |
| 1750. | H | H | I | A.8 |
| 1751. | F | H | I | A.8 |
| 1752. | Cl | H | I | A.8 |
| 1753. | Br | H | I | A.8 |
| 1754. | I | H | I | A.8 |
| 1755. | CN | H | I | A.8 |
| 1756. | CH₃ | H | I | A.8 |
| 1757. | CF₃ | H | I | A.8 |
| 1758. | OCH₃ | H | I | A.8 |
| 1759. | OCF₃ | H | I | A.8 |
| 1760. | CH₂CH₃ | H | I | A.8 |
| 1761. | H | F | I | A.8 |
| 1762. | F | F | I | A.8 |
| 1763. | Cl | F | I | A.8 |
| 1764. | Br | F | I | A.8 |
| 1765. | I | F | I | A.8 |
| 1766. | CN | F | I | A.8 |
| 1767. | CH₃ | F | I | A.8 |
| 1768. | CF₃ | F | I | A.8 |
| 1769. | OCH₃ | F | I | A.8 |
| 1770. | OCF₃ | F | I | A.8 |
| 1771. | CH₂CH₃ | F | I | A.8 |
| 1772. | H | Cl | I | A.8 |
| 1773. | F | Cl | I | A.8 |
| 1774. | Cl | Cl | I | A.8 |
| 1775. | Br | Cl | I | A.8 |
| 1776. | I | Cl | I | A.8 |
| 1777. | CN | Cl | I | A.8 |
| 1778. | CH₃ | Cl | I | A.8 |
| 1779. | CF₃ | Cl | I | A.8 |
| 1780. | OCH₃ | Cl | I | A.8 |
| 1781. | OCF₃ | Cl | I | A.8 |
| 1782. | CH₂CH₃ | Cl | I | A.8 |
| 1783. | H | H | CN | A.8 |
| 1784. | F | H | CN | A.8 |
| 1785. | Cl | H | CN | A.8 |
| 1786. | Br | H | CN | A.8 |
| 1787. | I | H | CN | A.8 |
| 1788. | CN | H | CN | A.8 |
| 1789. | CH₃ | H | CN | A.8 |
| 1790. | CF₃ | H | CN | A.8 |
| 1791. | OCH₃ | H | CN | A.8 |
| 1792. | OCF₃ | H | CN | A.8 |
| 1793. | CH₂CH₃ | H | CN | A.8 |
| 1794. | H | F | CN | A.8 |
| 1795. | F | F | CN | A.8 |
| 1796. | Cl | F | CN | A.8 |
| 1797. | Br | F | CN | A.8 |
| 1798. | I | F | CN | A.8 |
| 1799. | CN | F | CN | A.8 |
| 1800. | CH₃ | F | CN | A.8 |
| 1801. | CF₃ | F | CN | A.8 |
| 1802. | OCH₃ | F | CN | A.8 |
| 1803. | OCF₃ | F | CN | A.8 |
| 1804. | CH₂CH₃ | F | CN | A.8 |
| 1805. | H | Cl | CN | A.8 |
| 1806. | F | Cl | CN | A.8 |
| 1807. | Cl | Cl | CN | A.8 |
| 1808. | Br | Cl | CN | A.8 |
| 1809. | I | Cl | CN | A.8 |
| 1810. | CN | Cl | CN | A.8 |
| 1811. | CH₃ | Cl | CN | A.8 |
| 1812. | CF₃ | Cl | CN | A.8 |
| 1813. | OCH₃ | Cl | CN | A.8 |
| 1814. | OCF₃ | Cl | CN | A.8 |
| 1815. | CH₂CH₃ | Cl | CN | A.8 |
| 1816. | H | H | CH₃ | A.8 |
| 1817. | F | H | CH₃ | A.8 |
| 1818. | Cl | H | CH₃ | A.8 |
| 1819. | Br | H | CH₃ | A.8 |
| 1820. | I | H | CH₃ | A.8 |
| 1821. | CN | H | CH₃ | A.8 |
| 1822. | CH₃ | H | CH₃ | A.8 |
| 1823. | CF₃ | H | CH₃ | A.8 |
| 1824. | OCH₃ | H | CH₃ | A.8 |
| 1825. | OCF₃ | H | CH₃ | A.8 |
| 1826. | CH₂CH₃ | H | CH₃ | A.8 |
| 1827. | H | F | CH₃ | A.8 |
| 1828. | F | F | CH₃ | A.8 |
| 1829. | Cl | F | CH₃ | A.8 |
| 1830. | Br | F | CH₃ | A.8 |
| 1831. | I | F | CH₃ | A.8 |
| 1832. | CN | F | CH₃ | A.8 |
| 1833. | CH₃ | F | CH₃ | A.8 |
| 1834. | CF₃ | F | CH₃ | A.8 |
| 1835. | OCH₃ | F | CH₃ | A.8 |
| 1836. | OCF₃ | F | CH₃ | A.8 |
| 1837. | CH₂CH₃ | F | CH₃ | A.8 |
| 1838. | H | Cl | CH₃ | A.8 |
| 1839. | F | Cl | CH₃ | A.8 |
| 1840. | Cl | Cl | CH₃ | A.8 |
| 1841. | Br | Cl | CH₃ | A.8 |
| 1842. | I | Cl | CH₃ | A.8 |
| 1843. | CN | Cl | CH₃ | A.8 |
| 1844. | CH₃ | Cl | CH₃ | A.8 |
| 1845. | CF₃ | Cl | CH₃ | A.8 |
| 1846. | OCH₃ | Cl | CH₃ | A.8 |
| 1847. | OCF₃ | Cl | CH₃ | A.8 |
| 1848. | CH₂CH₃ | Cl | CH₃ | A.8 |
| 1849. | H | H | H | A.9 |
| 1850. | F | H | H | A.9 |
| 1851. | Cl | H | H | A.9 |
| 1852. | Br | H | H | A.9 |
| 1853. | I | H | H | A.9 |
| 1854. | CN | H | H | A.9 |
| 1855. | CH₃ | H | H | A.9 |
| 1856. | CF₃ | H | H | A.9 |
| 1857. | OCH₃ | H | H | A.9 |
| 1858. | OCF₃ | H | H | A.9 |
| 1859. | CH₂CH₃ | H | H | A.9 |
| 1860. | H | F | H | A.9 |
| 1861. | F | F | H | A.9 |
| 1862. | Cl | F | H | A.9 |
| 1863. | Br | F | H | A.9 |
| 1864. | I | F | H | A.9 |
| 1865. | CN | F | H | A.9 |
| 1866. | CH₃ | F | H | A.9 |
| 1867. | CF₃ | F | H | A.9 |
| 1868. | OCH₃ | F | H | A.9 |
| 1869. | OCF₃ | F | H | A.9 |
| 1870. | CH₂CH₃ | F | H | A.9 |
| 1871. | H | Cl | H | A.9 |
| 1872. | F | Cl | H | A.9 |
| 1873. | Cl | Cl | H | A.9 |
| 1874. | Br | Cl | H | A.9 |
| 1875. | I | Cl | H | A.9 |
| 1876. | CN | Cl | H | A.9 |
| 1877. | CH₃ | Cl | H | A.9 |
| 1878. | CF₃ | Cl | H | A.9 |
| 1879. | OCH₃ | Cl | H | A.9 |
| 1880. | OCF₃ | Cl | H | A.9 |
| 1881. | CH₂CH₃ | Cl | H | A.9 |
| 1882. | H | H | F | A.9 |
| 1883. | F | H | F | A.9 |
| 1884. | Cl | H | F | A.9 |
| 1885. | Br | H | F | A.9 |
| 1886. | I | H | F | A.9 |
| 1887. | CN | H | F | A.9 |
| 1888. | CH₃ | H | F | A.9 |
| 1889. | CF₃ | H | F | A.9 |
| 1890. | OCH₃ | H | F | A.9 |
| 1891. | OCF₃ | H | F | A.9 |
| 1892. | CH₂CH₃ | H | F | A.9 |
| 1893. | H | F | F | A.9 |
| 1894. | F | F | F | A.9 |
| 1895. | Cl | F | F | A.9 |
| 1896. | Br | F | F | A.9 |
| 1897. | I | F | F | A.9 |
| 1898. | CN | F | F | A.9 |
| 1899. | CH₃ | F | F | A.9 |
| 1900. | CF₃ | F | F | A.9 |
| 1901. | OCH₃ | F | F | A.9 |
| 1902. | OCF₃ | F | F | A.9 |
| 1903. | CH₂CH₃ | F | F | A.9 |
| 1904. | H | Cl | F | A.9 |
| 1905. | F | Cl | F | A.9 |
| 1906. | Cl | Cl | F | A.9 |
| 1907. | Br | Cl | F | A.9 |
| 1908. | I | Cl | F | A.9 |
| 1909. | CN | Cl | F | A.9 |
| 1910. | CH₃ | Cl | F | A.9 |
| 1911. | CF₃ | Cl | F | A.9 |
| 1912. | OCH₃ | Cl | F | A.9 |
| 1913. | OCF₃ | Cl | F | A.9 |
| 1914. | CH₂CH₃ | Cl | F | A.9 |
| 1915. | H | H | Cl | A.9 |
| 1916. | F | H | Cl | A.9 |
| 1917. | Cl | H | Cl | A.9 |
| 1918. | Br | H | Cl | A.9 |
| 1919. | I | H | Cl | A.9 |
| 1920. | CN | H | Cl | A.9 |
| 1921. | CH₃ | H | Cl | A.9 |
| 1922. | CF₃ | H | Cl | A.9 |
| 1923. | OCH₃ | H | Cl | A.9 |
| 1924. | OCF₃ | H | Cl | A.9 |
| 1925. | CH₂CH₃ | H | Cl | A.9 |
| 1926. | H | F | Cl | A.9 |
| 1927. | F | F | Cl | A.9 |
| 1928. | Cl | F | Cl | A.9 |
| 1929. | Br | F | Cl | A.9 |
| 1930. | I | F | Cl | A.9 |
| 1931. | CN | F | Cl | A.9 |
| 1932. | CH₃ | F | Cl | A.9 |
| 1933. | CF₃ | F | Cl | A.9 |
| 1934. | OCH₃ | F | Cl | A.9 |
| 1935. | OCF₃ | F | Cl | A.9 |
| 1936. | CH₂CH₃ | F | Cl | A.9 |
| 1937. | H | Cl | Cl | A.9 |
| 1938. | F | Cl | Cl | A.9 |
| 1939. | Cl | Cl | Cl | A.9 |
| 1940. | Br | Cl | Cl | A.9 |
| 1941. | I | Cl | Cl | A.9 |
| 1942. | CN | Cl | Cl | A.9 |
| 1943. | CH₃ | Cl | Cl | A.9 |
| 1944. | CF₃ | Cl | Cl | A.9 |
| 1945. | OCH₃ | Cl | Cl | A.9 |
| 1946. | OCF₃ | Cl | Cl | A.9 |
| 1947. | CH₂CH₃ | Cl | Cl | A.9 |
| 1948. | H | H | Br | A.9 |
| 1949. | F | H | Br | A.9 |
| 1950. | CI | H | Br | A.9 |
| 1951. | Br | H | Br | A.9 |
| 1952. | I | H | Br | A.9 |
| 1953. | CN | H | Br | A.9 |
| 1954. | CH₃ | H | Br | A.9 |
| 1955. | CF₃ | H | Br | A.9 |
| 1956. | OCH₃ | H | Br | A.9 |
| 1957. | OCF₃ | H | Br | A.9 |
| 1958. | CH₂CH₃ | H | Br | A.9 |
| 1959. | H | F | Br | A.9 |
| 1960. | F | F | Br | A.9 |
| 1961. | Cl | F | Br | A.9 |
| 1962. | Br | F | Br | A.9 |
| 1963. | I | F | Br | A.9 |
| 1964. | CN | F | Br | A.9 |
| 1965. | CH₃ | F | Br | A.9 |
| 1966. | CF₃ | F | Br | A.9 |
| 1967. | OCH₃ | F | Br | A.9 |
| 1968. | OCF₃ | F | Br | A.9 |
| 1969. | CH₂CH₃ | F | Br | A.9 |
| 1970. | H | Cl | Br | A.9 |
| 1971. | F | Cl | Br | A.9 |
| 1972. | Cl | Cl | Br | A.9 |
| 1973. | Br | Cl | Br | A.9 |
| 1974. | I | Cl | Br | A.9 |
| 1975. | CN | Cl | Br | A.9 |
| 1976. | CH₃ | Cl | Br | A.9 |
| 1977. | CF₃ | Cl | Br | A.9 |
| 1978. | OCH₃ | Cl | Br | A.9 |
| 1979. | OCF₃ | Cl | Br | A.9 |
| 1980. | CH₂CH₃ | Cl | Br | A.9 |
| 1981. | H | H | I | A.9 |
| 1982. | F | H | I | A.9 |
| 1983. | Cl | H | I | A.9 |
| 1984. | Br | H | I | A.9 |
| 1985. | I | H | I | A.9 |
| 1986. | CN | H | I | A.9 |
| 1987. | CH₃ | H | I | A.9 |
| 1988. | CF₃ | H | I | A.9 |
| 1989. | OCH₃ | H | I | A.9 |
| 1990. | OCF₃ | H | I | A.9 |
| 1991. | CH₂CH₃ | H | I | A.9 |
| 1992. | H | F | I | A.9 |
| 1993. | F | F | I | A.9 |
| 1994. | Cl | F | I | A.9 |
| 1995. | Br | F | I | A.9 |
| 1996. | I | F | I | A.9 |
| 1997. | CN | F | I | A.9 |
| 1998. | CH₃ | F | I | A.9 |
| 1999. | CF₃ | F | I | A.9 |
| 2000. | OCH₃ | F | I | A.9 |
| 2001. | OCF₃ | F | I | A.9 |
| 2002. | CH₂CH₃ | F | I | A.9 |
| 2003. | H | Cl | I | A.9 |
| 2004. | F | Cl | I | A.9 |
| 2005. | Cl | Cl | I | A.9 |
| 2006. | Br | Cl | I | A.9 |
| 2007. | I | Cl | I | A.9 |
| 2008. | CN | Cl | I | A.9 |
| 2009. | CH₃ | Cl | I | A.9 |
| 2010. | CF₃ | Cl | I | A.9 |
| 2011. | OCH₃ | Cl | I | A.9 |
| 2012. | OCF₃ | Cl | I | A.9 |
| 2013. | CH₂CH₃ | Cl | I | A.9 |
| 2014. | H | H | CN | A.9 |
| 2015. | F | H | CN | A.9 |
| 2016. | Cl | H | CN | A.9 |
| 2017. | Br | H | CN | A.9 |
| 2018. | I | H | CN | A.9 |
| 2019. | CN | H | CN | A.9 |
| 2020. | CH₃ | H | CN | A.9 |
| 2021. | CF₃ | H | CN | A.9 |
| 2022. | OCH₃ | H | CN | A.9 |
| 2023. | OCF₃ | H | CN | A.9 |
| 2024. | CH₂CH₃ | H | CN | A.9 |
| 2025. | H | F | CN | A.9 |
| 2026. | F | F | CN | A.9 |
| 2027. | Cl | F | CN | A.9 |
| 2028. | Br | F | CN | A.9 |
| 2029. | I | F | CN | A.9 |
| 2030. | CN | F | CN | A.9 |
| 2031. | CH₃ | F | CN | A.9 |
| 2032. | CF₃ | F | CN | A.9 |
| 2033. | OCH₃ | F | CN | A.9 |
| 2034. | OCF₃ | F | CN | A.9 |
| 2035. | CH₂CH₃ | F | CN | A.9 |
| 2036. | H | Cl | CN | A.9 |
| 2037. | F | Cl | CN | A.9 |
| 2038. | Cl | Cl | CN | A.9 |
| 2039. | Br | Cl | CN | A.9 |
| 2040. | I | Cl | CN | A.9 |
| 2041. | CN | Cl | CN | A.9 |
| 2042. | CH₃ | Cl | CN | A.9 |
| 2043. | CF₃ | Cl | CN | A.9 |
| 2044. | OCH₃ | Cl | CN | A.9 |
| 2045. | OCF₃ | Cl | CN | A.9 |
| 2046. | CH₂CH₃ | Cl | CN | A.9 |
| 2047. | H | H | CH₃ | A.9 |
| 2048. | F | H | CH₃ | A.9 |
| 2049. | Cl | H | CH₃ | A.9 |
| 2050. | Br | H | CH₃ | A.9 |
| 2051. | I | H | CH₃ | A.9 |
| 2052. | CN | H | CH₃ | A.9 |
| 2053. | CH₃ | H | CH₃ | A.9 |
| 2054. | CF₃ | H | CH₃ | A.9 |
| 2055. | OCH₃ | H | CH₃ | A.9 |
| 2056. | OCF₃ | H | CH₃ | A.9 |
| 2057. | CH₂CH₃ | H | CH₃ | A.9 |
| 2058. | H | F | CH₃ | A.9 |
| 2059. | F | F | CH₃ | A.9 |
| 2060. | Cl | F | CH₃ | A.9 |
| 2061. | Br | F | CH₃ | A.9 |
| 2062. | I | F | CH₃ | A.9 |
| 2063. | CN | F | CH₃ | A.9 |
| 2064. | CH₃ | F | CH₃ | A.9 |
| 2065. | CF₃ | F | CH₃ | A.9 |
| 2066. | OCH₃ | F | CH₃ | A.9 |
| 2067. | OCF₃ | F | CH₃ | A.9 |
| 2068. | CH₂CH₃ | F | CH₃ | A.9 |
| 2069. | H | Cl | CH₃ | A.9 |
| 2070. | F | Cl | CH₃ | A.9 |
| 2071. | Cl | Cl | CH₃ | A.9 |
| 2072. | Br | Cl | CH₃ | A.9 |
| 2073. | I | Cl | CH₃ | A.9 |
| 2074. | CN | Cl | CH₃ | A.9 |
| 2075. | CH₃ | Cl | CH₃ | A.9 |
| 2076. | CF₃ | Cl | CH₃ | A.9 |
| 2077. | OCH₃ | Cl | CH₃ | A.9 |
| 2078. | OCF₃ | Cl | CH₃ | A.9 |
| 2079. | CH₂CH₃ | Cl | CH₃ | A.9 |
| 2080. | H | H | H | A.10 |
| 2081. | F | H | H | A.10 |
| 2082. | Cl | H | H | A.10 |
| 2083. | Br | H | H | A.10 |
| 2084. | I | H | H | A.10 |
| 2085. | CN | H | H | A.10 |
| 2086. | CH₃ | H | H | A.10 |
| 2087. | CF₃ | H | H | A.10 |
| 2088. | OCH₃ | H | H | A.10 |
| 2089. | OCF₃ | H | H | A.10 |
| 2090. | CH₂CH₃ | H | H | A.10 |
| 2091. | H | F | H | A.10 |
| 2092. | F | F | H | A.10 |
| 2093. | Cl | F | H | A.10 |
| 2094. | Br | F | H | A.10 |
| 2095. | I | F | H | A.10 |
| 2096. | CN | F | H | A.10 |
| 2097. | CH₃ | F | H | A.10 |
| 2098. | CF₃ | F | H | A.10 |
| 2099. | OCH₃ | F | H | A.10 |
| 2100. | OCF₃ | F | H | A.10 |
| 2101. | CH₂CH₃ | F | H | A.10 |
| 2102. | H | Cl | H | A.10 |
| 2103. | F | Cl | H | A.10 |
| 2104. | Cl | Cl | H | A.10 |
| 2105. | Br | Cl | H | A.10 |
| 2106. | I | Cl | H | A.10 |
| 2107. | CN | Cl | H | A.10 |
| 2108. | CH₃ | Cl | H | A.10 |
| 2109. | CF₃ | Cl | H | A.10 |
| 2110. | OCH₃ | Cl | H | A.10 |
| 2111. | OCF₃ | Cl | H | A.10 |
| 2112. | CH₂CH₃ | Cl | H | A.10 |
| 2113. | H | H | F | A.10 |
| 2114. | F | H | F | A.10 |
| 2115. | Cl | H | F | A.10 |
| 2116. | Br | H | F | A.10 |
| 2117. | I | H | F | A.10 |
| 2118. | CN | H | F | A.10 |
| 2119. | CH₃ | H | F | A.10 |
| 2120. | CF₃ | H | F | A.10 |
| 2121. | OCH₃ | H | F | A.10 |
| 2122. | OCF₃ | H | F | A.10 |
| 2123. | CH₂CH₃ | H | F | A.10 |
| 2124. | H | F | F | A.10 |
| 2125. | F | F | F | A.10 |
| 2126. | Cl | F | F | A.10 |
| 2127. | Br | F | F | A.10 |
| 2128. | I | F | F | A.10 |
| 2129. | CN | F | F | A.10 |
| 2130. | CH₃ | F | F | A.10 |
| 2131. | CF₃ | F | F | A.10 |
| 2132. | OCH₃ | F | F | A.10 |
| 2133. | OCF₃ | F | F | A.10 |
| 2134. | CH₂CH₃ | F | F | A.10 |
| 2135. | H | Cl | F | A.10 |
| 2136. | F | Cl | F | A.10 |
| 2137. | Cl | Cl | F | A.10 |
| 2138. | Br | Cl | F | A.10 |
| 2139. | I | Cl | F | A.10 |
| 2140. | CN | Cl | F | A.10 |
| 2141. | CH₃ | Cl | F | A.10 |
| 2142. | CF₃ | Cl | F | A.10 |
| 2143. | OCH₃ | Cl | F | A.10 |
| 2144. | OCF₃ | Cl | F | A.10 |
| 2145. | CH₂CH₃ | Cl | F | A.10 |
| 2146. | H | H | Cl | A.10 |
| 2147. | F | H | Cl | A.10 |
| 2148. | Cl | H | Cl | A.10 |
| 2149. | Br | H | Cl | A.10 |
| 2150. | I | H | Cl | A.10 |
| 2151. | CN | H | Cl | A.10 |
| 2152. | CH₃ | H | Cl | A.10 |
| 2153. | CF₃ | H | Cl | A.10 |
| 2154. | OCH₃ | H | Cl | A.10 |
| 2155. | OCF₃ | H | Cl | A.10 |
| 2156. | CH₂CH₃ | H | Cl | A.10 |
| 2157. | H | F | Cl | A.10 |
| 2158. | F | F | Cl | A.10 |
| 2159. | Cl | F | Cl | A.10 |
| 2160. | Br | F | Cl | A.10 |
| 2161. | I | F | Cl | A.10 |
| 2162. | CN | F | Cl | A.10 |
| 2163. | CH₃ | F | Cl | A.10 |
| 2164. | CF₃ | F | Cl | A.10 |
| 2165. | OCH₃ | F | Cl | A.10 |
| 2166. | OCF₃ | F | Cl | A.10 |
| 2167. | CH₂CH₃ | F | Cl | A.10 |
| 2168. | H | Cl | Cl | A.10 |
| 2169. | F | Cl | Cl | A.10 |
| 2170. | Cl | Cl | Cl | A.10 |
| 2171. | Br | Cl | Cl | A.10 |
| 2172. | I | Cl | Cl | A.10 |
| 2173. | CN | Cl | Cl | A.10 |
| 2174. | CH₃ | Cl | Cl | A.10 |
| 2175. | CF₃ | Cl | Cl | A.10 |
| 2176. | OCH₃ | Cl | Cl | A.10 |
| 2177. | OCF₃ | Cl | Cl | A.10 |
| 2178. | CH₂CH₃ | Cl | Cl | A.10 |
| 2179. | H | H | Br | A.10 |
| 2180. | F | H | Br | A.10 |
| 2181. | Cl | H | Br | A.10 |
| 2182. | Br | H | Br | A.10 |
| 2183. | I | H | Br | A.10 |
| 2184. | CN | H | Br | A.10 |
| 2185. | CH₃ | H | Br | A.10 |
| 2186. | CF₃ | H | Br | A.10 |
| 2187. | OCH₃ | H | Br | A.10 |
| 2188. | OCF₃ | H | Br | A.10 |
| 2189. | CH₂CH₃ | H | Br | A.10 |
| 2190. | H | F | Br | A.10 |
| 2191. | F | F | Br | A.10 |
| 2192. | Cl | F | Br | A.10 |
| 2193. | Br | F | Br | A.10 |
| 2194. | I | F | Br | A.10 |
| 2195. | CN | F | Br | A.10 |
| 2196. | CH₃ | F | Br | A.10 |
| 2197. | CF₃ | F | Br | A.10 |
| 2198. | OCH₃ | F | Br | A.10 |
| 2199. | OCF₃ | F | Br | A.10 |
| 2200. | CH₂CH₃ | F | Br | A.10 |
| 2201. | H | Cl | Br | A.10 |
| 2202. | F | Cl | Br | A.10 |
| 2203. | Cl | Cl | Br | A.10 |
| 2204. | Br | Cl | Br | A.10 |
| 2205. | I | Cl | Br | A.10 |
| 2206. | CN | Cl | Br | A.10 |
| 2207. | CH₃ | Cl | Br | A.10 |
| 2208. | CF₃ | Cl | Br | A.10 |
| 2209. | OCH₃ | Cl | Br | A.10 |
| 2210. | OCF₃ | Cl | Br | A.10 |
| 2211. | CH₂CH₃ | Cl | Br | A.10 |
| 2212. | H | H | I | A.10 |
| 2213. | F | H | I | A.10 |
| 2214. | Cl | H | I | A.10 |
| 2215. | Br | H | I | A.10 |
| 2216. | I | H | I | A.10 |
| 2217. | CN | H | I | A.10 |
| 2218. | CH₃ | H | I | A.10 |
| 2219. | CF₃ | H | I | A.10 |
| 2220. | OCH₃ | H | I | A.10 |
| 2221. | OCF₃ | H | I | A.10 |
| 2222. | CH₂CH₃ | H | I | A.10 |
| 2223. | H | F | I | A.10 |
| 2224. | F | F | I | A.10 |
| 2225. | Cl | F | I | A.10 |
| 2226. | Br | F | I | A.10 |
| 2227. | I | F | I | A.10 |
| 2228. | CN | F | I | A.10 |
| 2229. | CH₃ | F | I | A.10 |
| 2230. | CF₃ | F | I | A.10 |
| 2231. | OCH₃ | F | I | A.10 |
| 2232. | OCF₃ | F | I | A.10 |
| 2233. | CH₂CH₃ | F | I | A.10 |
| 2234. | H | Cl | I | A.10 |
| 2235. | F | Cl | I | A.10 |
| 2236. | Cl | Cl | I | A.10 |
| 2237. | Br | Cl | I | A.10 |
| 2238. | I | Cl | I | A.10 |
| 2239. | CN | Cl | I | A.10 |
| 2240. | CH₃ | Cl | I | A.10 |
| 2241. | CF₃ | Cl | I | A.10 |
| 2242. | OCH₃ | Cl | I | A.10 |
| 2243. | OCF₃ | Cl | I | A.10 |
| 2244. | CH₂CH₃ | Cl | I | A.10 |
| 2245. | H | H | CN | A.10 |
| 2246. | F | H | CN | A.10 |
| 2247. | Cl | H | CN | A.10 |
| 2248. | Br | H | CN | A.10 |
| 2249. | I | H | CN | A.10 |
| 2250. | CN | H | CN | A.10 |
| 2251. | CH₃ | H | CN | A.10 |
| 2252. | CF₃ | H | CN | A.10 |
| 2253. | OCH₃ | H | CN | A.10 |
| 2254. | OCF₃ | H | CN | A.10 |
| 2255. | CH₂CH₃ | H | CN | A.10 |
| 2256. | H | F | CN | A.10 |
| 2257. | F | F | CN | A.10 |
| 2258. | Cl | F | CN | A.10 |
| 2259. | Br | F | CN | A.10 |
| 2260. | I | F | CN | A.10 |
| 2261. | CN | F | CN | A.10 |
| 2262. | CH₃ | F | CN | A.10 |
| 2263. | CF₃ | F | CN | A.10 |
| 2264. | OCH₃ | F | CN | A.10 |
| 2265. | OCF₃ | F | CN | A.10 |
| 2266. | CH₂CH₃ | F | CN | A.10 |
| 2267. | H | Cl | CN | A.10 |
| 2268. | F | Cl | CN | A.10 |
| 2269. | Cl | Cl | CN | A.10 |
| 2270. | Br | Cl | CN | A.10 |
| 2271. | I | Cl | CN | A.10 |
| 2272. | CN | Cl | CN | A.10 |
| 2273. | CH₃ | Cl | CN | A.10 |
| 2274. | CF₃ | Cl | CN | A.10 |
| 2275. | OCH₃ | Cl | CN | A.10 |
| 2276. | OCF₃ | Cl | CN | A.10 |
| 2277. | CH₂CH₃ | Cl | CN | A.10 |
| 2278. | H | H | CH₃ | A.10 |
| 2279. | F | H | CH₃ | A.10 |
| 2280. | Cl | H | CH₃ | A.10 |
| 2281. | Br | H | CH₃ | A.10 |
| 2282. | I | H | CH₃ | A.10 |
| 2283. | CN | H | CH₃ | A.10 |
| 2284. | CH₃ | H | CH₃ | A.10 |
| 2285. | CF₃ | H | CH₃ | A.10 |
| 2286. | OCH₃ | H | CH₃ | A.10 |
| 2287. | OCF₃ | H | CH₃ | A.10 |
| 2288. | CH₂CH₃ | H | CH₃ | A.10 |
| 2289. | H | F | CH₃ | A.10 |
| 2290. | F | F | CH₃ | A.10 |
| 2291. | Cl | F | CH₃ | A.10 |
| 2292. | Br | F | CH₃ | A.10 |
| 2293. | I | F | CH₃ | A.10 |
| 2294. | CN | F | CH₃ | A.10 |
| 2295. | CH₃ | F | CH₃ | A.10 |
| 2296. | CF₃ | F | CH₃ | A.10 |
| 2297. | OCH₃ | F | CH₃ | A.10 |
| 2298. | OCF₃ | F | CH₃ | A.10 |
| 2299. | CH₂CH₃ | F | CH₃ | A.10 |
| 2300. | H | Cl | CH₃ | A.10 |
| 2301. | F | Cl | CH₃ | A.10 |
| 2302. | Cl | Cl | CH₃ | A.10 |
| 2303. | Br | Cl | CH₃ | A.10 |
| 2304. | I | Cl | CH₃ | A.10 |
| 2305. | CN | Cl | CH₃ | A.10 |
| 2306. | CH₃ | Cl | CH₃ | A.10 |
| 2307. | CF₃ | Cl | CH₃ | A.10 |
| 2308. | OCH₃ | Cl | CH₃ | A.10 |
| 2309. | OCF₃ | Cl | CH₃ | A.10 |
| 2310. | CH₂CH₃ | Cl | CH₃ | A.10 |
| 2311. | H | H | H | A.11 |
| 2312. | F | H | H | A.11 |
| 2313. | Cl | H | H | A.11 |
| 2314. | Br | H | H | A.11 |
| 2315. | I | H | H | A.11 |
| 2316. | CN | H | H | A.11 |
| 2317. | CH₃ | H | H | A.11 |
| 2318. | CF₃ | H | H | A.11 |
| 2319. | OCH₃ | H | H | A.11 |
| 2320. | OCF₃ | H | H | A.11 |
| 2321. | CH₂CH₃ | H | H | A.11 |
| 2322. | H | F | H | A.11 |
| 2323. | F | F | H | A.11 |
| 2324. | Cl | F | H | A.11 |
| 2325. | Br | F | H | A.11 |
| 2326. | I | F | H | A.11 |
| 2327. | CN | F | H | A.11 |
| 2328. | CH₃ | F | H | A.11 |
| 2329. | CF₃ | F | H | A.11 |
| 2330. | OCH₃ | F | H | A.11 |
| 2331. | OCF₃ | F | H | A.11 |
| 2332. | CH₂CH₃ | F | H | A.11 |
| 2333. | H | Cl | H | A.11 |
| 2334. | F | Cl | H | A.11 |
| 2335. | Cl | Cl | H | A.11 |
| 2336. | Br | Cl | H | A.11 |
| 2337. | I | Cl | H | A.11 |
| 2338. | CN | Cl | H | A.11 |
| 2339. | CH₃ | Cl | H | A.11 |
| 2340. | CF₃ | Cl | H | A.11 |
| 2341. | OCH₃ | Cl | H | A.11 |
| 2342. | OCF₃ | Cl | H | A.11 |
| 2343. | CH₂CH₃ | Cl | H | A.11 |
| 2344. | H | H | F | A.11 |
| 2345. | F | H | F | A.11 |
| 2346. | Cl | H | F | A.11 |
| 2347. | Br | H | F | A.11 |
| 2348. | I | H | F | A.11 |
| 2349. | CN | H | F | A.11 |
| 2350. | CH₃ | H | F | A.11 |
| 2351. | CF₃ | H | F | A.11 |
| 2352. | OCH₃ | H | F | A.11 |
| 2353. | OCF₃ | H | F | A.11 |
| 2354. | CH₂CH₃ | H | F | A.11 |
| 2355. | H | F | F | A.11 |
| 2356. | F | F | F | A.11 |
| 2357. | Cl | F | F | A.11 |
| 2358. | Br | F | F | A.11 |
| 2359. | I | F | F | A.11 |
| 2360. | CN | F | F | A.11 |
| 2361. | CH₃ | F | F | A.11 |
| 2362. | CF₃ | F | F | A.11 |
| 2363. | OCH₃ | F | F | A.11 |
| 2364. | OCF₃ | F | F | A.11 |
| 2365. | CH₂CH₃ | F | F | A.11 |
| 2366. | H | Cl | F | A.11 |
| 2367. | F | Cl | F | A.11 |
| 2368. | Cl | Cl | F | A.11 |
| 2369. | Br | Cl | F | A.11 |
| 2370. | I | Cl | F | A.11 |
| 2371. | CN | Cl | F | A.11 |
| 2372. | CH₃ | Cl | F | A.11 |
| 2373. | CF₃ | Cl | F | A.11 |
| 2374. | OCH₃ | Cl | F | A.11 |
| 2375. | OCF₃ | Cl | F | A.11 |
| 2376. | CH₂CH₃ | Cl | F | A.11 |
| 2377. | H | H | Cl | A.11 |
| 2378. | F | H | Cl | A.11 |
| 2379. | Cl | H | Cl | A.11 |
| 2380. | Br | H | Cl | A.11 |
| 2381. | I | H | Cl | A.11 |
| 2382. | CN | H | Cl | A.11 |
| 2383. | CH₃ | H | Cl | A.11 |
| 2384. | CF₃ | H | Cl | A.11 |
| 2385. | OCH₃ | H | Cl | A.11 |
| 2386. | OCF₃ | H | Cl | A.11 |
| 2387. | CH₂CH₃ | H | Cl | A.11 |
| 2388. | H | F | Cl | A.11 |
| 2389. | F | F | Cl | A.11 |
| 2390. | Cl | F | Cl | A.11 |
| 2391. | Br | F | Cl | A.11 |
| 2392. | I | F | Cl | A.11 |
| 2393. | CN | F | Cl | A.11 |
| 2394. | CH₃ | F | Cl | A.11 |
| 2395. | CF₃ | F | Cl | A.11 |
| 2396. | OCH₃ | F | Cl | A.11 |
| 2397. | OCF₃ | F | Cl | A.11 |
| 2398. | CH₂CH₃ | F | Cl | A.11 |
| 2399. | H | Cl | Cl | A.11 |
| 2400. | F | Cl | Cl | A.11 |
| 2401. | Cl | Cl | Cl | A.11 |
| 2402. | Br | Cl | Cl | A.11 |
| 2403. | I | Cl | Cl | A.11 |
| 2404. | CN | Cl | Cl | A.11 |
| 2405. | CH₃ | Cl | Cl | A.11 |
| 2406. | CF₃ | Cl | Cl | A.11 |
| 2407. | OCH₃ | Cl | Cl | A.11 |
| 2408. | OCF₃ | Cl | Cl | A.11 |
| 2409. | CH₂CH₃ | Cl | Cl | A.11 |
| 2410. | H | H | Br | A.11 |
| 2411. | F | H | Br | A.11 |
| 2412. | Cl | H | Br | A.11 |
| 2413. | Br | H | Br | A.11 |
| 2414. | I | H | Br | A.11 |
| 2415. | CN | H | Br | A.11 |
| 2416. | CH₃ | H | Br | A.11 |
| 2417. | CF₃ | H | Br | A.11 |
| 2418. | OCH₃ | H | Br | A.11 |
| 2419. | OCF₃ | H | Br | A.11 |
| 2420. | CH₂CH₃ | H | Br | A.11 |
| 2421. | H | F | Br | A.11 |
| 2422. | F | F | Br | A.11 |
| 2423. | Cl | F | Br | A.11 |
| 2424. | Br | F | Br | A.11 |
| 2425. | I | F | Br | A.11 |
| 2426. | CN | F | Br | A.11 |
| 2427. | CH₃ | F | Br | A.11 |
| 2428. | CF₃ | F | Br | A.11 |
| 2429. | OCH₃ | F | Br | A.11 |
| 2430. | OCF₃ | F | Br | A.11 |
| 2431. | CH₂CH₃ | F | Br | A.11 |
| 2432. | H | Cl | Br | A.11 |
| 2433. | F | Cl | Br | A.11 |
| 2434. | Cl | Cl | Br | A.11 |
| 2435. | Br | Cl | Br | A.11 |
| 2436. | I | Cl | Br | A.11 |
| 2437. | CN | Cl | Br | A.11 |
| 2438. | CH₃ | Cl | Br | A.11 |
| 2439. | CF₃ | Cl | Br | A.11 |
| 2440. | OCH₃ | Cl | Br | A.11 |
| 2441. | OCF₃ | Cl | Br | A.11 |
| 2442. | CH₂CH₃ | Cl | Br | A.11 |
| 2443. | H | H | I | A.11 |
| 2444. | F | H | I | A.11 |
| 2445. | Cl | H | I | A.11 |
| 2446. | Br | H | I | A.11 |
| 2447. | I | H | I | A.11 |
| 2448. | CN | H | I | A.11 |
| 2449. | CH₃ | H | I | A.11 |
| 2450. | CF₃ | H | I | A.11 |
| 2451. | OCH₃ | H | I | A.11 |
| 2452. | OCF₃ | H | I | A.11 |
| 2453. | CH₂CH₃ | H | I | A.11 |
| 2454. | H | F | I | A.11 |
| 2455. | F | F | I | A.11 |
| 2456. | Cl | F | I | A.11 |
| 2457. | Br | F | I | A.11 |
| 2458. | I | F | I | A.11 |
| 2459. | CN | F | I | A.11 |
| 2460. | CH₃ | F | I | A.11 |
| 2461. | CF₃ | F | I | A.11 |
| 2462. | OCH₃ | F | I | A.11 |
| 2463. | OCF₃ | F | I | A.11 |
| 2464. | CH₂CH₃ | F | I | A.11 |
| 2465. | H | Cl | I | A.11 |
| 2466. | F | Cl | I | A.11 |
| 2467. | Cl | Cl | I | A.11 |
| 2468. | Br | Cl | I | A.11 |
| 2469. | I | Cl | I | A.11 |
| 2470. | CN | Cl | I | A.11 |
| 2471. | CH₃ | Cl | I | A.11 |
| 2472. | CF₃ | Cl | I | A.11 |
| 2473. | OCH₃ | Cl | I | A.11 |
| 2474. | OCF₃ | Cl | I | A.11 |
| 2475. | CH₂CH₃ | Cl | I | A.11 |
| 2476. | H | H | CN | A.11 |
| 2477. | F | H | CN | A.11 |
| 2478. | Cl | H | CN | A.11 |
| 2479. | Br | H | CN | A.11 |
| 2480. | I | H | CN | A.11 |
| 2481. | CN | H | CN | A.11 |
| 2482. | CH₃ | H | CN | A.11 |
| 2483. | CF₃ | H | CN | A.11 |
| 2484. | OCH₃ | H | CN | A.11 |
| 2485. | OCF₃ | H | CN | A.11 |
| 2486. | CH₂CH₃ | H | CN | A.11 |
| 2487. | H | F | CN | A.11 |
| 2488. | F | F | CN | A.11 |
| 2489. | Cl | F | CN | A.11 |
| 2490. | Br | F | CN | A.11 |
| 2491. | I | F | CN | A.11 |
| 2492. | CN | F | CN | A.11 |
| 2493. | CH₃ | F | CN | A.11 |
| 2494. | CF₃ | F | CN | A.11 |
| 2495. | OCH₃ | F | CN | A.11 |
| 2496. | OCF₃ | F | CN | A.11 |
| 2497. | CH₂CH₃ | F | CN | A.11 |
| 2498. | H | Cl | CN | A.11 |
| 2499. | F | Cl | CN | A.11 |
| 2500. | Cl | Cl | CN | A.11 |
| 2501. | Br | Cl | CN | A.11 |
| 2502. | I | Cl | CN | A.11 |
| 2503. | CN | Cl | CN | A.11 |
| 2504. | CH₃ | Cl | CN | A.11 |
| 2505. | CF₃ | Cl | CN | A.11 |
| 2506. | OCH₃ | Cl | CN | A.11 |
| 2507. | OCF₃ | Cl | CN | A.11 |
| 2508. | CH₂CH₃ | Cl | CN | A.11 |
| 2509. | H | H | CH₃ | A.11 |
| 2510. | F | H | CH₃ | A.11 |
| 2511. | Cl | H | CH₃ | A.11 |
| 2512. | Br | H | CH₃ | A.11 |
| 2513. | I | H | CH₃ | A.11 |
| 2514. | CN | H | CH₃ | A.11 |
| 2515. | CH₃ | H | CH₃ | A.11 |
| 2516. | CF₃ | H | CH₃ | A.11 |
| 2517. | OCH₃ | H | CH₃ | A.11 |
| 2518. | OCF₃ | H | CH₃ | A.11 |
| 2519. | CH₂CH₃ | H | CH₃ | A.11 |
| 2520. | H | F | CH₃ | A.11 |
| 2521. | F | F | CH₃ | A.11 |
| 2522. | Cl | F | CH₃ | A.11 |
| 2523. | Br | F | CH₃ | A.11 |
| 2524. | I | F | CH₃ | A.11 |
| 2525. | CN | F | CH₃ | A.11 |
| 2526. | CH₃ | F | CH₃ | A.11 |
| 2527. | CF₃ | F | CH₃ | A.11 |
| 2528. | OCH₃ | F | CH₃ | A.11 |
| 2529. | OCF₃ | F | CH₃ | A.11 |
| 2530. | CH₂CH₃ | F | CH₃ | A.11 |
| 2531. | H | Cl | CH₃ | A.11 |
| 2532. | F | Cl | CH₃ | A.11 |
| 2533. | Cl | Cl | CH₃ | A.11 |
| 2534. | Br | Cl | CH₃ | A.11 |
| 2535. | I | Cl | CH₃ | A.11 |
| 2536. | CN | Cl | CH₃ | A.11 |
| 2537. | CH₃ | Cl | CH₃ | A.11 |
| 2538. | CF₃ | Cl | CH₃ | A.11 |
| 2539. | OCH₃ | Cl | CH₃ | A.11 |
| 2540. | OCF₃ | Cl | CH₃ | A.11 |
| 2541. | CH₂CH₃ | Cl | CH₃ | A.11 |

| | | | | |
|---|---|---|---|---|
| *⁾ the variables A.1 to A.11 representing W in Table 1 above have the following meanings: | | | | |

| | |
|---|---|
| A.1 | 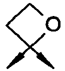 |
| A.2 | 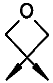 |
| A.3 | 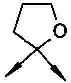 |
| A.4 | 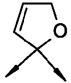 |
| A.5 | 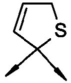 |
| A.6 | 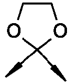 |
| A.7 | 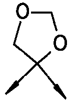 |
| A.8 | 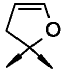 |
| A.9 | 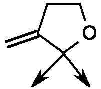 |
| A.10 | 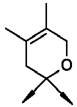 |
| A.11 | 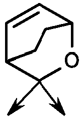 |

Particular preference is also given to the following compounds I.1 to I.118, which are compounds of formula (I), wherein the substituents R¹, R², R⁶ and R⁹ are all hydrogen.

Compounds of formula I.1., wherein R³, R⁴, R⁵ and R⁷, R⁸ (forming W together with the carbon atom to which they are bound) have the meanings as defined lines in 1 to 2541 of Table 1 above, i.e. individual compounds I.1.1 - I.1.2541, are particularly preferred:

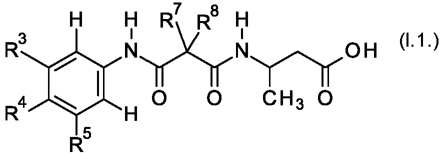

Compounds of formula I.2., wherein R³, R⁴, R⁵ and R⁷, R⁸ (forming W together with the carbon atom to which they are bound) have the meanings as defined lines in 1 to 2541 of Table 1 above, i.e. individual compounds I.2.1 - I.2.2541, are particularly preferred:

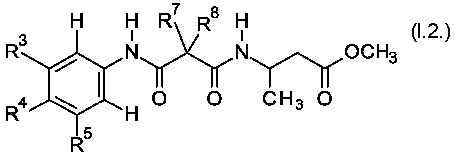

Compounds of formula I.3., wherein R³, R⁴, R⁵ and R⁷, R⁸ (forming W together with the carbon atom to which they are bound) have the meanings as defined lines in 1 to 2541 of Table 1 above, i.e. individual compounds I.3.1 - I.3.2541, are particularly preferred:

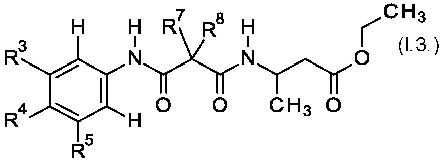

Compounds of formula I.4., wherein R³, R⁴, R⁵ and R⁷, R⁸ (forming W together with the carbon atom to which they are bound) have the meanings as defined lines in 1 to 2541 of Table 1 above, i.e. individual compounds I.4.1 - I.4.2541, are particularly preferred:

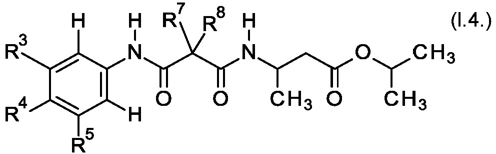

Compounds of formula I.5., wherein R³, R⁴, R⁵ and R⁷, R⁸ (forming W together with the carbon atom to which they are bound) have the meanings as defined lines in 1 to 2541 of Table 1 above, i.e. individual compounds I.5.1 - I.5.2541, are particularly preferred:

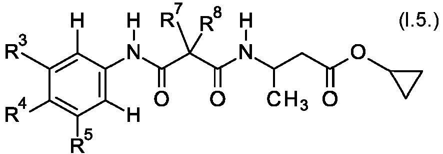

Compounds of formula I.6., wherein R³, R⁴, R⁵ and R⁷, R⁸ (forming W together with the carbon atom to which they are bound) have the meanings as defined lines in 1 to 2541 of Table 1 above, i.e. individual compounds I.6.1 - I.6.2541, are particularly preferred:

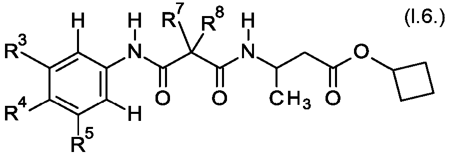

Compounds of formula I.7., wherein R³, R⁴, R⁵ and R⁷, R⁸ (forming W together with the carbon atom to which they are bound) have the meanings as defined lines in 1 to 2541 of Table 1 above, i.e. individual compounds I.7.1 - I.7.2541, are particularly preferred:

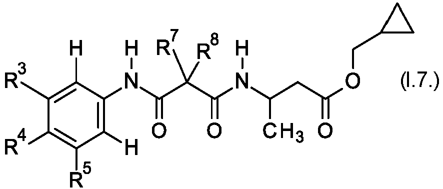

Compounds of formula I.8., wherein R³, R⁴, R⁵ and R⁷, R⁸ (forming W together with the carbon atom to which they are bound) have the meanings as defined lines in 1 to 2541 of Table 1 above, i.e. individual compounds I.8.1 - I.8.2541, are particularly preferred:

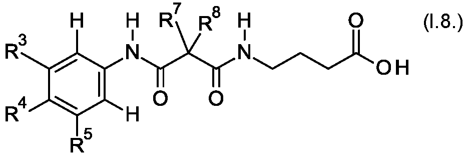

Compounds of formula I.9., wherein R³, R⁴, R⁵ and R⁷, R⁸ (forming W together with the carbon atom to which they are bound) have the meanings as defined lines in 1 to 2541 of Table 1 above, i.e. individual compounds I.9.1 - I.9.2541, are particularly preferred:

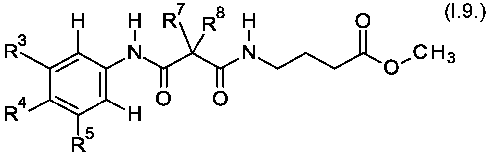

Compounds of formula I.10., wherein R³, R⁴, R⁵ and R⁷, R⁸ (forming W together with the carbon atom to which they are bound) have the meanings as defined lines in 1 to 2541 of Table 1 above, i.e. individual compounds I.10.1 - I.10.2541, are particularly preferred:

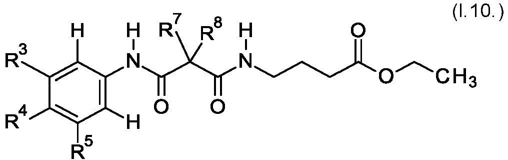

Compounds of formula I.11., wherein R³, R⁴, R⁵ and R⁷, R⁸ (forming W together with the carbon atom to which they are bound) have the meanings as defined lines in 1 to 2541 of Table 1 above, i.e. individual compounds I.11.1 - I.11.2541, are particularly preferred:

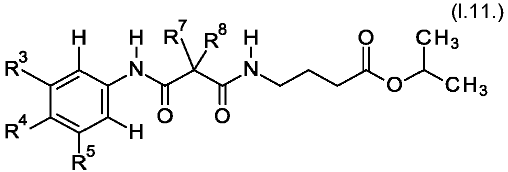

Compounds of formula I.12., wherein R³, R⁴, R⁵ and R⁷, R⁸ (forming W together with the carbon atom to which they are bound) have the meanings as defined lines in 1 to 2541 of Table 1 above, i.e. individual compounds I.12.1 - I.12.2541, are particularly preferred:

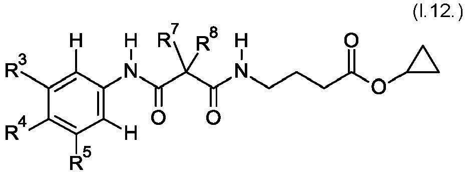

Compounds of formula I.13., wherein R³, R⁴, R⁵ and R⁷, R⁸ (forming W together with the carbon atom to which they are bound) have the meanings as defined lines in 1 to 2541 of Table 1 above, i.e. individual compounds I.13.1 - I.13.2541, are particularly preferred:

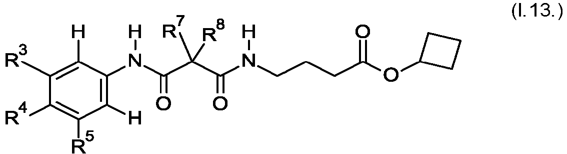

Compounds of formula I.14., wherein R³, R⁴, R⁵ and R⁷, R⁸ (forming W together with the carbon atom to which they are bound) have the meanings as defined lines in 1 to 2541 of Table 1 above, i.e. individual compounds I.14.1 - I.14.2541, are particularly preferred:

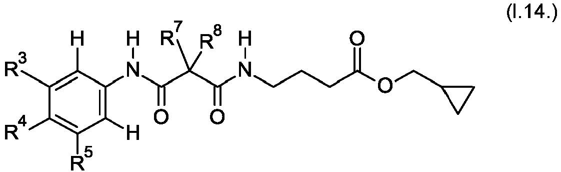

Compounds of formula I.15., wherein R³, R⁴, R⁵ and R⁷, R⁸ (forming W together with the carbon atom to which they are bound) have the meanings as defined lines in 1 to 2541 of Table 1 above, i.e. individual compounds I.15.1 - I.15.2541, are particularly preferred:

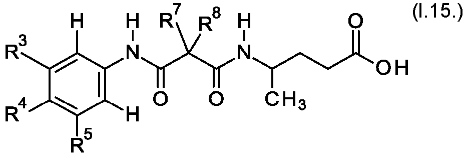

Compounds of formula I.16., wherein R³, R⁴, R⁵ and R⁷, R⁸ (forming W together with the carbon atom to which they are bound) have the meanings as defined lines in 1 to 2541 of Table 1 above, i.e. individual compounds I.16.1 - I.16.2541, are particularly preferred:

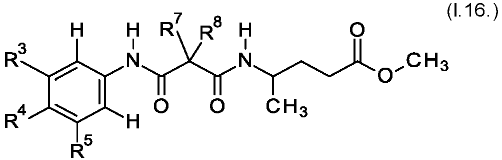

Compounds of formula I.17., wherein R³, R⁴, R⁵ and R⁷, R⁸ (forming W together with the carbon atom to which they are bound) have the meanings as defined lines in 1 to 2541 of Table 1 above, i.e. individual compounds I.17.1 - I.17.2541, are particularly preferred:

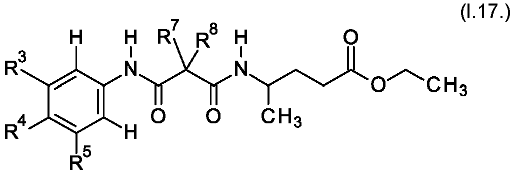

Compounds of formula I.18., wherein R³, R⁴, R⁵ and R⁷, R⁸ (forming W together with the carbon atom to which they are bound) have the meanings as defined lines in 1 to 2541 of Table 1 above, i.e. individual compounds I.18.1 - I.18.2541, are particularly preferred:

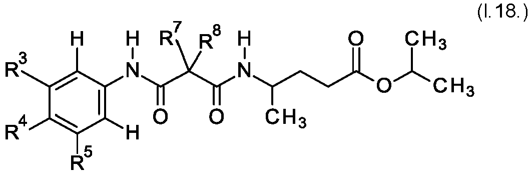

Compounds of formula I.19., wherein R³, R⁴, R⁵ and R⁷, R⁸ (forming W together with the carbon atom to which they are bound) have the meanings as defined lines in 1 to 2541 of Table 1 above, i.e. individual compounds I.19.1 - I.19.2541, are particularly preferred:

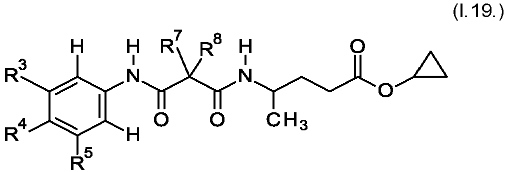

Compounds of formula I.20., wherein R³, R⁴, R⁵ and R⁷, R⁸ (forming W together with the carbon atom to which they are bound) have the meanings as defined lines in 1 to 2541 of Table 1 above, i.e. individual compounds I.20.1 - I.20.2541, are particularly preferred:

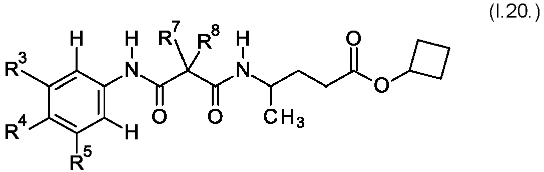

Compounds of formula I.21., wherein R³, R⁴, R⁵ and R⁷, R⁸ (forming W together with the carbon atom to which they are bound) have the meanings as defined lines in 1 to 2541 of Table 1 above, i.e. individual compounds I.21.1 - I.21.2541, are particularly preferred:

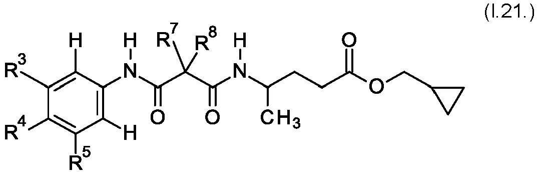

Compounds of formula I.22., wherein R³, R⁴, R⁵ and R⁷, R⁸ (forming W together with the carbon atom to which they are bound) have the meanings as defined lines in 1 to 2541 of Table 1 above, i.e. individual compounds I.22.1 - I.22.2541, are particularly preferred:

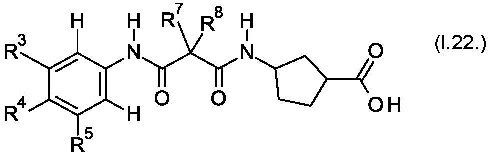

Compounds of formula I.23., wherein R³, R⁴, R⁵ and R⁷, R⁸ (forming W together with the carbon atom to which they are bound) have the meanings as defined lines in 1 to 2541 of Table 1 above, i.e. individual compounds I.23.1 - I.23.2541, are particularly preferred:

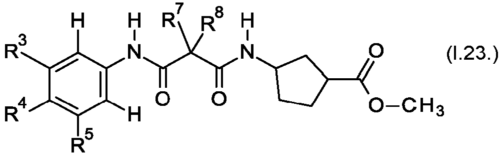

Compounds of formula I.24., wherein R³, R⁴, R⁵ and R⁷, R⁸ (forming W together with the carbon atom to which they are bound) have the meanings as defined lines in 1 to 2541 of Table 1 above, i.e. individual compounds I.24.1 - I.24.2541, are particularly preferred:

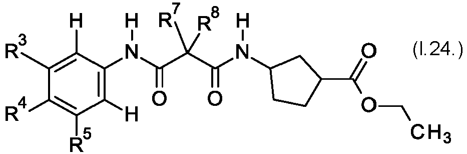

Compounds of formula I.25., wherein R³, R⁴, R⁵ and R⁷, R⁸ (forming W together with the carbon atom to which they are bound) have the meanings as defined lines in 1 to 2541 of Table 1 above, i.e. individual compounds I.25.1 - I.25.2541, are particularly preferred:

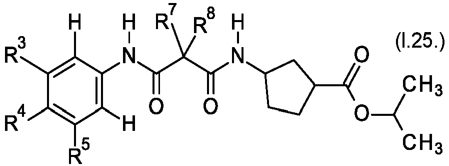

Compounds of formula I.26., wherein R³, R⁴, R⁵ and R⁷, R⁸ (forming W together with the carbon atom to which they are bound) have the meanings as defined lines in 1 to 2541 of Table 1 above, i.e. individual compounds I.26.1 - I.26.2541, are particularly preferred:

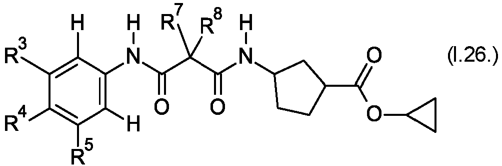

Compounds of formula I.27., wherein R³, R⁴, R⁵ and R⁷, R⁸ (forming W together with the carbon atom to which they are bound) have the meanings as defined lines in 1 to 2541 of Table 1 above, i.e. individual compounds I.27.1 - I.27.2541, are particularly preferred:

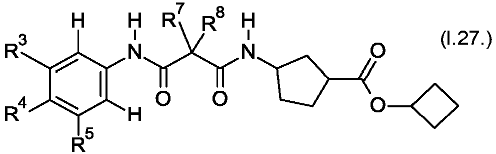

Compounds of formula I.28., wherein R³, R⁴, R⁵ and R⁷, R⁸ (forming W together with the carbon atom to which they are bound) have the meanings as defined lines in 1 to 2541 of Table 1 above, i.e. individual compounds I.28.1 - I.28.2541, are particularly preferred:

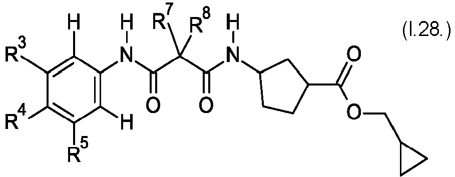

Compounds of formula I.29., wherein R³, R⁴, R⁵ and R⁷, R⁸ (forming W together with the carbon atom to which they are bound) have the meanings as defined lines in 1 to 2541 of Table 1 above, i.e. individual compounds I.29.1 - I.29.2541, are particularly preferred:

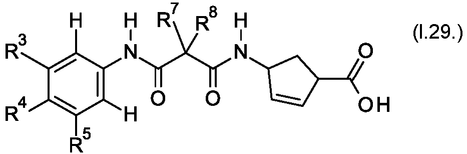

Compounds of formula I.30., wherein R³, R⁴, R⁵ and R⁷, R⁸ (forming W together with the carbon atom to which they are bound) have the meanings as defined lines in 1 to 2541 of Table 1 above, i.e. individual compounds I.30.1 - I.30.2541, are particularly preferred:

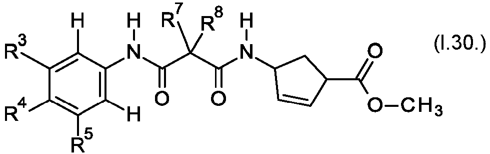

Compounds of formula I.31., wherein R³, R⁴, R⁵ and R⁷, R⁸ (forming W together with the carbon atom to which they are bound) have the meanings as defined lines in 1 to 2541 of Table 1 above, i.e. individual compounds I.31.1 - I.31.2541, are particularly preferred:

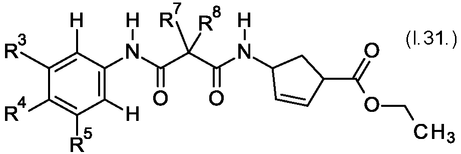

Compounds of formula I.32., wherein R³, R⁴, R⁵ and R⁷, R⁸ (forming W together with the carbon atom to which they are bound) have the meanings as defined lines in 1 to 2541 of Table 1 above, i.e. individual compounds I.32.1 - I.32.2541, are particularly preferred:

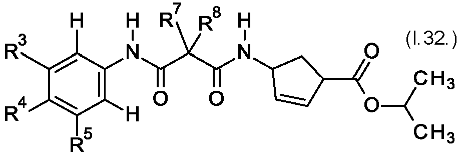

Compounds of formula I.33., wherein R³, R⁴, R⁵ and R⁷, R⁸ (forming W together with the carbon atom to which they are bound) have the meanings as defined lines in 1 to 2541 of Table 1 above, i.e. individual compounds I.33.1 - I.33.2541, are particularly preferred:

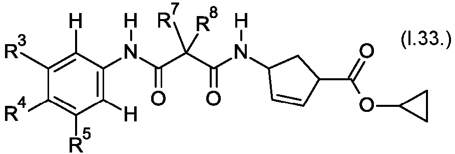

Compounds of formula I.34., wherein R³, R⁴, R⁵ and R⁷, R⁸ (forming W together with the carbon atom to which they are bound) have the meanings as defined lines in 1 to 2541 of Table 1 above, i.e. individual compounds I.34.1 - I.34.2541, are particularly preferred:

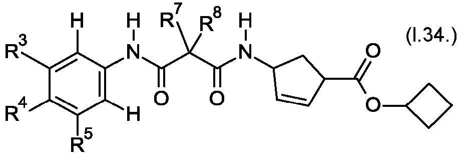

Compounds of formula I.35., wherein R³, R⁴, R⁵ and R⁷, R⁸ (forming W together with the carbon atom to which they are bound) have the meanings as defined lines in 1 to 2541 of Table 1 above, i.e. individual compounds I.35.1 - I.35.2541, are particularly preferred:

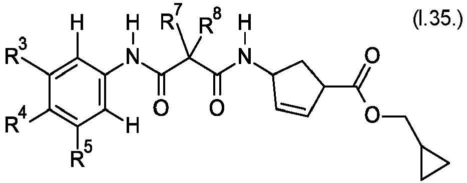

Compounds of formula I.36., wherein R³, R⁴, R⁵ and R⁷, R⁸ (forming W together with the carbon atom to which they are bound) have the meanings as defined lines in 1 to 2541 of Table 1 above, i.e. individual compounds I.36.1 - I.36.2541, are particularly preferred:

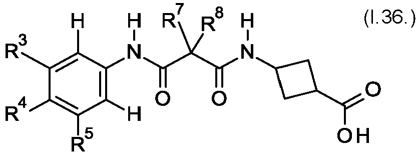

Compounds of formula I.37., wherein R³, R⁴, R⁵ and R⁷, R⁸ (forming W together with the carbon atom to which they are bound) have the meanings as defined lines in 1 to 2541 of Table 1 above, i.e. individual compounds I.37.1 - I.37.2541, are particularly preferred:

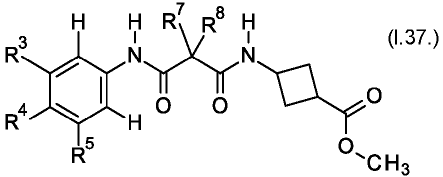

Compounds of formula I.38., wherein R³, R⁴, R⁵ and R⁷, R⁸ (forming W together with the carbon atom to which they are bound) have the meanings as defined lines in 1 to 2541 of Table 1 above, i.e. individual compounds I.38.1 - I.38.2541, are particularly preferred:

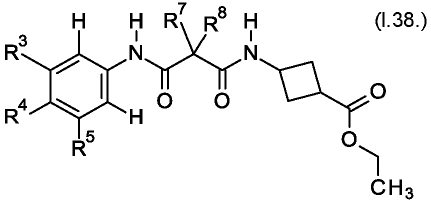

Compounds of formula I.39., wherein R³, R⁴, R⁵ and R⁷, R⁸ (forming W together with the carbon atom to which they are bound) have the meanings as defined lines in 1 to 2541 of Table 1 above, i.e. individual compounds I.39.1 - I.39.2541, are particularly preferred:

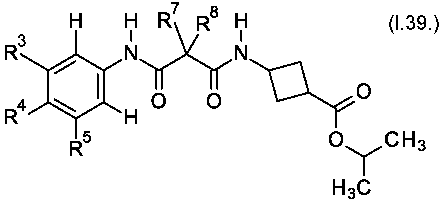

Compounds of formula I.40., wherein R³, R⁴, R⁵ and R⁷, R⁸ (forming W together with the carbon atom to which they are bound) have the meanings as defined lines in 1 to 2541 of Table 1 above, i.e. individual compounds I.40.1 - I.40.2541, are particularly preferred:

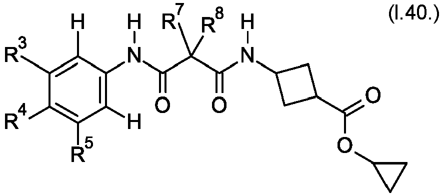

Compounds of formula I.41., wherein R³, R⁴, R⁵ and R⁷, R⁸ (forming W together with the carbon atom to which they are bound) have the meanings as defined lines in 1 to 2541 of Table 1 above, i.e. individual compounds I.41.1 - I.41.2541, are particularly preferred:

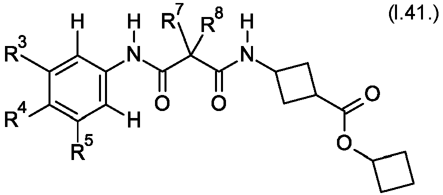

Compounds of formula I.42., wherein R³, R⁴, R⁵ and R⁷, R⁸ (forming W together with the carbon atom to which they are bound) have the meanings as defined lines in 1 to 2541 of Table 1 above, i.e. individual compounds I.42.1 - I.42.2541, are particularly preferred:

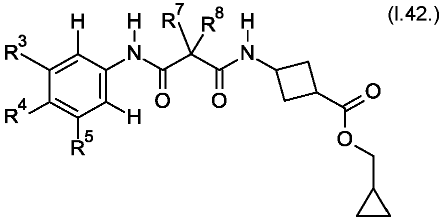

Compounds of formula I.43., wherein R³, R⁴, R⁵ and R⁷, R⁸ (forming W together with the carbon atom to which they are bound) have the meanings as defined lines in 1 to 2541 of Table 1 above, i.e. individual compounds I.43.1 - I.43.2541, are particularly preferred:

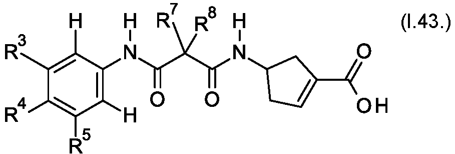

Compounds of formula I.44., wherein R³, R⁴, R⁵ and R⁷, R⁸ (forming W together with the carbon atom to which they are bound) have the meanings as defined lines in 1 to 2541 of Table 1 above, i.e. individual compounds I.44.1 - I.44.2541, are particularly preferred:

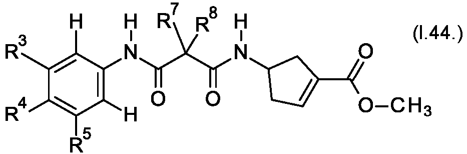

Compounds of formula I.45., wherein R³, R⁴, R⁵ and R⁷, R⁸ (forming W together with the carbon atom to which they are bound) have the meanings as defined lines in 1 to 2541 of Table 1 above, i.e. individual compounds I.45.1 - I.45.2541, are particularly preferred:

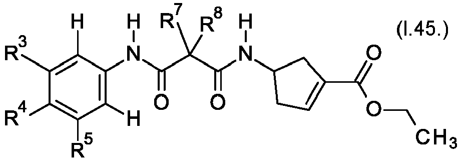

Compounds of formula I.46., wherein R³, R⁴, R⁵ and R⁷, R⁸ (forming W together with the carbon atom to which they are bound) have the meanings as defined lines in 1 to 2541 of Table 1 above, i.e. individual compounds I.46.1 - I.46.2541, are particularly preferred:

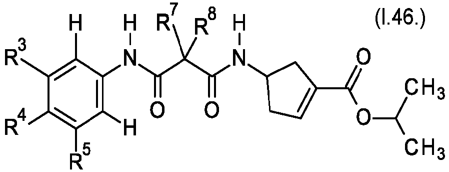

Compounds of formula I.47., wherein R³, R⁴, R⁵ and R⁷, R⁸ (forming W together with the carbon atom to which they are bound) have the meanings as defined lines in 1 to 2541 of Table 1 above, i.e. individual compounds I.47.1 - I.47.2541, are particularly preferred:

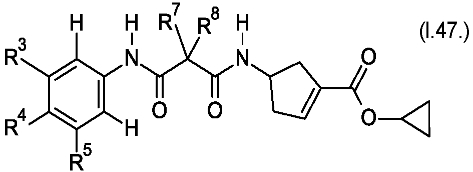

Compounds of formula I.48., wherein R³, R⁴, R⁵ and R⁷, R⁸ (forming W together with the carbon atom to which they are bound) have the meanings as defined lines in 1 to 2541 of Table 1 above, i.e. individual compounds I.48.1 - I.48.2541, are particularly preferred:

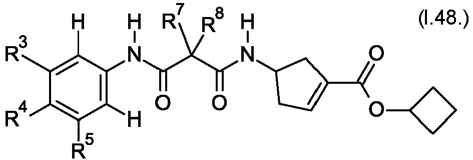

Compounds of formula I.49., wherein R³, R⁴, R⁵ and R⁷, R⁸ (forming W together with the carbon atom to which they are bound) have the meanings as defined lines in 1 to 2541 of Table 1 above, i.e. individual compounds I.49.1 - I.49.2541, are particularly preferred:

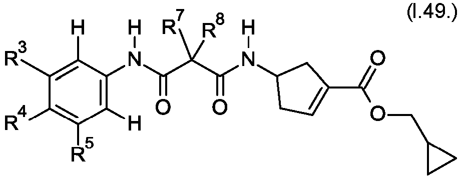

Compounds of formula I.50., wherein R³, R⁴, R⁵ and R⁷, R⁸ (forming W together with the carbon atom to which they are bound) have the meanings as defined lines in 1 to 2541 of Table 1 above, i.e. individual compounds I.50.1 - I.50.2541, are particularly preferred:

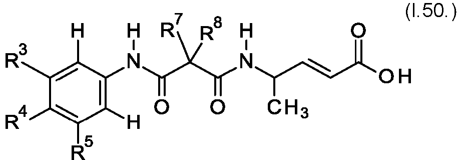

Compounds of formula I.51., wherein R³, R⁴, R⁵ and R⁷, R⁸ (forming W together with the carbon atom to which they are bound) have the meanings as defined lines in 1 to 2541 of Table 1 above, i.e. individual compounds I.51.1 - I.51.2541, are particularly preferred:

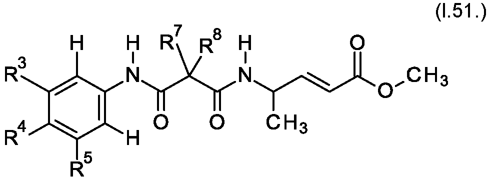

Compounds of formula I.52., wherein R³, R⁴, R⁵ and R⁷, R⁸ (forming W together with the carbon atom to which they are bound) have the meanings as defined lines in 1 to 2541 of Table 1 above, i.e. individual compounds I.52.1 - I.52.2541, are particularly preferred:

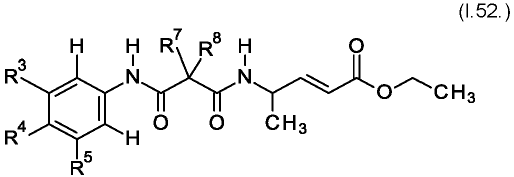

Compounds of formula I.53., wherein R³, R⁴, R⁵ and R⁷, R⁸ (forming W together with the carbon atom to which they are bound) have the meanings as defined lines in 1 to 2541 of Table 1 above, i.e. individual compounds I.53.1 - I.53.2541, are particularly preferred:

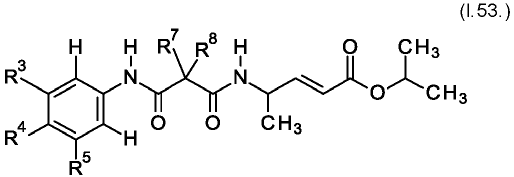

Compounds of formula I.54., wherein R³, R⁴, R⁵ and R⁷, R⁸ (forming W together with the carbon atom to which they are bound) have the meanings as defined lines in 1 to 2541 of Table 1 above, i.e. individual compounds I.54.1 - I.54.2541, are particularly preferred:

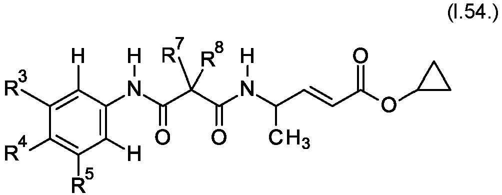

Compounds of formula I.55., wherein R³, R⁴, R⁵ and R⁷, R⁸ (forming W together with the carbon atom to which they are bound) have the meanings as defined lines in 1 to 2541 of Table 1 above, i.e. individual compounds I.55.1 - I.55.2541, are particularly preferred:

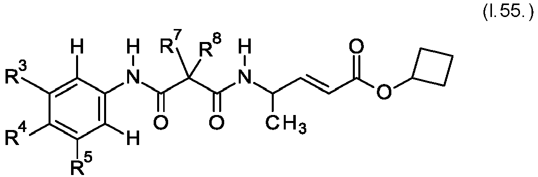

Compounds of formula I.56., wherein R³, R⁴, R⁵ and R⁷, R⁸ (forming W together with the carbon atom to which they are bound) have the meanings as defined lines in 1 to 2541 of Table 1 above, i.e. individual compounds I.56.1 - I.56.2541, are particularly preferred:

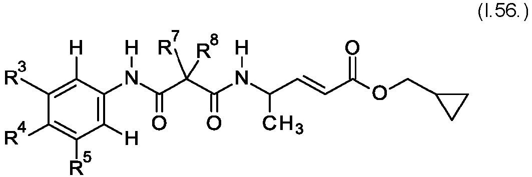

Compounds of formula I.57., wherein R³, R⁴, R⁵ and R⁷, R⁸ (forming W together with the carbon atom to which they are bound) have the meanings as defined lines in 1 to 2541 of Table 1 above, i.e. individual compounds I.57.1 - I.57.2541, are particularly preferred:

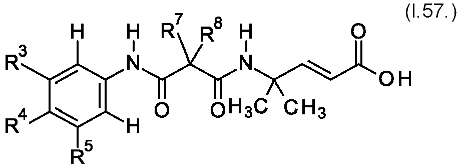

Compounds of formula I.58., wherein R³, R⁴, R⁵ and R⁷, R⁸ (forming W together with the carbon atom to which they are bound) have the meanings as defined lines in 1 to 2541 of Table 1 above, i.e. individual compounds I.58.1 - I.58.2541, are particularly preferred:

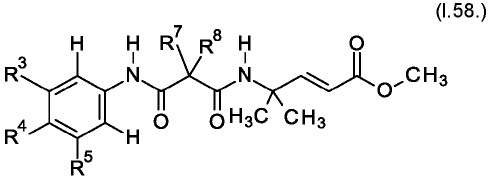

Compounds of formula I.59., wherein R³, R⁴, R⁵ and R⁷, R⁸ (forming W together with the carbon atom to which they are bound) have the meanings as defined lines in 1 to 2541 of Table 1 above, i.e. individual compounds I.59.1 - I.59.2541, are particularly preferred:

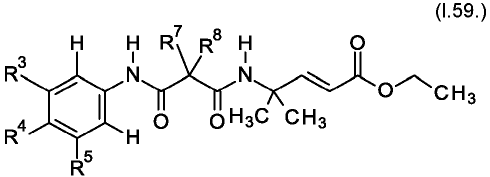

Compounds of formula I.60., wherein R³, R⁴, R⁵ and R⁷, R⁸ (forming W together with the carbon atom to which they are bound) have the meanings as defined lines in 1 to 2541 of Table 1 above, i.e. individual compounds I.60.1 - I.60.2541, are particularly preferred:

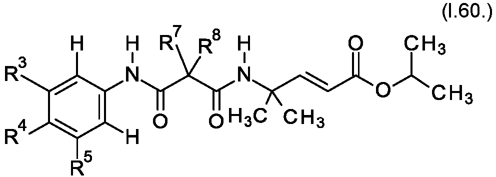

Compounds of formula I.61., wherein R³, R⁴, R⁵ and R⁷, R⁸ (forming W together with the carbon atom to which they are bound) have the meanings as defined lines in 1 to 2541 of Table 1 above, i.e. individual compounds I.61.1 - I.61.2541, are particularly preferred:

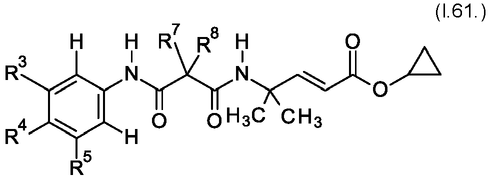

Compounds of formula I.62., wherein R³, R⁴, R⁵ and R⁷, R⁸ (forming W together with the carbon atom to which they are bound) have the meanings as defined lines in 1 to 2541 of Table 1 above, i.e. individual compounds I.62.1 - I.62.2541, are particularly preferred:

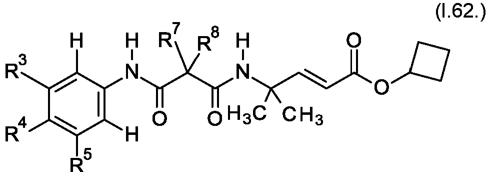

Compounds of formula I.63., wherein R³, R⁴, R⁵ and R⁷, R⁸ (forming W together with the carbon atom to which they are bound) have the meanings as defined lines in 1 to 2541 of Table 1 above, i.e. individual compounds I.63.1 - I.63.2541, are particularly preferred:

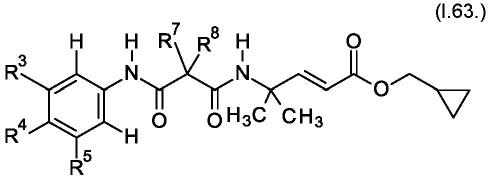

Compounds of formula I.64., wherein R³, R⁴, R⁵ and R⁷, R⁸ (forming W together with the carbon atom to which they are bound) have the meanings as defined lines in 1 to 2541 of Table 1 above, i.e. individual compounds I.64.1 - I.64.2541, are particularly preferred:

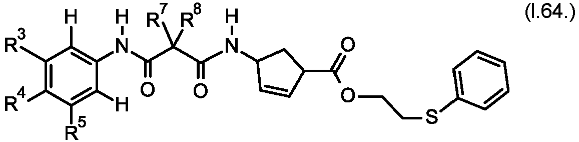

Compounds of formula I.65., wherein R³, R⁴, R⁵ and R⁷, R⁸ (forming W together with the carbon atom to which they are bound) have the meanings as defined lines in 1 to 2541 of Table 1 above, i.e. individual compounds I.65.1 - I.65.2541, are particularly preferred:

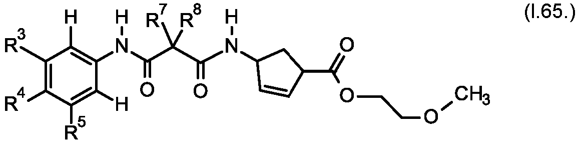

Compounds of formula I.66., wherein R³, R⁴, R⁵ and R⁷, R⁸ (forming W together with the carbon atom to which they are bound) have the meanings as defined lines in 1 to 2541 of Table 1 above, i.e. individual compounds I.66.1 - I.66.2541, are particularly preferred:

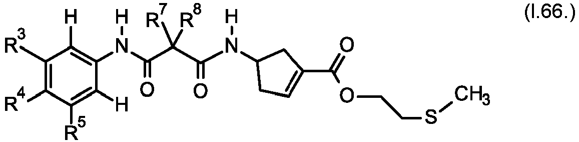

Compounds of formula I.67., wherein R³, R⁴, R⁵ and R⁷, R⁸ (forming W together with the carbon atom to which they are bound) have the meanings as defined lines in 1 to 2541 of Table 1 above, i.e. individual compounds I.67.1 - I.67.2541, are particularly preferred:

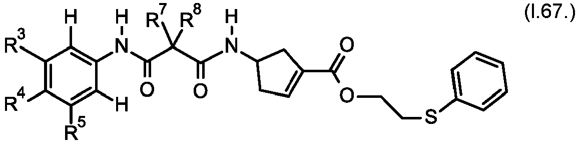

Compounds of formula I.68., wherein R³, R⁴, R⁵ and R⁷, R⁸ (forming W together with the carbon atom to which they are bound) have the meanings as defined lines in 1 to 2541 of Table 1 above, i.e. individual compounds I.68.1 - I.68.2541, are particularly preferred:

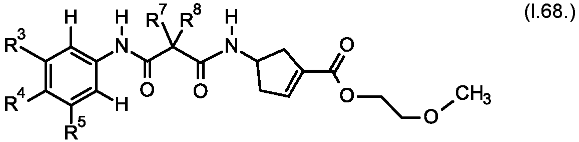

Compounds of formula I.69., wherein R³, R⁴, R⁵ and R⁷, R⁸ (forming W together with the carbon atom to which they are bound) have the meanings as defined lines in 1 to 2541 of Table 1 above, i.e. individual compounds I.69.1 - I.69.2541, are particularly preferred:

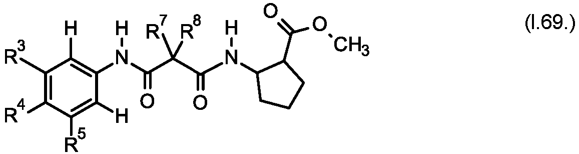

Compounds of formula I.70., wherein R³, R⁴, R⁵ and R⁷, R⁸ (forming W together with the carbon atom to which they are bound) have the meanings as defined lines in 1 to 2541 of Table 1 above, i.e. individual compounds I.70.1 - I.70.2541, are particularly preferred:

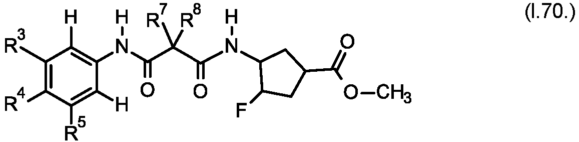

Compounds of formula I.71., wherein R³, R⁴, R⁵ and R⁷, R⁸ (forming W together with the carbon atom to which they are bound) have the meanings as defined lines in 1 to 2541 of Table 1 above, i.e. individual compounds I.71.1 - I.71.2541, are particularly preferred:

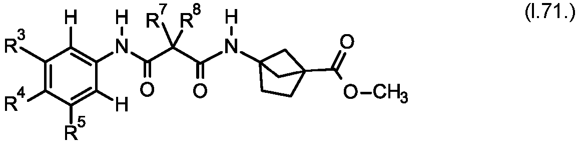

Compounds of formula I.72., wherein R³, R⁴, R⁵ and R⁷, R⁸ (forming W together with the carbon atom to which they are bound) have the meanings as defined lines in 1 to 2541 of Table 1 above, i.e. individual compounds I.72.1 - I.72.2541, are particularly preferred:

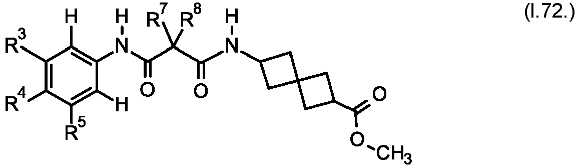

Compounds of formula I.73., wherein R³, R⁴, R⁵ and R⁷, R⁸ (forming W together with the carbon atom to which they are bound) have the meanings as defined lines in 1 to 2541 of Table 1 above, i.e. individual compounds I.73.1 - I.73.2541, are particularly preferred:

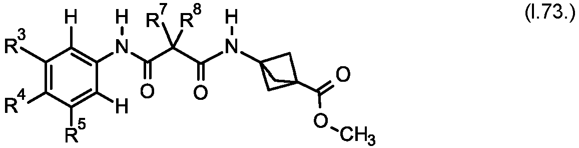

Compounds of formula I.74., wherein R³, R⁴, R⁵ and R⁷, R⁸ (forming W together with the carbon atom to which they are bound) have the meanings as defined lines in 1 to 2541 of Table 1 above, i.e. individual compounds I.74.1 - I.74.2541, are particularly preferred:

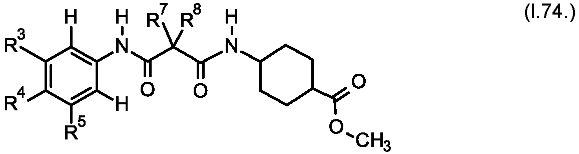

Compounds of formula I.75., wherein R³, R⁴, R⁵ and R⁷, R⁸ (forming W together with the carbon atom to which they are bound) have the meanings as defined lines in 1 to 2541 of Table 1 above, i.e. individual compounds I.75.1 - I.75.2541, are particularly preferred:

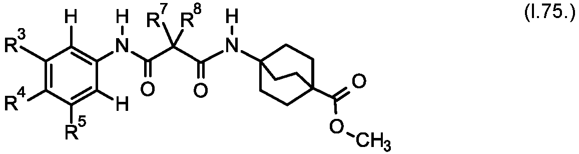

Compounds of formula I.76., wherein R³, R⁴, R⁵ and R⁷, R⁸ (forming W together with the carbon atom to which they are bound) have the meanings as defined lines in 1 to 2541 of Table 1 above, i.e. individual compounds I.76.1 - I.76.2541, are particularly preferred:

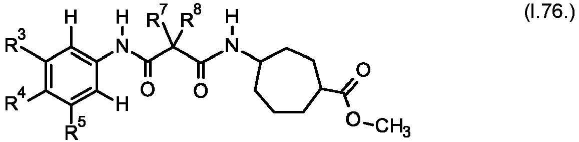

Compounds of formula I.77., wherein R³, R⁴, R⁵ and R⁷, R⁸ (forming W together with the carbon atom to which they are bound) have the meanings as defined lines in 1 to 2541 of Table 1 above, i.e. individual compounds I.77.1 - I.77.2541, are particularly preferred:

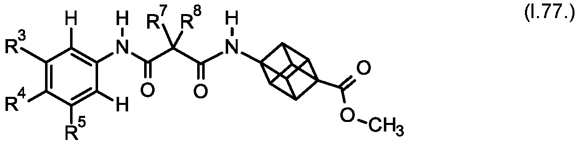

Compounds of formula I.78., wherein R³, R⁴, R⁵ and R⁷, R⁸ (forming W together with the carbon atom to which they are bound) have the meanings as defined lines in 1 to 2541 of Table 1 above, i.e. individual compounds I.78.1 - I.78.2541, are particularly preferred:

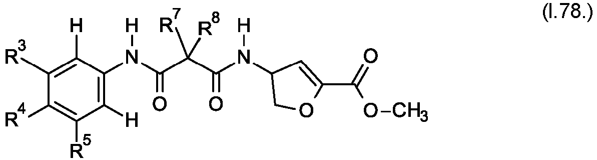

Compounds of formula I.79., wherein R³, R⁴, R⁵ and R⁷, R⁸ (forming W together with the carbon atom to which they are bound) have the meanings as defined lines in 1 to 2541 of Table 1 above, i.e. individual compounds I.79.1 - I.79.2541, are particularly preferred:

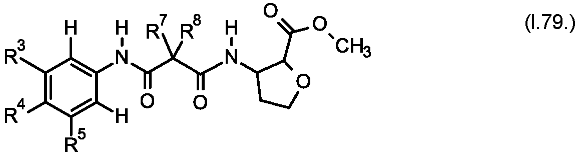

Compounds of formula I.80., wherein R³, R⁴, R⁵ and R⁷, R⁸ (forming W together with the carbon atom to which they are bound) have the meanings as defined lines in 1 to 2541 of Table 1 above, i.e. individual compounds I.80.1 - I.80.2541, are particularly preferred:

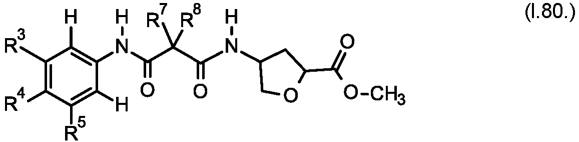

Compounds of formula I.81., wherein R³, R⁴, R⁵ and R⁷, R⁸ (forming W together with the carbon atom to which they are bound) have the meanings as defined lines in 1 to 2541 of Table 1 above, i.e. individual compounds I.81.1 - I.81.2541, are particularly preferred:

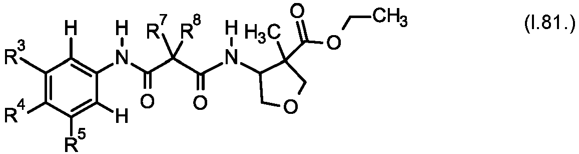

Compounds of formula I.82., wherein R³, R⁴, R⁵ and R⁷, R⁸ (forming W together with the carbon atom to which they are bound) have the meanings as defined lines in 1 to 2541 of Table 1 above, i.e. individual compounds I.82.1 - I.82.2541, are particularly preferred:

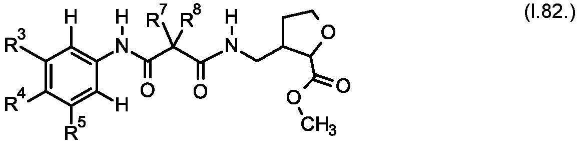

Compounds of formula I.83., wherein R³, R⁴, R⁵ and R⁷, R⁸ (forming W together with the carbon atom to which they are bound) have the meanings as defined lines in 1 to 2541 of Table 1 above, i.e. individual compounds I.83.1 - I.83.2541, are particularly preferred:

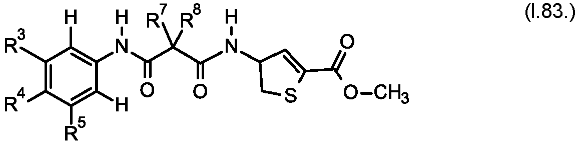

Compounds of formula I.84., wherein R³, R⁴, R⁵ and R⁷, R⁸ (forming W together with the carbon atom to which they are bound) have the meanings as defined lines in 1 to 2541 of Table 1 above, i.e. individual compounds I.84.1 - I.84.2541, are particularly preferred:

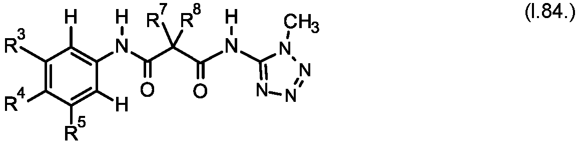

Compounds of formula I.85., wherein R³, R⁴, R⁵ and R⁷, R⁸ (forming W together with the carbon atom to which they are bound) have the meanings as defined lines in 1 to 2541 of Table 1 above, i.e. individual compounds I.85.1 - I.85.2541, are particularly preferred:

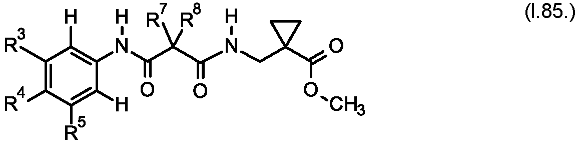

Compounds of formula I.86., wherein R³, R⁴, R⁵ and R⁷, R⁸ (forming W together with the carbon atom to which they are bound) have the meanings as defined lines in 1 to 2541 of Table 1 above, i.e. individual compounds I.86.1 - I.86.2541, are particularly preferred:

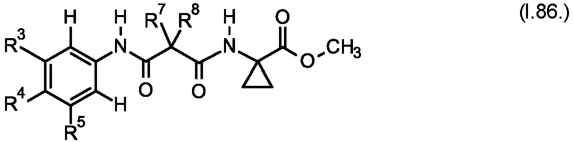

Compounds of formula I.87., wherein R³, R⁴, R⁵ and R⁷, R⁸ (forming W together with the carbon atom to which they are bound) have the meanings as defined lines in 1 to 2541 of Table 1 above, i.e. individual compounds I.87.1 - I.87.2541, are particularly preferred:

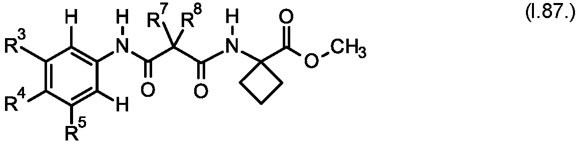

Compounds of formula I.88., wherein R³, R⁴, R⁵ and R⁷, R⁸ (forming W together with the carbon atom to which they are bound) have the meanings as defined lines in 1 to 2541 of Table 1 above, i.e. individual compounds I.88.1 - I.88.2541, are particularly preferred:

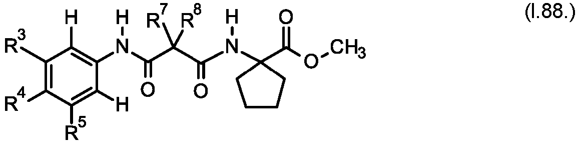

Compounds of formula I.89., wherein R³, R⁴, R⁵ and R⁷, R⁸ (forming W together with the carbon atom to which they are bound) have the meanings as defined lines in 1 to 2541 of Table 1 above, i.e. individual compounds I.89.1 - I.89.2541, are particularly preferred:

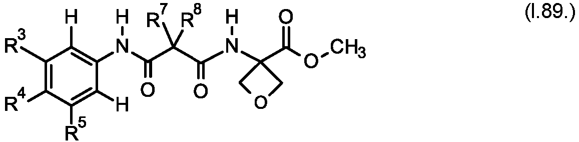

Compounds of formula I.90., wherein R³, R⁴, R⁵ and R⁷, R⁸ (forming W together with the carbon atom to which they are bound) have the meanings as defined lines in 1 to 2541 of Table 1 above, i.e. individual compounds I.90.1 - I.90.2541, are particularly preferred:

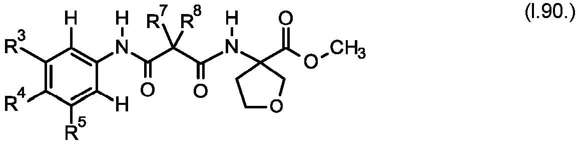

Compounds of formula I.91., wherein R³, R⁴, R⁵ and R⁷, R⁸ (forming W together with the carbon atom to which they are bound) have the meanings as defined lines in 1 to 2541 of Table 1 above, i.e. individual compounds I.91.1 - I.91.2541, are particularly preferred:

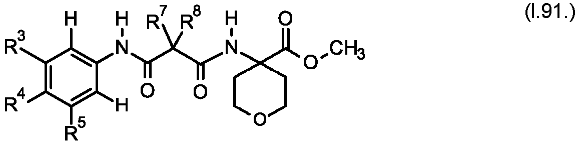

Compounds of formula I.92., wherein R³, R⁴, R⁵ and R⁷, R⁸ (forming W together with the carbon atom to which they are bound) have the meanings as defined lines in 1 to 2541 of Table 1 above, i.e. individual compounds I.92.1 - I.92.2541, are particularly preferred:

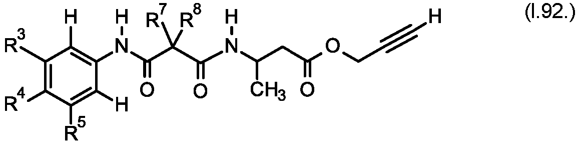

Compounds of formula I.93., wherein R³, R⁴, R⁵ and R⁷, R⁸ (forming W together with the carbon atom to which they are bound) have the meanings as defined lines in 1 to 2541 of Table 1 above, i.e. individual compounds I.93.1 - I.93.2541, are particularly preferred:

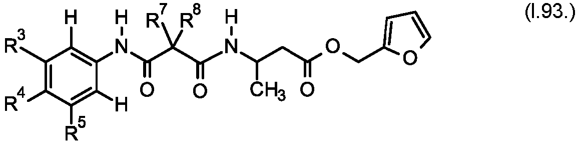

Compounds of formula I.94., wherein R³, R⁴, R⁵ and R⁷, R⁸ (forming W together with the carbon atom to which they are bound) have the meanings as defined lines in 1 to 2541 of Table 1 above, i.e. individual compounds I.94.1 - I.94.2541, are particularly preferred:

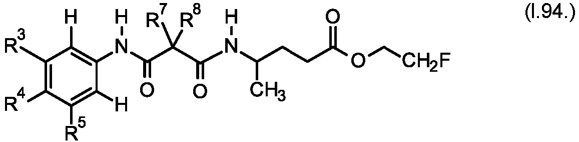

Compounds of formula I.95., wherein R³, R⁴, R⁵ and R⁷, R⁸ (forming W together with the carbon atom to which they are bound) have the meanings as defined lines in 1 to 2541 of Table 1 above, i.e. individual compounds I.95.1 - I.95.2541, are particularly preferred:

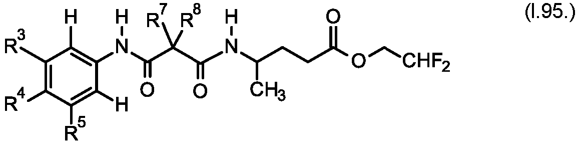

Compounds of formula I.96., wherein R³, R⁴, R⁵ and R⁷, R⁸ (forming W together with the carbon atom to which they are bound) have the meanings as defined lines in 1 to 2541 of Table 1 above, i.e. individual compounds I.96.1 - I.96.2541, are particularly preferred:

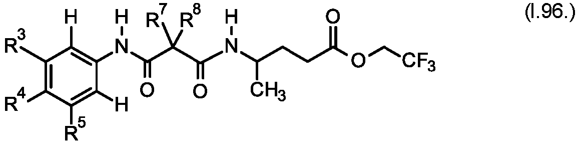

Compounds of formula I.97., wherein R³, R⁴, R⁵ and R⁷, R⁸ (forming W together with the carbon atom to which they are bound) have the meanings as defined lines in 1 to 2541 of Table 1 above, i.e. individual compounds I.97.1 - I.97.2541, are particularly preferred:

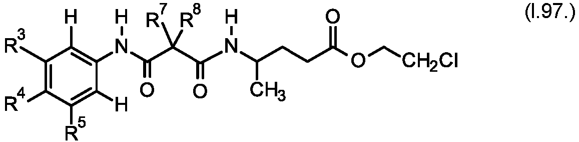

Compounds of formula I.98., wherein R³, R⁴, R⁵ and R⁷, R⁸ (forming W together with the carbon atom to which they are bound) have the meanings as defined lines in 1 to 2541 of Table 1 above, i.e. individual compounds I.98.1 - I.98.2541, are particularly preferred:

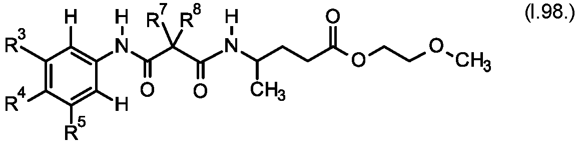

Compounds of formula I.99., wherein R³, R⁴, R⁵ and R⁷, R⁸ (forming W together with the carbon atom to which they are bound) have the meanings as defined lines in 1 to 2541 of Table 1 above, i.e. individual compounds I.99.1 - I.99.2541, are particularly preferred:

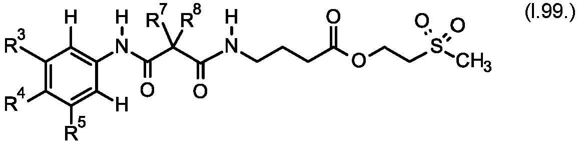

Compounds of formula I.100., wherein R³, R⁴, R⁵ and R⁷, R⁸ (forming W together with the carbon atom to which they are bound) have the meanings as defined lines in 1 to 2541 of Table 1 above, i.e. individual compounds I.100.1 - I.100.2541, are particularly preferred:

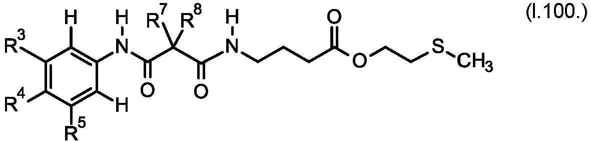

Compounds of formula I.101., wherein R³, R⁴, R⁵ and R⁷, R⁸ (forming W together with the carbon atom to which they are bound) have the meanings as defined lines in 1 to 2541 of Table 1 above, i.e. individual compounds I.101.1 - I.101.2541, are particularly preferred:

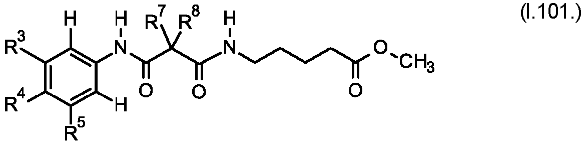

Compounds of formula I.102., wherein R³, R⁴, R⁵ and R⁷, R⁸ (forming W together with the carbon atom to which they are bound) have the meanings as defined lines in 1 to 2541 of Table 1 above, i.e. individual compounds I.102.1 - I.102.2541, are particularly preferred:

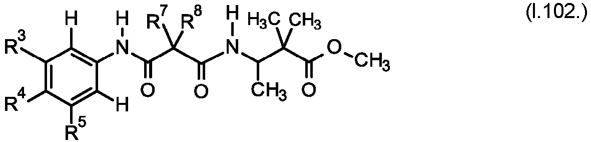

Compounds of formula I.103., wherein R³, R⁴, R⁵ and R⁷, R⁸ (forming W together with the carbon atom to which they are bound) have the meanings as defined lines in 1 to 2541 of Table 1 above, i.e. individual compounds I.103.1 - I.103.2541, are particularly preferred:

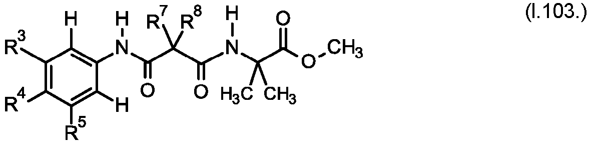

Compounds of formula I.104., wherein R³, R⁴, R⁵ and R⁷, R⁸ (forming W together with the carbon atom to which they are bound) have the meanings as defined lines in 1 to 2541 of Table 1 above, i.e. individual compounds I.104.1 - I.104.2541, are particularly preferred:

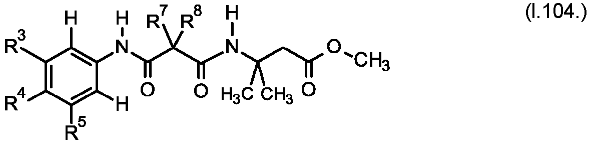

Compounds of formula I.105., wherein R³, R⁴, R⁵ and R⁷, R⁸ (forming W together with the carbon atom to which they are bound) have the meanings as defined lines in 1 to 2541 of Table 1 above, i.e. individual compounds I.105.1 - I.105.2541, are particularly preferred:

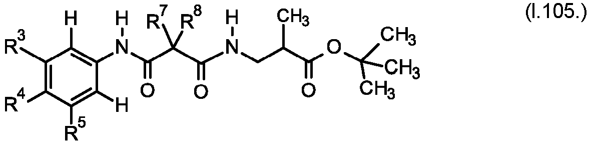

Compounds of formula I.106., wherein R³, R⁴, R⁵ and R⁷, R⁸ (forming W together with the carbon atom to which they are bound) have the meanings as defined lines in 1 to 2541 of Table 1 above, i.e. individual compounds I.106.1 - I.106.2541, are particularly preferred:

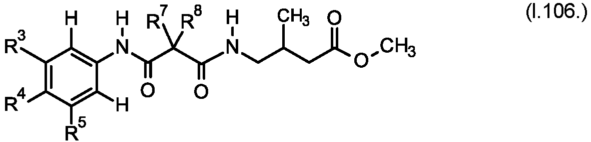

Compounds of formula I.107., wherein R³, R⁴, R⁵ and R⁷, R⁸ (forming W together with the carbon atom to which they are bound) have the meanings as defined lines in 1 to 2541 of Table 1 above, i.e. individual compounds I.107.1 - I.107.2541, are particularly preferred:

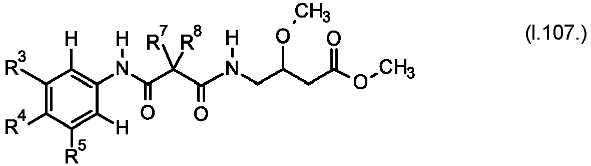

Compounds of formula I.108., wherein R³, R⁴, R⁵ and R⁷, R⁸ (forming W together with the carbon atom to which they are bound) have the meanings as defined lines in 1 to 2541 of Table 1 above, i.e. individual compounds I.108.1 - I.108.2541, are particularly preferred:

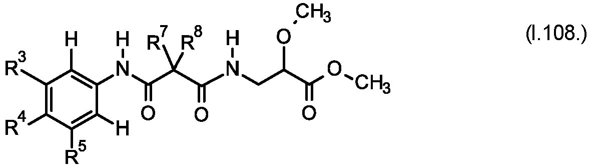

Compounds of formula I.109., wherein R³, R⁴, R⁵ and R⁷, R⁸ (forming W together with the carbon atom to which they are bound) have the meanings as defined lines in 1 to 2541 of Table 1 above, i.e. individual compounds I.109.1 - I.109.2541, are particularly preferred:

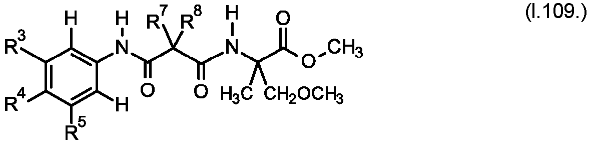

Compounds of formula I.110., wherein R³, R⁴, R⁵ and R⁷, R⁸ (forming W together with the carbon atom to which they are bound) have the meanings as defined lines in 1 to 2541 of Table 1 above, i.e. individual compounds I.110.1 - I.110.2541, are particularly preferred:

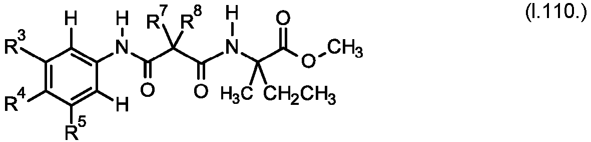

Compounds of formula I.111., wherein R³, R⁴, R⁵ and R⁷, R⁸ (forming W together with the carbon atom to which they are bound) have the meanings as defined lines in 1 to 2541 of Table 1 above, i.e. individual compounds I.111.1 - I.111.2541, are particularly preferred:

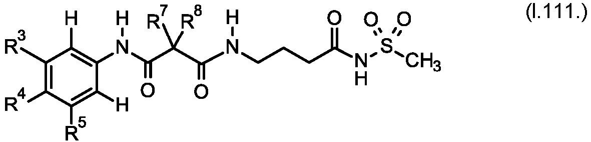

Compounds of formula I.112., wherein R³, R⁴, R⁵ and R⁷, R⁸ (forming W together with the carbon atom to which they are bound) have the meanings as defined lines in 1 to 2541 of Table 1 above, i.e. individual compounds I.112.1 - I.112.2541, are particularly preferred:

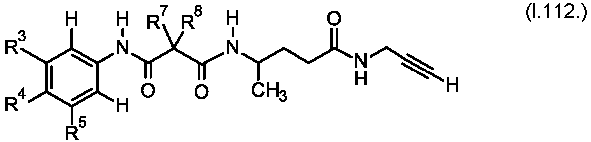

Compounds of formula I.113., wherein R³, R⁴, R⁵ and R⁷, R⁸ (forming W together with the carbon atom to which they are bound) have the meanings as defined lines in 1 to 2541 of Table 1 above, i.e. individual compounds I.113.1 - I.113.2541, are particularly preferred:

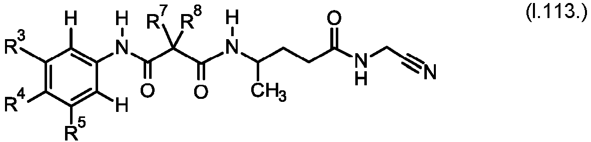

Compounds of formula I.114., wherein R³, R⁴, R⁵ and R⁷, R⁸ (forming W together with the carbon atom to which they are bound) have the meanings as defined lines in 1 to 2541 of Table 1 above, i.e. individual compounds I.114.1 - I.114.2541, are particularly preferred:

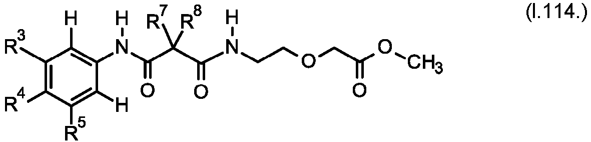

Compounds of formula I.115., wherein R³, R⁴, R⁵ and R⁷, R⁸ (forming W together with the carbon atom to which they are bound) have the meanings as defined lines in 1 to 2541 of Table 1 above, i.e. individual compounds I.115.1 - I.115.2541, are particularly preferred:

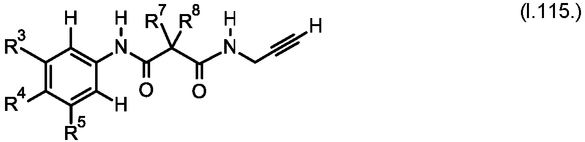

Compounds of formula I.116., wherein R³, R⁴, R⁵ and R⁷, R⁸ (forming W together with the carbon atom to which they are bound) have the meanings as defined lines in 1 to 2541 of Table 1 above, i.e. individual compounds I.116.1 - I.116.2541, are particularly preferred:

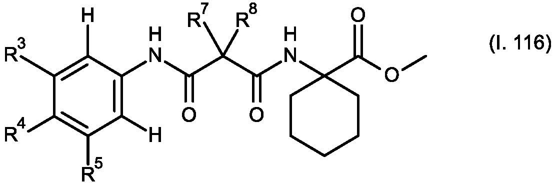

Compounds of formula I.117., wherein R³, R⁴, R⁵ and R⁷, R⁸ (forming W together with the carbon atom to which they are bound) have the meanings as defined lines in 1 to 2541 of Table 1 above, i.e. individual compounds I.117.1 - I.117.2541, are particularly preferred:

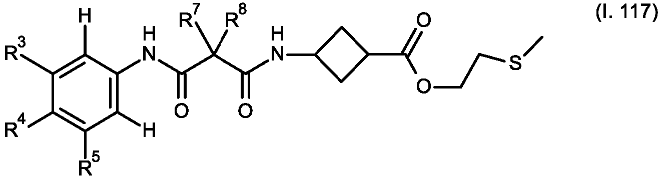

Compounds of formula I.118., wherein R³, R⁴, R⁵ and R⁷, R⁸ (forming W together with the carbon atom to which they are bound) have the meanings as defined lines in 1 to 2541 of Table 1 above, i.e. individual compounds I.118.1 - I.118.2541, are particularly preferred:

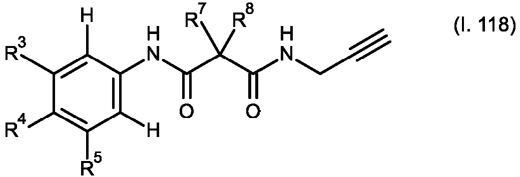

The compounds of formula (I) according to the invention can be prepared by standard processes of organic chemistry, for example by the following processes:

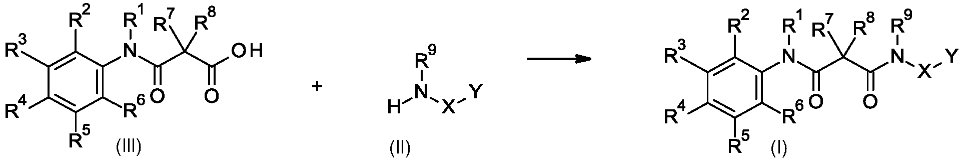

The compounds of formula (I) can be prepared according to methods or in analogy to methods that are described in the prior art. The synthesis takes advantage of starting materials that are commercially available or may be prepared according to conventional procedures starting from readily available compounds.

Compounds of formula (I) can be prepared from carboxylic acids (III) and commercially available amines (II) using an organic base and a coupling reagent. Thus, compounds of formula (I) can be synthesized from the corresponding carboxylic acids (1 eq.) using a coupling reagent (1-2 eq.), for example T₃P (propanephosphonic acid anhydride) or HATU (O-(7-azabenzotriazole-*1-yl)-N,N,N' ,N'* -tetramethyluronium-hexafluorphosphate), an organic base (1-3 eq.) and the amines (II) (1-3 eq.). The reaction is typically carried out in an organic solvent. Preferably an aprotic organic solvent is used. Most preferably tetrahydrofuran (THF), *N*,*N*-dimethylformamide (DMF) or acetonitrile (ACN) are used. The reaction is carried out at temperatures between 0°C and reflux. Preferably the reaction is carried out at room temperature. Preferably the organic base is triethylamine or N,N-diisopropylethylamine.

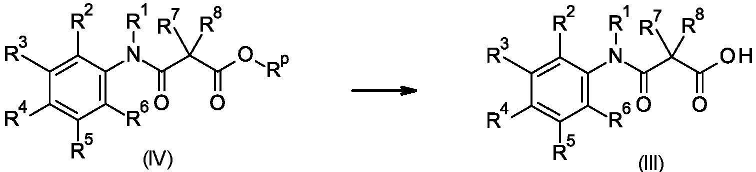

The carboxylic acids (III) are commercially available or can be prepared from the corresponding esters (IV) (wherein R^{P} is alkyl or benzyl). If R^{P} is alkyl, esters (IV) may be cleaved using aqueous alkali metal hydroxides. Preferably lithium hydroxide, sodium hydroxide or potassium hydroxide (1-2 eq.) are employed. The reaction is typically carried out in mixtures of water and an organic solvent. Preferably the organic solvent is THF, methanol or acetonitrile. The reaction is carried out at temperatures between 0°C and 100°C. Preferably the reaction is carried at room temperature. If R^{p} is benzyl in (IV), then the ester may be cleaved using palladium on charcoal (0.001-1 eq.) as catalyst and hydrogen gas at temperatures between 0°C and reflux. Preferably the reaction is carried out at room temperature. Typically, an organic solvent is employed. Preferably THF, methanol or ethanol are employed.

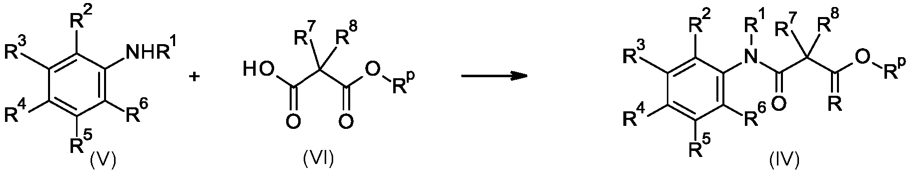

Cyclic compounds of the formula (IV) can be prepared from cyclic carboxylic acids (VI) and commercially available amines (V) using a base and a coupling reagent. Thus, compounds of formula (IV) can be synthesized from the corresponding carboxylic acids (1eq.) using a coupling reagent (1-2 eq.), for example T₃P (propanephosphonic acid anhydride) or HATU (O-(7-azabenzotriazole-1- yl)-N,N,N' ,N' -tetramethyluronium-hexafluorphosphate), an organic base (1-3 eq.) and the amines (V) (1-3 eq.). The reaction is typically carried out in an organic solvent. Preferably an aprotic organic solvent is used. Most preferably tetrahydrofuran (THF), N,N-dimethylformamide (DMF) or acetonitrile (ACN) are used. The reaction is carried out at temperatures between 0°C to refluxing temperatures. Preferably the reaction is carried out at room temperature. Preferably the organic base is triethylamine or N,N-diisopropylethylamine.

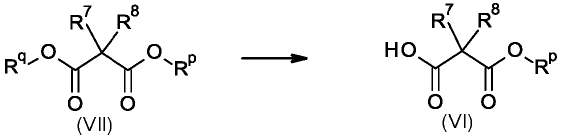

Cyclic carboxylic acids (VI) may be prepared from the corresponding diester by selective cleavage of one ester group. If Rq is an alkyl ester, selective ester cleavage may be achieved using an aqueous base. Preferably an alkali metal hydroxide is used. Most preferably lithium hydroxide, sodium hydroxide or potassium hydroxide are used. The reaction is typically carried out in mixtures of water and an organic solvent. Preferably THF, methanol or acetonitrile are employed. The reaction is carried out at temperatures between 0°C and 100°C, preferably at room temperature.

Alternatively, trimethyltin hydroxide (e.g. 1 eq.) in 1,2 dichlorethane at room temperature to reflux may be used (as described in Angew. Chem. Int. Ed, 2005, 44: 1378-1382), preferably at reflux. If Rq is benzyl in (VII), then the ester may be cleaved using palladium on charcoal (0.001-1eq.) as catalyst and hydrogen gas at temperatures between 0°C and reflux. Preferably the reaction is carried out at room temperature. Typically, an organic solvent is employed. Preferably THF, methanol or ethanol are employed.

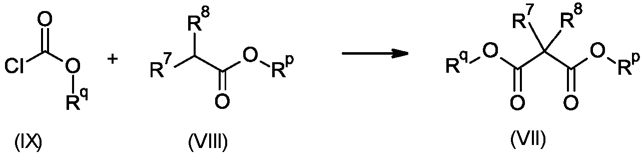

The cyclic diesters (VII) may be synthesized from a commercially available cyclic monoester (VIII), a base and a chloroformate (IX) (1-3 eq.) as described in Bioorganic & Medicinal Chemistry Letters, 12 (11), 1501-1505; 2002. The reaction is typically carried out in an organic solvent, preferably in tetrahydrofuran. Suitable temperatures range between -78°C and 25°C. Preferably the reaction is allowed to warm up from -78°C to 25°C over a period of 16 h. Preferably lithium-diisopropylamide (1eq.) is used as a base.

Alternatively, specific cyclic diesters can be prepared according to the following processes:

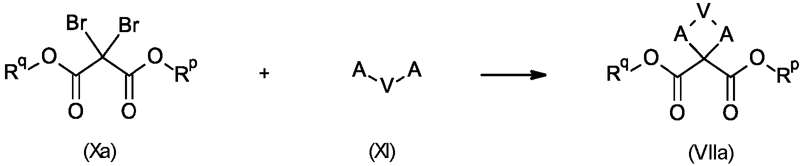

Alternatively, cyclic diester (Vlla) may be prepared from commercially available diethyl dibromo-malonates (Xa), a base and an alcohol or thiol of the formula (XI) (V is -CH₂CH₂- or -CH₂CH₂CH₂-, optionally substituted with R^{g}; and A is independently selected from OH, SH, NR^{d}). The reaction is typically carried out in an organic solvent, preferably in tetrahydrofuran. Suitable temperatures range between 0°C and 25 °C. Preferably sodium hydride (2eq.) is used as a base.

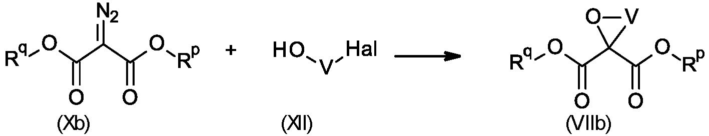

The cyclic diester of the formula (Vllb) containing oxy-heterocycles are either commercially available or may be prepared from the corresponding diazo-compounds (Xb) using dirhodiumtetraacetat ([Rh(OAc)₂]₂) (0.001-0.1 eq.) and commercially available halogenated (Hal = Cl, Br) alcohols of the formula (XII) (V is -CH₂CH₂-, -CH₂CH₂CH₂-, -CH₂CH₂CH₂CH₂-, optionally substituted with R^{g}), followed by cyclization using sodium hydride (1.2 eq.) in N,N-dimethylformamide (DMF) as described in Angew. Chem. Int. Ed. 2014, 53, 14230-14234.

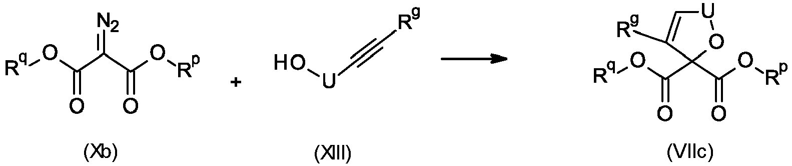

Alternatively, the cyclic unsaturated diester of the formula (Vllc) containing oxy-heterocycles may be prepared from the corresponding diazo-compounds (Xb) using dirhodiumtetraacetat ([Rh(OAc)₂]₂) (0.001-0.1 eq.) and alcohols of the formula (XIII) (U is -CH₂- or -CH₂CH₂-, optionally substituted with R^{g}) followed by cyclization using cesium carbonate (2.0 eq.) in acetonitrile. Suitable temperatures range from room temperature to 60°C.

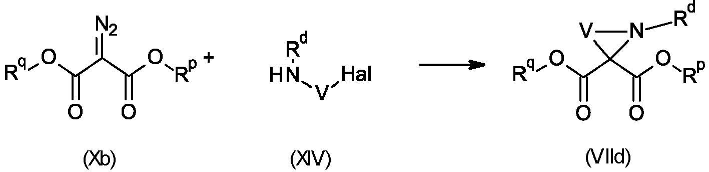

The cyclic diesters containing N-heterocycles (Vlld) may be prepared from the corresponding diazo-compounds (Xb) using Bis[rhodium(α,α,α',α'-tetramethyl-1,3-benzenedipropionic acid)] ([Rh(esp)]₂, CAS [819050-89-0] (0.001-0.1 eq.) and amines containing halogens (Hal = Br, Cl) of the formula (XIV) (V is -CH₂CH₂-, -CH₂CH₂CH₂-, or -CH₂CH₂CH₂CH₂-, optionally substituted with R^{g}) in toluene at 60°C followed by addition of cesium hydroxid monohydrate (2 eq.) and tetrabutylammonium bromide (0.1 eq.) as described in Angew. Chem. Int. Ed. 2019, 58, 1458-1462.

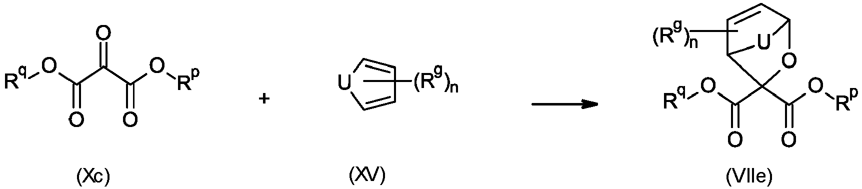

The unsaturated diesters containing O-heterocycles of the formula (VIIe) (U is -CH₂- or - CH₂CH₂-) may be prepared from the commercially available keto malonate (CAS 609-09-6) (Xc) using cyclic or acyclic dienes of the formula (XV) in acetonitrile at 130 °C as described in J. Org. Chem. 1977, 42, 4095-4103. Alternatively, heating at 130 °C for 4 h is performed by irradiating the reaction mixture with a microwave (300 W).

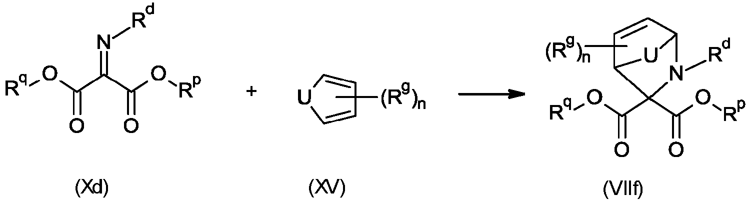

The corresponding N-heterocyclic unsaturated diesters of the formula (VIIf) (U is -CH₂- or - CH₂CH₂-) may be prepared analogously from the corresponding imido malonate (Xd) using cyclic or acyclic dienes of the formular (XV) in tetrahydrofuran at 100 °C as described in Tetrahedron Lett. 1981, 22, 4607, Synth. Commun. 1972, 2, 211 and J. Med. Chem. 1973, 16, 853 or by irradiating the reaction mixture with a microwave (100°C, 4 h, 300 W).

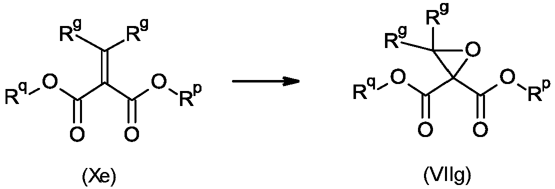

The diesters containing epoxides of the formula (Vllg) may be prepared from the commercially available alkenes of the formula (Xe) using sodium tungstate dihydrate (CAS 13472-45-2) and hydrogen peroxide (40% aqueous solution) in ethanol as described in Tetrahedron 2010, 66, 9401-9404.

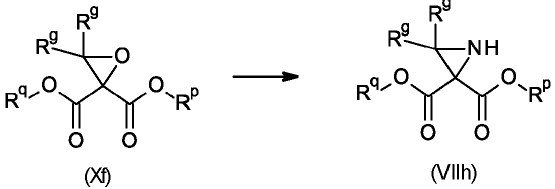

The corresponding aziridines of the formula (Vllh) may be prepared from the epoxide of the formula (Xf) using sodium azide and ammonium chloride in aqueous dioxane followed by reduction with triphenylphosphine in acetonitrile as described in Tetrahedron 2010, 66, 9401-9404.

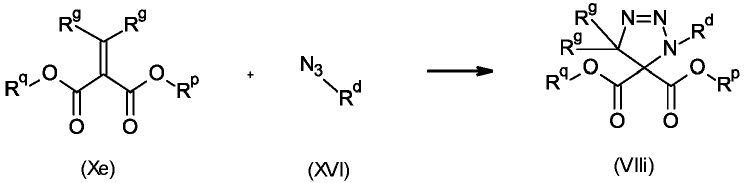

The corresponding triazolines of the formula (Vlli) may be prepared from the commercially available alkenes of the formula (Xe) using azides of the formula (XVI) using catalytic amounts of N,N-dimethylurea in toluene at 60°C as described in Org. Lett. 2015, 17, 4568-4571.

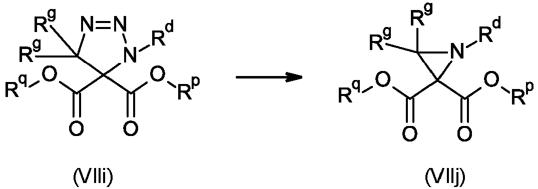

The corresponding aziridines of the formula (Vllj) may be prepared from the triazoline of the formula (Vlli) as a solid or in solution upon irradiation with using wavelengths λ ≥ 220 nm as described in Org. Lett. 2015, 17, 4568-4571.

To widen the spectrum of action, the compounds of formula (I) may be mixed with many representatives of other herbicidal or growth-regulating active ingredient groups and then applied concomitantly. Suitable components for combinations are, for example, herbicides from the classes of the acetamides, amides, aryloxyphenoxypropionates, benzamides, benzofuran, benzoic acids, benzothiadiazinones, bipyridylium, carbamates, chloroacetamides, chlorocarboxylic acids, cyclohexanediones, dinitroanilines, dinitrophenol, diphenyl ether, glycines, imidazolinones, isoxazoles, isoxazolidinones, nitriles, N-phenylphthalimides, oxadiazoles, oxazolidinediones, oxyacetamides, phenoxycarboxylic acids, phenylcarbamates, phenylpyrazoles, phenylpyrazolines, phenylpyridazines, phosphinic acids, phosphoroamidates, phosphorodithioates, phthalamates, pyrazoles, pyridazinones, pyridines, pyridinecarboxylic acids, pyridinecarboxamides, pyrimidinediones, pyrimidinyl(thio)benzoates, quinolinecarboxylic acids, semicarbazones, sulfonylaminocarbonyltriazolinones, sulfonylureas, tetrazolinones, thiadiazoles, thiocarbamates, triazines, triazinones, triazoles, triazolinones, triazolocarboxamides, triazolopyrimidines, triketones, uracils, ureas.

It may furthermore be beneficial to apply the compounds of formula (I) alone or in combination with other herbicides, or else in the form of a mixture with other crop protection agents, for example together with agents for controlling pests or phytopathogenic fungi or bacteria. Also of interest is the miscibility with mineral salt solutions, which are employed for treating nutritional and trace element deficiencies. Other additives such as non-phytotoxic oils and oil concentrates may also be added.

In one embodiment of the present invention the combinations according to the present invention comprise at least one compound of formula (I) (compound A or component A) and at least one further active compound selected from herbicides B (compound B), preferably herbicides B of class b1) to b15), and safeners C (compound C).

In another embodiment of the present invention the combinations according to the present invention comprise at least one compound of formula (I) and at least one further active compound B (herbicide B).

Examples of herbicides B which can be used in combination with the *compounds* A of formula (I) according to the present invention are:
b1) from the group of the lipid biosynthesis inhibitors:
   ACC-herbicides such as alloxydim, alloxydim-sodium, butroxydim, clethodim, clodinafop, clodinafop-propargyl, cycloxydim, cyhalofop, cyhalofop-butyl, diclofop, diclofop-methyl, fenoxaprop, fenoxaprop-ethyl, fenoxaprop-P, fenoxaprop-P-ethyl, fluazifop, fluazifop-butyl, fluazifop-P, fluazifop-P-butyl, haloxyfop, haloxyfop-methyl, haloxyfop-P, haloxyfop-P-methyl, metamifop, pi-noxaden, profoxydim, propaquizafop, quizalofop, quizalofop-ethyl, quizalofop-tefuryl, quizalofop-P, quizalofop-P-ethyl, quizalofop-P-tefuryl, sethoxydim, tepraloxydim, tralkoxydim, 4-(4'-Chloro-4-cyclopropyl-2'-fluoro[1,1'-biphenyl]-3-yl)-5-hydroxy-2,2,6,6-tetramethyl-2H-pyran-3(6H)-one (CAS 1312337-72-6); 4-(2',4'-Dichloro-4-cyclopropyl[1,1'-biphenyl]-3-yl)-5-hydroxy-2,2,6,6-tetramethyl-2H-pyran-3(6H)-one (CAS 1312337-45-3); 4-(4'-Chloro-4-ethyl-2'-fluoro[1,1'-biphenyl]-3-yl)-5-hydroxy-2,2,6,6-tetramethyl-2H-pyran-3(6H)-one (CAS 1033757-93-5); 4-(2',4'-Dichloro-4-ethyl[1,1'-biphenyl]-3-yl)-2,2,6,6-tetramethyl-2H-pyran-3,5(4H,6H)-dione (CAS 1312340-84-3); 5-(Acetyloxy)-4-(4'-chloro-4-cyclopropyl-2'-fluoro[1,1'-biphenyl]-3-yl)-3,6-dihydro-2,2,6,6-tetramethyl-2H-pyran-3-one (CAS 1312337-48-6); 5-(Acetyloxy)-4-(2',4'-dichloro-4-cyclopropyl- [1,1'-biphenyl]-3-yl)-3,6-dihydro-2,2,6,6-tetramethyl-2H-pyran-3-one; 5-(Acetyloxy)-4-(4'-chloro-4-ethyl-2'-fluoro[1,1'-biphenyl]-3-yl)-3,6-dihydro-2,2,6,6-tetramethyl-2H-pyran-3-one (CAS 1312340-82-1); 5-(Acetyloxy)-4-(2',4'-dichloro-4-ethyl[1,1'-biphenyl]-3-yl)-3,6-dihydro-2,2,6,6-tetramethyl-2H-pyran-3-one (CAS 1033760-55-2); 4-(4'-Chloro-4-cyclopropyl-2'-fluoro[1,1'-biphenyl]-3-yl)-5,6-dihydro-2,2,6,6-tetramethyl-5-oxo-2H-pyran-3-yl carbonic acid methyl ester (CAS 1312337-51-1); 4-(2',4'-Dichloro -4-cyclopropyl- [1,1'-biphenyl]-3-yl)-5,6-dihydro-2,2,6,6-tetramethyl-5-oxo-2H-pyran-3-yl carbonic acid methyl ester; 4-(4'-Chloro-4-ethyl-2'-fluoro[1,1'-biphenyl]-3-yl)-5,6-dihydro-2,2,6,6-tetramethyl-5-oxo-2H-pyran-3-yl carbonic acid methyl ester (CAS 1312340-83-2); 4-(2',4'-Dichloro-4-ethyl[1,1'-biphenyl]-3-yl)-5,6-dihydro-2,2,6,6-tetramethyl-5-oxo-2H-pyran-3-yl carbonic acid methyl ester (CAS 1033760-58-5); and non ACC herbicides such as benfuresate, butylate, cycloate, dalapon, dimepiperate, EPTC, esprocarb, ethofumesate, flupropanate, molinate, orbencarb, pebulate, prosulfocarb, TCA, thiobencarb, tiocarbazil, triallate and vernolate;
b2) from the group of the ALS inhibitors:
   sulfonylureas such as amidosulfuron, azimsulfuron, bensulfuron, bensulfuron-methyl, chlorimuron, chlorimuron-ethyl, chlorsulfuron, cinosulfuron, cyclosulfamuron, ethametsulfuron, ethametsulfuron-methyl, ethoxysulfuron, flazasulfuron, flucetosulfuron, flupyrsulfuron, flupyrsulfuron-methyl-sodium, foramsulfuron, halosulfuron, halosulfuron-methyl, imazosulfuron, iodosulfuron, iodosulfuron-methyl-sodium, iofensulfuron, iofensulfuron-sodium, mesosulfuron, metazosulfuron, metsulfuron, metsulfuron-methyl, nicosulfuron, orthosulfamuron, oxasulfuron, primisulfuron, primisulfuron-methyl, propyrisulfuron, prosulfuron, pyrazosulfuron, pyrazosulfuron-ethyl, rimsulfuron, sulfometuron, sulfometuron-methyl, sulfosulfuron, thifensulfuron, thifensulfuron-methyl, triasulfuron, tribenuron, tribenuron-methyl, trifloxysulfuron, triflusulfuron, triflusulfuron-methyl and tritosulfuron,
   imidazolinones such as imazamethabenz, imazamethabenz-methyl, imazamox, imazapic, imazapyr, imazaquin and imazethapyr, triazolopyrimidine herbicides and sulfonanilides such as cloransulam, cloransulam-methyl, diclosulam, flumetsulam, florasulam, metosulam, penoxsulam, pyrimisulfan and pyroxsulam,
   pyrimidinylbenzoates such as bispyribac, bispyribac-sodium, pyribenzoxim, pyriftalid, pyriminobac, pyriminobac-methyl, pyrithiobac, pyrithiobac-sodium, 4-[[[2-[(4,6-dimethoxy-2-pyrimidinyl)oxy]phenyl]methyl]amino]-benzoic acid-1-methylethyl ester (CAS 420138-41-6), 4-[[[2-[(4,6-dimethoxy-2-pyrimidinyl)oxy]phenyl]methyl]amino]-benzoic acid propyl ester (CAS 420138-40-5), N-(4-bromophenyl)-2-[(4,6-dimethoxy-2-pyrimidinyl)oxy]benzenemethanamine (CAS 420138-01-8),
   sulfonylaminocarbonyl-triazolinone herbicides such as flucarbazone, flucarbazone-sodium, propoxycarbazone, propoxycarbazone-sodium, thiencarbazone and thiencarbazone-methyl; and triafamone;
   among these, a preferred embodiment of the invention relates to those compositions comprising at least one imidazolinone herbicide;
b3) from the group of the photosynthesis inhibitors:
   amicarbazone, inhibitors of the photosystem II, e.g. 1-(6-tert-butylpyrimidin-4-yl)-2-hydroxy-4-methoxy-3-methyl-2H-pyrrol-5-one (CAS 1654744-66-7), 1-(5-tert-butylisoxazol-3-yl)-2-hydroxy-4-methoxy-3-methyl-2H-pyrrol-5-one (CAS 1637455-12-9), 1-(5-tert-butylisoxazol-3-yl)-4-chloro-2-hydroxy-3-methyl-2H-pyrrol-5-one (CAS 1637453-94-1), 1-(5-tert-butyl-1-methyl-pyrazol-3-yl)-4-chloro-2-hydroxy-3-methyl-2H-pyrrol-5-one (CAS 1654057-29-0), 1-(5-tert-butyl-1-methyl-py-razol-3-yl)-3-chloro-2-hydroxy-4-methyl-2H-pyrrol-5-one (CAS 1654747-80-4), 4-hydroxy-1-methoxy-5-methyl-3-[4-(trifluoromethyl)-2-pyridyl]imidazolidin-2-one; (CAS 2023785-78-4), 4-hydroxy-1,5-dimethyl-3-[4-(trifluoromethyl)-2-pyridyl]imidazolidin-2-one (CAS 2023785-79-5), 5-ethoxy-4-hydroxy-1-methyl-3-[4-(trifluoromethyl)-2-pyridyl]imidazolidin-2-one (CAS 1701416-69-4), 4-hydroxy-1-methyl-3-[4-(trifluoromethyl)-2-pyridyl]imidazolidin-2-one (CAS 1708087-22-2), 4-hydroxy-1,5-dimethyl-3-[1-methyl-5-(trifluoromethyl)pyrazol-3-yl]imidazolidin-2-one (CAS 2023785-80-8), 1-(5-tert-butylisoxazol-3-yl)-4-ethoxy-5-hydroxy-3-methyl-imidazolidin-2-one (CAS 1844836-64-1), triazine herbicides, including of chlorotriazine, triazinones, triazindiones, methylthiotriazines and pyridazinones such as ametryn, atrazine, chloridazone, cyanazine, desmetryn, dimethametryn,hexazinone, metribuzin, prometon, prometryn, propazine, simazine, simetryn, terbumeton, terbuthylazin, terbutryn and trietazin, aryl urea such as chlorobromuron, chlorotoluron, chloroxuron, dimefuron, diuron, fluometuron, isoproturon, isouron, linuron, metamitron, methabenzthiazuron, metobenzuron, metoxuron, monolinuron, neburon, siduron, tebuthiuron and thiadiazuron, phenyl carbamates such as desmedipham, karbutilat, phenmedipham, phenmedipham-ethyl, nitrile herbicides such as bromofenoxim, bromoxynil and its salts and esters, ioxynil and its salts and esters, uraciles such as bromacil, lenacil and terbacil, and bentazon and bentazon-sodium, pyridate, pyridafol, pentanochlor and propanil and inhibitors of the photosystem I such as diquat, diquat-dibromide, paraquat, paraquat-dichloride and paraquat-dimetilsulfate. Among these, a preferred embodiment of the invention relates to those compositions comprising at least one aryl urea herbicide. Among these, likewise a preferred embodiment of the invention relates to those compositions comprising at least one triazine herbicide. Among these, likewise a preferred embodiment of the invention relates to those compositions comprising at least one nitrile herbicide;
b4) from the group of the protoporphyrinogen-IX oxidase inhibitors:
   acifluorfen, acifluorfen-sodium, azafenidin, bencarbazone, benzfendizone, bifenox, butafenacil, carfentrazone, carfentrazone-ethyl, chlomethoxyfen, chlorphthalim, cinidon-ethyl, cyclopyranil, fluazolate, flufenpyr, flufenpyr-ethyl, flumiclorac, flumiclorac-pentyl, flumioxazin, fluoroglycofen, fluoroglycofen-ethyl, fluthiacet, fluthiacet-methyl, fomesafen, halosafen, lactofen, oxadiargyl, oxadiazon, oxyfluorfen, pentoxazone, profluazol, pyraclonil, pyraflufen, pyraflufen-ethyl, saflufenacil, sulfentrazone, thidiazimin, tiafenacil, trifludimoxazin, ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (CAS 353292-31-6; S-3100), N-ethyl-3-(2,6-dichloro-4-trifluoromethylphenoxy)-5-methyl-1*H*-pyrazole-1-carboxamide (CAS 452098-92-9), N-tetrahydrofurfuryl-3-(2,6-dichloro-4-trifluoro-methylphenoxy)-5-methyl-1*H*-pyrazole-1-carboxamide (CAS 915396-43-9), N-ethyl-3-(2-chloro-6-fluoro-4-trifluoromethylphenoxy)-5-methyl-1*H*-pyrazole-1-carboxamide (CAS 452099-05-7), N-tetrahydrofurfuryl-3-(2-chloro-6-fluoro-4-trifluoromethylphenoxy)-5-methyl-1*H*-pyrazole-1-carboxamide (CAS 452100-03-7), 3-[7-fluoro-3-oxo-4-(prop-2-ynyl)-3,4-dihydro-2H-benzo[1,4]oxa-zin-6-yl]-1,5-dimethyl-6-thioxo-[1,3,5]triazinan-2,4-dione (CAS 451484-50-7), 2-(2,2,7-trifluoro-3-oxo-4-prop-2-ynyl-3,4-dihydro-2H-benzo[1,4]oxazin-6-yl)-4,5,6,7-tetrahydro-isoindole-1,3-dione (CAS 1300118-96-0), 1-methyl-6-trifluoromethyl-3-(2,2,7-trifluoro-3-oxo-4-prop-2-ynyl-3,4-dihy-dro-2H-benzo[1,4]oxazin-6-yl)-1H-pyrimidine-2,4-dione (CAS 1304113-05-0), methyl (E)-4-[2-chloro-5-[4-chloro-5-(difluoromethoxy)-1*H*-methyl-pyrazol-3-yl]-4-fluoro-phenoxy]-3-methoxy-but-2-enoate (CAS 948893-00-3), and 3-[7-chloro-5-fluoro-2-(trifluoromethyl)-1H-benzimidazol-4-yl]-1-methyl-6-(trifluoromethyl)-1H-pyrimidine-2,4-dione (CAS 212754-02-4), 2-[2-chloro-5-[3-chloro-5-(trifluoromethyl)-2-pyridinyl]-4-fluorophenoxy]-2-methoxy-acetic acid methyl ester (CAS 1970221-16-9), 2-[2-[[3-chloro-6-[3,6-dihydro-3-methyl-2,6-dioxo-4-(trifluoromethyl)-1(2H)-pyrimidinyl]-5-fluoro-2-pyridinyl]oxy]phenoxy]-acetic acid methyl ester (CAS 2158274-96-3), 2-[2-[[3-chloro-6-[3,6-dihydro-3-methyl-2,6-dioxo-4-(trifluoromethyl)-1(2H)-pyrimidinyl]-5-fluoro-2-pyridinyl]oxy]phenoxy] acetic acid ethyl ester (CAS 158274-50-9), methyl 2-[[3-[2-chloro-5-[4-(difluoromethyl)-3-methyl-5-oxo-1,2,4-triazol-1-yl]-4-fluoro-phenoxy]-2-pyridyl]oxy]acetate (CAS 2271389-22-9), ethyl 2-[[3-[2-chloro-5-[4-(difluoromethyl)-3-methyl-5-oxo-1,2,4-triazol-1-yl]-4-fluoro-phenoxy]-2-pyridyl]oxy]acetate (CAS 2230679-62-4), 2-[[3-[[3-chloro-6-[3,6-dihydro-3-methyl-2,6-dioxo-4-(trifluoromethyl)-1(2H)-pyrimidinyl]-5-fluoro-2-pyridinyl]oxy]-2-pyridinyl]oxy]-acetic acid methyl ester (CAS 2158275-73-9), 2-[[3-[[3-chloro-6-[3,6-dihydro-3-methyl-2,6-dioxo-4-(trifluoromethyl)-1(2H)-pyrimidinyl]-5-fluoro-2-pyridinyl]oxy]-2-pyridinyl]oxy] acetic acid ethyl ester (CAS 2158274-56-5), 2-[2-[[3-chloro-6-[3,6-dihydro-3-methyl-2,6-dioxo-4-(trifluoromethyl)-1(2H)-pyrimidinyl]-5-fluoro-2-pyridinyl]oxy]phenoxy]-N-(methylsulfonyl)-acetamide (CAS 2158274-53-2), 2-[[3-[[3-chloro-6-[3,6-dihydro-3-methyl-2,6-dioxo-4-(trifluoromethyl)-1(2H)-pyrimidinyl]-5-fluoro-2-pyridinyl]oxy]-2-pyridinyl]oxy]-N-(methylsulfonyl)-acetamide (CAS 2158276-22-1);
b5) from the group of the bleacher herbicides:
   PDS inhibitors: beflubutamid, diflufenican, fluridone, flurochloridone, flurtamone, norflurazon, picolinafen, and 4-(3-trifluoromethylphenoxy)-2-(4-trifluoromethylphenyl)pyrimidine (CAS 180608-33-7), HPPD inhibitors: benzobicyclon, benzofenap, bicyclopyrone, clomazone, fenquinotrione, isoxaflutole, mesotrione, oxotrione (CAS 1486617-21-3), pyrasulfotole, pyrazol-ynate, pyrazoxyfen, sulcotrione, tefuryltrione, tembotrione, tolpyralate, topramezone , bleacher, unknown target: aclonifen, amitrole flumeturon 2-chloro-3-methylsulfanyl-N-(1-methyltetrazol-5-yl)-4-(trifluoromethyl)benzamide (CAS 1361139-71-0), bixlozone and 2-(2,5-dichlorophenyl)methyl-4,4-dimethyl-3-isoxazolidinone (CAS 81778-66-7);
b6) from the group of the EPSP synthase inhibitors:
   glyphosate, glyphosate-isopropylammonium, glyposate-potassium and glyphosate-trimesium (sulfosate);
b7) from the group of the glutamine synthase inhibitors:
   bilanaphos (bialaphos), bilanaphos-sodium, glufosinate, glufosinate-P and glufosinate-ammonium;
b8) from the group of the DHP synthase inhibitors: asulam;
b9) from the group of the mitosis inhibitors:
   compounds of group K1: dinitroanilines such as benfluralin, butralin, dinitramine, ethalfluralin, fluchloralin, oryzalin, pendimethalin, prodiamine and trifluralin, phosphoramidates such as amiprophos, amiprophos-methyl, and butamiphos, benzoic acid herbicides such as chlorthal, chlorthal-dimethyl, pyridines such as dithiopyr and thiazopyr, benzamides such as propyzamide and tebutam; compounds of group K2: carbetamide, chlorpropham, flamprop, flamprop-isopropyl, flamprop-methyl, flamprop-M-isopropyl, flamprop-M-methyl and propham ; among these, compounds of group K1, in particular dinitroanilines are preferred;
b10) from the group of the VLCFA inhibitors:
   chloroacetamides such as acetochlor, alachlor, amidochlor, butachlor, dimethachlor, dimethenamid, dimethenamid-P, metazachlor, metolachlor, metolachlor-S, pethoxamid, pretilachlor, propachlor, propisochlor and thenylchlor, oxyacetanilides such as flufenacet and mefenacet, acetanilides such as diphenamid, naproanilide, napropamide and napropamide-M, tetrazolinones such fentrazamide, and other herbicides such as anilofos, cafenstrole, fenoxasulfone, ipfen-carbazone, piperophos, pyroxasulfone and isoxazoline compounds of the formulae II.1, II.2, II.3, II.4, II.5, II.6, II.7, II.8 and II.9 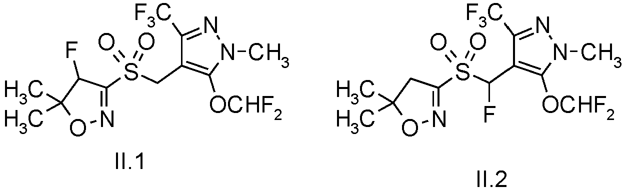 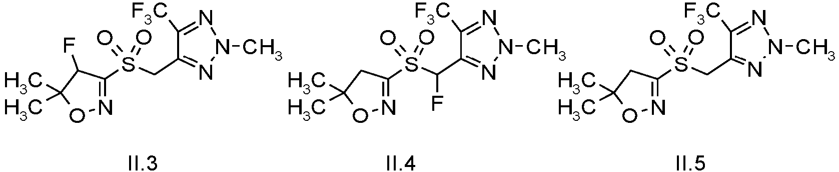 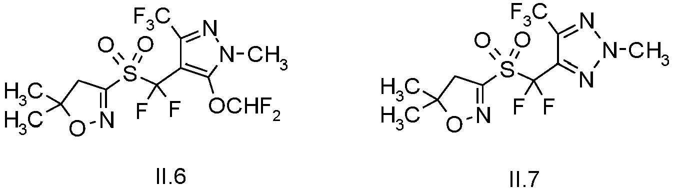 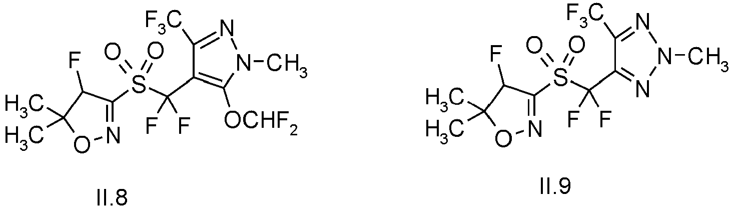
   the isoxazoline compounds of the formula (II) are known in the art, e.g. from WO 2006/024820, WO 2006/037945, WO 2007/071900 and WO 2007/096576;
   among the VLCFA inhibitors, preference is given to chloroacetamides and oxyacetamides;
b11) from the group of the cellulose biosynthesis inhibitors:
   chlorthiamid, dichlobenil, flupoxam, indaziflam, isoxaben, triaziflam and 1-cyclohexyl-5-pen-tafluorphenyloxy-1⁴-[1,2,4,6]thiatriazin-3-ylamine (CAS 175899-01-1);
b12) from the group of the decoupler herbicides:
   dinoseb, dinoterb and DNOC and its salts;
b13) from the group of the auxinic herbicides:
   2,4-D and its salts and esters such as clacyfos, 2,4-DB and its salts and esters, aminocyclopy-rachlor and its salts and esters, aminopyralid and its salts such as aminopyralid-dimethylammonium, aminopyralid-tris(2-hydroxypropyl)ammonium and its esters, benazolin, benazolin-ethyl, chloramben and its salts and esters, clomeprop, clopyralid and its salts and esters, dicamba and its salts and esters, dichlorprop and its salts and esters, dichlorprop-P and its salts and esters, flopyrauxifen, fluroxypyr, fluroxypyr-butometyl, fluroxypyr-meptyl, halauxifen and its salts and esters (CAS 943832-60-8); MCPA and its salts and esters, MCPA-thioethyl, MCPB and its salts and esters, mecoprop and its salts and esters, mecoprop-P and its salts and esters, piclo-ram and its salts and esters, quinclorac, quinmerac, TBA (2,3,6) and its salts and esters, triclopyr and its salts and esters, florpyrauxifen, florpyrauxifen-benzyl (CAS 1390661-72-9) and 4-amino-3-chloro-5-fluoro-6-(7-fluoro-1H-indol-6-yl)picolinic acid (CAS 1629965-65-6);
b14) from the group of the auxin transport inhibitors: diflufenzopyr, diflufenzopyr-sodium, naptalam and naptalam-sodium;
b15) from the group of the other herbicides: bromobutide, chlorflurenol, chlorflurenol-methyl, cinmethylin, cumyluron, cyclopyrimorate (CAS 499223-49-3) and its salts and esters, dalapon, dazomet, difenzoquat, difenzoquat-metilsulfate, dimethipin, DSMA, dymron, endothal and its salts, etobenzanid, flurenol, flurenol-butyl, flurprimidol, fosamine, fosamine-ammonium, indanofan, maleic hydrazide, mefluidide, metam, methiozolin, methyl azide, methyl bromide, methyldymron, methyl iodide, MSMA, oleic acid, oxaziclomefone, pelargonic acid, pyributicarb, quinoclamine tetflupyrolimet, and tridiphane.

Moreover, it may be useful to apply the compounds of formula (I) in combination with safeners. Safeners are chemical compounds which prevent or reduce damage on useful plants without having a major impact on the herbicidal action of the compounds of the formula (I) towards undesired vegetation. They can be applied either before sowings (e.g. on seed treatments, shoots or seedlings) or in the pre-emergence application or post-emergence application of the useful plant. The safeners and the compounds of formula (I) and optionally the herbicides B can be applied simultaneously or in succession.

In another embodiment of the present invention the combinations according to the present invention comprise at least one compound of formula (I) and at least one safener C (component C).

Examples of safeners are e.g. (quinolin-8-oxy)acetic acids, 1-phenyl-5-haloalkyl-1H-1,2,4-triazol-3-carboxylic acids, 1-phenyl-4,5-dihydro-5-alkyl-1H-pyrazol-3,5-dicarboxylic acids, 4,5-dihydro-5,5-diaryl-3-isoxazol carboxylic acids, dichloroacetamides, alpha-oximinophenylacetonitriles, acetophenonoximes, 4,6-dihalo-2-phenylpyrimidines, N-[[4-(aminocarbonyl)phenyl]sulfonyl]-2-benzoic amides, 1,8-naphthalic anhydride, 2-halo-4-(haloalkyl)-5-thiazol carboxylic acids, phosphorthiolates and N-alkyl-O-phenylcarbamates and their agriculturally acceptable salts and their agriculturally acceptable derivatives such amides, esters, and thioesters, provided they have an acid group.

Examples of safener compounds C are benoxacor, cloquintocet, cyometrinil, cyprosulfamide, dichlormid, dicyclonon, dietholate, fenchlorazole, fenclorim, flurazole, fluxofenim, furilazole, isoxadifen, mefenpyr, mephenate, naphthalic anhydride, oxabetrinil, 4-(dichloroacetyl)-1-oxa-4-azaspiro[4.5]decane (MON4660, CAS 71526-07-3), 2,2,5-trimethyl-3-(dichloroacetyl)-1,3-oxa-zolidine (R-29148, CAS 52836-31-4), metcamifen and BPCMS (CAS 54091-06-4).

The active compounds B of groups b1) to b15) and the active compounds C are known herbicides and safeners, see, for example, The Compendium of Pesticide Common Names (http://www.alanwood.net/pesticides/); Farm Chemicals Handbook 2000 volume 86, Meister Publishing Company, 2000; B. Hock, C. Fedtke, R. R. Schmidt, Herbizide [Herbicides], Georg Thieme Verlag, Stuttgart 1995; W. H. Ahrens, Herbicide Handbook, 7th edition, Weed Science Society of America, 1994; and K. K. Hatzios, Herbicide Handbook, Supplement for the 7th edition, Weed Science Society of America, 1998. 2,2,5-Trimethyl-3-(dichloroacetyl)-1,3-oxazolidine [CAS No. 52836-31-4] is also referred to as R-29148. 4-(Dichloroacetyl)-1-oxa-4-azaspiro[4.5]decane [CAS No. 71526-07-3] is also referred to as AD-67 and MON 4660.

The assignment of the active compounds to the respective mechanisms of action is based on current knowledge. If several mechanisms of action apply to one active compound, this substance was only assigned to one mechanism of action.

The invention also relates to formulations comprising at least an auxiliary and at least one compound of formula (I) according to the invention.

A formulation comprises a pesticidally effective amount of a compound of formula (I). The term "effective amount" denotes an amount of the combination or of the compound of formula (I), which is sufficient for controlling undesired vegetation, especially for controlling undesired vegetation in crops (i.e. cultivated plants) and which does not result in a substantial damage to the treated crop plants. Such an amount can vary in a broad range and is dependent on various factors, such as the undesired vegetation to be controlled, the treated crop plants or material, the climatic conditions and the specific compound of formula (I) used.

The compounds of formula (I) or their salts can be converted into customary types of formulations, e. g. solutions, emulsions, suspensions, dusts, powders, pastes, granules, pressings, capsules, and mixtures thereof. Examples for formulation types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). These and further formulation types are defined in the "Catalogue of pesticide formulation types and international coding system", Technical Monograph No. 2, 6th Ed. May 2008, CropLife International.

The formulations are prepared in a known manner, such as described by Mollet and Grube-mann, Formulation technology, Wiley VCH, Weinheim, 2001; or Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetting agents, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e.g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharides, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emulsifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-substituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-substituted fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolygluco-sides. Examples of polymeric surfactants are home- or copolymers of vinylpyrrolidone, vinylal-cohols, or vinylacetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines or polyethyleneamines.

Suitable adjuvants are compounds, which have a neglectable or even no pesticidal activity themselves, and which improve the biological performance of the compounds of formula (I) on the target. Examples are surfactants, mineral or vegetable oils, and other auxiliaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), inorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea and glycerin.

Suitable anti-foaming agents are silicones, long chain alcohols, and salts of fatty acids.

Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo- and phthalocyanine colorants).

Suitable tackifiers or binders are polyvinylpyrrolidons, polyvinylacetates, polyvinyl alcohols, polyacrylates, biological or synthetic waxes, and cellulose ethers.

Examples for formulation types and their preparation are:
i) Water-soluble concentrates (SL, LS)
   10-60 wt% of a compound of formula (I) or a combination comprising at least one compound of formula (I) (component A) and at least one further compound selected from the herbicidal compounds B (component B) and safeners C (component C) according to the invention and 5-15 wt% wetting agent (e.g. alcohol alkoxylates) are dissolved in water and/or in a water-soluble solvent (e.g. alcohols) ad 100 wt%. The active substance dissolves upon dilution with water.
ii) Dispersible concentrates (DC)
   5-25 wt% of a compound of formula (I) or a combination comprising at least one compound of formula (I) (component A) and at least one further compound selected from the herbicidal compounds B (component B) and safeners C (component C) according to the invention and 1-10 wt% dispersant (e. g. polyvinylpyrrolidone) are dissolved in organic solvent (e.g. cyclohexanone) ad 100 wt%. Dilution with water gives a dispersion.
iii) Emulsifiable concentrates (EC) 15-70 wt% of compound of formula (I) or a combination comprising at least one compound of formula (I) (component A) and at least one further compound selected from the herbicidal compounds B (component B) and safeners C (component C) according to the invention and 5-10 wt% emulsifiers (e.g. calcium dodecylbenzenesulfonate and castor oil ethoxylate) are dissolved in water-insoluble organic solvent (e.g. aromatic hydrocarbon) ad 100 wt%. Dilution with water gives an emulsion.
iv) Emulsions (EW, EO, ES)
   5-40 wt% of compound of formula (I) or a combination comprising at least one compound of formula (I) (component A) and at least one further compound selected from the herbicidal compounds B (component B) and safeners C (component C) according to the invention and 1-10 wt% emulsifiers (e.g. calcium dodecylbenzenesulfonate and castor oil ethoxylate) are dissolved in 20-40 wt% water-insoluble organic solvent (e.g. aromatic hydrocarbon). This mixture is introduced into water ad 100 wt% by means of an emulsifying machine and made into a homogeneous emulsion. Dilution with water gives an emulsion.
v) Suspensions (SC, OD, FS)
   In an agitated ball mill, 20-60 wt% of a compound of formula (I) or a combination comprising at least one compound of formula (I) (component A) and at least one further compound selected from the herbicidal compounds B (component B) and safeners C (component C)according to the invention are comminuted with addition of 2-10 wt% dispersants and wetting agents (e.g. sodium lignosulfonate and alcohol ethoxylate), 0,1-2 wt% thickener (e.g. xanthan gum) and water ad 100 wt% to give a fine active substance suspension. Dilution with water gives a stable suspension of the active substance. For FS type formulation up to 40 wt% binder (e.g. polyvinylalcohol) is added.
vi) Water-dispersible granules and water-soluble granules (WG, SG)
   50-80 wt% of a compound of formula (I) or a combination comprising at least one compound of formula (I) (component A) and at least one further compound selected from the herbicidal compounds B (component B) and safeners C (component C)according to the invention are ground finely with addition of dispersants and wetting agents (e.g. sodium lignosulfonate and alcohol ethoxylate) ad 100 wt% and prepared as water-dispersible or water-soluble granules by means of technical appliances (e. g. extrusion, spray tower, fluidized bed). Dilution with water gives a stable dispersion or solution of the active substance.
vii) Water-dispersible powders and water-soluble powders (WP, SP, WS)
   50-80 wt% of a compound of formula (I) or a combination comprising at least one compound of formula (I) (component A) and at least one further compound selected from the herbicidal compounds B (component B) and safeners C (component C) according to the invention are ground in a rotor-stator mill with addition of 1-5 wt% dispersants (e.g. sodium lignosulfonate), 1-3 wt% wetting agents (e.g. alcohol ethoxylate) and solid carrier (e.g. silica gel) ad 100 wt%. Dilution with water gives a stable dispersion or solution of the active substance.
viii) Gel (GW, GF)
   In an agitated ball mill, 5-25 wt% of a compound of formula (I) or a combination comprising at least one compound of formula (I) (component A) and at least one further compound selected from the herbicidal compounds B (component B) and safeners C (component C) according to the invention are comminuted with addition of 3-10 wt% dispersants (e.g. sodium lignosulfonate), 1-5 wt% thickener (e.g. carboxymethylcellulose) and water ad 100 wt% to give a fine suspension of the active substance. Dilution with water gives a stable suspension of the active substance.
iv) Microemulsion (ME)
   5-20 wt% of a compound of formula (I) or a combination comprising at least one compound of formula (I) (component A) and at least one further compound selected from the herbicidal compounds B (component B) and safeners C (component C) according to the invention are added to 5-30 wt% organic solvent blend (e.g. fatty acid dimethylamide and cyclohexanone), 10-25 wt% surfactant blend (e.g. alcohol ethoxylate and arylphenol ethoxylate), and water ad 100 %. This mixture is stirred for 1 h to produce spontaneously a thermodynamically stable microemulsion.
iv) Microcapsules (CS)
   An oil phase comprising 5-50 wt% of a compound of formula (I) or a combination comprising at least one compound of formula (I) (component A) and at least one further compound selected from the herbicidal compounds B (component B) and safeners C (component C) according to the invention, 0-40 wt% water insoluble organic solvent (e.g. aromatic hydrocarbon), 2-15 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 wt% of a compound of formula (I) according to the invention, 0-40 wt% water insoluble organic solvent (e.g. aromatic hydrocarbon), and an isocyanate monomer (e.g. diphenylmethene-4,4'-diisocyanate) are dispersed into an aqueous solution of a protective colloid (e.g. polyvinyl alcohol). The addition of a polyamine (e.g. hexamethylenediamine) results in the formation of polyurea microcapsules. The monomers amount to 1-10 wt%. The wt% relate to the total CS formulation.
ix) Dustable powders (DP, DS)
   1-10 wt% of a compound of formula (I) or a combination comprising at least one compound of formula (I) (component A) and at least one further compound selected from the herbicidal compounds B (component B) and safeners C (component C) according to the invention are ground finely and mixed intimately with solid carrier (e.g. finely divided kaolin) ad 100 wt%.
x) Granules (GR, FG)
   0.5-30 wt% of a compound of formula (I) or a combination comprising at least one compound of formula (I) (component A) and at least one further compound selected from the herbicidal compounds B (component B) and safeners C (component C) according to the invention is ground finely and associated with solid carrier (e.g. silicate) ad 100 wt%. Granulation is achieved by extrusion, spray-drying or the fluidized bed.
xi) Ultra-low volume liquids (UL)
   1-50 wt% of a compound of formula (I) or a combination comprising at least one compound of formula (I) (component A) and at least one further compound selected from the herbicidal compounds B (component B) and safeners C (component C) according to the invention are dissolved in organic solvent (e.g. aromatic hydrocarbon) ad 100 wt%.

The formulation types i) to xi) may optionally comprise further auxiliaries, such as 0,1-1 wt% bactericides, 5-15 wt% anti-freezing agents, 0,1-1 wt% anti-foaming agents, and 0,1-1 wt% colorants.

The formulations and/or combinations generally comprise between 0.01 and 95%, preferably between 0.1 and 90%, and in particular between 0.5 and 75%, by weight of the compounds of formula (I).

The compounds of formula (I) are employed in a purity of from 90% to 100%, preferably from 95% to 100% (according to NMR spectrum).

Solutions for seed treatment (LS), suspoemulsions (SE), flowable concentrates (FS), powders for dry treatment (DS), water-dispersible powders for slurry treatment (WS), water-soluble powders (SS), emulsions (ES), emulsifiable concentrates (EC) and gels (GF) are usually employed for the purposes of treatment of plant propagation materials, particularly seeds. The formulations in question give, after two-to-tenfold dilution, active substance concentrations of from 0.01 to 60% by weight, preferably from 0.1 to 40% by weight, in the ready-to-use preparations. (nach unten verschoben)

Methods for applying compounds of formula (I), formulations and /or combinations thereof, on to plant propagation material, especially seeds, include dressing, coating, pelleting, dusting, soaking and in-furrow application methods of the propagation material. Preferably, compounds of formula (I), formulations and /or combinations thereof, respectively, are applied on to the plant propagation material by a method such that germination is not induced, e. g. by seed dressing, pelleting, coating and dusting.

Various types of oils, wetting agents, adjuvants, fertilizer, or micronutrients, and further pesticides (e.g. herbicides, insecticides, fungicides, growth regulators, safeners) may be added to the compounds of formula (I), the formulations and/or the combinations comprising them as premix or, if appropriate not until immediately prior to use (tank mix). These agents can be admixed with the formulations according to the invention in a weight ratio of 1:100 to 100:1, preferably 1:10 to 10:1.

The user applies the compounds of formula (I) according to the invention, the formulations and/or the combinations comprising them usually from a pre-dosage device, a knapsack sprayer, a spray tank, a spray plane, or an irrigation system. Usually, the formulation is made up with water, buffer, and/or further auxiliaries to the desired application concentration and the ready-to-use spray liquor or the formulation according to the invention is thus obtained. Usually, 20 to 2000 liters, preferably 50 to 400 liters, of the ready-to-use spray liquor are applied per hectare of agricultural useful area.

According to one embodiment, either individual components of the formulation according to the invention or partially premixed components, e. g. components comprising compounds of formula (I) and optionally active substances from the groups B and/or C), may be mixed by the user in a spray tank and further auxiliaries and additives may be added, if appropriate.

In a further embodiment, individual components of the formulation according to the invention such as parts of a kit or parts of a binary or ternary mixture may be mixed by the user himself in a spray tank and further auxiliaries may be added, if appropriate.

In a further embodiment, either individual components of the formulation according to the invention or partially premixed components, e. g components comprising compounds of formula (I) and optionally active substances from the groups B and/or C), can be applied jointly (e.g. after tank mix) or consecutively.

The compounds of formula (I), are suitable as herbicides. They are suitable as such, as an appropriate formulation or in combination with at least one further compound selected from the herbicidal active compounds B (component B) and safeners C (component C).

The compounds of formula (I), or the formulations and /or combinations comprising the compounds of formula (I), control undesired vegetation on non-crop areas very efficiently, especially at high rates of application. They act against broad-leaved weeds and grass weeds in crops such as wheat, rice, maize, soya and cotton without causing any significant damage to the crop plants. This effect is mainly observed at low rates of application.

In particular, the compounds of formula (I), or the formulations and /or combinations comprising the compounds of formula (I) are useful for controlling at least one of the following undesired plant species: *Abutilon theophrasti* (ABUTH), *Alopercurus myosuroides* (ALOMY), *Amaranthus retroflexus* (AMARE), *Apera spica-venti* (APESV), *Avena fatua* (AVEFA), *Echinocloa crus-galli* (ECHCG), *Lolium multiflorum* (LOLMU), *Fallopia convolvulus* (POLCO), *Setaria viridis* (SETVI), *Setaria faberi* (SETFA).

The compounds of formula (I), or the formulations and/or the combinations comprising them, are applied to the plants mainly by spraying the leaves. Here, the application can be carried out using, for example, water as carrier by customary spraying techniques using spray liquor amounts of from about 100 to 1000 I/ha (for example from 300 to 400 I/ha). The compounds of formula (I), or the formulations and/or the combinations comprising them, may also be applied by the low-volume or the ultra-low-volume method, or in the form of microgranules.

Application of the compounds of formula (I), or the formulations and/or the combinations comprising them, can be done before, during and/or after, preferably during and/or after, the emergence of the undesired vegetation.

Application of the compounds of formula (I), or the formulations and/or the combinations can be carried out before or during sowing.

The compounds of formula (I), or the formulations and/or the combinations comprising them, can be applied pre-, post-emergence or pre-plant, or together with the seed of a crop plant. It is also possible to apply the compounds of formula (I), or the formulations and/or the combinations comprising them, by applying seed, pretreated with the compounds of formula (I), or the formulations and/or the combinations comprising them, of a crop plant. If the active ingredients are less well tolerated by certain crop plants, application techniques may be used in which the combinations are sprayed, with the aid of the spraying equipment, in such a way that as far as possible they do not come into contact with the leaves of the sensitive crop plants, while the active ingredients reach the leaves of undesired vegetation growing underneath, or the bare soil surface (post-directed, lay-by).

In a further embodiment, the compounds of formula (I), or the formulations and/or the combinations comprising them, can be applied by treating seed. The treatment of seeds comprises essentially all procedures familiar to the person skilled in the art (seed dressing, seed coating, seed dusting, seed soaking, seed film coating, seed multilayer coating, seed encrusting, seed dripping and seed pelleting) based on the compounds of formula (I), or the formulations and/or the combinations prepared therefrom. Here, the combinations can be applied diluted or undiluted.

The term "seed" comprises seed of all types, such as, for example, corns, seeds, fruits, tubers, seedlings and similar forms. Here, preferably, the term seed describes corns and seeds. The seed used can be seed of the crop plants mentioned above, but also the seed of transgenic plants or plants obtained by customary breeding methods.

When employed in plant protection, the amounts of active substances applied, i.e. the compounds of formula (I), component B and, if appropriate, component C without formulation auxiliaries, are, depending on the kind of effect desired, from 0.001 to 2 kg per ha, preferably from 0.005 to 2 kg per ha, more preferably from 0.05 to 0.9 kg per ha and in particular from 0.1 to 0.75 kg per ha.

In another embodiment of the invention, the application rate of the compounds of formula (I), component B and, if appropriate, component C, is from 0.001 to 3 kg/ha, preferably from 0.005 to 2.5 kg/ha and in particular from 0.01 to 2 kg/ha of active substance (a.s.).

In another preferred embodiment of the invention, the rates of application of the compounds of formula (I) according to the present invention (total amount of compounds of formula (I)) are from 0.1 g/ha to 3000 g/ha, preferably 10 g/ha to 1000 g/ha, depending on the control target, the season, the target plants and the growth stage.

In another preferred embodiment of the invention, the application rates of the compounds of formula (I) are in the range from 0.1 g/ha to 5000 g/ha and preferably in the range from 1 g/ha to 2500 g/ha or from 5 g/ha to 2000 g/ha.

In another preferred embodiment of the invention, the application rate of the compounds of formula (I) is 0.1 to 1000 g/ha, preferably1 to 750 g/ha, more preferably 5 to 500 g/ha.

The required application rates of herbicidal compounds B are generally in the range of from 0.0005 kg/ha to 2.5 kg/ha and preferably in the range of from 0.005 kg/ha to 2 kg/ha or 0.01 kg/ha to 1.5 kg/h of a.s.

The required application rates of safeners C are generally in the range of from 0.0005 kg/ha to 2.5 kg/ha and preferably in the range of from 0.005 kg/ha to 2 kg/ha or 0.01 kg/ha to 1.5 kg/h of a.s.

In treatment of plant propagation materials such as seeds, e. g. by dusting, coating or drenching seed, amounts of active substance of from 0.1 to 1000 g, preferably from 1 to 1000 g, more preferably from 1 to 100 g and most preferably from 5 to 100 g, per 100 kilogram of plant propagation material (preferably seeds) are generally required.

In another embodiment of the invention, to treat the seed, the amounts of active substances applied, i.e. the compounds of formula (I), component B and, if appropriate, component C are generally employed in amounts of from 0.001 to 10 kg per 100 kg of seed.

When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

In case of combinations according to the present invention it is immaterial whether the compounds of formula (I), and the further component B and/or the component C are formulated and applied jointly or separately.

In the case of separate application, it is of minor importance, in which order the application takes place. It is only necessary, that the compounds of formula (I), and the further component B and/or the component C are applied in a time frame that allows simultaneous action of the active ingredients on the plants, preferably within a time-frame of at most 14 days, in particular at most 7 days.

Depending on the application method in question, the compounds of formula (I), or the formulations and /or combinations comprising them, can additionally be employed in a further number of crop plants for eliminating undesired vegetation. Examples of suitable crops are the following:
*Allium cepa, Ananas comosus, Arachis hypogaea, Asparagus officinalis, Avena sativa, Beta vulgaris spec. altissima, Beta vulgaris spec. rapa, Brassica napus var. napus, Brassica napus var. napobrassica, Brassica rapa var. silvestris, Brassica oleracea, Brassica nigra, Camellia sinensis, Carthamus tinctorius, Carya illinoinensis, Citrus limon, Citrus sinensis,* Coffea *arabica* (Coffea *canephora,* Coffea *liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pistacia vera, Pisum sativum, Prunus avium, Prunus persica, Pyrus communis, Prunus armeniaca, Prunus cerasus, Prunus dulcis and Prunus domestica, Ribes sylvestre, Ricinus communis, Saccharum officinarum, Secale cereale, Sinapis alba, Solanum tuberosum, Sorghum bicolor (s. vulgare), Theobroma* cacao, *Trifolium pratense, Triticum aestivum, Triticale, Triticum durum, Vicia faba, Vitis vinifera* and *Zea mays.*

Preferred crops are *Arachis hypogaea, Beta vulgaris spec. altissima, Brassica napus var. napus, Brassica oleracea, Citrus limon, Citrus sinensis,* Coffea *arabica (*Coffea *canephora,* Coffea *liberica), Cynodon dactylon, Glycine max, Gossypium hirsutum, (Gossypium arboreum, Gossypium herbaceum, Gossypium vitifolium), Helianthus annuus, Hordeum vulgare, Juglans regia, Lens culinaris, Linum usitatissimum, Lycopersicon lycopersicum, Malus spec., Medicago sativa, Nicotiana tabacum (N.rustica), Olea europaea, Oryza sativa, Phaseolus lunatus, Phaseolus vulgaris, Pistacia vera, Pisum sativum, Prunus dulcis, Saccharum officinarum, Secale cereale, Solanum tuberosum, Sorghum bicolor (s. vulgare), Triticale, Triticum aestivum, Triticum durum, Vicia faba, Vitis vinifera* and *Zea mays.*

Especially preferred crops are crops of cereals, corn, soybeans, rice, oilseed rape, cotton, potatoes, peanuts or permanent crops.

The compounds of formula (I) according to the invention, or the formulations and /or combinations comprising them, can also be used in crops which have been modified by mutagenesis or genetic engineering in order to provide a new trait to a plant or to modify an already present trait.

The term "crops" as used herein includes also (crop) plants which have been modified by mutagenesis or genetic engineering in order to provide a new trait to a plant or to modify an already present trait.

Mutagenesis includes techniques of random mutagenesis using X-rays or mutagenic chemicals, but also techniques of targeted mutagenesis, in order to create mutations at a specific locus of a plant genome. Targeted mutagenesis techniques frequently use oligonucleotides or proteins like CRISPR/Cas, zinc-finger nucleases, TALENs or meganucleases to achieve the targeting effect.

Genetic engineering usually uses recombinant DNA techniques to create modifications in a plant genome which under natural circumstances cannot readily be obtained by cross breeding, mutagenesis or natural recombination. Typically, one or more genes are integrated into the genome of a plant in order to add a trait or improve a trait. These integrated genes are also referred to as transgenes in the art, while plant comprising such transgenes are referred to as transgenic plants. The process of plant transformation usually produces several transformation events, which differ in the genomic locus in which a transgene has been integrated. Plants comprising a specific transgene on a specific genomic locus are usually described as comprising a specific "event", which is referred to by a specific event name. Traits which have been introduced in plants or have been modified include in particular herbicide tolerance, insect resistance, increased yield and tolerance to abiotic conditions, like drought.

Herbicide tolerance has been created by using mutagenesis as well as using genetic engineering. Plants which have been rendered tolerant to acetolactate synthase (ALS) inhibitor herbicides by conventional methods of mutagenesis and breeding comprise plant varieties commercially available under the name Clearfield^{®}. However, most of the herbicide tolerance traits have been created via the use of transgenes.

Herbicide tolerance has been created to glyphosate, glufosinate, 2,4-D, dicamba, oxynil herbicides, like bromoxynil and ioxynil, sulfonylurea herbicides, ALS inhibitor herbicides and 4-hy-droxyphenylpyruvate dioxygenase (HPPD) inhibitors, like isoxaflutole and mesotrione.

Transgenes which have been used to provide herbicide tolerance traits comprise: for tolerance to glyphosate: cp4 epsps, epsps grg23ace5, mepsps, 2mepsps, gat4601, gat4621 and goxv247, for tolerance to glufosinate: pat and bar, for tolerance to 2,4-D: aad-1 and aad-12, for tolerance to dicamba: dmo, for tolerance to oxynil herbicies: bxn, for tolerance to sulfonylurea herbicides: zm-hra, csr1-2, gm-hra, S4-HrA, for tolerance to ALS inhibitor herbicides: csr1-2, for tolerance to HPPD inhibitor herbicides: hppdPF, W336 and avhppd-03.

Transgenic corn events comprising herbicide tolerance genes are for example, but not excluding others, DAS40278, MON801, MON802, MON809, MON810, MON832, MON87411, MON87419, MON87427, MON88017, MON89034, NK603, GA21, MZHG0JG, HCEM485, VCO-Ø1981-5, 676, 678, 680, 33121, 4114, 59122, 98140, Bt10, Bt176, CBH-351, DBT418, DLL25, MS3, MS6, MZIR098, T25, TC1507 and TC6275.

Transgenic soybean events comprising herbicide tolerance genes are for example, but not excluding others, GTS 40-3-2, MON87705, MON87708, MON87712, MON87769, MON89788, A2704-12, A2704-21, A5547-127, A5547-35, DP356043, DAS44406-6, DAS68416-4, DAS-81419-2, GU262, SYHTØH2, W62, W98, FG72 and CV127.

Transgenic cotton events comprising herbicide tolerance genes are for example, but not excluding others, 19-51a, 31707, 42317, 81910, 281-24-236, 3006-210-23, BXN10211, BXN10215, BXN10222, BXN10224, MON1445, MON1698, MON88701, MON88913, GHB119, GHB614, LLCotton25, T303-3 and T304-40.

Transgenic canola events comprising herbicide tolerance genes are for example, but not excluding others, MON88302, HCR-1, HCN10, HCN28, HCN92, MS1, MS8, PHY14, PHY23, PHY35, PHY36, RF1, RF2 and RF3.

Insect resistance has mainly been created by transferring bacterial genes for insecticidal proteins to plants. Transgenes which have most frequently been used are toxin genes of *Bacillus spec.* and synthetic variants thereof, like cry1A, cry1Ab, cry1Ab-Ac, cry1Ac, cry1A.105, cry1F, cry1Fa2, cry2Ab2, cry2Ae, mcry3A, ecry3.1Ab, cry3Bb1, cry34Ab1, cry35Ab1, cry9C, vip3A(a), vip3Aa20. However, also genes of plant origin have been transferred to other plants. In particular genes coding for protease inhibitors, like CpTI and pinll. A further approach uses transgenes in order to produce double stranded RNA in plants to target and downregulate insect genes. An example for such a transgene is dvsnf7.

Transgenic corn events comprising genes for insecticidal proteins or double stranded RNA are for example, but not excluding others, Bt10, Bt11, Bt176, MON801, MON802, MON809, MON810, MON863, MON87411, MON88017, MON89034, 33121, 4114, 5307, 59122, TC1507, TC6275, CBH-351, MIR162, DBT418 and MZIR098.

Transgenic soybean events comprising genes for insecticidal proteins are for example, but not excluding others, MON87701, MON87751 and DAS-81419.

Transgenic cotton events comprising genes for insecticidal proteins are for example, but not excluding others, SGK321, MON531, MON757, MON1076, MON15985, 31707, 31803, 31807, 31808, 42317, BNLA-601, Event1, COT67B, COT102, T303-3, T304-40, GFM Cry1A, GK12, MLS 9124, 281-24-236, 3006-210-23, GHB119 and SGK321.

Increased yield has been created by increasing ear biomass using the transgene athb17, being present in corn event MON87403, or by enhancing photosynthesis using the transgene bbx32, being present in the soybean event MON87712.

Crops comprising a modified oil content have been created by using the transgenes: gm-fad2-1, Pj.D6D, Nc.Fad3, fad2-1A and fatb1-A. Soybean events comprising at least one of these genes are: 260-05, MON87705 and MON87769.

Tolerance to abiotic conditions, in particular to tolerance to drought, has been created by using the transgene cspB, comprised by the corn event MON87460 and by using the transgene Hahb-4, comprised by soybean event IND-ØØ41Ø-5.

Traits are frequently combined by combining genes in a transformation event or by combining different events during the breeding process. Preferred combination of traits are herbicide tolerance to different groups of herbicides, insect tolerance to different kind of insects, in particular tolerance to lepidopteran and coleopteran insects, herbicide tolerance with one or several types of insect resistance, herbicide tolerance with increased yield as well as a combination of herbicide tolerance and tolerance to abiotic conditions.

Plants comprising singular or stacked traits as well as the genes and events providing these traits are well known in the art. For example, detailed information as to the mutagenized or integrated genes and the respective events are available from websites of the organizations "International Service for the Acquisition of Agri-biotech Applications (ISAAA)" (http://www.isaaa.org/gmapprovaldatabase) and the "Center for Environmental Risk Assessment (CERA)" (http://cera-gmc.org/GMCropDatabase), as well as in patent applications, like EP3028573 and WO2017/011288.

The use of the compounds of formula (I) or formulations or combinations comprising them according to the invention on crops may result in effects which are specific to a crop comprising a certain gene or event. These effects might involve changes in growth behavior or changed resistance to biotic or abiotic stress factors. Such effects may in particular comprise enhanced yield, enhanced resistance or tolerance to insects, nematodes, fungal, bacterial, mycoplasma, viral or viroid pathogens as well as early vigor, early or delayed ripening, cold or heat tolerance as well as changed amino acid or fatty acid spectrum or content.

Furthermore, plants are also covered that contain by the use of recombinant DNA techniques a modified amount of ingredients or new ingredients, specifically to improve raw material production, e.g., potatoes that produce increased amounts of amylopectin (e.g. Amflora^{®} potato, BASF SE, Germany).

Furthermore, it has been found that the compounds of formula (I) according to the invention, or the formulations and /or combinations comprising them, are also suitable for the defoliation and/or desiccation of plant parts of crops such as cotton, potato, oilseed rape, sunflower, soybean or field beans, in particular cotton. In this regard, formulations and /or combinations for the desiccation and/or defoliation of crops, processes for preparing these formulations and /or combinations and methods for desiccating and/or defoliating plants using the compounds of formula (I) have been found.

As desiccants, the compounds of formula (I) are particularly suitable for desiccating the above-ground parts of crop plants such as potato, oilseed rape, sunflower and soybean, but also cereals. This makes possible the fully mechanical harvesting of these important crop plants.

Also of economic interest is to facilitate harvesting, which is made possible by concentrating within a certain period of time the dehiscence, or reduction of adhesion to the tree, in citrus fruit, olives and other species and varieties of pernicious fruit, stone fruit and nuts. The same mechanism, i.e. the promotion of the development of abscission tissue between fruit part or leaf part and shoot part of the plants is also essential for the controlled defoliation of useful plants, in particular cotton.

Moreover, a shortening of the time interval in which the individual cotton plants mature leads to an increased fiber quality after harvesting.

### Examples

### A Chemistry Examples

Chemical bonds, drawn as bars in chemical formulae, indicate the relative stereochemistry on the ring system.

### Example 1: Synthesis of 2-[(3,5-dichlorophenyl)carbamoyl]oxetane-2-carboxylic acid (Inter A)

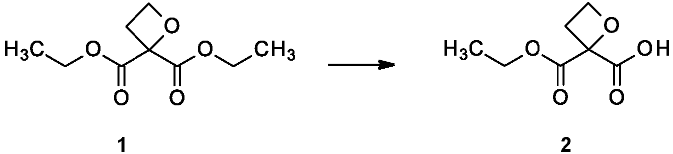

A solution of lithium hydroxide (118 mg, 4.95 mmol) in water was added dropwise to mixture of 2,2-diethoxyoxetane-2,2-dicarboxylat (**1**) (CAS [1384465-73-9]) (1000 mg, 4.95 mmol), tetrahydrofuran (THF) (50 ml) and water (50 ml) and the reaction mixture was stirred at room temperature overnight. THF was evaporated *in vacuo* and the remainder washed with methyl t-butyl ether. The aqueous solution was concentrated *in vacuo* and the remainder dried to give the product (2) (740 mg, 86% yield). 1H NMR (500 MHz, Deuterium Oxide) δ 4.55 (t, 2H), 4.28 (m, 2H), 3.13 (m, 1H), 2.91 (m, 1H), 1.29 (t, 3H).

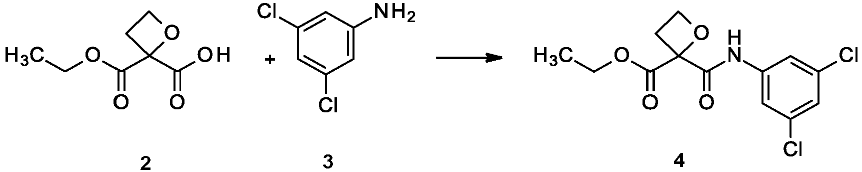

To a solution of ethyl 2-[(3,5-dichlorophenyl)carbamoyl]oxetane-2-carboxylate (**2**) (3.52 g, 19.6 mmol) in dimethylformamide (DMF) aniline **3** (3.17 g, 19.6 mmol) was added. To the resulting solution was added HATU (2-(7-aza-1H-benzotriazole-1-yl)-1,1,3,3-tetramethyluronium hexafluorophosphate, CAS [148893-10-1]), (8.18 g, 21.5 mmol) and then diisopropylethylamine (16.6 mL). The resulting reaction mixture was stirred at room temperature overnight. To the reaction mixture water and sodium bicarbonate solution were added. The reaction mixture was extracted with ethyl acetate, washed with water, dried (sodium sulfate) and the solvent was evaporated under reduced pressure. The crude product was purified by column chromatography using ethyl acetate as solvent yielding ethyl 2-[(3,5-dichlorophenyl)carbamoyl]oxetane-2-carboxylate (**4**) (1.6 g, 26%). 1H NMR (500 MHz, Chloroform-d) δ 8.71 (s, 1H), 7.61 (d, 2H), 7.16 (d, 1H), 4.74 (dt, 1H), 4.66 (m, 1H), 4.31 (q, 2H), 3.42 (ddd, 1H), 2.95 (ddd, 1H), 1.32 (t, 3H).

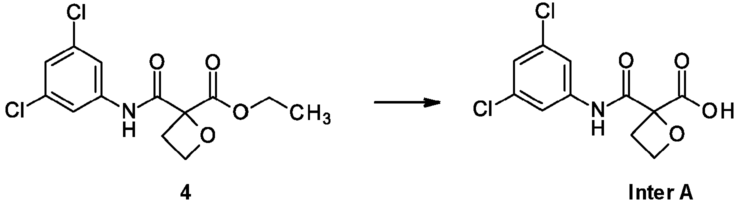

A solution of lithium hydroxide (23 mg, 3.8 mmol) in water was added dropwise to mixture of ethyl 2-[(3,5-dichlorophenyl)carbamoyl]oxetane-2-carboxylate (**4**) (150 mg, 0.472 mmol), tetrahydrofuran (THF) (10 ml) and water (10 ml) and the reaction mixture was stirred at room temperature overnight. THF was evaporated *in vacuo* and the remainder washed with methyl t-butyl ether (MTBE). The aqueous solution was concentrated *in vacuo* and the remainder dried to give the carboxylic acid Inter A (130 mg, 95% yield). 1H NMR (500 MHz, Methanol-d4) δ 7.76 (d, 2H), 7.21 (t, 1H), 6.56 (m, 1H), 4.67 (t, 2H), 3.31 (m, 1H), 2.94 (dt, 1H).

### Example 2:

### Synthesis of methyl (1S,4R)-4-[[2-[(3,5-dichlorophenyl)carbamoyl]oxetane-2-carbonyl]amino]cy-clopent-2-ene-1-carboxylate (1:1 mixture of diastereomers) - Compound I.6

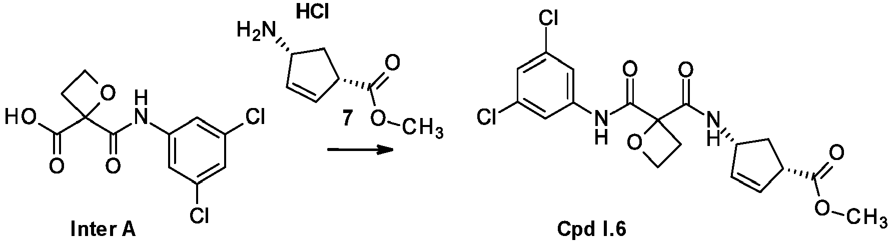

To a solution of carboxylic acid Inter A (330 mg, 1.14 mmol) in dimethylformamide (DMF) methyl (1S,4R)-4-aminocyclopent-2-ene-1-carboxylate hydrochloride (**7**, CAS [180196-56-9]) (202 mg, 1.14 mmol) was added. To the resulting solution was added HATU (2-(7-aza-1H-benzotriazole-1-yl)-1,1,3,3-tetramethyluronium hexafluorophosphate, CAS [148893-10-1]), (476 mg, 1.25 mmol) and then diisopropylethylamine (0.76 mL). The resulting reaction mixture was stirred at room temperature overnight. To the reaction mixture water and sodium bicarbonate solution were added. The reaction mixture was extracted with ethyl acetate, washed with water, dried (sodium sulfate) and the solvent was evaporated under reduced pressure. The crude product was purified by column chromatography using ethyl acetate as solvent yielding compound I.6 (62 mg, 13%, 1:1 mixture of diastereomers). 1H NMR (400 MHz, Chloroform-d) δ 9.37 (s, 1H), 9.25 (s, 1H), 7.59 (m, 4H), 7.12 (m, 2H), 5.94 (m, 4H), 5.08 (m, 2H), 4.73 (m, 4H), 3.74 (s, 3H), 3.73 (s, 3H), 3.56 (m, 2H), 3.05 (m, 4H), 2.51 (m, 2H), 2.05 (dt, 1H), 1.95 (dt, 1H).

### Example 3:

### Synthesis of methyl (3S)-3-[[2-[(3,5-dichlorophenyl)carbamoyl]oxetane-2-carbonyl]amino]butanoate (1:1 mixture of diastereomers) - Compound I.7

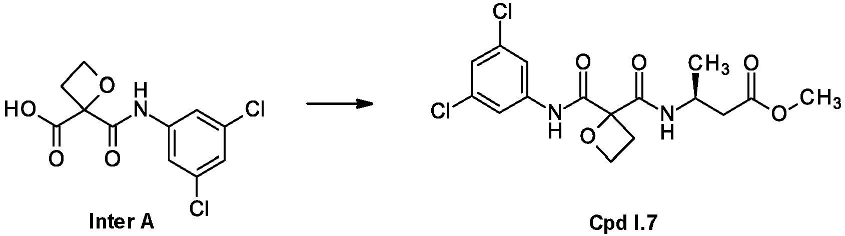

To a solution of carboxylic acid Inter A (130 mg, 0.448 mmol) in dimethylformamide (DMF, 5 mL) (3S)-3-aminobutanoate (S-homoalanine) hydrochloride (89 mg, 0.081 mmol) (CAS [139243-55-3]) was added. To the resulting solution was added HATU (2-(7-aza-1H-benzotriazole-1-yl)-1,1,3,3-tetramethyluronium hexafluorophosphate, CAS [148893-10-1]), (187 mg, 0.493 mmol) and then triethylamine (0.22 mL). The resulting reaction mixture was stirred at room temperature overnight. To the reaction mixture water and sodium bicarbonate solution were added. The reaction mixture was extracted with ethyl acetate, washed with water, dried (sodium sulfate) and the solvent was evaporated under reduced pressure. The crude product was purified by column chromatography using ethyl acetate as solvent yielding compound I.7 (102 mg, 58%, 1:1 mixture of diastereomers). 1H NMR (400 MHz, Chloroform-d) δ 9.68 (s, 1H), 9.48 (s, 1H), 7.76 (m, 6H), 7.15 (m, 2H), 4.57 (m, 5H), 4.32 (dqd, 2H), 3.62 (s, 3H), 3.58 (s, 3H), 2.97 (m, 4H), 2.55 (m, 4H), 1.24 (d, 3H), 1.21 (d, 3H).

### Example 4:

### Synthesis of methyl (1S,4R)-4-[[2-[(3,5-difluorophenyl)carbamoyl]oxetane-2-carbonyl]amino]cy-clopent-2-ene-1-carboxylate (1:1 mixture of diastereomers) - Compound I.1

In a similar way to the synthesis for Inter A described above starting from ethyl 2-[(3,5-dichlorophenyl)carbamoyl]oxetane-2-carboxylate, Inter B was obtained as an off-white solid by saponification of ethyl 1-(3,5-fluorophenyl)-3-methyl-2-oxo-azetidine-3-carboxylate.

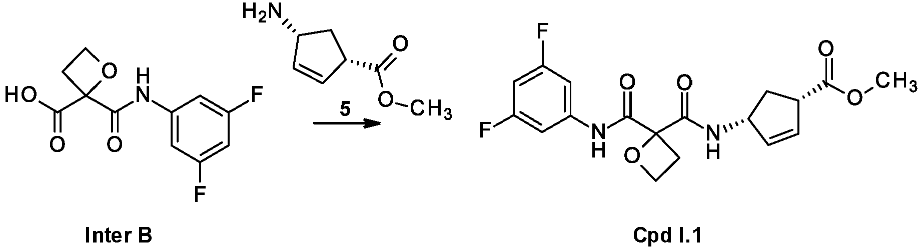

To a solution of carboxylic acid Inter B (130 mg, 0.506 mmol) in dimethylformamide (DMF) methyl (1S,4R)-4-aminocyclopent-2-ene-1-carboxylate (5, CAS [229613-83-6]) (99 mg, 0.56 mmol) was added. To the resulting solution was added HATU (2-(7-aza-1H-benzotriazole-1-yl)-1,1,3,3-tetramethyluronium hexafluorophosphate, CAS [148893-10-1]), (0.21 g, 0.56 mmol) and then diisopropylethylamine (2.5 mL). The resulting reaction mixture was stirred at room temperature overnight. To the reaction mixture water and sodium bicarbonate solution were added. The reaction mixture was extracted with ethyl acetate, washed with water, dried (sodium sulfate) and the solvent was evaporated under reduced pressure. The crude product was purified by column chromatography using ethyl acetate as solvent yielding compound I.1 (105 mg, 56%, 1:1 mixture of diastereomers). 1H NMR: (400 MHz, Chloroform-d) δ 9.39 (s, 1H), 9.28 (s, 1H), 7.58 (s, 2H), 7.23 (d, 4H), 6.58 (t, 2H), 5.99 (d, 2H), 5.08 (s, 2H), 4.75 (m, 3H), 3.74 (s, 3H), 3.73 (s, 3H), 3.57 (m, 2H), 3.04 (dd, 3H), 2.50 (m, 2H), 2.05 (d, 1H), 1.95 (d, 1H).

### Example 5:

### Synthesis of methyl (3S)-3-[[2-[(3,5-difluorophenyl)carbamoyl]oxetane-2-carbonyl]amino]butanoate (1:1 mixture of diastereomers) - Compound I.2.

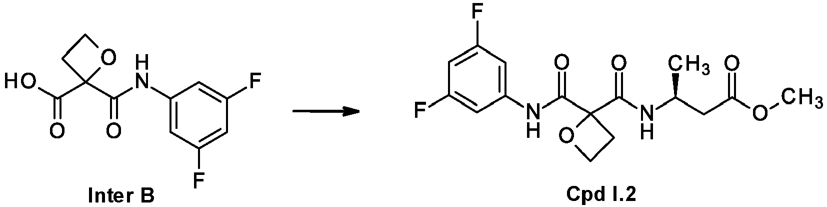

To a solution of carboxylic acid Inter B (19 mg, 0.074 mmol) in dimethylformamide (DMF) (3S)-3-aminobutanoate (S-homoalanine) hydrochloride (12 mg, 0.081 mmol) (CAS [139243-55-3]) was added. To the resulting solution was added HATU (2-(7-aza-1H-benzotriazole-1-yl)-1,1,3,3-tetramethyluronium hexafluorophosphate, CAS [148893-10-1]), (31 mg, 0.081 mmol) and then diisopropylethylamine (0.22 mL). The resulting reaction mixture was stirred at room temperature overnight. To the reaction mixture water and sodium bicarbonate solution were added. The reaction mixture was extracted with ethyl acetate, washed with water, dried (sodium sulfate) and the solvent was evaporated under reduced pressure. The crude product was purified by column chromatography using ethyl acetate as solvent yielding compound I.2 (17 mg, 65%, 1:1 mixture of diastereomers). 1H NMR (400 MHz, THF-d8) δ 9.72 (s, 1H), 9.52 (s, 1H), 7.75 (m, 2H), 7.40 (m, 4H), 6.67 (m, 2H), 4.56 (m, 3H), 4.31 (dq, 1H), 3.58 (m, 6H), 2.98 (m, 2H), 2.51 (m, 4H), 1.25 (d, 3H), 1.21 (d, 3H).

### Example 6:

### Synthesis of 5-[(3,5-dichlorophenyl)carbamoyl]-hydrofuran-5-carboxylic acid (Inter C)

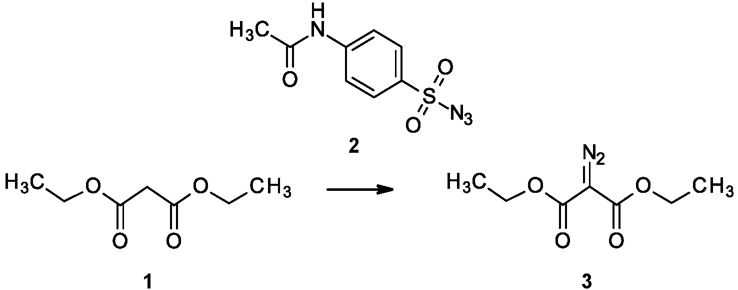

To a mixture of diethyl malonate (1) (50 g, 310 mmol) (CAS [53051-81-3]) and triethylamine (75 g, 744 mmol) in MeCN (500 ml) was added 4-acetamidobenzenesulfonyl azide (2) (p-ABSA, 12 g, 465 mmol) (CAS [2158-14-7]) at 20°C. The mixture was stirred at 20°C for 16 h. The reaction was filtered and the filtrate was concentrated. Dichloromethane (100 mL) was added to the filtrate. The mixture was filtered and the filtrate was concentrated. The crude was purified by HPLC (EtOAc/PE = 0%~100%) to give diethyl 2-diazopropanedioate (2) (62 g, quant.) as yellow oil. 1H NMR (400 MHz, Chloroform-d) δ 4.31 (q, 4H), 1.32 (t, 6H).

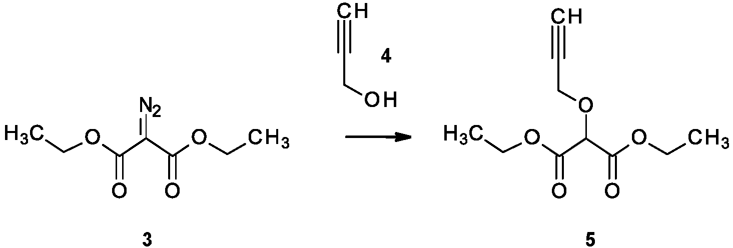

To a solution of diethyl 2-diazopropanedioate (3) (62 g, 330 mmol) and propargyl alcohol (4) (18.5 g, 330 mmol) (CAS [107-19-7]) in toluene (600 ml) was added Rh₂(OAc)₄ (1.3 g, 2.9 mmol) (CAS [15956-28-2]) at 20°C. The mixture was stirred at 60°C for 1h. The reaction was filtered and the filtrated was concentrated. The crude was purified by HPLC (EtOAc/PE = 0%~100%) to give the diethyl 2-prop-2-ynoxypropanedioate (5) (53 g, 80% over two steps) as yellow oil. 1H NMR (400 MHz, Chloroform-d) δ 4.82 - 4.80 (s, 1H), 4.41 (d, 2H), 4.28 (m, 4H), 2.53 (t, 1H), 1.31 (t, 6H).

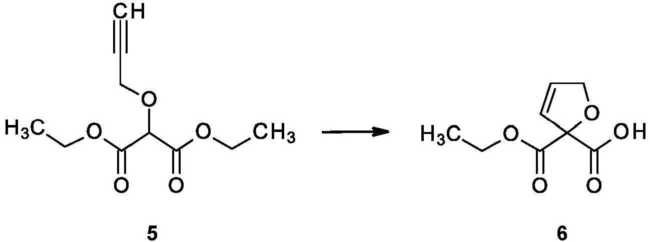

To a solution of diethyl 2-prop-2-ynoxypropanedioate (5) (30 g, 140 mmol) in EtOH/H₂O (200/200 mL) was added KOH (7.85 g, 140 mmol) at 20°C in portions. The mixture was stirred at 20°C for 16 h. The mixture was quenched with H₂O and adjusted to pH = 3 with 6N HCI, extracted with EtOAc. The combined organics were washed with brine, dried and concentrated to give 2-ethoxycarbonylhydrofuran-2-carboxylic acid (6) (20 g, 77%) as yellow oil. 1H NMR (400 MHz, Chloroform-d) δ 6.23 (m, 1H), 6.02 (tdd, 1H), 4.93 (m, 1H), 4.87 (m, 1H), 4.29 (q, 2H), 1.32 (t, 3H).

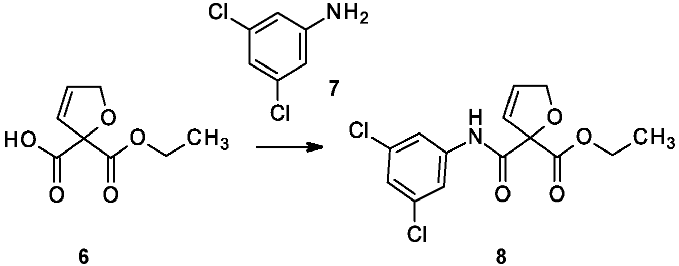

In a similar way to the synthesis for Inter A described above, starting with 2-ethoxycarbonylhy-drofuran-2-carboxylic acid (20 g, 108 mmol), ethyl 5-[(3,5-dichlorophenyl)carbamoyl]-hydrofu-ran-5-carboxylate (8) was obtained as a yellow solid (23 g, 66%). 1H NMR (400 MHz, Chloroform-d) δ 8.32 (br s, 1H), 7.56 (d, J=1.8 Hz, 2H), 7.14 (m, 1H), 6.20 (m, 1H), 6.01 (m, 1H), 4.89 - 4.86 (m, 1H), 4.81 (s, 1H), 4.28 (m, 4H), 1.32 (t, 3H).

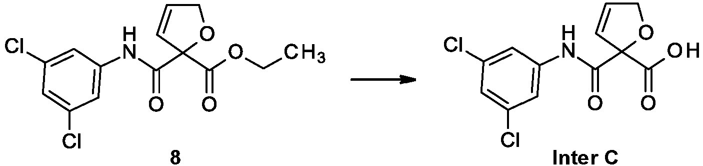

To a solution of ethyl 5-[(3,5-dichlorophenyl)carbamoyl]-hydrofuran-5-carboxylate (8) (1.33 g, 4.03 mmol) in EtOH/H₂O (40/20 mL) was added KOH (452 mg, 8.06 mmol) at 20°C in portions. The mixture was stirred at 20°C for 16 h. The mixture was quenched with H₂O and adjusted to pH= 3 with 6N HCI, extracted with EtOAc. The combined organics were washed with brine, dried and concentrated to give 5-[(3,5-dichlorophenyl)carbamoyl]-hydrofuran-5-carboxylic acid Inter C (950 mg, 78%) as yellow oil. 1H NMR (400 MHz, Chloroform-d) δ 6.23 (m, 1H), 6.02 (tdd, 1H), 4.93 (m, 1H), 4.87 (m, 1H), 4.29 (q, 2H), 1.32 (t, 3H).

### Example 7:

### Synthesis of methyl (1S,4R)-4-[[5-[(3,5-dichlorophenyl)carbamoyl]-2H-furan-5-carbonyl]amino]cyclopent-2-ene-1-carboxylate (1:1 mixture of diastereomers) - Compound I.24

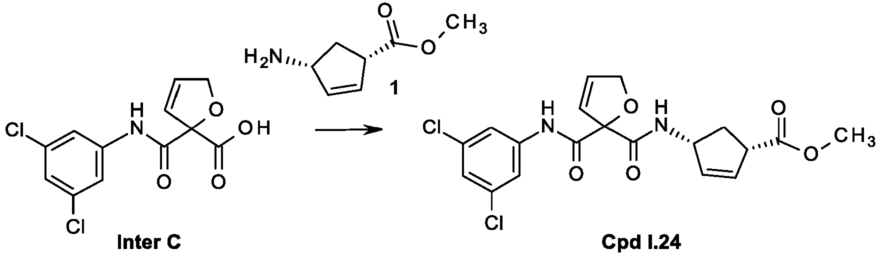

In a similar way to the synthesis for Compound I.6 described above, starting with 5-[(3,5-dichlorophenyl)carbamoyl]-hydrofuran-5-carboxylic acid (Inter C) (48 mg, 0.16 mmol), Compound I.24 was obtained as an off-white solid (49 mg, 73%, 1:1 mixture of diastereomers). 1H NMR (400 MHz, Chloroform-d) δ 9.60 (s, 1H), 9.51 (s, 1H), 7.56 (m, 6H), 7.09 (m, 2H), 6.13 (m, 2H), 6.07 (m, 2H), 5.96 (m, 2H), 5.88 (m, 2H), 5.02 (m, 6H), 3.74 (m, 3H), 3.73 (s, 3H), 3.54 (m, 2H), 2.47 (dtd, 2H), 1.93 (m, 2H).

The two diastereoisomers could be separated by SFC (column: (S,S)-WHELK-O1,50x6mm i.D., 3.5 µm; mobile phase A: CO2; mobile phase B: IPA (0.1% IPAm, v/v; flow rate: 3.4 mL/min, column temp.: 35 °C, ABPR: 1800 psi; gradient: time (A/B): 0.0 (95/5), 0.2 min (95/5), 1.2 min (50/50), 2.2 (50/50), 2.6 min (95/5), 3.0 (95/5). In Table 2 below they are depicted as compounds I.183 and I.184. They are characterized as follows:
Compound I.183: t_{R} = 1.395 min, 1H NMR (400 MHz, Chloroform-d) δ 9.52 (s, 1H), 7.56 (m, 3H), 6.13 (m, 1H), 6.07 (m, 1H), 5.96 (m, 1H), 5.88 (m, 1H), 5.02 (m, 3H), 3.75 (s, 3H), 3.55 (m 1H), 2.47 (dtd, 1H), 1.93 (m, 2H).
Compound I.184: t_{R} = 1.616 min, 1H NMR (400 MHz, Chloroform-d) δ 9.63 (s, 1H), 7.56 (m, 3H), 6.13 (m, 1H), 6.07 (m, 1H), 5.96 (m, 1H), 5.88 (m, 1H), 5.02 (m, 3H), 3.74 (s, 3H), 3.55 (m 1H), 2.47 (dtd, 1H), 1.93 (m, 2H).

### Example 8:

### Synthesis of 5-[(3,5-dichlorophenyl)carbamoyl]-1,3-dimethylhydrofuran-5-carboxylic acid (1:1 mixture of diastereomers) - Inter D

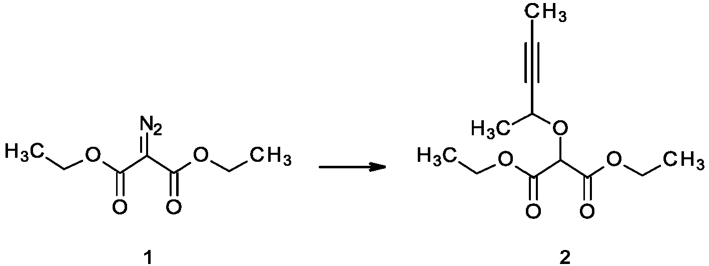

To a solution of diethyl 2-diazopropanedioate (1) (1.13 g, 6.07 mmol) in toluene (25 mL) pent-3-yn-2-ol (0.85 ml, 9.1 mmol) was added. To the resulting solution [Rh(OAc)₂]₂ (0.12 g, 0.30 mmol) was added and the resulting reaction mixture was stirred at 60 °C for 2 h. After cooling to room temperature the reaction mixture was washed with water, dried (sodium sulfate) and the solvent was evaporated under reduced pressure. The crude product 2 (1.10 g, 75%) was used without further purification in the next step. 1H NMR (400 MHz, Chloroform-d, 1:1 mixture of diastereomers) δ 4.39 (m, 1H), 4.26 (m, 4H), 1.82 (d, 3H), 1.47 (d, 3H), 1.28 (m, 6H).

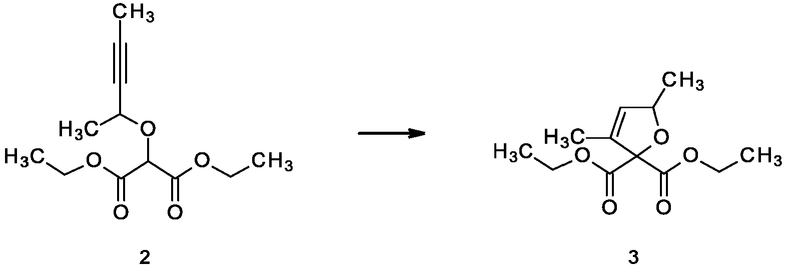

To a solution of diethyl 2-prop-2-ynoxypropanedioate (2) (1.10 g, 4.54 mmol) in acetonitrile (50 mL) cesium carbonate (2.96 g, 9.08 mmol) were added. The resulting reaction mixture was stirred at room temperature for 4 h. The solvent was evaporated under reduced pressure and the residue was dissolved in ethyl acetate and water. The organic layer was separated, washed with water (2x), dried (sodium sulfate) and the solvent was evaporated under reduced pressure. The crude product was purified by column chromatography using ethyl acetate as solvent yielding 3 (1.00 g, 59%, 1:1 mixture of diastereomers). 1H NMR (400 MHz, Chloroform-d, 1:1 mixture of diastereomers) δ 5.71 (t, 1H), 5.07 (dtt, 1H), 4.25 (m, 4H), 1.90 (m, 3H), 1.29 (m, 9H).

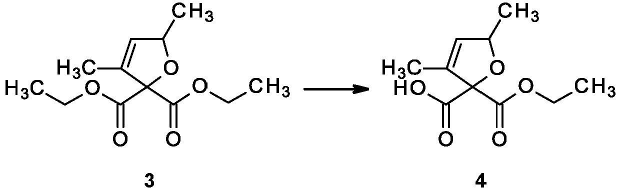

In a similar way to the synthesis for Inter A described above, starting with diethyl 3,5-dimethylhydrofuran-2,2-dicarboxylate (750 mg, 3.1 mmol), 2-ethoxycarbonyl-3,5-dimethylhydro-furan-2-carboxylic acid (4) was obtained as an oil (580 mg, 87%, 1:1 mixture of diastereomers). 1H NMR (400 MHz, Chloroform-d) δ 5.71 (t, 1H), 5.07 (dtt, 1H), 4.25 (m, 2H), 1.90 (t, 3H), 1.29 (m, 6H).

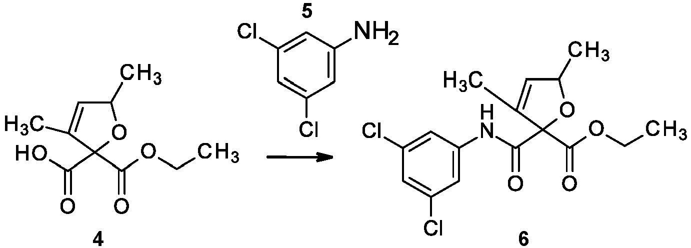

In a similar way to the synthesis for Inter A described above, starting with 2-ethoxycarbonyl-3,5-dimethylhydrofuran-2-carboxylic acid (305 mg, 1.42 mmol), ethyl 5-[(3,5-dichlorophenyl)carbamoyl]-1,3-dimethylhydrofuran-5-carboxylate was obtained as a yellow solid (250 mg, 49%). 1H NMR (400 MHz, Chloroform-d) δ 8.69 (s, 1H), 7.56 (d, 2H), 7.12 (m, 1H), 5.71 (m, 1H), 4.27 (m, 2H), 1.34 (d, 3H), 1.30 (t, 3H).1H NMR (400 MHz, Chloroform-d, minor diastereomer) δ = 8.69 (s, 1H), 7.53 (d, 2H), 7.12 (m, 1H), 5.71 (m, 1H), 4.27 (m, 2H), 1.45 (d, 3H), 1.30 (t, 3H).

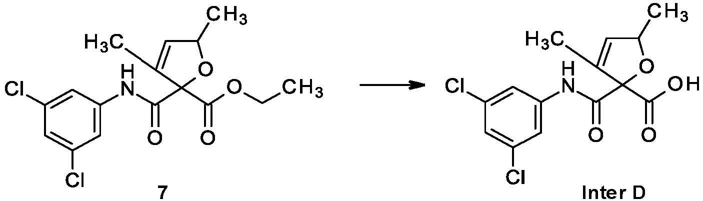

In a similar way to the synthesis for Inter A described above starting with ethyl 5-[(3,5-dichlorophenyl)carbamoyl]-1,3-dimethylhydrofuran-5-carboxylate (250 g, 0.70 mmol), 5-[(3,5-dichlorophenyl)carbamoyl]-1,3-dimethylhydrofuran-5-carboxylic acid (Inter D) was obtained as a white solid (128 mg, 56%, 1:3-mixture of diastereomers). 1H NMR (400 MHz, Chloroform-d, major diastereomer) δ = 8.84 (s, 1H), 7.53 (d, 2H), 7.19 (t, 1H), 5.75 (dt, 1H), 5.29 (dtd, 1H), 1.97 (t, 3H), 1.51 (dd, 3H). 1H NMR (400 MHz, Chloroform-d, minor diastereomer) δ = 8.84 (s, 1H), 7.50 (d, 2H), 7.21 (t, 1H), 5.75 (dt, 1H), 5.40 (dt, 1H), 1.95 (t, 3H), 1.51 (dd, 3H).

### Example 9:

### Synthesis of methyl (1S,4R)-4-[[5-[(3,5-dichlorophenyl)carbamoyl]-2,4-dimethyl-2H-furan-5-carbonyl]amino]cyclopent-2-ene-1-carboxylate (1:2:3 mixture of diastereomers) - Compound I.29

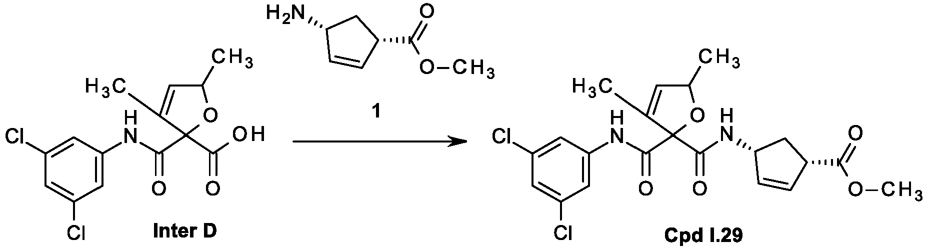

In a similar way to the synthesis for Compound I.6 described above, starting with 5-[(3,5-dichlorophenyl)carbamoyl]-1,3-dimethylhydrofuran-5-carboxylic acid (Inter D) (58 mg, 0.17 mmol), Compound 1.29 was obtained as an off-white solid (65 mg, 82%, 1:2:3 mixture of diastereomers). 1H NMR (400 MHz, Chloroform-d) δ 9.84 (m, 1H), 7.66 (m, 1H), 7.57 (m, 2H), 7.10 (m, 1H), 5.96 (m, 1H), 5.87 (m, 1H), 5.64 (m, 1H), 5.30 (m, 1H), 5.03 (m, 1H), 3.73 (m, 3H), 3.54 (m, 1H), 2.49 (m, 1H), 1.93 (m, 4H), 1.46 (m, 3H).

### Example 10:

### Synthesis of methyl (4S)-4-[[5-[(3,5-dichlorophenyl)carbamoyl]-2,4-dimethyl-2H-furan-5-carbonyl]amino]pentanoate (1:1:1:1 mixture of diastereomers) - Compound I.30

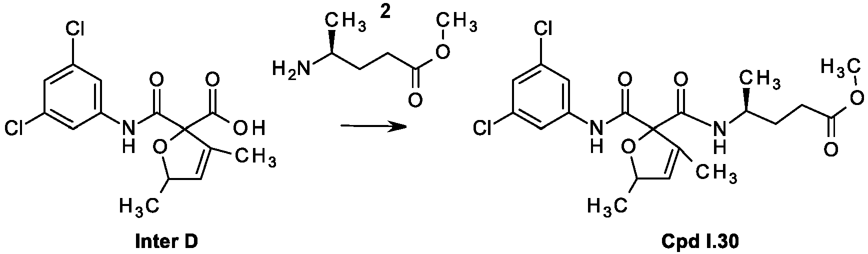

In a similar way to the synthesis for Compound I.6 described above, starting with 5-[(3,5-dichlorophenyl)carbamoyl]-hydrofuran-5-carboxylic acid (Inter D) (50 mg, 0.15 mmol), Compound I.30 was obtained as an off-white solid (60 mg, 89%, 1:1:1:1 mixture of diastereomers). 1H NMR (400 MHz, Chloroform-d) δ 9.99 (s, 1H), 9.92 (s, 1H), 9.87 (s, 1H), 9.80 (s, 1H), 7.56 (m, 8H), 7.15 (m, 4H), 7.10 (m, 4H), 5.64 (m, 4H), 5.30 (m, 4H), 3.99 (m, 4H), 3.67 (m, 12H), 2.33 (m, 8H), 1.93 (m, 12H), 1.86 (m, 4H), 1.78 (m, 4H), 1.50 (m, 6H), 1.43 (t, 6H), 1.19 (m, 12H).

### Example 11:

### Synthesis of [(3,5-dichlorophenyl)carbamoyl]tetrahydrofuran-2-carboxylic acid (Inter E)

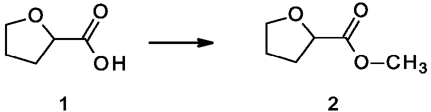

To a mixture of tetrahydrofuran-2-carboxylic acid (1) (5 g, 43 mmol) in MeOH (15 ml) was added H₂SO₄ (0.2 ml) at 25°C and stirred at 75°C for 16 h. The mixture was poured into H₂O, extracted with dichloromethane. The combined organics were washed with aq. NaHCO₃, dried and concentrated. The crude was purified by distillation to give methyl tetrahydrofuran-2-carboxylic acid (2) (3.5 g, 62.5 %) as a yellow oil. 1H NMR (400 MHz, Chloroform-d) δ 4.48 (m, 1H), 3.97 (m, 2H), 3.74 (s, 3H), 2.28 (m, 2H), 2.10 (m, 1H), 1.96 (m, 1H).

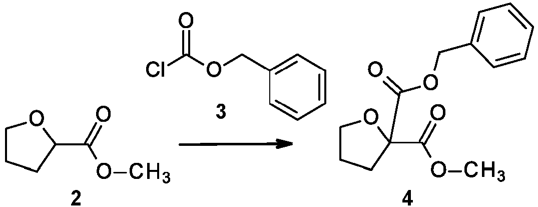

To a mixture of methyl tetrahydrofuran-2-carboxylic acid (2) (3 g, 23 mmol) in THF (50 ml) was added lithium diisopropylamide (LDA) (17 ml, 34.5 mmol) dropwise at -78°C. The mixture was stirred at -78°C for 0.5 h before adding benzyl chloroformate (3) (15.7 g, 92 mmol). The mixture was stirred at -78°C to 20 °C for 1 h. The mixture was poured into H₂O, adjusted pH = 3, extracted with EtOAc. The combined organics were washed with brine, dried and concentrated. The crude was purified by column using ethyl acetate and hexane to give benzyl methyl tetrahydrofuran-2,2-dicarboxylate (4) (2.5 g, 33 %) as yellow oil. 1H NMR (400 MHz, Chloroform-d) δ 7.35 (m, 5H), 5.24 (d, 2H), 4.07 (t, 2H), 3.75 (s, 3H). 2.46 (m, 2H), 2.01 (m, 2H).

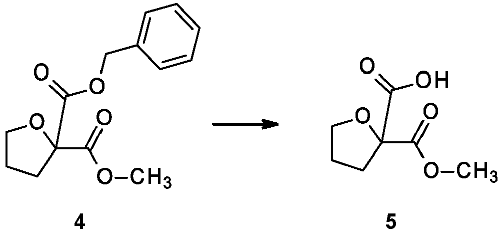

To a mixture of Pd/C (200 mg) was added to a solution of benzyl methyl tetrahydrofuran-2,2-dicarboxylate (4) (2.2 g, 8.33 mmol) in MeOH (200 ml) and stirred at 25°C under H₂ (50 psi) for 2 h. The mixture was filtered and concentrated give compound 2-methoxycarbonyltetrahydrofu-ran-2-carboxylic acid (5) (1.4 g, 97 %) as yellow oil. 1H NMR (400 MHz, Chloroform-d) δ 4.12 (quin, 2H), 3.82 (s, 3H), 2.47 (m, 2H), 2.05 (m, 3H).

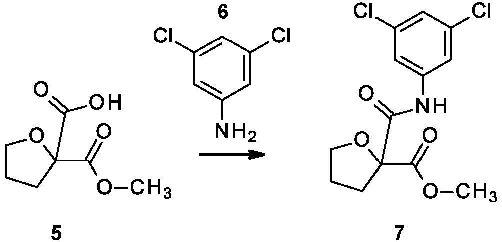

To a mixture of carboxylic acid 5 (1 g, 5.75 mmol) in DMF (20 mL) was added 3,5-dichloroaniline (6) (1.4 g, 8.6 mmol) and HATU (2.6 g, 6.9 mmol) at 15 °C and stirred at 15 °C for 24 h. The mixture was poured into ice water and extracted with methyl tert-butyl ether. The combined organics were washed with brine, dried and concentrated. The crude was purified by prep-HPLC (TFA-ACN-H₂O) to give methyl 2-[(3,5-dichlorophenyl)carbamoyl]tetrahydrofuran-2-carboxylate (7) (900 mg, 49%) as a white solid. 1H NMR (400 MHz, Chloroform-d) δ 8.61 (br s, 1H), 7.58 (d, 2H), 7.14 (t, 1H), 4.12 (m, 2H), 3.81 (s, 3H), 2.75 (td, 1H), 2.44 (ddd, 1H), 2.11 (m, 1H), 1.99 (m, 1H).

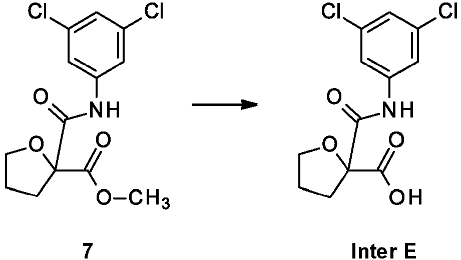

A solution of lithium hydroxide (133 mg, 5.56 mmol) in water was added dropwise to mixture of methyl 2-[(3,5-dichlorophenyl)carbamoyl]tetrahydrofuran-2-carboxylate (7) (885 mg, 2.78 mmol), tetrahydrofuran (THF) (50 ml) and water (50 ml) and the reaction mixture was stirred at room temperature overnight. THF was evaporated *in vacuo* and the remainder washed with methyl *t-*butyl ether. The aqueous solution was concentrated *in vacuo* and the remainder dried to give [(3,5-dichlorophenyl)carbamoyl]tetrahydrofuran-2-carboxylic acid (Inter E) (750 mg, 89% yield). 1H NMR: (400 MHz, Chloroform-d) δ 8.77 (s, 1H), 7.55 (d, 2H), 7.18 (t, 1H), 4.29 (tq, 2H), 2.63 (ddd, 1H), 2.46 (ddd, 1H), 2.16 (tt, 1H), 2.05 (m, 1H).

### Example 12:

### Synthesis of methyl (1S,4R)-4-[[2-[(3,5-dichlorophenyl)carbamoyl]tetrahydrofuran-2-carbonyl]amino]cyclopent-2-ene-1-carboxylate (1:1 mixture of diastereomers) - Compound I.16

In a similar way to the synthesis of methyl (1S,4R)-4-[[2-[(3,5-dichlorophenyl)carbamoyl]ox-etane-2-carbonyl]amino]cyclopent-2-ene-1-carboxylate (Compound I.6) commencing from Inter A, Inter E was converted to Compound I.16

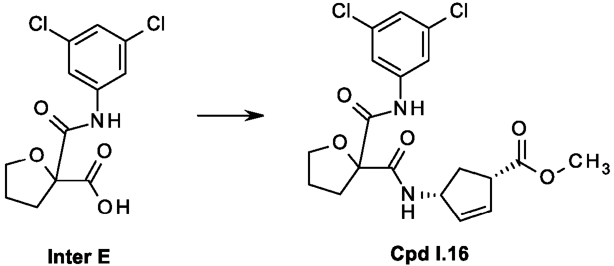

To a solution of [(3,5-dichlorophenyl)carbamoyl]tetrahydrofuran-2-carboxylic acid (Inter E) (100 mg, 0.329 mmol) in dimethylformamide (DMF) methyl (1S,4R)-4-aminocyclopent-2-ene-1-carboxylate (5, CAS [229613-83-6]) (76 mg, 0.43 mmol) was added. To the resulting solution was added HATU (2-(7-aza-1H-benzotriazole-1-yl)-1,1,3,3-tetramethyluronium hexafluorophosphate, CAS [148893-10-1]), (162 mg, 0.427 mmol) and then diisopropylethylamine (0.17 mL). The resulting reaction mixture was stirred at room temperature overnight. To the reaction mixture water and sodium bicarbonate solution were added. The reaction mixture was extracted with ethyl acetate, washed with water, dried (sodium sulfate) and the solvent was evaporated under reduced pressure. The crude product was purified by column chromatography using ethyl acetate as solvent yielding compound I.16 (119 mg, 85%, 1:1 mixture of diastereomers). 1H NMR: (400 MHz, Chloroform-d) δ 9.71 (s, 1H), 9.66 (s, 1H), 7.73 (m, 4H), 7.54 (m, 2H), 7.11 (m, 2H), 5.90 (dq, 2H), 5.81 (tq, 2H), 4.95 (m, 2H), 4.08 (m, 4H), 3.65 (s, 3H), 3.65 (s, 3H), 3.50 (m, 2H), 2.45 (m, 5H), 2.35 (m, 1H), 1.90 (m, 5H), 1.84 (dd, 1H).

### Example 13:

### Synthesis of (1S,4R)-4-[[2-[(3,5-dichlorophenyl)carbamoyl]tetrahydrofuran-2-carbonyl]amino]cyclopentane-1-carboxylate (1:1 mixture of diastereomers) - Compound I.15

In a similar way to the synthesis of methyl (1S,4R)-4-[[2-[(3,5-dichlorophenyl)carbamoyl]tetrahydrofuran-2-carbonyl]amino]cyclopent-2-ene-1-carboxylate (Compound I.16), Inter E was converted to Compound I.15.

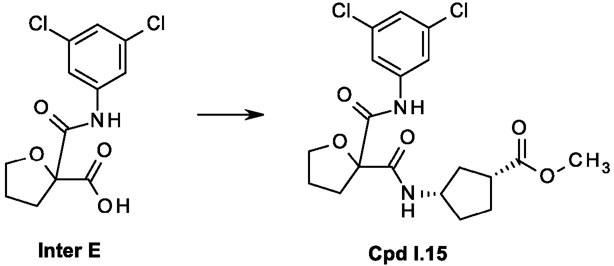

[(3,5-dichlorophenyl)carbamoyl]tetrahydrofuran-2-carboxylic acid Inter E (100 mg, 0.329 mmol) was treated with (1S,4R)-4-aminocyclopentane-1-carboxylate hydrochloride (CAS [222530-29-2]) (84 mg, 0.43 mmol) to give (1S,4R)-4-[[2-[(3,5-dichlorophenyl)carbamoyl]tetrahydrofuran-2-carbonyl]amino]cyclopentane-1-carboxylate (Compound I.15) as a colorless oil (82 mg, 58%, 1:1 mixture of diastereomers). 1H NMR: (400 MHz, Chloroform-d) δ 9.75 (s, 1H), 9.69 (s, 1H), 7.71 (d, 2H), 7.70 (d, 2H), 7.55 (m, 2H), 7.12 (t, 2H), 4.23 (m, 3H), 4.09 (m, 5H), 3.62 (s, 4H), 3.62 (s, 3H), 2.86 (m, 2H), 2.38 (m, 6H), 2.14 (m, 2H), 1.90 (m, 7H), 1.72 (m, 1H), 1.62 (m, 1H).

### Example 14:

### Synthesis of methyl 4-[[2-[(3,5-dichlorophenyl)carbamoyl]tetrahydrofuran-2-carbonyl]amino]butanoate - Compound I.18

In a similar way to the synthesis of methyl (1S,4R)-4-[[2-[(3,5-dichlorophenyl)carbamoyl]tetrahydrofuran-2-carbonyl]amino]cyclopent-2-ene-1-carboxylate (Compound I.16), Inter E was converted to Compound I.18.

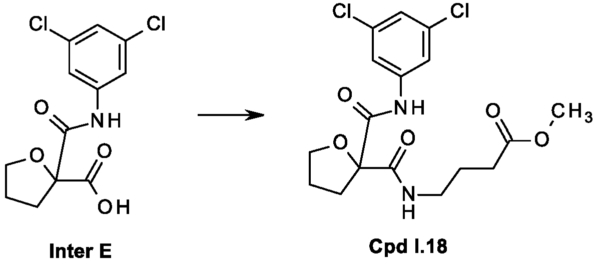

[(3,5-dichlorophenyl)carbamoyl]tetrahydrofuran-2-carboxylic acid (Inter E) (100 mg, 0.329 mmol) was treated with methyl 3-aminopropanoate to give methyl 4-[[2-[(3,5-dichlorophenyl)carbamoyl]tetrahydrofuran-2-carbonyl]amino]butanoate (Compound I.18) as a colorless oil (96 mg, 72%). 1H NMR: (400 MHz, Chloroform-d) δ 9.43 (s, 1H), 7.55 (d, 2H), 7.24 (m, 1H), 7.10 (t, 1H), 4.23 (dp, 2H), 3.68 (s, 3H), 3.34 (tt, 2H), 2.47 (m, 1H), 2.44 (m, 1H), 2.36 (t, 2H), 2.04 (dt, 1H), 1.98 (m, 1H), 1.87 (m, 2H).

### Example 15:

### Synthesis of (3S)-3-[[2-[(3,5-dichlorophenyl)carbamoyl]tetrahydrofuran-2-carbonyl]amino]butanoate (1:1 mixture of diastereomers) - Compound I.17

In a similar way to the synthesis of of methyl (1S,4R)-4-[[2-[(3,5-dichlorophenyl)carbamoyl]tetrahydrofuran-2-carbonyl]amino]cyclopent-2-ene-1-carboxylate (Compound I.16), Inter E was converted to Compound I.17.

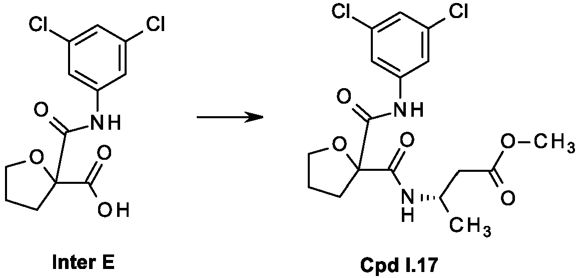

[(3,5-dichlorophenyl)carbamoyl]tetrahydrofuran-2-carboxylic acid Inter E(100 mg, 0.329 mmol) was treated with methyl (3S)-aminobutanoate hydrochlorid to give methyl (3S)-3-[[2-[(3,5-dichlorophenyl)carbamoyl]tetrahydrofuran-2-carbonyl]amino]butanoate (Compound I.17) as a colorless oil (120 mg, 90%, 1:1 mixture of diastereomers). 1H NMR: (400 MHz, Chloroform-d) δ 9.48 (s, 1H), 9.28 (s, 1H), 7.58 (d, 2H), 7.56 (d, 2H), 7.48 (m, 2H), 7.08 (m, 2H), 4.33 (qd, 2H), 4.20 (tdd, 4H), 3.69 (s, 3H), 3.67 (s, 3H), 2.50 (m, 8H), 2.00 (m, 4H), 1.26 (m, 6H).

### Example 16:

### 2-[(3,5-dichlorophenyl)carbamoyl]-1,3-oxathiolane-2-carboxylic acid (Inter F)

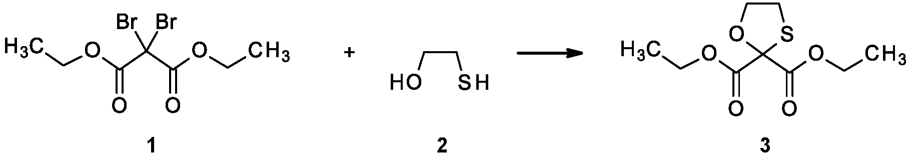

To a mixture of 2-hydroxyethanthiol (2) (23.4 g, 0.3 mol) in THF (2 L) was added sodium hydride (26 g, 0.65 mol) at 20°C, stirred for 2 h. and then added dropwise to a solution of diethyl dibromomalonate (1) (75.4 g, 0.3 mol) in THF (100 ml) at 20°C for 4 h. The mixture was stirred at 20°C for 16 h under N₂. The mixture was poured into ice water (1.5 L), adjusted to pH = 7, concentrated, extracted with EtOAc (1.5 L). The combined organic layer was washed with brine, dried over Na₂SO₄, concentrated and purified by prep- HPLC (TFA-MeCN-H2O) to give diethyl 1,3-oxathiolane-2,2-dicarboxylate (3) (1.8 g, 2.5%) as yellow solid. 1H NMR (400 MHz Chloroform-d) δ 4.40 (t, 2H), 4.29 (m, 4H), 3.19 (t, 2H), 1.31 (t, 6H).

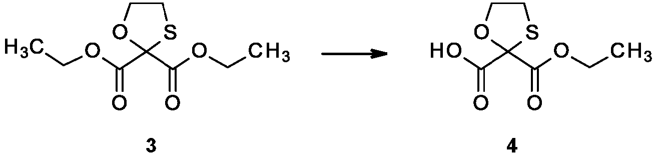

To a mixture of compound diethyl 1,3-oxathiolane-2,2-dicarboxylate (3) (800 mg, 3.42 mmol) in EtOH (8 mL) and H₂O (8 ml) was added KOH (191 mg, 3.418 mmol) at 0°C. The mixture was stirred at 0°C for 2 h. The mixture was quenched with H₂O (50 ml) and adjusted to pH= 3 with 6N HCI, extracted with EtOAc (100 ml). The combined organics were washed with brine, dried and concentrated to give 2-ethoxycarbonyl-1,3-oxathiolane-2-carboxylic acid (4) (350 mg, 50%) as yellow oil. 1H NMR (400 MHz, Chloroform-d) δ 9.20 (br s, 1H), 4.45 (m, 2H), 4.33 (q, J = 7.1 Hz, 2H), 3.23 (t, J = 5.8 Hz, 2H), 1.33 (t, J = 7.2 Hz, 3H).

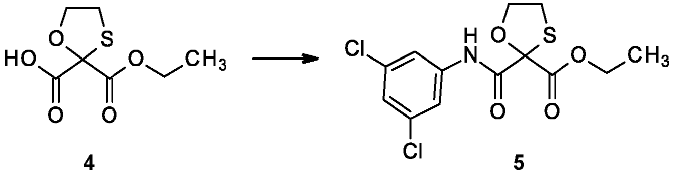

To a solution of compound 2-ethoxycarbonyl-1,3-oxathiolane-2-carboxylic acid (4) (350 mg, 1.7 mmol) and 3,5-dichloroaniline (328.25 mg, 2.04 mmol) in THF (5 mL) was added HATU (775.2 mg, 2.04 mmol), triethylamine (0.47 ml, 3.4 mmol) at 20°C. The mixture was stirred at 20°C for 4 h. The mixture was quenched with H₂O (20 ml) and extracted with methyl ter-butyl ether (MTBE) (50 ml). The combined organic layers were washed with brine, dried and concentrated. The crude was purified by prep-HPLC (MeCN-TFA-H₂O) to give ethyl 2-[(3,5-dichlorophenyl)carbamoyl]-1,3-oxathiolane-2-carboxylate (5) (320 mg, 51 %) as white solid. 1H NMR (400 MHz, Chloroform-d ) δ = 8.56 (br s, 1H), 7.56 (d, J = 1.8 Hz, 2H), 7.14 (t, J = 1.8 Hz, 1H), 4.53 (td, J = 5.2, 9.2 Hz, 1H), 4.42 - 4.27 (m, 3H), 3.29 - 3.17 (m, 2H), 1.34 (t, J = 7.1 Hz, 3H)

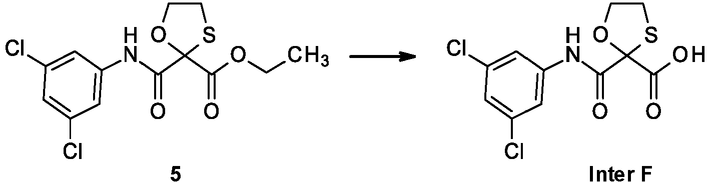

In a similar way to the synthesis of diethyl 1,3-oxathiolane-2,2-dicarboxylate (4), commencing with ethyl 2-[(3,5-dichlorophenyl)carbamoyl]-1,3-oxathiolane-2-carboxylate (5), 2-[(3,5-dichlorophenyl)carbamoyl]-1,3-oxathiolane-2-carboxylic acid (Inter F) was obtained as an off-white solid (Inter F). 1H NMR (500 MHz, THF-d8) δ 9.98 (s, 1H), 9.50 (s, 1H), 7.75 (d, 2H), 7.15 (t, 1H), 4.42 (dt, 1H), 4.36 (dt, 1H), 3.19 (m, 2H).

### Example 17:

### Synthesis of methyl (1S,4R)-4-[[2-[(3,5-dichlorophenyl)carbamoyl]-1,3-oxathiolane-2-carbonyl]amino]cyclopent-2-ene-1-carboxylate (1:1 mixture of diastereomers) - Compound I.20

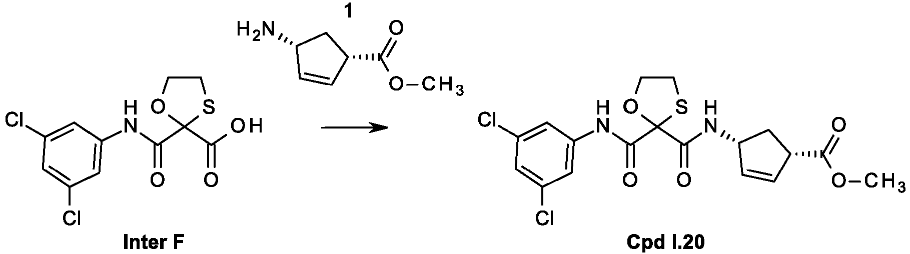

In a similar way to the synthesis for Compound I.6 described above, starting with 2-[(3,5-dichlorophenyl)carbamoyl]-1,3-oxathiolane-2-carboxylic acid (Inter F), Compound I.20 was obtained as an off-white solid (0.1 g, 72%, 1:1 mixture of diastereomers). 1H NMR (400 MHz, Chloroform-d) δ 9.85 (s, 1H), 9.76 (s, 1H), 7.56 (m, 6H), 7.09 (m, 2H), 5.98 (m, 2H), 5.93 (dt, 1H), 5.88 (dt, 1H), 5.03 (m, 2H), 4.63 (m, 4H), 3.73 (d, 7H), 3.55 (m, 2H), 3.23 (m, 4H), 2.49 (dtd, 2H), 1.97 (ddt, 2H).

### Example 18:

### Synthesis of methyl (4S)-4-[[2-[(3,5-dichlorophenyl)carbamoyl]-1,3-oxathiolane-2-carbonyl]amino]pentanoate (1:1 mixture of diastereomers) - Compound I.21

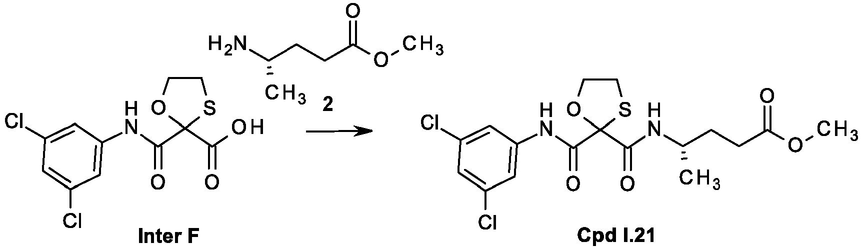

In a similar way to the synthesis for Compound I.7 described above, starting with 2-[(3,5-dichlorophenyl)carbamoyl]-1,3-oxathiolane-2-carboxylic acid (Inter F), methyl (4S)-4-[[2-[(3,5-dichlorophenyl)carbamoyl]-1,3-oxathiolane-2-carbonyl]amino]pentanoate (Compound I.21) was obtained as an off-white solid (40 mg, 59%, 1:1 mixture of diastereomers). 1H NMR (400 MHz, Chloroform-d) δ = 9.79 (s, 1H), 9.68 (s, 1H), 7.53 (t, 4H), 7.10 (dt, 2H), 7.01 (m, 2H), 4.64 (m, 4H), 4.01 (m, 2H), 3.69 (s, 3H), 3.66 (s, 3H), 3.27 (m, 2H), 3.21 (m, 2H), 2.39 (m, 4H), 1.86 (m, 4H), 1.22 (t, 6H).

### Example 19:

### Synthesis of 4-[(3,5-dichlorophenyl)carbamoyl]-1,3-dioxolane-4-carboxylic acid (Inter G)

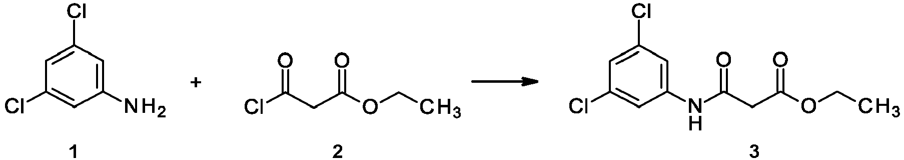

To a solution of 3,5-dichloroaniline (1) (10 g, 61.7 mmol) in dichloromethane (200 mL) was added ethyl 3-chloro-3-oxo-propanoate (2) (9.3 g, 61.7 mmol) at 0°C. The mixture solution was stirred for 15 min. After dropwise addition of triethylamine (6.6 g, 64.8 mmol) at 0°C and then stirred for 5 h at 0 to 15°C. The reaction was poured into water, extracted with DCM. The organic phase was washed with brine and dried over Na₂SO₄. The dried organic phase was filtrated, concentrated and purified by column (pentane/EtOAc = 10:1 to 2:1) to give ethyl 3-(3,5-dichloroanilino)-3-oxo-propanoate (3) (19.8 g, 97%) as brown solid. 1H NMR (400 MHz, Chloroform-d) δ = 9.49 (br s, 1H) 7.54 (d, 2H) 7.12 (t, 1 H) 4.28 (q, 2H) 3.48 (s, 2 H) 1.34 (t, 3H).

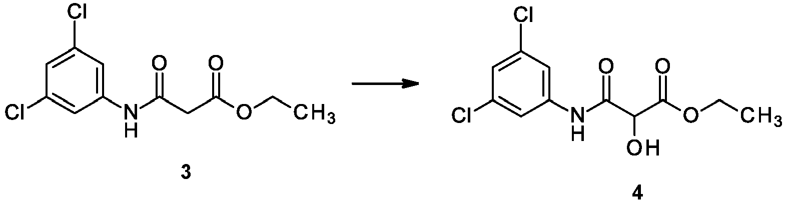

To a solution of ethyl 3-(3,5-dichloroanilino)-3-oxo-propanoate (3) (4.5 g, 16.3 mmol) in trifluoroethanol (50 mL) was added bis(trifluoroacetoxy)iodo)benzene (PIFA) (9.1 g, 21 mmol) (CAS [2712-78-9]) at 18°C in several portions. Then the mixture solution was stirred for 16 h. The reaction was quenched with water and diluted with ethyl acetate (EtOAc). The aqueous phase was separated and extracted with EtOAc. The organic phase was washed with brine and dried over Na₂SO₄. The dried organic phase was filtrated, concentrated and purified by column (pentane/EtOAc=10:1 to 1:1) to give ethyl 3-(3,5-dichloroanilino)-2-hydroxy-3-oxo-propanoate (4) (2.3 g, 49%) as brown oil. 1H NMR (400 MHz, Chloroform-d) δ = 8.60 (br s, 1H), 7.55 (d, 2H), 7.27 (s, 1H), 4.76 (s, 1H), 4.30 (m, 2H), 1.40 (t, 3H).

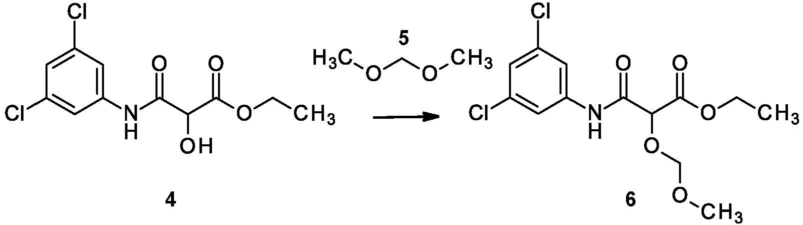

To a solution of ethyl 3-(3,5-dichloroanilino)-2-hydroxy-3-oxo-propanoate (4) (1.0 g, 3.4 mmol) in dimethoxymethane (5) (5 mL) was added BF₃ etherate (0.3 g, 1.7 mmol) at 20°C in one portion. Then the mixture solution was heated to 60°C and stirred for 4 h. The reaction was quenched with water and extracted with EtOAc. The organic phase was washed with brine and dried over Na₂SO₄. The dried organic phase was filtrated, concentrated and purified by column (pentane/EtOAc = 10:1 to 2:1) to give ethyl 3-(3,5-dichloroanilino)-2-(methoxymethoxy)-3-oxo-propanoate (6) (0.6 g, 54%) as a yellow oil. 1H NMR (400 MHz, Chloroform-d) δ = 8.39 (br s, 1H), 7.56 (d, 2H), 7.14 (t, 1H), 4.84 (d, 2H), 4.72 (s, 1H), 4.31 (m, 2H), 3.45 (s, 3H), 1.34 (t, 3H).

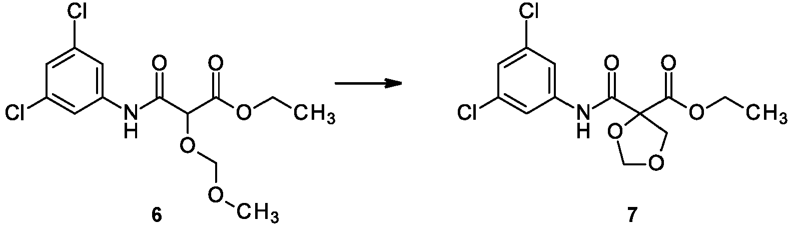

To a solution of ethyl 3-(3,5-dichloroanilino)-2-(methoxymethoxy)-3-oxo-propanoate (6) (0.3 g, 0.9 mmol) in toluene (3 mL) was added paraformaldehyde (0.27 g, 2.7 mmol) and para-toluenesulfonic acid (PTSA) (16 mg, 0.09 mmol) at 20°C. Then the mixture solution was heated to 90°C and stirred for 0.5 h. The reaction was concentrated to move toluene and the residue was purified by column (pentane/ EtOAc = 10:1 to 1:1) to give ethyl 2-[(3,5-dichlorophenyl)carbamoyl]-1,3-dioxolane-2-carboxylate (7) (190 mg, 63%) as white solid. 1H NMR (400 MHz, Chloroform-d) δ = 8.45 (br s, 1H), 7.56 (d, 2H), 7.16 (t, 1H), 5.29 (s, 1H), 5.11 (s, 1H), 4.52 (m, 1H), 4.43 (m, 1H), 4.31 (q, 2H), 1.32 (t, 3H).

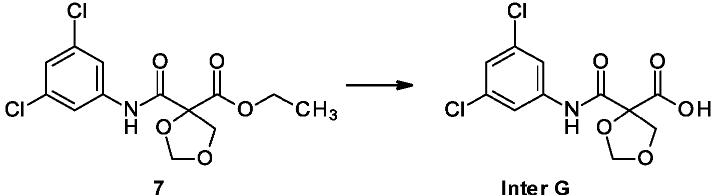

In a similar way to the synthesis of 2-[(3,5-dichlorophenyl)carbamoyl]-1,3-oxathiolane-2-carboxylic acid (Inter F), starting with ethyl 4-[(3,5-dichlorophenyl)carbamoyl]-1,3-dioxolane-4-carboxylate (0.17 g, 0.50 mmol), 4-[(3,5-dichlorophenyl)carbamoyl]-1,3-dioxolane-4-carboxylic acid (Inter G) was obtained as an off-white solid (111 mg, 72%). LC-MS (M+H)⁺: 307.8

### Example 20:

### Synthesis of methyl (1S,4R)-4-[[4-[(3,5-dichlorophenyl)carbamoyl]-1,3-dioxolane-4-carbonyl]amino]cyclopent-2-ene-1-carboxylate (1:1 mixture of diastereomers) - Compound I.22

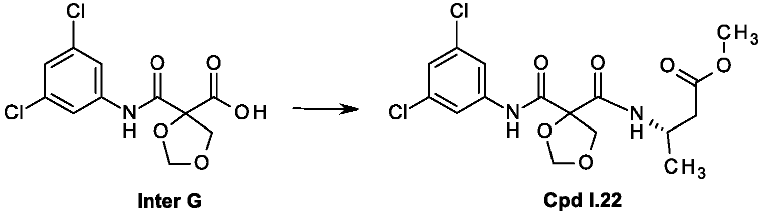

In a similar way to the synthesis for Compound I.7 described above, starting with 4-[(3,5-dichlorophenyl)carbamoyl]-1,3-dioxolane-4-carboxylic acid (Inter G), methyl (1S,4R)-4-[[4-[(3,5-dichlorophenyl)carbamoyl]-1,3-dioxolane-4-carbonyl]amino]cyclopent-2-ene-1-carboxylate (Compound I.22) was obtained as an off-white solid (55 mg, 68%, 1:1 mixture of diastereomers). 1H NMR (400 MHz, Chloroform-d) δ 9.47 (s, 1H), 9.28 (s, 0H), 7.57 (d, 1H), 7.55 (m, 2H), 7.13 (m, 1H), 5.33 (d, 1H), 5.25 (d, 1H), 4.30 (m, 2H), 4.22 (d, 1H), 3.70 (s, 2H), 3.69 (s, 1H), 2.55 (m, 2H), 1.29 (d, 1H), 1.27 (d, 1H).

### Example 21:

### Synthesis of methyl 4-[[4-[(3,5-dichlorophenyl)carbamoyl]-1,3-dioxolane-4-carbonyl]amino]butanoate - Compound I.23

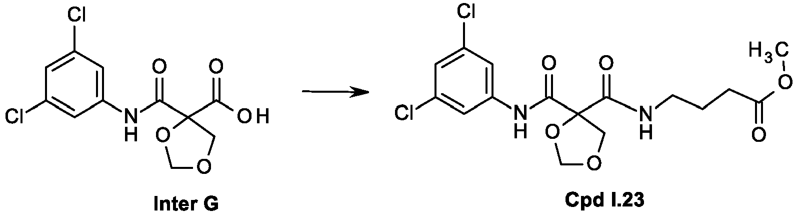

In a similar way to the synthesis for Compound I.7 described above, starting with 4-[(3,5-dichlorophenyl)carbamoyl]-1,3-dioxolane-4-carboxylic acid (Inter G), methyl 4-[[4-[(3,5-dichlorophenyl)carbamoyl]-1,3-dioxolane-4-carbonyl]amino]butanoate (Compound I.23) was obtained as an off-white solid (40 mg, 60%). 1H NMR (400 MHz, Chloroform-d) δ 9.50 (s, 1H), 7.55 (d, 2H), 7.29 (s, 1H), 7.13 (t, 1H), 5.35 (s, 1H), 5.25 (s, 1H), 4.32 (d, 1H), 4.21 (d, 1H), 3.69 (s, 3H), 3.38 (m, 2H), 2.38 (t, 2H), 1.90 (m, 2H).

### Example 22:

### Synthesis of tert-butyl 2-[(3,5-dichlorophenyl)carbamoyl]-2-[[(1R,4S)-4-methoxycarbonylcyclo-pent-2-en-1-yl]carbamoyl]pyrrolidine-1-carboxylate (1:1 mixture of diastereomers) - Compound I.35

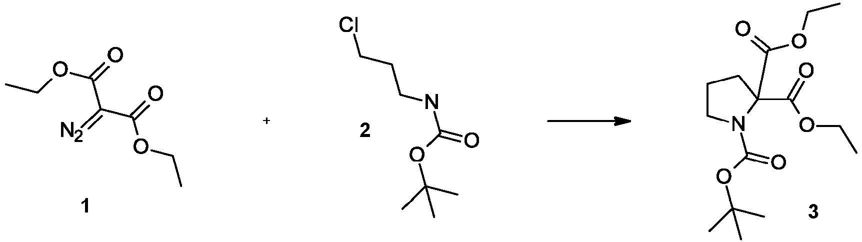

To a solution of diethyl 2-diazopropanedioate (1) (5.86 g, 31.5 mmol) and *tert-butyl* N-(3-bromopropyl)carbamate (5 g, 21 mmol) in toluene (50 mL) bis[Rhodium(α,α,α',α'-tetramethyl-1,3-ben-zenedipropionic acid)] ([Rh(esp)]₂ CAS [819050-89-0], 100 mg, 0.13 mmol) was added and the mixture was stirred for 2.5 h at 60°C. After cooling to room temperature, tetrabutylammonium bromide (677 mg, 2.1 mmol) and cesium hydroxide monohydrate (7.1 g, 42 mmol) were added and the mixture stirred for 18 h. After filtration through Celite^{®} and washing with EtOAc, the filtrate was purified by column chromatography (silica RP18, MeCN/H₂O) to afford compound 3 (3.5 g, 53%, 1:1 mixture of atropisomers) as a colorless oil. 1H NMR (400 MHz, Chloroform-d) δ 4.26 (m, 4H), 3.56 (m, 2H), 2.48 (m, 2H), 1.86 (m, 2H), 1.47 (s, 9H), 1.41 (s, 9H), 1.29 (m, 6H).

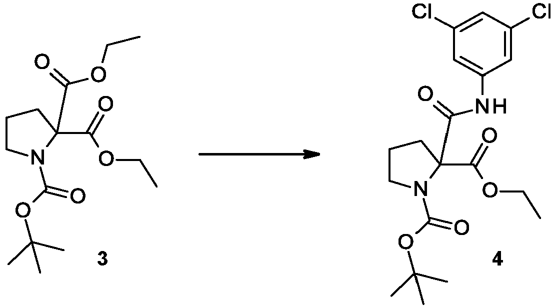

To a solution of compound 3 (3.5 g, 11 mmol) in a 1:1 mixture of H₂O and THF (50 mL) lithium hydroxide (266 mg, 11.1 mmol) was added and the mixture stirred for 2 h. After concentrating the mixture, the residue was dissolved in THF (50 mL) and treated with 3,5-dichloroaniline (1.77 g, 10.9 mmol) and triethylamine (4.56 mL, 32.7 mmol). The reaction was quenched with H₂O. The organic layer was separated and extracted with ethyl acetate. The combined filtrates were washed with brine and concentrated. The residue was purified by flash column chromatography (pentane/EtOAc) to afford compound 4 (0.7 g, 15% over two steps) as an off-white solid. 1H NMR (400 MHz, Chloroform-d) δ 7.86 (m, 2H), 7.28 (m, 1H), 7.25 (m, 0H), 3.57 (m, 2H), 2.43 (m, 2H), 2.07 (m, 2H), 1.45 (s, 4H), 1.30 (s, 6H).

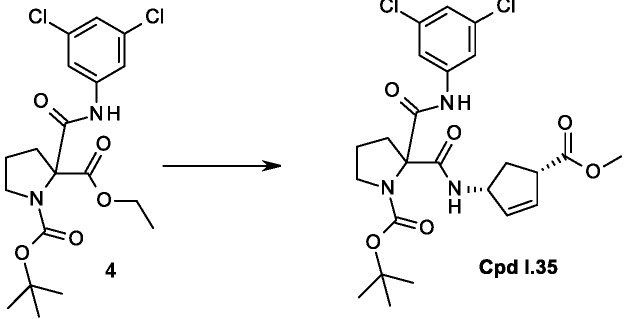

To a solution of compound 4 (0.7 g, 1.6 mmol) in a 1:1 mixture of H₂O and THF (50 mL) lithium hydroxide (266 mg, 11.1 mmol) was added and the mixture stirred for 2 h. After concentrating the mixture, the residue was dissolved in dimethylformamide (DMF) (10 mL) and treated with [(1R,4S)-4-methoxycarbonylcyclopent-2-en-1-yl]ammonium chloride (329 mg, 1.85 mmol) and HATU (705 mg, 1.85 mmol). After stirring for 18 h, the reaction was quenched with water. The aqueous layer was separated and extracted with ethyl acetate. The combined filtrates were washed with brine and concentrated. The residue was purified by column chromatography (silica RP18, MeCN/H₂O) to afford compound I.35 (380 mg, 48%, 1:1 mixture of diastereomers, mixture of rotamers) as an off-white solid. 1H NMR (400 MHz, Chloroform-d) δ 11.27 (s, 1H), 11.22 (s, 1H), 7.61 (d, 4H), 7.09 (m, 2H), 5.96 (m, 4H), 5.04 (m, 2H), 3.80 (m, 2H), 3.72 (m, 10H), 3.56 (m, 2H), 2.40 (m, 6H), 1.95 (m, 6H), 1.32 (s, 9H), 1.27 (s, 9H).

### Example 23:

### Synthesis of methyl (1S,4R)-4-[[2-[(3,5-dichlorophenyl)carbamoyl]pyrrolidine-2-carbonyl]amino]cyclopent-2-ene-1-carboxylate (1:1 mixture of diastereomers) - Compound I.33

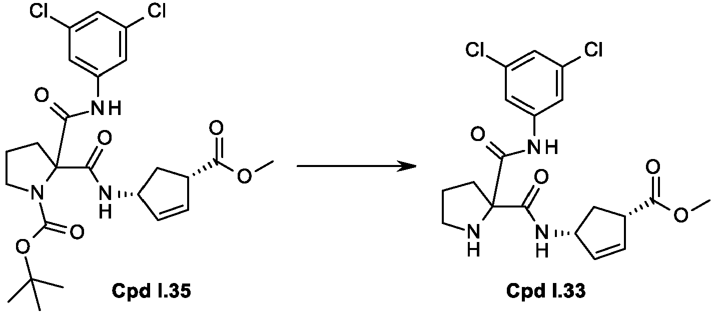

A solution of hydrochloric acid (1.25 M in methanol, 2 mL) was added to compound I.35 (350 mg, 0.665 mmol) and the mixture stirred for 1 h under refluxing conditions. After concentrating, the ammonium salt of compound I.33 (280 mg, 99%, 1:1 mixture of diastereomers) was obtained as an off-white solid. 1H NMR (400 MHz, Chloroform-d) δ 11.04 (s, 1H), 10.96 (s, 1H), 8.41 (s, 2H), 7.77 (d, 4H), 7.11 (s, 1H), 7.09 (s, 1H), 5.95 (m, 4H), 4.94 (s, 2H), 3.71 (m, 8H), 3.53 (m, 2H), 2.94 (s, 2H), 2.74 (s, 2H), 2.45 (m, 2H), 2.10 (m, 8H).

### Example 24:

### Synthesis of methyl (1S,4R)-4-[[2-[(3,5-dichlorophenyl)carbamoyl]-1-methyl-pyrrolidine-2-carbonyl]amino]cyclopent-2-ene-1-carboxylate (1:1 mixture of diastereomers) - Compound I.34

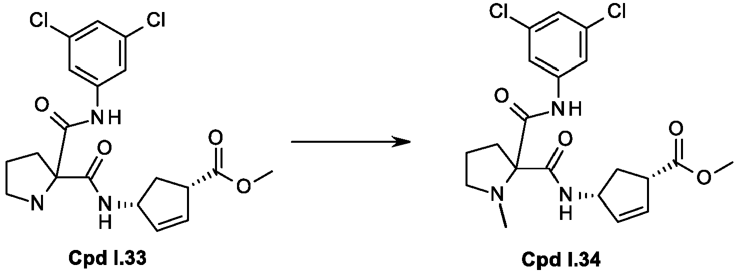

To a solution of compound I.33 (50 mg, 0.12 mmol) in acetonitrile (2 mL) an aqueous solution of formaldehyde (5 equiv) and sodium cyanoborohydride (12 mg, 0.19 mmol) were added. After stirring for 15 min, the reaction was quenched with acetic acid until pH = 7 was reached and the mixture stirred for another 30 min. After concentrating the mixture, the residue dissolved in ethyl acetate, washed with an aqueous saturated solution of sodium bicarbonate (3x) and dried over Na₂SO₄. After concentrating, the crude compound I.34 was obtained as a colorless oil (39 mg, 76%, 1:1 mixture of diastereomers). 1H NMR (400 MHz, Chloroform-d) δ 10.84 (s, 1H), 10.82 (s, 1H), 8.28 (s, 1H), 8.25 (s, 1H), 7.57 (s, 2H), 7.57 (s, 2H), 7.08 (m, 2H), 5.92 (m, 4H), 5.01 (m, 2H), 3.73 (s, 3H), 3.73 (s, 3H), 3.56 (m, 2H), 3.21 (m, 4H), 2.51 (m, 8H), 2.32 (m, 4H), 1.95 (m, 6H).

### Example 25:

### Synthesis of methyl (3S)-3-[[3-[(3,5-dichlorophenyl)carbamoyl]-2-oxabicyclo[2.2.2]oct-5-ene-3-carbonyl]amino]butanoate (1:1 mixture of diastereoisomers) - Compound I.174

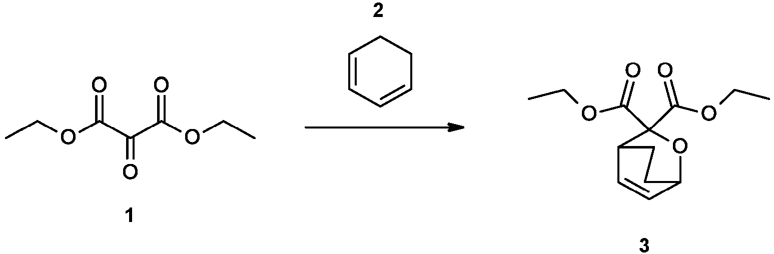

According to the literature J. Org. Chem. 1997, 42, 4095-4103, diethyl ketomalonate (**1**) (5.0 g, 29 mmol) was added to a solution of hydroguinone (50 mg, 0.45 mmol) and freshly destilled 1,3-cyclohexadiene (**2**) (5.06 g, 63.2 mmol) in acetonitrile (20 mL). The mixture was placed in a microwave and heated to 130 °C for 4 h. After cooling to room temperature, the mixture was concentrated under reduced pressure and the residue purified by column chromatography (silica RP18, MeCN/H₂O) to yield the title compound **3** (4.5 g, 62%) as a colourless oil. 1H NMR (400 MHz, Chloroform-d) δ 6.53 (ddd, 1H), 6.46 (ddd, 1H), 4.68 (ddt, 1H), 4.22 (m, 4H), 2.18 (m, 1H), 1.63 (m, 1H), 1.26 (m, 9H).), which is in alignment with the reported literature.

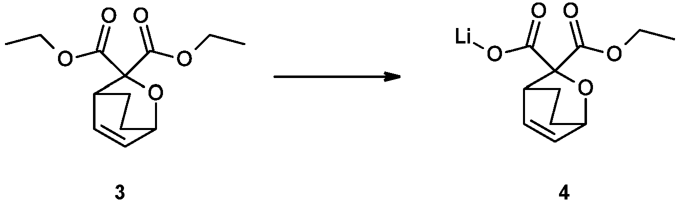

In analogy to example 1, lithium hydroxide (418 mg, 17.4 mmol) was added to a solution of diethyl 2-oxabicyclo[2.2.2]oct-5-ene-3,3-dicarboxylate (**3**) (4.43 g, 17.4 mmol) in a 1:1-mixture of THF and water (50 mL). After stirring the mixture at room temperature for 2 h, THF was evaporated *in vacuo* and the remainder dried to give the product (**4**) (4,0 g, 99% yield). 1H NMR (500 MHz, Deuterium Oxide) δ 6.56 (m, 1H), 6.47 (m, 1H), 4.60 (m, 1H), 4.14 (m, 2H), 3.37 (m, 1H), 2.08 (m, 1H), 1.60 (m, 1H), 1.26 (m, 5H).

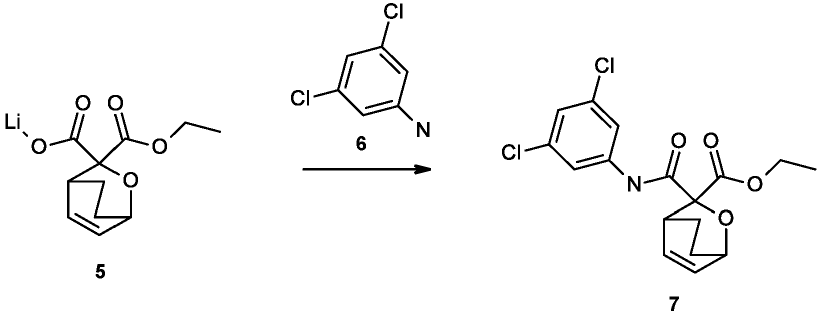

In analogy to example 1, 1-propanephosphonic anhydride (16.6 g, 29.3 mmol) was added to a solution of lithium (3-ethoxycarbonyl-2-oxabicyclo[2.2.2]oct-5-ene-3-carboxylate (**5**) (4.0 g, 17 mmol) , 3,5-dichloroaniline (**6**) (2.8 g, 17 mmol), triethylamine (5.2 g, 52 mmol) in THF (50 mL) at 0 °C. The resulting reaction mixture was stirred at room temperature overnight. To the reaction mixture water and sodium bicarbonate solution were added. The reaction mixture was extracted with ethyl acetate, washed with water, dried (sodium sulfate) and the solvent was evaporated under reduced pressure. The crude product was purified by column chromatography (silica RP18, MeCN/H₂O) yielding ethyl 3-[(3,5-dichlorophenyl)carbamoyl]-2-oxabicyclo[2.2.2]oct-5-ene-3-carboxylate (**7**) (1.3 g, 20%) as a colorless oil. 1H NMR (400 MHz, Chloroform-d) δ 8.94 (s, 1H), 7.58 (d, 2H), 7.13 (t, 1H), 6.57 (ddd, 1H), 6.47 (ddd, 1H), 4.80 (m, 1H), 4.19 (m, 2H), 3.69 (m, 1H), 2.03 (tt, 1H), 1.66 (ddt, 1H), 1.35 (m, 2H), 1.26 (t, 3H).

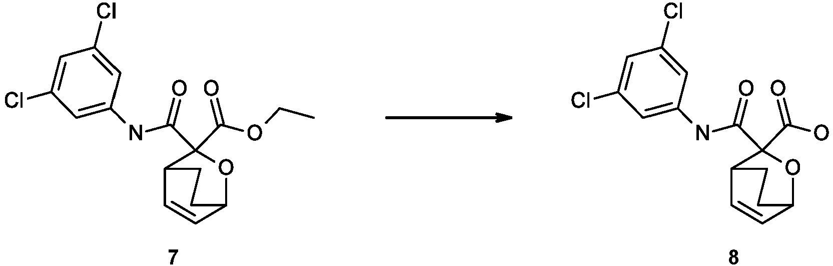

In analogy to example 1, lithium hydroxide (168 mg, 7.0 mmol) was added to a solution of ethyl 3-[(3,5-dichlorophenyl)carbamoyl]-2-oxabicyclo[2.2.2]oct-5-ene-3-carboxylate (**7**) (1.3 g, 3.5 mmol) in a 1:1-mixture of THF and water (20 mL). After stirring the mixture at room temperature for 2 h, THF was evaporated *in vacuo* and the residue was acidified to pH=1 with HCI (1 M). The aqueous acidic phase was extracted with ethyl acetate (3 x 10 mL) and the combined extracts were dried over MgSO₄. After concentration, the product (**8**) (900 g, 99% yield) was obtained as colorless amorphous crystals and was used in the next step without further purification. 1H NMR (400 MHz, Chloroform-d) δ 8.94 (s, 1H), 7.58 (d, 2H), 7.16 (t, 1H), 6.54 (m, 2H), 4.89 (m, 1H), 3.63 (dd, 1H), 2.08 (m, 1H), 1.71 (ddt, 1H), 1.39 (m, 2H).

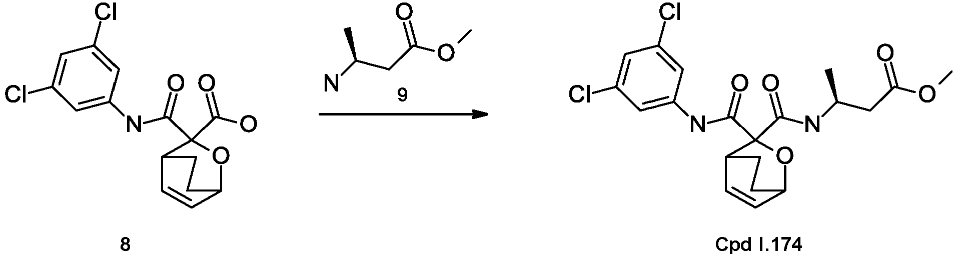

In analogy to example 3, to a solution of carboxylic acid **8** (150 mg, 0.438 mmol) in dimethylformamide (DMF, 5 mL) (3S)-3-aminobutanoate (**9**) hydrochloride (80.8 mg, 0.503 mmol) (CAS [139243-55-3]) was added. To the resulting solution was added HATU (2-(7-aza-1H-benzotriazole-1-yl)-1,1,3,3-tetramethyluronium hexafluorophosphate, CAS [148893-10-1]), (210 mg, 0.526 mmol) and then diisopropyl ethyl amine (0.22 mL, 1.3 mmol). The resulting reaction mixture was stirred at room temperature overnight. To the reaction mixture water and sodium bicarbonate solution were added. The reaction mixture was extracted with ethyl acetate, washed with water, dried (sodium sulfate) and the solvent was evaporated under reduced pressure. The crude product was purified by column chromatography (silica RP18, MeCN/H₂O) yielding compound I.174 (128 mg, 66%, 1:1 mixture of diastereomers). 1H NMR (400 MHz, Chloroform-d) δ 9.35 (s, 1H), 9.28 (s, 1H), 7.57 (m, 4H), 7.08 (m, 2H), 6.44 (m, 4H), 4.85 (t, 2H), 4.27 (m, 2H), 3.67 (m, 7H), 3.53 (ddd, 1H), 2.55 (m, 2H), 2.47 (dd, 4H), 2.18 (m, 1H), 1.72 (m, 1H), 1.29 (m, 4H), 1.18 (m, 6H).

### Example 26:

### Synthesis of methyl (1S,4R)-4-[[5-[(3,5-dichlorophenyl)carbamoyl]-3-methyl-4H-isoxazole-5-carbonyl]amino]cyclopent-2-ene-1-carboxylate (1:1 mixture of diastereoisomers) - Compound I.178

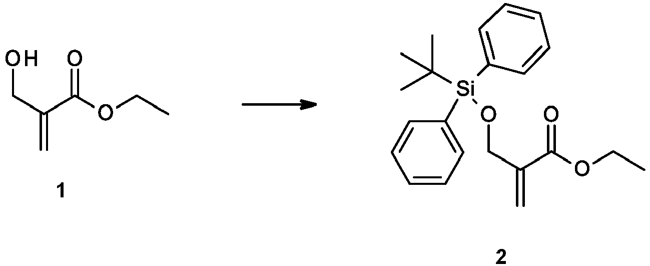

To a solution of compound ethyl 2-(hydroxymethyl)prop-2-enoate (**1**) (CAS: 10029-04-6, 4.3 g, 33 mmol) in dichloromethane (45 mL) was added tert-butyldiphenylsilyl chloride (CAS: 58479-61-1, 9.97 g, 36.3 mmol) and imidazole (2.69 g, 39.6 mmol) at room temperature. The mixture was stirred for 2 h at room temperature. The mixture was filtered and the filtrate was concentrated. The crude was purified by column chromatography (pentane/EtOAc) to afford compound 2 (11.3 g, 93%) as yellow oil. 1H NMR (400 MHz, Chloroform-d) δ 7.68 (dd, 4H), 7.41 (m, 6H), 6.34 (q, 1H), 6.11 (q, 1H), 4.44 (t, 2H), 4.18 (m, 2H), 1.27 (m, 3H), 1.10 (m, 9H).

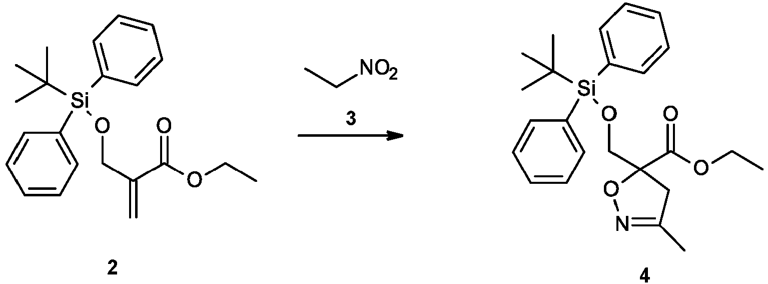

To a mixture of compound **2** (20 g, 54.3 mmol), di-tert-butyl dicarbonate (CAS: 24424-99-5, 23.7 g, 108.6 mmol) and 4-dimethylaminopyridine (1.3 g, 10.9 mmol) in chloroform (200 mL) was added compound nitroethane (**3**) (10.2 g, 135.8 mmol) dropwise. The mixture was stirred for 16 h at room temperature. The mixture was poured into the solution of NH₄Cl (aq), extracted with dichloromethane, the organic layers was washed with brine, dried, concentrated. The residue was purified by column chromatography (pentane/EtOAc) to afford compound **4** (17.5 g, 75%) as brown oil. 1H NMR (400 MHz, Chloroform-d) δ 7.67 (m, 4H), 7.49 - 7.36 (m, 6H), 4.23 (q, 2H), 3.99 (m, 1H), 3.89 (m, 1H), 3.44 (dd, 1H), 3.50 - 3.39 (m, 1H), 3.12 (dd, 1H), 1.99 (s, 3H), 1.29 (m, 3H), 1.04 (s, 9H)

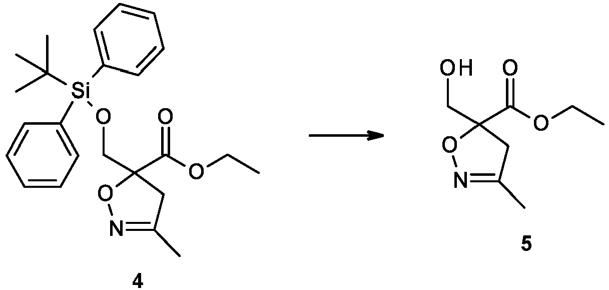

To a solution of compound **4** (17.5 g, 41.2 mmol) in THF (175 mL) was added tetrabutylammonium fluoride (TBAF, 1 M in THF, 61.8 mL, 61.8 mmol) dropwise at 0 °C. The mixture was stirred for 2 h at the same temperatue. The mixture was poured into ice water, extracted with ethyl acetate, the organic layers was washed with brine, dried, concentrated. The residue was purified by column chromatography (pentane/EtOAc) to afford compound **5** (4 g, 52%) as brown oil. 1H NMR (400 MHz, Chloroform-d) δ 4.24 (m, 2H), 3.73 (m, 2H), 3.22 (m, 2H), 2.54 (br s, 1H), 1.98 (s, 3H), 1.29 (t, 3H).

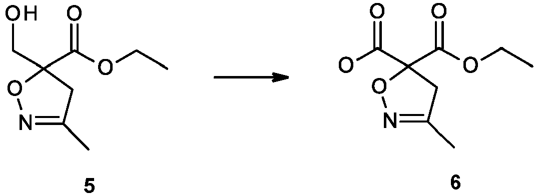

The mixture of compound **5** (2 g, 10.7 mmol) and pyridinium dichromate (PDC, CAS: 20039-37-6, 40.2 g, 107 mmol) in DMF (40 mL) was stirred for 16 h at room temperature. The mixture was poured into ice water, adjusted pH= 3 with 3N HCI, extracted with EtOAc, the organic layers was washed with brine, dried, concentrated to give compound **6** (1.8 g, Crude) as brown oil.

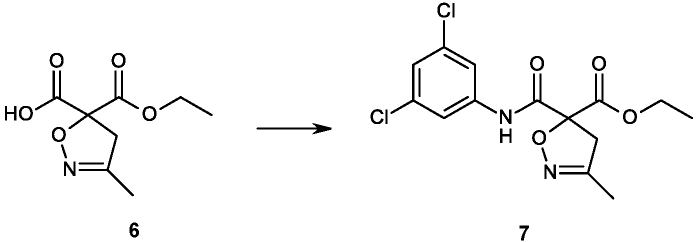

In analogy to example 1, to a solution of compound **6** (1.8 g, 8.0 mmol) in THF (18 mL) was added 3,5-dichloroaniline (1.7 g, 10.8 mmol), HATU (4.1 g, 10.8 mmol), triethylamine (1.8 g, 18 mmol) at room temperature. After stirring for 3 h at the same temperature, the mixture was poured into ice water, extracted with EtOAc, the organic layers was washed with brine, dried, concentrated. The residue was purified by prep-HPLC (NH₄HCO₃, MeCN-H₂O) to give compound 7 (651 mg, 28 %) as brown solid. 1H NMR (400 MHz, Chloroform-d) δ 8.65 (br s, 1H), 7.58 (d, 2H), 7.17 (s, 1H), 4.32 (m, 2H), 3.92 (d, 1H), 3.47 (d, 1H), 2.05 (s, 3H), 1.31 (t, 3H).

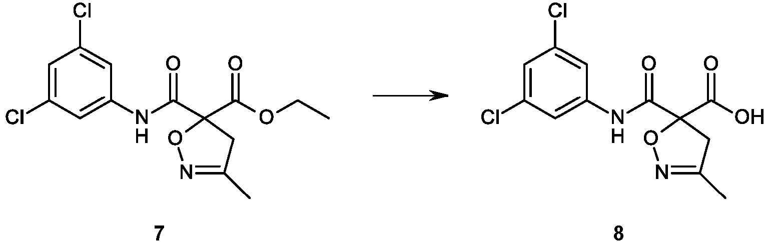

In analogy to example 1, lithium hydroxide (66.2 mg, 2.85 mmol) was added to a solution of compound **7** (575 mg, 1.67 mmol) in a 1:1-mixture of THF and water (5 mL). After stirring the mixture at room temperature for 2 h, THF was evaporated *in vacuo* and the residue was acidified to pH=1 with HCI (1 M). The aqueous acidic phase was extracted with ethyl acetate (3 x 10 mL) and the combined extracts were dried over MgSO₄. After concentration, the product (**8**) (900 g, 99% yield) was obtained as colorless amorphous crystals and was used in the next step without further purification. LC-MS (M+H)⁺: 317.1

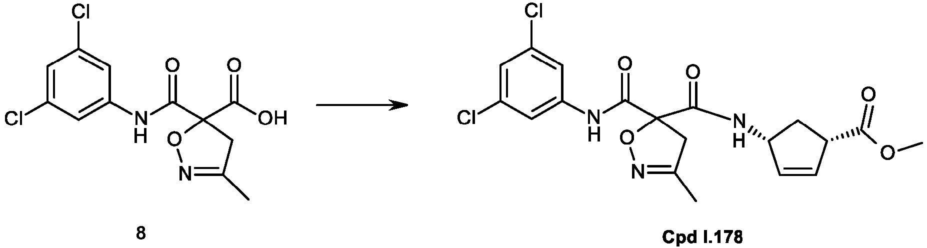

In analogy to example 2, to a solution of carboxylic acid **8** (150 mg, 0.438 mmol) in dimethylformamide (DMF, 5 mL) methyl (1S,4R)-4-aminocyclopent-2-ene-1-carboxylate hydrochloride (CAS: 180196-56-9, 148 mg, 0.833 mmol) was added. To the resulting solution was added HATU (2-(7-aza-1H-benzotriazole-1-yl)-1,1,3,3-tetramethyluronium hexafluorophosphate, CAS [148893-10-1]), (316 mg, 0.833 mmol) and then diisopropyl ethyl amine (0.35 mL, 2.1 mmol). The resulting reaction mixture was stirred at room temperature overnight. To the reaction mixture water and sodium bicarbonate solution were added. The reaction mixture was extracted with ethyl acetate, washed with water, dried (sodium sulfate) and the solvent was evaporated under reduced pressure. The crude product was purified by column chromatography (silica RP18, MeCN/H₂O) yielding compound I.178 (245 mg, 80%, 1:1 mixture of diastereomers). 1H NMR (400 MHz, Chloroform-d) δ 8.86 (s, 1H), 8.74 (s, 1H), 7.61 (d, 2H), 7.56 (d, 2H), 7.35 (d, 1H), 7.27 (m, 1H), 7.12 (m, 2H), 6.01 (dq, 1H), 5.96 (ddd, 1H), 5.91 (m, 2H), 5.00 (m, 2H), 3.73 (m, 6H), 3.61 (m, 2H), 3.54 (m, 2H), 2.45 (dt, 1H), 2.36 (dt, 1H), 2.17 (s, 6H), 2.03 (m, 4H).

### Example 27:

### Synthesis of methyl (1S,4R)-4-[[5-[(3,5-dichlorophenyl)carbamoyl]-2H-thiophene-5-carbonyl]amino]cyclopent-2-ene-1-carboxylate (1:1 mixture of diastereoisomers) - Compound I.177

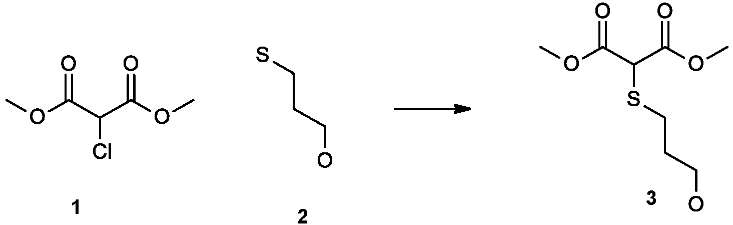

To a solution of dimethyl 2-chloropropanedioate (1) (45.5 g, 274 mmol) in THF (300 ml) was added 3-sulfanylpropan-1-ol (**2**) (19.5 g, 212 mmol) and Na₂CO₃ (67.3 g, 635 mmol) at room temperature. The mixture was stirred at 45 °C for 16h. The reaction mixture was filtered and the filtrate was concentrated. The crude was purified by column chromatography by applying a gradient (EtOAc/pentane = 100:0 to 1:1) to give compound 3 (54 g, 89%) as yellow oil. 1H NMR (400 MHz, Chloroform-d) δ 4.22 (s, 1H), 3.80 (s, 6H), 3.76 (t, 2H), 2.86 (t, 2H), 1.86 (m, 2H).

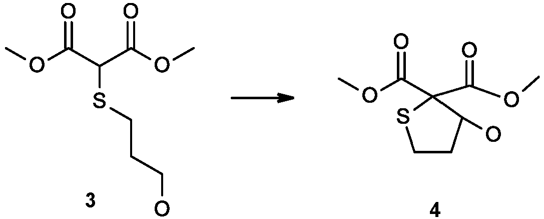

In two parallel reactions, 4 Å molecular sieve (27 g*2) and pyridinium chlorochromate (PCC, 39 g*2 g, 182 mmol*2) was added to a solution of compound (27*2 g, 121.6*2 mmol) in dichloromethane (1.5*2 L) at room temperature. The two mixtures were stirred in two different flasks for 4h. The two mixtures were filtered and the combined filtrates were concentrated. The residue was purified by column chromatography by applying a gradient (EtOAc/pentane = 100:0 to 1:1) to give compound 4 (21 g, 39%) as yellow oil. 1H NMR (400 MHz, Chloroform-d) δ 4.80 (br d, 1H), 3.82 (s, 3H), 3.78 (s, 3H), 3.17 (m, 1H), 2.97 (m, 2H), 2.38 (m, 2H).

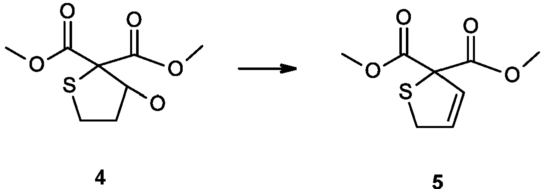

To a solution of compound **4** (19.5 g, 88.6 mmol) and 4-dimethylaminopyridine (54 g, 443 mmol) in dichloromethane (400 mL) was added trifluoromethanesulfonic anhydride (62.5 g, 221.6 mmol) dropwise at -40 °C. After warming to room temperature, the mixture was stirred for 16h at the same temperature before the reaction was quenched with H₂O (500 mL). The aqueous layer was separated and extracted with dichloromethane. The combined extracts were washed with brine, dried over Na₂SO₄ and concentrated. The residue was purified by column chromatography by applying a gradient (EtOAc/pentane = 100:0 to 1:1) to give compound **5** (10 g, 55.9%) as yellow solid. 1H NMR (400 MHz, Chloroform-d) δ 6.11 (td, 1H), 5.98 (td, 1H), 3.88 (t,), 3.80 (m, 6H).

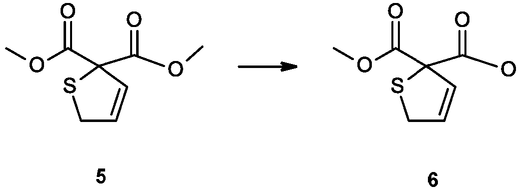

To a solution of compound **5** (7.25 g, 35.9 mmol) in MeOH (60 mL) was added lithium hydroxide (1.5 g, 35.9 mmol) in H₂O (60 mL) dropwise at 0°C. The mixture was stirred for 2h at 0°C. The reaction mixture was quenched with H₂O (100 mL) and adjusted to pH= 3, extracted with EtOAc (80 mL*2). The combined organics were washed with brine, dried over Na₂SO₄ and concentrated to give compound **6** (6 g, 89%) as yellow solid. 1H NMR (400 MHz, Chloroform-d) δ 6.15 (m, 1H), 5.99 (td, 1H), 3.92 (t, 2H), 3.83 (m, 4H).

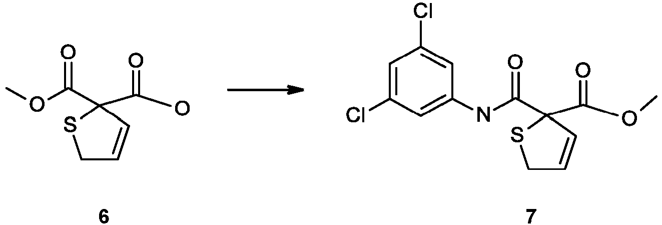

To a solution of compound **6** (5 g, 26.6 mmol) in dichloromethane (50 mL) was added DMF (1 drop) and oxalyl chloride (6.9 g, 53.2 mmol) dropwise at 0°C. The mixture was stirred for 2h at 0°C. The mixture was added dropwise to the solution of 3,5-dichloroaniline (6.5 g, 39.9 mmol) and pyridine (8.4 g, 106.4 mmol) in dichloromethane (50 mL) at 0°C. After stirring for 2 h at the same temperature, the reaction was quenched with H₂O (50 mL). The aqueous layer was extracted with EtOAc (50 mL*2). The combined extracts were washed with brine, dried over Na₂SO₄ and concentrated. The crude was purified by column chromatography by applying a gradient (EtOAc/pentane = 100:0 to 70:30) to give compound **7** (8 g, 91%) as yellow solid. 1H NMR (400 MHz, Chloroform-d) δ 9.13 (br s, 1H), 7.56 (d, 2H), 7.15 (t, 1H), 6.13 (m, 2H), 3.99 (m, 2H), 3.82 (s, 3H).

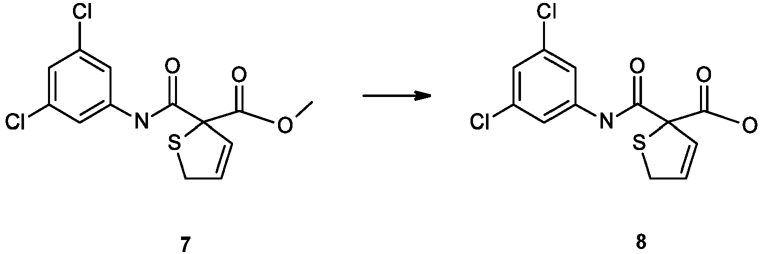

To a solution of compound **7** (3.31 g, 10 mmol) in a 1:1 mixture of methanol and water (40 mL) was added lithium hydroxide (420 mmol, 10 mmol) at 0°C. The mixture was stirred for 1h at 0 °C. The reaction was quenched with H₂O (50 mL), washed with EtOAc (50 mL). The aqueous phase was adjusted to pH = 3 with 6N HCI and extracted with EtOAc (50 mL*2). The combined organics were washed with brine, dried over Na₂SO₄ and concentrated to give compound **8** (2 g, 63%) as yellow solid. 1H NMR (400 MHz, MeOD) δ 7.67 (d, 2H), 7.20 (t, 1H), 6.18 (dt, 1H), 6.04 (dt, 1H), 4.97 (m, 2H).

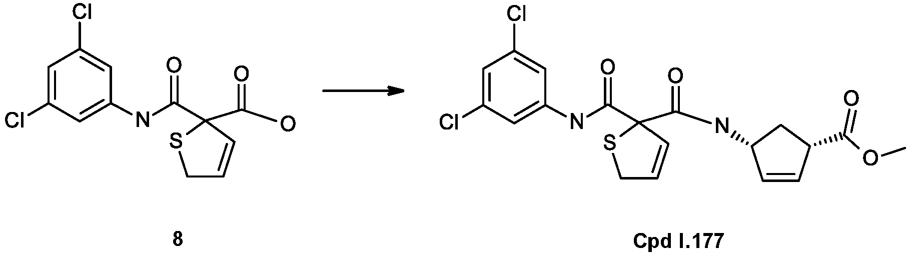

To a solution of compound **8** (500 mg, 4.58 mmol) and methyl (1S,4R)-4-aminocyclopent-2-ene-1-carboxylate (418 mg, 2.36 mmol) in THF (10 mL) was added triethylamine (478 mg, 9.26 mmol) and 1-propanephosphonic anhydride (636 mg, 5.58 mmol) dropwise at 0°C. The mixture was stirred for 1h at 0°C. The reaction mixture was quenched with H₂O (20 mL) and extracted with EtOAc (20 mL*2). The combined organics were washed with brine, dried over Na₂SO₄ and concentrated. The crude was purified by column chromatography by applying a gradient (EtOAc/pentane = 100:0 to 0:100) to give compound I.177 (370 mg, 53%, 1:1 mixture of diastereomers) as yellow solid. 1H NMR (400 MHz, MeOD)δ 7.65 (m, 4H), 7.18 (m, 2H), 6.20 (m, 8H), 4.95 (m, 2H), 3.99 (m, 4H), 3.72 (s, 3H), 3.71 (s, 3H), 3.59 (m, 2H), 2.51 (m, 2H), 1.96 (m, 2H).

High Performance Liquid Chromatography: HPLC-column Kinetex XB C18 1,7µ (50 x 2,1 mm); eluent: acetonitrile / water + 0.1% trifluoroacetic acid (gradient from 5:95 to 100 : 0 in 1.5 min at 60°C, flow gradient from 0.8 to 1.0 ml/min in 1.5 min).

In analogy to the examples described above, the following compounds of formula (I) were prepared, wherein R¹ is hydrogen, and W is formed by R⁷ and R⁸ together with the carbon atom to which they are bound, starting from commercially available diesters and using commercially available amines:

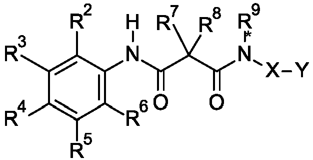

**Table 2**

| HPLC/MS = MassChargeRatio | | | | | | | | | |
|---|---|---|---|---|---|---|---|---|---|
| Cpd. | R² | R³ | R⁴ | R⁵ | R⁶ | W | R⁹ | N*-X-Y | HPLC/MS |
| I.1 | H | F | H | F | H | 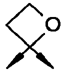 | H | 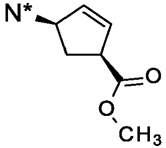 | 314.8 |
| I.2 | H | F | H | F | H | 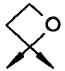 | H | 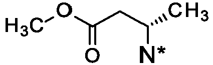 | 379.0 |
| I.3 | H | F | H | F | H | 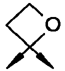 | H | 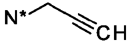 | 294.8 |
| I.4 | H | F | H | F | H | 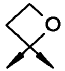 | H | 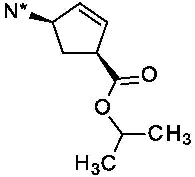 | 408.8 |
| I.5 | H | F | F | F | H | 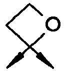 | H | 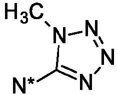 | 357.0 |
| I.6 | H | Cl | H | Cl | H | 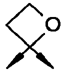 | H | 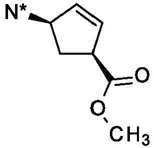 | 388.8 |
| I.7 | H | Cl | H | Cl | H | 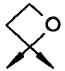 | H | 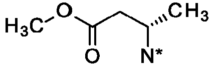 | 374.6 |
| I.8 | H | Cl | H | Cl | H | 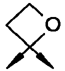 | H | 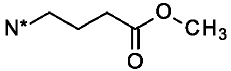 | 388.9 |
| I.9 | H | Cl | H | Cl | H | 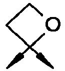 | H | 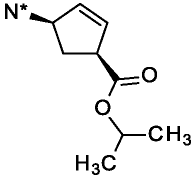 | 440.8 |
| I.10 | H | Cl | H | Cl | H | 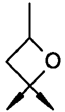 | H | 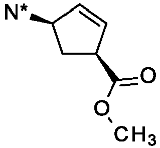 | 426.9 |
| I.11 | H | Cl | H | Cl | H | 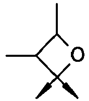 | H | 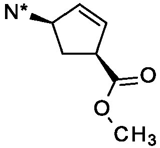 | 440.9 |
| I.12 | H | Cl | H | Cl | H | 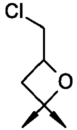 | H | 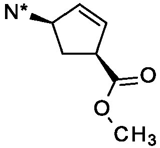 | 462.9 |
| I.13 | H | Cl | H | Cl | H | 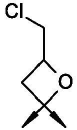 | H | 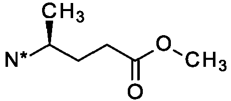 | 451.1 |
| I.14 | H | Cl | H | Cl | H | 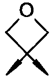 | H | 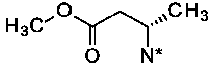 | 374.6 |
| I.15 | H | Cl | H | Cl | H | 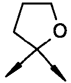 | H | 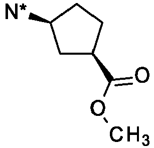 | 428.8 |
| I.16 | H | Cl | H | Cl | H | 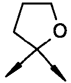 | H | 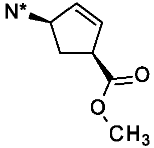 | 427.0 |
| I.17 | H | Cl | H | Cl | H | 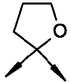 | H | 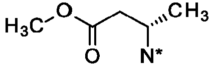 | 402.9 |
| I.18 | H | Cl | H | Cl | H | 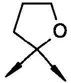 | H | 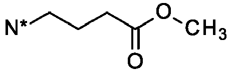 | 402.9 |
| I.19 | H | Cl | H | Cl | H | 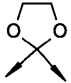 | H | 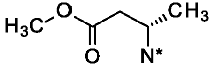 | 405.0 |
| I.20 | H | Cl | H | Cl | H | 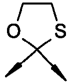 | H | 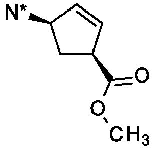 | 444.7 |
| I.21 | H | Cl | H | Cl | H | 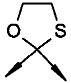 | H | 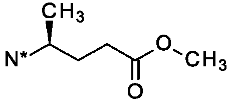 | 435.0 |
| I.22 | H | Cl | H | Cl | H | 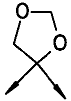 | H | 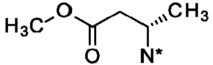 | 405.0 |
| I.23 | H | Cl | H | Cl | H | 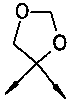 | H | 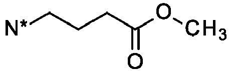 | 404.9 |
| I.24 | H | Cl | H | Cl | H | 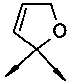 | H | 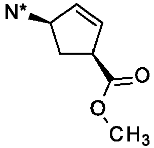 | 424.7 |
| I.25 | H | Cl | H | Cl | H | 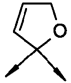 | H | 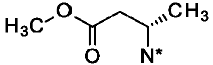 | 400.9 |
| I.26 | H | Cl | H | Cl | H | 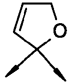 | H | 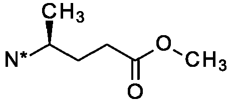 | 414.9 |
| I.27 | H | Cl | H | Cl | H | 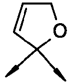 | Me | 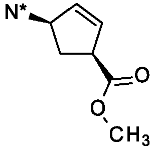 | 438.9 |
| I.28 | H | Cl | H | Cl | H | 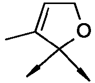 | H | 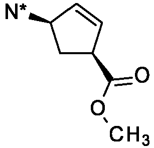 | 439.1 |
| I.29 | H | Cl | H | Cl | H | 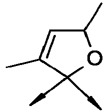 | H | 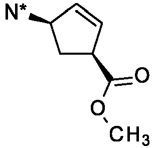 | 453.1 |
| I.30 | H | Cl | H | Cl | H | 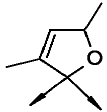 | H | 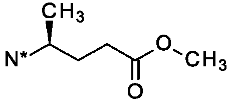 | 442.9 |
| I.31 | H | Cl | H | Cl | H | 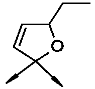 | H | 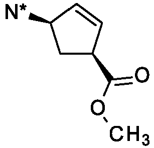 | 453.2 |
| I.32 | H | Cl | H | Cl | H | 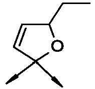 | H | 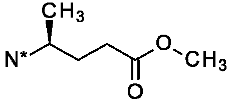 | 443.2 |
| I.33 | H | Cl | H | Cl | H | 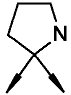 | H | 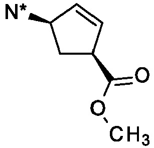 | 425.9 |
| I.34 | H | Cl | H | Cl | H | 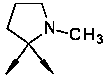 | H | 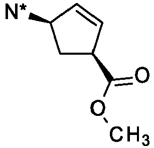 | 439.9 |
| I.35 | H | Cl | H | Cl | H | 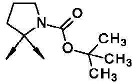 | H | 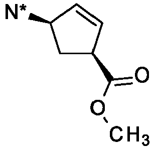 | 526.1 |
| I.36 | H | Cl | H | Cl | H | 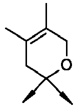 | H | 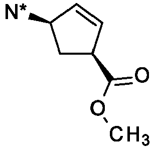 | 466.9 |
| I.37 | H | Cl | H | Cl | H | 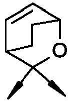 | H | 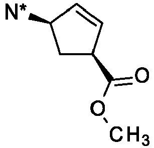 | 464.9 |
| I.38 | H | F | H | F | H | 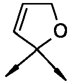 | H | 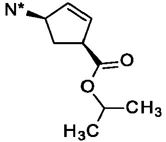 | 421.2 |
| I.39 | H | F | H | F | H | 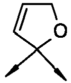 | H | 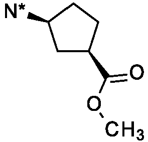 | 395.2 |
| I.40 | H | F | H | F | H | 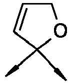 | H | 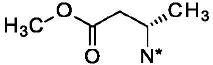 | 369.2 |
| I.41 | H | F | H | F | H | 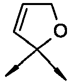 | H | 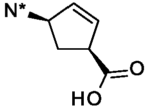 | 379.2 |
| I.42 | H | F | H | F | H | 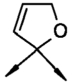 | H | 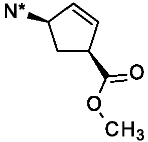 | 393.3 |
| I.43 | H | F | H | F | H | 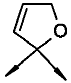 | H | 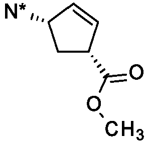 | 392.9 |
| I.44 | H | Cl | H | Cl | H | 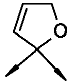 | H | 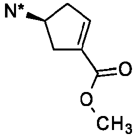 | 421.2 |
| I.45 | H | Cl | H | Cl | H | 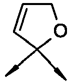 | H | 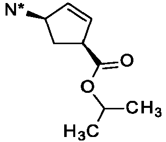 | 452.9 |
| I.46 | H | F | H | F | H | 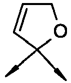 | H | 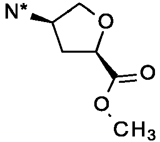 | 397.1 |
| I.47 | H | F | H | F | H | 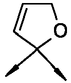 | H | 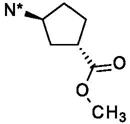 | 395 |
| I.48 | H | F | H | F | H | 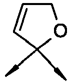 | H | 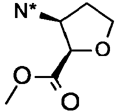 | 396.9 |
| I.49 | H | F | H | F | H | 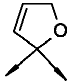 | H | 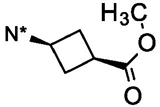 | 381.1 |
| I.50 | H | F | H | F | H | 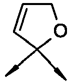 | H | 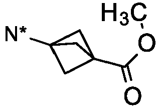 | 393.1 |
| I.51 | H | F | H | F | H | 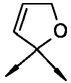 | H | 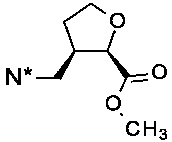 | 411.1 |
| I.52 | H | F | H | F | H | 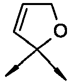 | H | 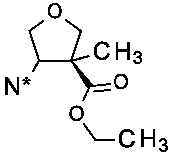 | 425.2 |
| I.53 | H | Cl | H | Cl | H | 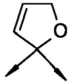 | H | 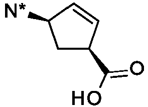 | 411.2 |
| I.54 | H | CH₂CH₃ | H | H | H | 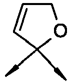 | H | 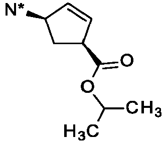 | 413.3 |
| I.55 | H | I | H | H | H | 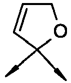 | H | 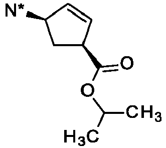 | 511 |
| I.56 | H | Cl | Cl | Cl | H | 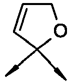 | H | 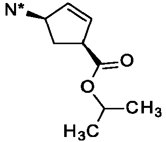 | 488.9 |
| I.57 | H | F | F | F | H | 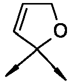 | H | 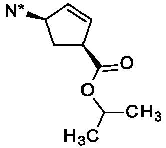 | 439 |
| I.58 | H | OCF₃ | H | H | H | 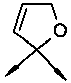 | H | 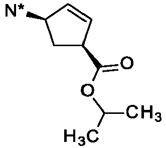 | 469 |
| I.59 | H | OCF₃ | H | F | H | 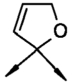 | H | 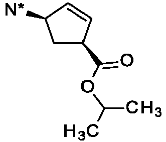 | 487 |
| I.60 | H | OCF₃ | H | Cl | H | 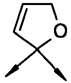 | H | 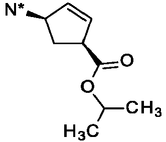 | 503 |
| I.61 | H | F | H | Cl | H | 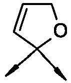 | H | 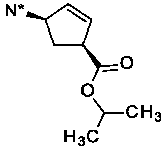 | 437.1 |
| 1.62 | H | CN | H | F | H | 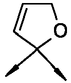 | H | 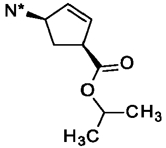 | 428 |
| I.63 | H | Cl | H | H | H | 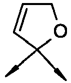 | H | 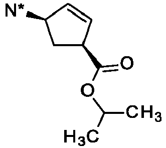 | 419.1 |
| I.64 | H | F | H | H | H | 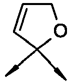 | H | 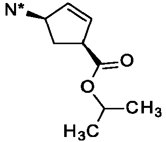 | 403.3 |
| I.65 | H | CF₃ | H | H | H | 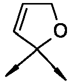 | H | 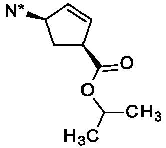 | 453 |
| I.66 | H | CF₃ | H | Cl | H | 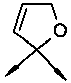 | H | 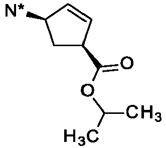 | 487 |
| I.67 | H | Cl | Cl | Cl | H | 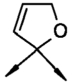 | H | 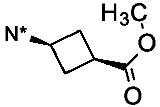 | 447.2 |
| I.68 | H | Cl | Cl | Cl | H | 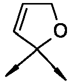 | H | 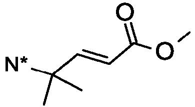 | 462.8 |
| I.69 | H | Cl | Cl | Cl | H | 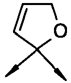 | H | 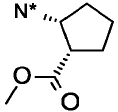 | 462.8 |
| I.70 | H | Cl | Cl | Cl | H | 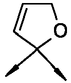 | H | 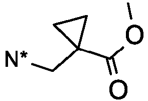 | 448.8 |
| I.71 | H | OCF₃ | H | H | H | 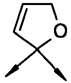 | H | 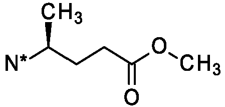 | 431.1 |
| I.72 | H | OCF₃ | H | H | H | 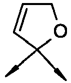 | H | 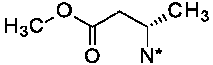 | 417.1 |
| I.73 | H | OCF₃ | H | H | H | 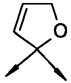 | H | 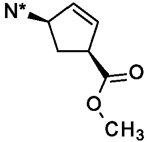 | 441 |
| I.74 | H | OCF₃ | H | H | H | 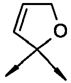 | H | 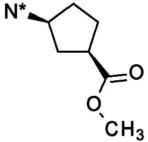 | 443 |
| I.75 | H | OCF₃ | H | H | H | 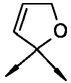 | H | 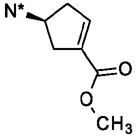 | 440.4 |
| I.76 | H | Cl | F | CI | H | 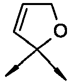 | H | 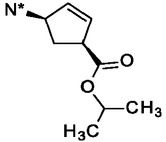 | 471.3 |
| I.77 | H | OCHF₂ | H | H | H | 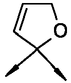 | H | 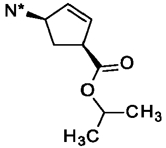 | 451.3 |
| I.78 | H | OCF₃ | H | CH₃ | H | 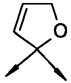 | H | 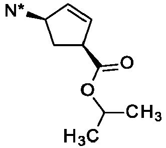 | 483.4 |
| I.79 | H | OCH₃ | H | H | H | 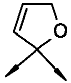 | H | 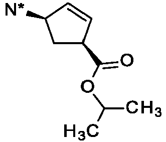 | 415.3 |
| I.80 | H | OCH₃ | H | F | H | 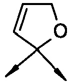 | H | 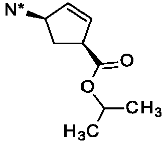 | 433.3 |
| I.81 | H | CH₃ | H | F | H | 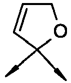 | H | 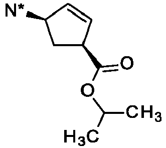 | 417.3 |
| I.82 | H | Cl | H | Cl | H | 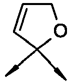 | H | 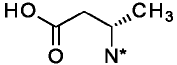 | 387.2 |
| I.83 | H | Cl | H | Cl | H | 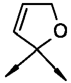 | H | 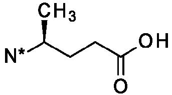 | 401.2 |
| I.84 | H | Cl | H | Cl | H | 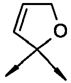 | H | 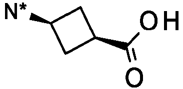 | 399.2 |
| I.85 | H | Cl | H | Cl | H | 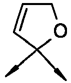 | H | 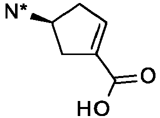 | 411.2 |
| I.86 | H | Cl | H | Cl | H | 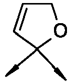 | H | 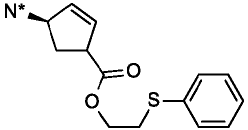 | 547.4 |
| I.87 | H | Cl | H | Cl | H | 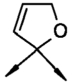 | H | 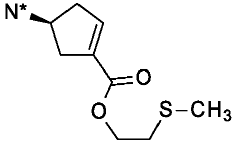 | 507.3 |
| I.88 | H | Cl | H | Cl | H | 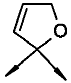 | H | 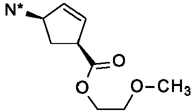 | 469.3 |
| I.89 | H | Cl | H | Cl | H | 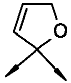 | H | 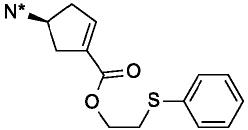 | 547.4 |
| I.90 | H | Cl | H | Cl | H | 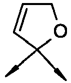 | H | 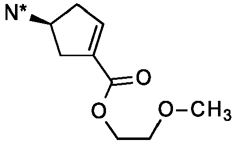 | 468.9 |
| I.91 | H | Cl | H | Cl | H | 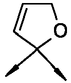 | H | 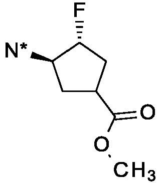 | 444.8 |
| I.92 | H | Cl | H | Cl | H | 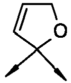 | H | 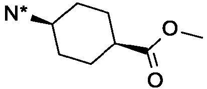 | 440.9 |
| I.93 | H | Cl | H | Cl | H | 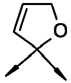 | H | 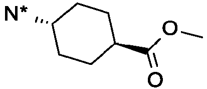 | 440.9 |
| I.94 | H | Cl | H | Cl | H | 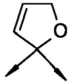 | H | 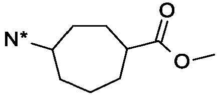 | 454.9 |
| I.95 | H | Cl | H | Cl | H | 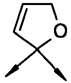 | H | 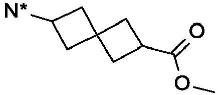 | 452.9 |
| I.96 | H | Cl | H | Cl | H | 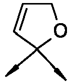 | H | 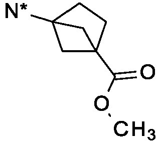 | 438.9 |
| I.97 | H | F | H | F | H | 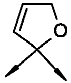 | H | 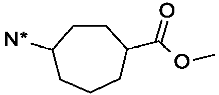 | 423 |
| I.98 | H | F | H | F | H | 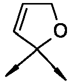 | H | 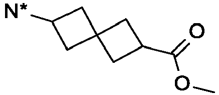 | 421 |
| I.99 | H | F | H | F | H | 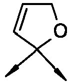 | H | 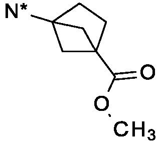 | 406.9 |
| I.100 | H | F | H | F | H | 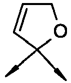 | H | 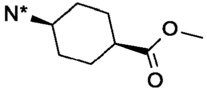 | 408.9 |
| I.101 | H | F | H | F | H | 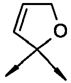 | H | 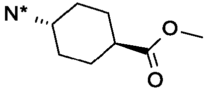 | 408.8 |
| I.102 | H | F | H | F | H | 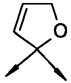 | H | 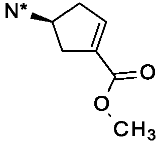 | 392.9 |
| I.103 | H | Cl | H | Cl | H | 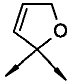 | H | 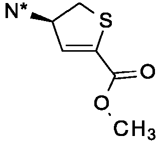 | 300.8 |
| I.104 | H | F | H | F | H | 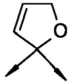 | H | 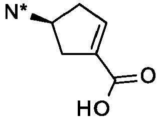 | 378.9 |
| I.105 | H | F | H | F | H | 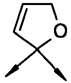 | H | 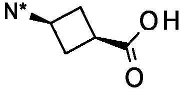 | 366.9 |
| I.106 | H | Cl | H | Cl | H | 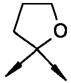 | H | 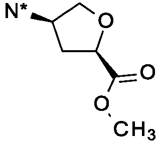 | 430.8 |
| I.107 | H | Cl | H | Cl | H | 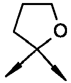 | H | 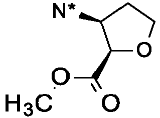 | 430.9 |
| I.108 | H | Cl | H | Cl | H | 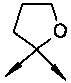 | H | 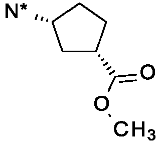 | 428.9 |
| I.109 | H | F | H | F | H | 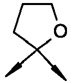 | H | 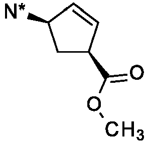 | 394.9 |
| I.110 | H | F | H | F | H | 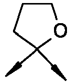 | H | 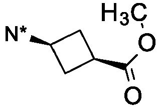 | 382.9 |
| I.111 | H | Cl | H | Cl | H | 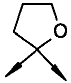 | H | 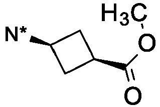 | 415.3 |
| I.112 | H | Cl | H | Cl | H | 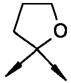 | H | 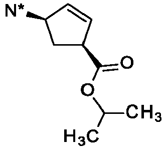 | 455 |
| I.113 | H | Cl | H | Cl | H | 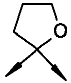 | H | 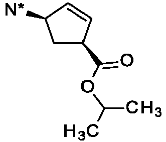 | 455.2 |
| I.114 | H | Cl | H | H | H | 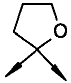 | H | 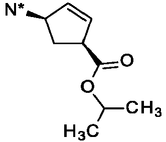 | 421.2 |
| I.115 | H | F | H | H | H | 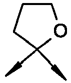 | H | 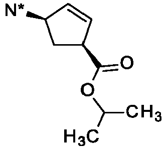 | 405.2 |
| I.116 | H | OCF₃ | H | H | H | 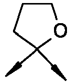 | H | 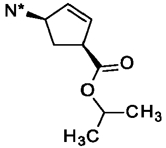 | 471 |
| I.117 | H | Cl | Cl | Cl | H | 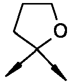 | H | 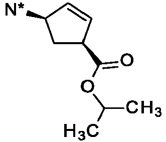 | 490.9 |
| I.118 | H | F | F | F | H | 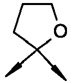 | H | 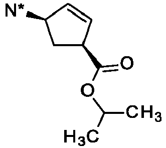 | 441.2 |
| I.119 | H | Cl | H | Cl | H | 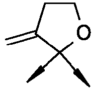 | H | 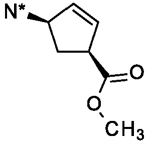 | 439.0 |
| I.120 | H | Cl | H | Cl | H | 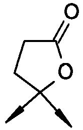 | H | 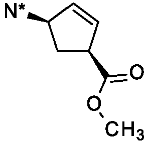 | 440.8 |
| I.121 | H | Cl | H | Cl | H | 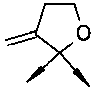 | H | 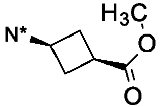 | 426.8 |
| I.122 | H | Cl | H | Cl | H | 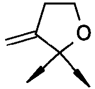 | H | 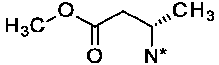 | 414.9 |
| I.123 | H | F | H | F | H | 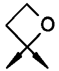 | H | 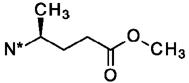 | 371.1 |
| I.124 | H | F | H | F | H | 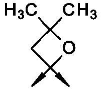 | H | 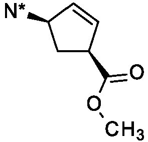 | 408.9 |
| I.125 | H | F | H | F | H | 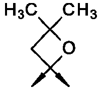 | H | 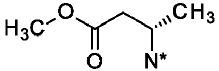 | 385 |
| I.126 | H | F | H | F | H | 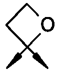 | H | 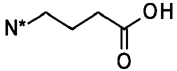 | 343.2 |
| I.127 | H | F | H | F | H | 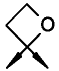 | H | 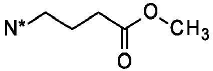 | 356.9 |
| I.128 | H | F | H | F | H | 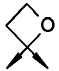 | H | 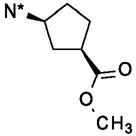 | 382.9 |
| I.129 | H | F | H | F | H | 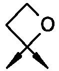 | H | 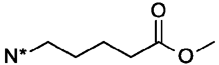 | 343.2 |
| I.130 | H | F | H | F | H | 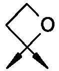 | H | 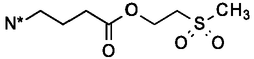 | 448.9 |
| I.131 | H | F | H | F | H | 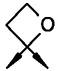 | H | 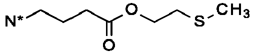 | 417.2 |
| I.132 | H | F | H | H | H | 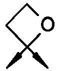 | H | 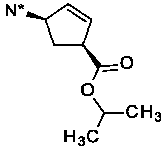 | 390.9 |
| I.133 | H | F | F | F | H | 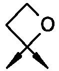 | H | 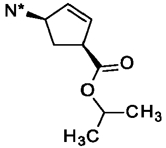 | 426.9 |
| I.134 | H | OCF₃ | H | H | H | 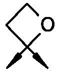 | H | 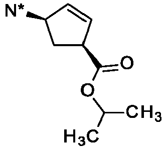 | 457 |
| I.135 | H | F | H | F | H | 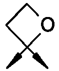 | H | 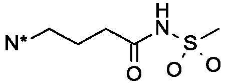 | 420.3 |
| I.136 | H | F | H | F | H | 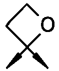 | H | 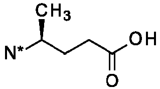 | 357.2 |
| I.137 | H | F | H | F | H | 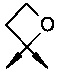 | H | 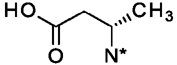 | 343.2 |
| I.138 | H | F | H | F | H | 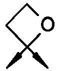 | H | 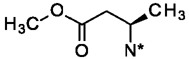 | 357 |
| I.139 | H | F | H | F | H | 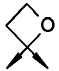 | H | 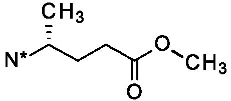 | 370.9 |
| I.140 | H | F | H | F | H | 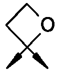 | H | 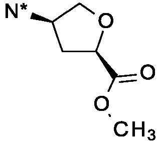 | 385 |
| I.141 | H | F | H | F | H | 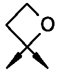 | H | 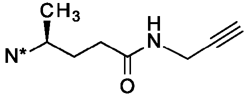 | 393.9 |
| I.142 | H | F | H | F | H | 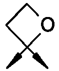 | H | 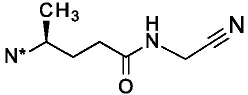 | 394.9 |
| I.143 | H | F | H | F | H | 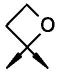 | H | 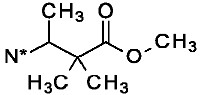 | 385.3 |
| I.144 | H | F | H | F | H | 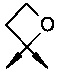 | H | 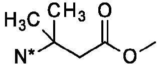 | 371.2 |
| I.145 | H | F | H | F | H | 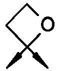 | H | 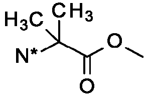 | 357.2 |
| I.146 | H | F | H | F | H | 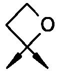 | H | 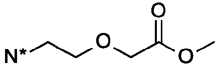 | 372.9 |
| I.147 | H | F | H | F | H | 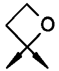 | H | 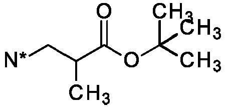 | 343.1 |
| I.148 | H | F | H | F | H | 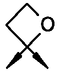 | H | 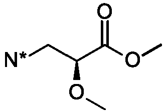 | 372.8 |
| I.149 | H | F | H | F | H | 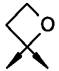 | H | 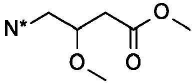 | 386.9 |
| I.150 | H | F | H | F | H | 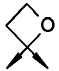 | H | 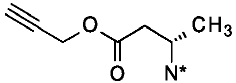 | 381.2 |
| I.151 | H | F | H | F | H | 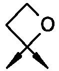 | H | 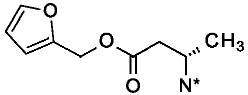 | 423.3 |
| I.152 | H | F | H | F | H | 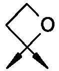 | H | 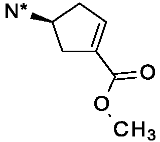 | 381.3 |
| I.153 | H | F | H | F | H | 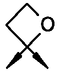 | H | 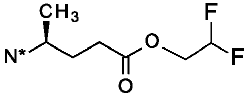 | 420.9 |
| I.154 | H | F | H | F | H | 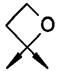 | H | 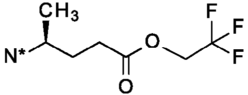 | 438.9 |
| I.155 | H | F | H | F | H | 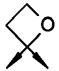 | H | 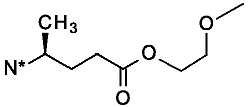 | 414.9 |
| I.156 | H | F | H | F | H | 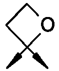 | H | 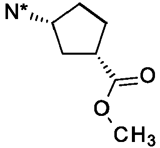 | 383.2 |
| I.157 | H | F | H | F | H | 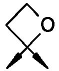 | H | 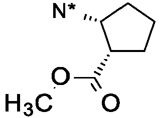 | 383.3 |
| I.158 | H | F | H | F | H | 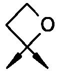 | H | 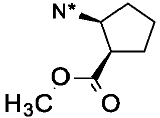 | 383.2 |
| I.159 | H | F | H | F | H | 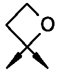 | H | 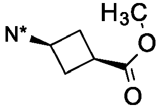 | 369.2 |
| I.160 | H | F | H | F | H | 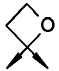 | H | 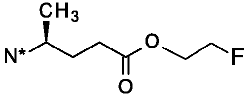 | 403.3 |
| I.161 | H | F | H | F | H | 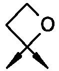 | H | 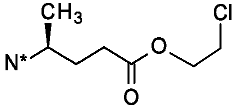 | 419.3 |
| I.162 | H | F | H | F | H | 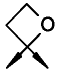 | H | 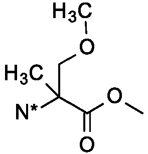 | 387 |
| I.163 | H | F | H | F | H | 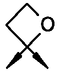 | H | 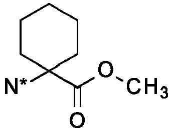 | 397 |
| I.164 | H | F | H | F | H | 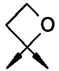 | H | 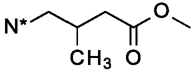 | 371 |
| I.165 | H | F | H | F | H | 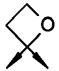 | H | 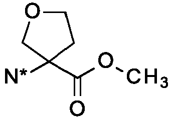 | 384.9 |
| I.166 | H | F | H | F | H | 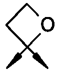 | H | 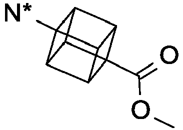 | 417.1 |
| I.167 | H | F | H | F | H | 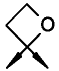 | H | 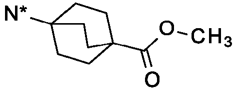 | 423.2 |
| I.168 | H | F | H | F | H | 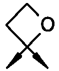 | H | 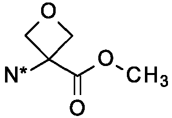 | 370.8 |
| I.169 | H | F | H | F | H | 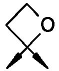 | H | 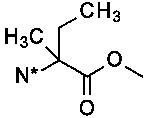 | 371 |
| I.170 | H | F | H | F | H | 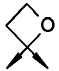 | H | 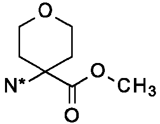 | 398.9 |
| I.171 | H | F | H | F | H | 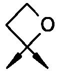 | H | 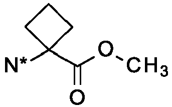 | 368.9 |
| I.172 | H | F | H | F | H | 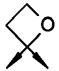 | H | 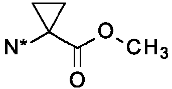 | 354.9 |
| I.173 | H | F | H | F | H | 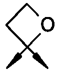 | H | 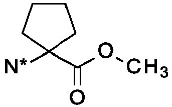 | 382.9 |
| I.174 | H | Cl | H | Cl | H | 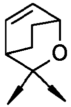 | H | 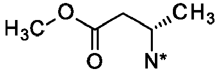 | 441.3 |
| I.175 | H | Cl | H | Cl | H | 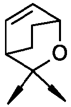 | H | 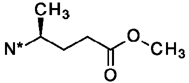 | 455.3 |
| I.176 | H | Cl | H | Cl | H | 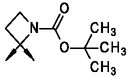 | H | 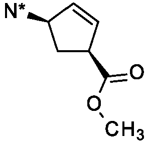 | 412.2 |
| I.177 | H | Cl | H | Cl | H | 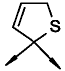 | H | 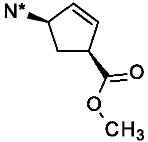 | 441 |
| I.178 | H | Cl | H | Cl | H | 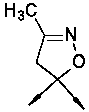 | H | 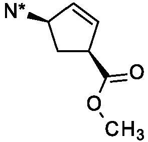 | 439.9 |
| I.179 | H | Cl | H | Cl | H | 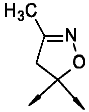 | H | 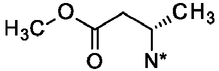 | 415.9 |
| I.180 | H | Cl | H | Cl | H | 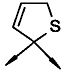 | H | 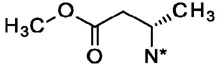 | 416.9 |
| I.181 | H | Cl | H | Cl | H | 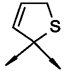 | H | 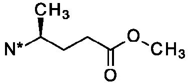 | 430.9 |
| I.182 * | H | Cl | H | Cl | H | 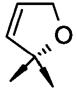 | H | 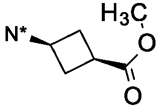 | 412.9 |
| I.183 * | H | Cl | H | Cl | H | 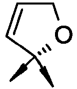 | H | 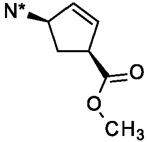 | 424.9 |
| I.184 * | H | Cl | H | Cl | H | 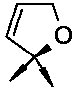 | H | 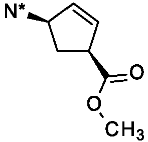 | 424.9 |
| I.185 * | H | Cl | H | Cl | H | 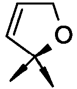 | H | 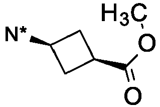 | 412.9 |
| I.186 | H | Cl | H | Cl | H | 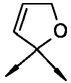 | H | 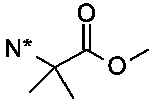 | 400.8 |
| I.187 | H | Cl | H | Cl | H | 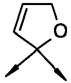 | H | 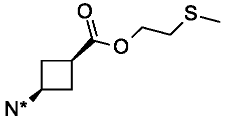 | 472.9 |
| I.188 | H | CN | H | H | H | 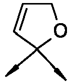 | H | 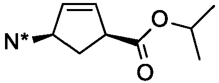 | 410.1 |
| I.189 | H | CN | H | Cl | H | 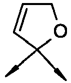 | H | 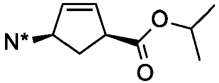 | 444.3 |
| I.190 | H | Cl | H | Cl | H | 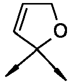 | H | 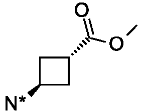 | 412.9 |
| I.191 * | H | Cl | H | Cl | H | 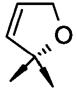 | H | 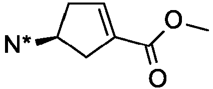 | 425.0 |
| I.192 * | H | Cl | H | Cl | H | 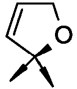 | H | 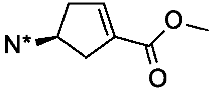 | 425.0 |
| I.193 * | H | Cl | H | Cl | H | 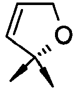 | H | 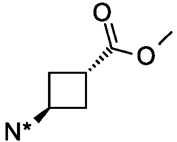 | 413.0 |
| I.194 * | H | Cl | H | Cl | H | 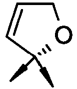 | H | 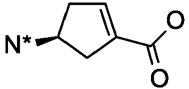 | 411.0 |
| I.195 * | H | Cl | H | Cl | H | 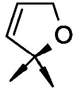 | H | 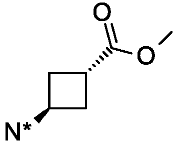 | 413.0 |
| I.196 * | H | Cl | H | Cl | H | 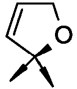 | H | 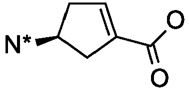 | 411.0 |
| I.197 | H | Cl | Cl | F | H | 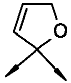 | H | 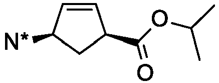 | 470.9 |
| I.198 | H | F | F | Cl | H | 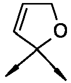 | H | 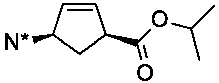 | 455.0 |
| I.199 | H | Cl | H | Cl | H | 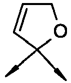 | H | 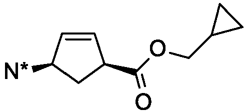 | 465.1 |
| I.200 | H | Cl | H | Cl | H | 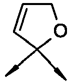 | H | 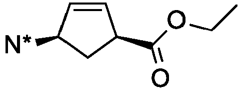 | 438.8 |
| I.201 | H | Cl | H | Cl | H | 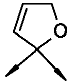 | H | 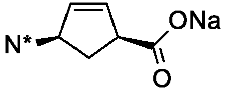 | 410.7^{a} |
| I.202 | H | Cl | H | Cl | H | 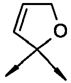 | H | 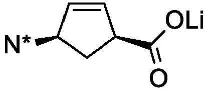 | 410.7^{b} |
| I.203 | H | F | H | CH₃ | H | 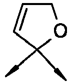 | H | 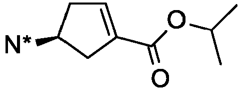 | 417.2 |
| I.204 | H | OCH₃ | H | F | H | 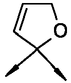 | H | 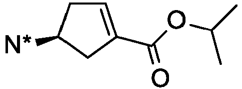 | 433.2 |
| I.205 | H | OCH₃ | H | H | H | 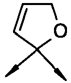 | H | 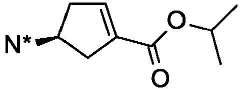 | 415.2 |
| I.206 | H | OCF₃ | H | CH₃ | H | 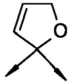 | H | 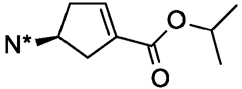 | 483.2 |
| I.207 | H | OCHF₂ | H | H | H | 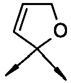 | H | 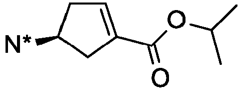 | 451.2 |
| I.208 | H | CF₃ | H | Cl | H | 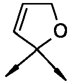 | H | 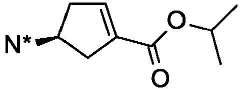 | 487.2 |
| I.209 | H | CF₃ | H | H | H | 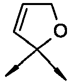 | H | 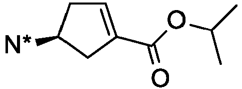 | 453.2 |
| I.210 | H | F | H | H | H | 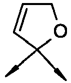 | H | 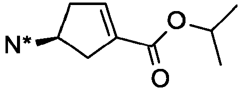 | 403.2 |
| I.211 | H | Cl | F | Cl | H | 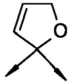 | H | 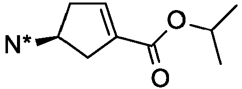 | 471.1 |
| I.212 | H | I | H | H | H | 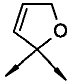 | H | 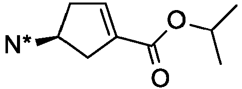 | 511.1 |
| I.213 | H | CH₂CH₃ | H | H | H | 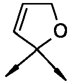 | H | 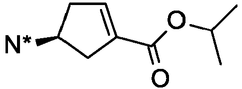 | 413.3 |
| I.214 | H | Cl | H | Cl | H | 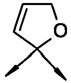 | H | 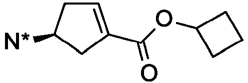 | 465.2 |
| I.215 | H | F | Cl | F | H | 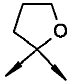 | H | 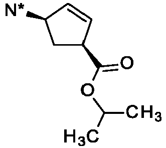 | 456.9 |
| I.216 | H | Cl | Cl | F | H | 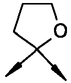 | H | 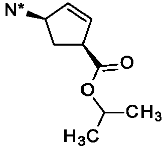 | 472.9 |
| I.217 | H | Cl | F | F | H | 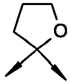 | H | 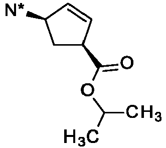 | 456.9 |
| I.218 | H | Cl | F | Cl | H | 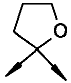 | H | 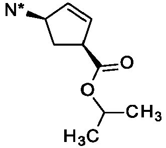 | 472.9 |
| I.219 | H | F | H | F | H | 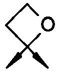 | H | 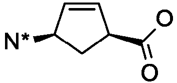 | 366.9 |
| I.220 | H | Cl | H | Cl | H | 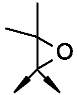 | H | 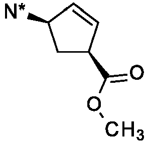 | 427.1 |
| I.221 | H | F | H | F | H | 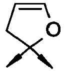 | H | 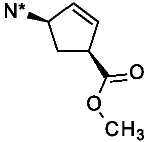 | 393.0 |
| I.222 * | H | F | H | F | H | 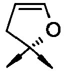 | H | 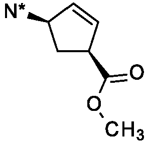 | 393.0 |
| I.223 * | H | F | H | F | H | 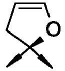 | H | 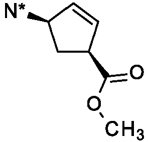 | 393.0 |

| | | | | | | | | | |
|---|---|---|---|---|---|---|---|---|---|
| ^{a} mass of the anion only. Salt obtained by neutralizing the corresponding acid with NaOH in aqueous THF and concentration of the reaction mixture. ^{b} mass of the anion only. Salt obtained by neutralizing the corresponding acid with LiOH in aqueous THF and concentration of the reaction mixture. * in context with the compound no.: Stereomerically pure compounds with an unknown absolute configurationen. These were obtained by SFC column chromatography under the indicated conditions: | | | | | | | | | |

Column: (S,S)-WHELK-O1,50x6mm i.D., 3.5 µm; mobile phase A: CO2; mobile phase B: IPA (0.1% IPAm, v/v); flow rate: 3.4 mL/min, column temp.: 35 °C, ABPR: 1800 psi; gradient: time (A/B): 0.0 (95/5), 0.2 min (95/5), 1.2 min (50/50), 2.2 (50/50), 2.6 min (95/5), 3.0 (95/5):
Cpd I.182: t_{R} = 1.384 min
Cpd I.183: t_{R} = 1.395 min
Cpd I.184: t_{R} = 1.616 min
Cpd I.185: t_{R} = 1.508 min

Column: Chiralpak AD-3, 50x4.6mm i. D., 3 µm; mobile phase A: CO2; mobile phase B: MeOH (0.1% IPAm, v/v); flow rate: 3.4 mL/min, column temp.: 35 °C, ABPR: 1800 psi; gradient: time (A/B): 0.0 (95/5), 0.2 min (95/5), 1.2 min (50/50), 2.2 (50/50), 2.6 min (95/5), 3.0 (95/5):
Cpd I.193: t_{R} = 1.386 min
Cpd I.194: t_{R} = 1.188 min
Cpd I.195: t_{R} = 1.677 min
Cpd I.196: t_{R} = 1.331 min
Cpd I.222: t_{R} = 2.020 min
Cpd I.223: t_{R} = 2.244 min

Column: Chiralpak IC-3, 50x4.6mm i. D., 3 µm; mobile phase A: CO2; mobile phase B: IPA (0.1% IPAm, v/v); flow rate: 3.4 mL/min, column temp.: 35 °C, ABPR: 1800 psi; gradient: time (A/B): 0.0 (95/5), 0.2 min (95/5), 1.2 min (50/50), 2.2 (50/50), 2.6 min (95/5), 3.0 (95/5):
Cpd I.191: t_{R} = 1.709 min
Cpd I.192: t_{R} = 2.000 min

### B Use examples

The herbicidal activity of the compounds of formula (I) was demonstrated by the following greenhouse experiments:
The culture containers used were plastic flowerpots containing loamy sand with approximately 3.0% of humus as the substrate. The seeds of the test plants were sown separately for each species.

For the pre-emergence treatment, the active ingredients, which had been suspended or emulsified in water, were applied directly after sowing by means of finely distributing nozzles. The containers were irrigated gently to promote germination and growth and subsequently covered with transparent plastic hoods until the test plants had rooted. This cover caused uniform germination of the test plants, unless this had been impaired by the active ingredients. For the post-emergence treatment, the test plants were first grown to a height of 3 to 15 cm, depending on the plant habit, and only then treated with the active ingredients which had been suspended or emulsified in water. For this purpose, the test plants were either sown directly and grown in the same containers, or they were first grown separately as seedlings and transplanted into the test containers a few days prior to treatment.

Depending on the species, the test plants were kept at 10 - 25°C or 20 - 35°C, respectively. The test period extended over 2 to 4 weeks. During this time, the test plants were tended, and their response to the individual treatments was evaluated.

Evaluation was carried out using a scale from 0 to 100. 100 means no emergence of the test plants, or complete destruction of at least the aerial moieties, and 0 means no damage, or normal course of growth. A good herbicidal activity is given at values of 70 to < 90 and a very good herbicidal activity is given at values of 90 to 100.

The test plants used in the greenhouse experiments were of the following species:

| Bayer code | Scientific name |
|---|---|
| ABUTH | *Abutilon theophrasti* |
| ALOMY | *Alopercurus myosuroides* |
| AMARE | *Amaranthus retroflexus* |
| APESV | *Apera spica-venti* |
| AVEFA | *Avena fatua* |
| ECHCG | *Echinocloa crus-galli* |
| LOLMU | *Lolium multiflorum* |
| POLCO | *Fallopia convolvulus* |
| SETVI | *Setaria viridis* |
| SETFA | *Setaria faberi* |

At an application rate of 0.500 kg/ha, applied by the pre-emergence method:
- compound I6 showed very good herbicidal activity against ABUTH.
- compounds I2, I7, I9, I17 showed very good herbicidal activity against AMARE.
- compounds I2, I6, I7, I9, I15, I16, I17, I18, I19 showed very good herbicidal activity against APESV.
- compound I14 showed good herbicidal activity against APESV.
- compounds I2, I6, I7, I9, I16 showed very good herbicidal activity against ECHCG.
- compound I15 showed good herbicidal activity against ECHCG.
- compounds I16, I17, showed very good herbicidal activity against SETFA.
- compound I15 showed good herbicidal activity against SETFA.

At an application rate of 0.250 kg/ha, applied by the pre-emergence method:
- compounds I1, I5, I21, I24, I26, I82, I83, I84, I85, I86, I87, I88, I89, I90, I91, I96, I97, I98, I99, I100, I101, I102, I105, I109, I110, I111, I112, I123, I136, I137, I138, I142, I143, I144, I145, I149, I150, I152, I153, I154, I155, I156, I159, I160, I161, I184, I185, I186, I187, I188, I190, I191 showed very good herbicidal activity against APESV.
- compounds I3, I10, I20, I93, I95, I135, I139, I141, I147, I189 showed good herbicidal activity against APESV.
- compounds I33, I114, I116 showed good herbicidal activity against ABUTH.
- compounds I87, I104, I105, I136, I138, I139, I141, I186, I191, I198, I202 showed very good herbicidal activity against AMARE.
- compounds I3, I5, I90, I96, I99, I174, I200, I201 showed good herbicidal activity against AMARE.
- compounds I1, I24, I26, I82, I83, I84, I86, I90, I91, I95, I99, I102, I104, I109, I110, I116, I118, I123, I135, I136, I137, I138, I142, I144, I145, I153, I154, I155, I156, I160, I161, I184, I185, I187 showed very good herbicidal activity against SETFA.
- compounds I29, I85, I87, I88, I89, I100, I111, I114, I139, I140, I152, I159, I188, I189, I191 showed good herbicidal activity against SETFA.
- compound I24 showed very good herbicidal activity against ALOMY.
- compound I1, I83, I84, I88, I102, I104, I105, I114, I123, I137, I145, I154, I160, I161, I184, I185, I188 showed very good herbicidal activity against ECHCG.
- compounds I26, I28, I82, I85, I91, I109, I110, I115, I142, I143, I144, I149, I153, I156, I159, I187, I189 showed good herbicidal activity against ECHCG.
- compound I5 showed very good herbicidal activity against LOLMU.
- compounds I200, I202 showed very good herbicidal activity against SETVI.
- compound I201 showed good herbicidal activity against SETVI.

At an application rate of 0.125 kg/ha, applied by the pre-emergence method:
- compound I176 showed very good herbicidal activity against ABUTH.
- compounds I30, I36 showed good herbicidal activity against ABUTH.
- compound I183 showed good herbicidal activity against APESV.

At an application rate of 0.13128 kg/ha, applied by the pre-emergence method:
- compound I133 showed very good herbicidal activity against ALOMY.
- compound I133 showed very good herbicidal activity against SETFA.
- compound I133 showed very good herbicidal activity against LOLMU.

At an application rate of 0.0625 kg/ha, applied by the pre-emergence method:
- compound I25 showed good herbicidal activity against ALOMY.
- compound I125 showed very good herbicidal activity against LOLMU.

At an application rate of 1.000 kg/ha, applied by the post-emergence method:
- compound I119 showed good herbicidal activity against ABUTH.

At an application rate of 0.500 kg/ha, applied by the post-emergence method:
- compounds I6, I9, I16 showed very good herbicidal activity against ABUTH.
- compounds I14, I15, I19 showed very good herbicidal activity against ALOMY.
- compounds I17, I18, I23 showed good herbicidal activity against ALOMY.
- compounds I7, I8, I9, I15 showed very good herbicidal activity against AMARE.
- compounds I2, I18 showed good herbicidal activity against AMARE.
- compounds I7, I14, I15 showed very good herbicidal activity against AVEFA.
- compounds I17, I18, I23 showed good herbicidal activity against AVEFA.
- compounds I2, I6, I7, I8, I9, I16, I19 showed very good herbicidal activity against ECHCG.
- compound I220 showed good herbicidal activity against ECHCG.
- compounds I6, I7, I8, I16, I17, I19 showed very good herbicidal activity against SETVI.
- compounds I2, I14 showed good herbicidal activity against SETVI.

At an application rate of 0.250 kg/ha, applied by the post-emergence method:
- compounds I1, I5, I10, I11, I86, I95, I100, I114, I115, I116, I123, I161, I185, I188 showed very good herbicidal activity against ABUTH.
- compounds I20, I21, I28, I33, I92, I117, I140, I141, I143, I144, I157, I186, I189 showed good herbicidal activity against ABUTH.
- compounds I1, I5, I20, I21, I103, I114, I118, I142, I151, I197, I200, I202 showed very good herbicidal activity against ALOMY.
- compound I141, I158, I198, I199, I201 showed good herbicidal activity against ALOMY.
- compounds I1, I5, I20, I21, I24, I82, I83, I102, I104, I109, I112, I118, I135, I138, I141, I144, I149, I152, I155, I156, I198, I199, I200, I201 showed very good herbicidal activity against AVEFA.
- compound I101, I139, I147, I148, I151, I158, I192, I197, I202 showed good herbicidal activity against AVEFA.
- Compound I10, I24, I82, I83, I84, I85, I86, I87, I88, I89, I90, I99, I102, I103, I104, I105, I109, I110, I111, I112, I114, I115, I116, I118, I123, I136, I137, I138, I140, I145, I146, I149, I150, I151, I152, I153, I154, I155, I156, I159, I160, I161, I184, I185, I186, I187, I188, I189, I191, I197, I198, I199, I200, I201, I202 showed very good herbicidal activity against ECHCG.
- compounds I26, I91, I92, I96, I100, I139, I178, I190 showed good herbicidal activity against ECHCG.
- Compound I10, I24, I26, I82, I83, I84, I85, I86, I87, I89, I90, I100, I103, I105, I109, I110, I111, I112, I116, I123, I135, I136, I137, I138, I139, I140, I142, I145, I146, I149, I150, I152, I153, I154, I155, I156, I159, I160, I184, I187, I189, I190, I191 showed very good herbicidal activity against SETVI.
- compounds I88, I91, I96, I99, I144, I147 showed good herbicidal activity against SETVI.
- compounds I84, I85, I87, I88, I89, I90, I95, I96, I99, I101, I102, I104, I105, I110, I111, I115, I135, I136, I137, I142, I145, I150, I153, I154, I159, I160, I161, I184, I185, I186, I187, I188, I191, I202 showed very good herbicidal activity against AMARE.
- compounds I26, I91, I93, I94, I98, I146, I190, I197, I198, I199, I200, I201 showed good herbicidal activity against AMARE.

At an application rate of 0.125 kg/ha, applied by the post-emergence method:
- compound I22 showed very good herbicidal activity against AVEFA.
- compound I22 showed very good herbicidal activity against ALOMY.
- compounds I125, I181 showed good herbicidal activity against ABUTH.
- compound I22 showed very good herbicidal activity against LOLMU.
- compound I122 showed good herbicidal activity against AMARE.

At an application rate of 0.0625 kg/ha, applied by the post-emergence method:
- compounds I126, I127, I128, I130, I131, I133, I134 showed very good herbicidal activity against AMARE.
- compound I129 showed good herbicidal activity against AMARE.
- compounds I25, I126, I131 showed very good herbicidal activity against AVEFA.
- compound I130, showed good herbicidal activity against AVEFA.
- compounds I128, I129, I130, I131, I132, I134 showed very good herbicidal activity against POLCO.
- compounds I106, I108 showed good herbicidal activity against POLCO.
- compounds I25, I127, I128, I132, I133 showed very good herbicidal activity against SETVI.
- compound I126 showed good herbicidal activity against SETVI.
- compounds I25, I108, I127, I129, I132, I133 showed very good herbicidal activity against LOLMU.
- compound I134 showed very good herbicidal activity against ECHCG.

At an application rate of 0.32 kg/ha, applied by the post-emergence method:
- compound I4 showed very good herbicidal activity against POLCO.
- compound I4 showed very good herbicidal activity against SETVI.
- compound I4 showed very good herbicidal activity against ECHCG.

## Claims

1. Compounds of formula (I) 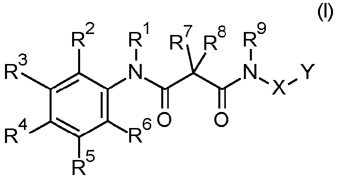
wherein the substituents have the following meanings:
R¹ is hydrogen;
R² is hydrogen or halogen;
R³ is hydrogen, halogen, cyano, (C₁-C₃)-alkyl, (C₁-C₃)-haloalkyl, (C₁-C₃)-alkoxy or (C₁-C₃)-haloalkoxy;
R⁴ is hydrogen or halogen;
R⁵ is hydrogen, halogen, cyano, (C₁-C₃)-alkyl, (C₁-C₃)-haloalkyl, (C₁-C₃)-alkoxy or (C₁-C₃)-haloalkoxy;
R⁶ is hydrogen;
R⁷ and R⁸ form, together with the carbon atom to which they are bound, a saturated or partially unsaturated 3-, 4-, 5- or 6-membered monocyclic heterocyclic ring W or a 6-, 7- or 8-membered bicyclic heterocyclic ring W, the ring containing, in addition to said carbon atom, q carbon atoms, u oxygen atoms, v nitrogen atoms, w sulfur atoms and x elements selected from the group consisting of NR^{d} and NC(O)OR^{d}, where one carbon atom bears p oxo groups and where the ring is substituted by n radicals R^{g};
R⁹ is hydrogen or (C₁-C₄)-alkyl;
X is a bond and Y is Z, where
Z is a 3-, 4-, 5-, 6-, 7- or 8-membered saturated or partly unsaturated monocyclic carbocyclic ring substituted by a group CO₂R^{e}, by 0 or 1 fluorine atoms and by 0 or 1 (C₁-C₄)-alkyl groups; or
Z is a 5-, 6-, 7- or 8-membered saturated or partly unsaturated bicyclic carbocyclic ring substituted by a group CO₂R^{e}, and by 0 or 1 (C₁-C₄)-alkyl groups; or
Z is an 8-membered saturated polycyclic carbocyclic ring substituted by a group CO₂R^{e} and by 0 or 1 (C₁-C₄)-alkyl groups; or
Z is a 5- or 6-membered saturated or partly unsaturated monocyclic heterocyclic ring containing one oxygen atom or one sulfur atom as ring member, where the heterocyclic ring is substituted by a group CO₂R^{e} and by 0 or 1 (C₁-C₄)-alkyl groups; or
Z is a 5- or 6-membered heteroaromatic ring containing 1, 2, 3 or 4 heteroatoms selected from N, O and S as ring members, where the heteroaromatic ring is substituted by 0 or 1 (C₁-C₄)-alkyl groups; or
X is a bond and Y is (C₁-C₆)-alkyl or (C₂-C₆)-alkenyl, where the two last-mentioned radicals are substituted by a group CO₂R^{e}, CONR^{b}R^{h} or CONR^{e}SO₂R^{a} and by 0 or 1 (C₁-C₄)-alkoxy groups; or
X is a bond and Y is (C₁-C₆)-alkyl substituted by Z, where Z is a 3-, 4-, 5- or 6-membered saturated monocyclic carbocyclic ring substituted by a group CO₂R^{e} and by 0 or 1 (C₁-C₄)-alkyl groups or is a 5- or 6-membered saturated monocyclic heterocyclic ring containing 1 oxygen atom as ring member, where the heterocyclic ring is substituted by a group CO₂R^{e} and by 0 or 1 (C₁-C₄)-alkyl groups; or
X is a bond and Y is (C₂-C₈)-alkynyl; or
X is X⁶ 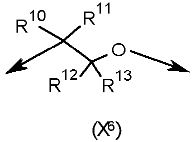 wherein R¹⁰ to R¹³ are independently hydrogen or methyl, preferably hydrogen; and Y is (C₁-C₆)-alkyl substituted by a group CO₂R^{e} and by 0 or 1 (C₁-C₄)-alkoxy groups;
R^{a} is (C₁-C₆)-alkyl;
R^{b} is hydrogen or (C₁-C₆)-alkyl;
each R^{d} is independently hydrogen or (C₁-C₆)-alkyl;
each R^{e} is independently hydrogen, (C₁-C₆)-alkyl which is unsubstituted or substituted by 1, 2 or 3 fluorine or chlorine atoms or by 1 radical selected from the group consisting of (C₁-C₂)-alkoxy, (C₁-C₃)-alkylsulfonyl, (C₁-C₃)-alkylthio, phenylthio, phenylsulfonyl and furanyl; or is (C₂-C₄)-alkynyl, (C₃-C₆)-cycloalkyl or (C₃-C₆)-cycloalkyl-(C₁-C₃)-alkyl;
R^{g} is (C₁-C₃)-alkyl or (C₁-C₃)-haloalkyl, or two R^{g}, bound on the same carbon atom, form together a methylene group (=CH₂);
R^{h} is hydrogen, (C₁-C₆)-alkyl substituted by 0 or 1 cyano groups; or (C₂-C₄)-alkynyl;
n is 0, 1 or 2;
p is 0 or 1;
q is 1, 2, 3, 4, 5 or 6;
u is 0, 1 or 2;
v is 0 or 1;
w is 0 or 1;
x is 0 or 1;
with the proviso that the sum of u, v, w and x is 1 or 2;
including their agriculturally acceptable salts, stereoisomers and tautomers.

2. The compounds as claimed in claim 1, wherein R⁹ is hydrogen.

3. The compounds as claimed in claim 1 or 2, wherein R² is hydrogen.

4. The compounds as claimed in any one of claims 1 to 3, wherein R⁵ is hydrogen, halogen or (C₁-C₃)-alkyl.

5. The compounds as claimed in any one of claims 1 to 4, wherein the substituents have the following meaning:
R³ is halogen, cyano or (C₁-C₃)-haloalkoxy;
R⁵ is hydrogen or halogen.

6. The compounds as claimed in any of claims 1 to 5, where
u is 1 or 2, v is 0, w is 0 and x is 0; or
u is 0 or 1, v is 1, w is 0 and x is 0; or
u is 0 or 1, v is 0, w is 1 and x is 0; or
u is 0, v is 0, w is 0 and x is 1.

7. The compounds as claimed in any one of claims 1 to 6, wherein the substituents have the following meaning:
R⁷ and R⁸ form, together with the carbon atom to which they are bound, a saturated or partially unsaturated four- to five-membered monocyclic heterocyclic ring W, containing, in addition to this carbon atom, q carbon atoms and u oxygen atoms, and where the ring is substituted by n radicals R^{g}, where u is 1 or 2 and q is 1, 2 or 3.

8. The compounds as claimed in any one of claims 1 to 7, where each R^{d} in the elements selected from the group consisting of NR^{d} and NC(O)OR^{d} as a ring member of the ring formed by R⁷ and R⁸ is independently hydrogen or (C₁-C₃)-alkyl.

9. The compounds as claimed in any one of claims 1 to 8, wherein X is a bond.

10. The compounds as claimed in any one of claims 1 to 9, where X is a bond and Y is Z, where
Z is a 3-, 4-, 5-, 6-, 7- or 8-membered saturated or partly unsaturated monocyclic carbocyclic ring substituted by a group CO₂R^{e}, by 0 or 1 fluorine atoms and by 0 or 1 (C₁-C₄)-alkyl groups; or
Z is a 5-, 6-, 7- or 8-membered saturated or partly unsaturated bicyclic carbocyclic ring substituted by a group CO₂R^{e}, and by 0 or 1 (C₁-C₄)-alkyl groups; or
Z is an 8-membered saturated polycyclic carbocyclic ring substituted by a group CO₂R^{e} and by 0 or 1 (C₁-C₄)-alkyl groups; or
Z is a 5- or 6-membered saturated or partly unsaturated monocyclic heterocyclic ring containing one oxygen atom or one sulfur atom as ring member, where the heterocyclic ring is substituted by a group CO₂R^{e} and by 0 or 1 (C₁-C₄)-alkyl groups; or
Z is a 5- or 6-membered heteroaromatic ring containing 1, 2, 3 or 4 heteroatoms selected from N, O and S as ring members, where the heteroaromatic ring is substituted by 0 or 1 (C₁-C₄)-alkyl groups.

11. The compounds as claimed in claim 1, wherein the substituents have the following meaning:
R¹ is hydrogen;
R² is hydrogen;
R³ is halogen, cyano or (C₁-C₃)-haloalkoxy;
R⁴ is hydrogen or halogen;
R⁵ is hydrogen, halogen or (C₁-C₃)-alkyl; in particular hydrogen or halogen;
R⁶ is hydrogen;
R⁷ and R⁸ form, together with the carbon atom to which they are bound, a saturated or partially unsaturated 3-, 4-, 5- or 6-membered monocyclic heterocyclic ring W or a 6-, 7- or 8-membered bicyclic heterocyclic ring W, the ring containing, in addition to said carbon atom, q carbon atoms, u oxygen atoms, v nitrogen atoms, w sulfur atoms and x elements selected from the group consisting of NR^{d} and NC(O)OR^{d}, where the ring is substituted by n radicals R^{g};
R⁹ is hydrogen;
X is a bond and Y is Z, where
Z is a 4-, 5-, 6-, 7- or 8-membered saturated or partly unsaturated monocyclic carbocyclic ring substituted by a group CO₂R^{e} and by 0 or 1 fluorine atoms; or
Z is a 5-, 6-, 7- or 8-membered saturated or partly unsaturated bicyclic carbocyclic ring substituted by a group CO₂R^{e} and by 0 or 1 (C₁-C₄)-alkyl groups; or
Z is a 5- or 6-membered saturated or partly unsaturated monocyclic heterocyclic ring containing 1 oxygen atom or one sulfur atom as ring member, where the heterocyclic ring is substituted by a group CO₂R^{e}; or
Z is a 5-membered heteroaromatic ring containing 1, 2, 3 or 4 nitrogen atoms as ring members, where the heteroaromatic ring is substituted by 0 or 1 (C₁-C₄)-alkyl groups; or
X is a bond and Y is (C₁-C₆)-alkyl substituted by a group CO₂R^{e}, CONR^{b}R^{h} or CONR^{e1}SO₂R^{a} and by 0 or 1 (C₁-C₄)-alkoxy groups; or
X is a bond and Y is (C₂-C₈)-alkynyl; or
X is X⁶, wherein R¹⁰ to R¹³ are independently hydrogen; and Y is (C₁-C₄)-alkyl substituted by a group CO₂R^{e};
R^{a} is (C₁-C₆)-alkyl;
R^{b} is hydrogen;
each R^{d} is independently hydrogen or (C₁-C₆)-alkyl;
R^{e1} is hydrogen or (C₁-C₄)-alkyl;
each R^{e} is independently hydrogen, (C₁-C₆)-alkyl which is unsubstituted or substituted by 1, 2 or 3 fluorine or chlorine atoms or by 1 radical selected from the group consisting of (C₁-C₂)-alkoxy, (C₁-C₃)-alkylsulfonyl, (C₁-C₃)-alkylthio, phenylsulfonyl, phenylthio and furanyl; or is (C₂-C₄)-alkynyl or (C₃-C₆)-cycloalkyl-(C₁-C₃)-alkyl;
R^{g} is (C₁-C₃)-alkyl, or two R^{g}, bound on the same carbon atom, form together a methylene group (=CH₂);
R^{h} is (C₁-C₆)-alkyl substituted by 0 or 1 cyano groups; or is (C₂-C₄)-alkynyl;
n is 0, 1 or 2;
q is 1, 2, 3, 4, 5 or 6;
u is 0, 1 or 2;
v is 0 or 1;
w is 0 or 1;
x is 0 or 1;
with the proviso that the sum of u, v, w and x is 1 or 2.

12. A composition comprising at least one compound as claimed in any one of claims 1 to 11, and at least one auxiliary which is customary for formulating crop protection compounds.

13. The composition as claimed in claim 12, comprising a further herbicide.

14. The use of a compound as claimed in any one of claims 1 to 11, or a composition as claimed in claims 12 or 13 for controlling unwanted vegetation.

15. A method for controlling unwanted vegetation which comprises allowing a herbicidally effective amount of at least one compound as claimed in any one of claims 1 to 11, or a composition as claimed in claim 12 or 13 to act on plants, their seed and/or their habitat.

## Patentansprüche

1. Verbindungen der Formel (I) 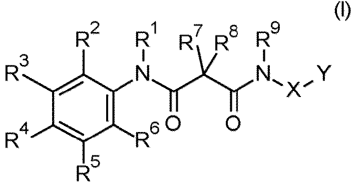 in denen die Substituenten die folgenden Bedeutungen haben:
R¹ steht für Wasserstoff,
R² steht für Wasserstoff oder Halogen,
R³ steht für Wasserstoff, Halogen, Cyano, (C₁-C₃)-Alkyl, (C₁-C₃)-Halogenalkyl, (C₁-C₃)-Alkoxy oder (C₁-C₃)-Halogenalkoxy,
R⁴ steht für Wasserstoff oder Halogen,
R⁵ steht für Wasserstoff, Halogen, Cyano, (C₁-C₃)-Alkyl, (C₁-C₃)-Halogenalkyl, (C₁-C₃)-Alkoxy oder (C₁-C₃)-Halogenalkoxy,
R⁶ steht für Wasserstoff,
R⁷ und R⁸ bilden zusammen mit dem Kohlenstoffatom, an das sie gebunden sind, einen gesättigten oder teilweise ungesättigten 3-, 4-, 5- oder 6-gliedrigen monocyclischen heterocyclischen Ring W oder einen 6-, 7- oder 8-gliedrigen bicyclischen heterocyclischen Ring W, wobei der Ring neben diesem Kohlenstoffatom q Kohlenstoffatome, u Sauerstoffatome, v Stickstoffatome, w Schwefelatome und x Elemente, die aus der Gruppe bestehend aus NR^{d} and NC(O)OR^{d} ausgewählt sind, enthält, wobei ein Kohlenstoffatom p Oxogruppen trägt und wobei der Ring durch n Reste R⁹ substituiert ist,
R⁹ steht für Wasserstoff oder C₁-C₄-Alkyl,
X steht für eine Bindung und Y steht für Z, wobei Z für einen 3-, 4-, 5-, 6-, 7- oder 8-gliedrigen gesättigten oder teilweise ungesättigten monocyclischen carbocyclischen Ring steht, der durch eine Gruppe CO₂R^{e}, durch 0 oder 1 Fluoratome und durch 0 oder 1 (C₁-C₄)-Alkylgruppen substituiert ist, oder
Z für einen 5-, 6-, 7- oder 8-gliedrigen gesättigten oder teilweise ungesättigten bicyclischen carbocyclischen Ring steht, der durch eine Gruppe CO₂R^{e} und durch 0 oder 1 (C₁-C₄)-Alkylgruppen substituiert ist, oder
Z für einen 8-gliedrigen gesättigten polycyclischen carbocyclischen Ring steht, der durch eine Gruppe CO₂R^{e} und durch 0 oder 1 (C₁-C₄)-Alkylgruppen substituiert ist, oder
Z für einen 5- oder 6-gliedrigen gesättigten oder teilweise ungesättigten monocyclischen heterocyclischen Ring mit einem Sauerstoffatom oder einem Schwefelatom als Ringglied steht, wobei der heterocyclische Ring durch eine Gruppe CO₂R^{e} und durch 0 oder 1 (C₁-C₄)-Alkylgruppen substituiert ist, oder
Z für einen 5- oder 6-gliedrigen heteroaromatischen Ring mit 1, 2, 3 oder 4 Heteroatomen, die aus N, O und S ausgewählt sind, als Ringglieder steht, wobei der heteroaromatische Ring durch 0 oder 1 (C₁-C₄)-Alkylgruppen substituiert ist, oder
X steht für eine Bindung und Y steht für (C₁-C₆)-Alkyl oder (C₂-C₆)-Alkenyl, wobei die beiden letztgenannten Reste durch eine Gruppe CO₂R^{e}, CONR^{b}R^{h} oder CONR^{e}SO₂R^{a} und durch 0 oder 1 (C₁-C₄)-Alkoxygruppen substituiert sind, oder
X steht für eine Bindung und Y steht für durch Z substituiertes (C₁-C₆)-Alkyl, wobei Z für einen 3-, 4-, 5- oder 6-gliedrigen gesättigten monocyclischen carbocyclischen Ring steht, der durch eine Gruppe CO₂R^{e} und durch 0 oder 1 (C₁-C₄)-Alkylgruppen substituiert ist, oder für einen 5- oder 6-gliedrigen gesättigten monocyclischen heterocyclischen Ring mit 1 Sauerstoffatom als Ringglied steht, wobei der heterocyclische Ring durch eine Gruppe CO₂R^{e} und durch 0 oder 1 (C₁-C₄)-Alkylgruppen substituiert ist, oder
X steht für eine Bindung und Y steht für (C₂-C₈)-Alkinyl oder
X steht für X⁶, 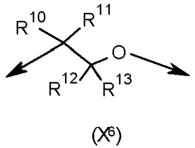
wobei R¹⁰ bis R¹³ unabhängig für Wasserstoff oder Methyl, vorzugsweise Wasserstoff, stehen, und Y steht für (C₁-C₆)-Alkyl, das durch eine Gruppe CO₂R^{e} und durch 0 oder 1 (C₁-C₄)-Alkoxygruppen substituiert ist,
R^{a} steht für C₁-C₆-Alkyl,
R^{b} steht für Wasserstoff oder (C₁-C₆)-Alkyl,
R^{d} steht jeweils unabhängig für Wasserstoff oder (C₁-C₆)-Alkyl,
R^{e} steht jeweils unabhängig für Wasserstoff, (C₁-C₆)-Alkyl, das unsubstituiert oder durch 1, 2 oder 3 Fluor- oder Chloratome oder durch 1 Rest, der aus der Gruppe bestehend aus (C₁-C₂)-Alkoxy, (C₁-C₃)-Alkylsulfonyl, (C₁-C₃)-Alkylthio, Phenylthio, Phenylsulfonyl und Furanyl ausgewählt ist, substituiert ist, oder für (C₂-C₄)-Alkinyl, (C₃-C₆) -Cycloalkyl oder (C₃-C₆) -Cycloalkyl- (C₁-C₃)-alkyl,
R^{g} steht für (C₁-C₃)-Alkyl oder (C₁-C₃)-Halogenalkyl oder zwei R^{g}, die an dasselbe Kohlenstoffatom gebunden sind, bilden zusammen eine Methylengruppe (=CH₂),
R^{h} steht für Wasserstoff, (C₁-C₆)-Alkyl, das durch 0 oder 1 Cyanogruppen substituiert ist, oder (C₂-C₄)-Alkinyl,
n steht für 0, 1 oder 2,
p steht für 0 oder 1,
q steht für 2, 3, 4, 5 oder 6,
u steht für 0, 1 oder 2,
v steht für 0 oder 1,
w steht für 0 oder 1,
x steht für 0 oder 1,
mit der Maßgabe, dass die Summe von u, v, w und x 1 oder 2 beträgt,
einschließlich ihrer landwirtschaftlich unbedenklichen Salze, Stereoisomere und Tautomere.

2. Verbindungen nach Anspruch 1, in denen R⁹ für Wasserstoff steht.

3. Verbindungen nach Anspruch 1 oder 2, in denen R² für Wasserstoff steht.

4. Verbindungen nach einem der Ansprüche 1 bis 3, in denen R⁵ für Wasserstoff, Halogen oder (C₁-C₃)-Alkyl steht.

5. Verbindungen nach einem der Ansprüche 1 bis 4, in denen die Substituenten die folgende Bedeutung haben:
R³ steht für Halogen, Cyano oder (C₁-C₃)-Halogenalkoxy,
R⁵ steht für Wasserstoff oder Halogen.

6. Verbindungen nach einem der Ansprüche 1 bis 5, wobei
u für 1 oder 2 steht, v für 0 steht, w für 0 steht und x für 0 steht oder
u für 0 oder 1 steht, v für 1 steht, w für 0 steht und x für 0 steht oder
u für 0 oder 1 steht, v für 0 steht, w für 1 steht und x für 0 steht oder
u für 0 steht, v für 0 steht, w für 0 steht und x für 1 steht.

7. Verbindungen nach einem der Ansprüche 1 bis 6, in denen die Substituenten die folgende Bedeutung haben:
R⁷ und R⁸ bilden zusammen mit dem Kohlenstoffatom, an das sie gebunden sind, einen gesättigten oder teilweise ungesättigten vier- bis fünfgliedrigen monocyclischen heterocyclischen Ring W, der neben diesem Kohlenstoffatom q Kohlenstoffatome und u Sauerstoffatome enthält, und wobei der Ring durch n Reste R⁹ substituiert ist, wobei u für 1 oder 2 steht und q für 1, 2 oder 3 steht.

8. Verbindungen nach einem der Ansprüche 1 bis 7, wobei jedes R^{d} in den Elementen, die aus der Gruppe bestehend aus NR^{d} und NC(O)OR^{d} als Ringglied des durch R⁷ und R⁸ gebildete Rings ausgewählt sind, unabhängig für Wasserstoff oder (C₁-C₃)-Alkyl steht.

9. Verbindungen nach einem der Ansprüche 1 bis 8, in denen X für eine Bindung steht.

10. Verbindungen nach einem der Ansprüche 1 bis 9, wobei
X für eine Bindung steht und Y für Z steht, wobei
Z für einen 3-, 4-, 5-, 6-, 7- oder 8-gliedrigen gesättigten oder teilweise ungesättigten monocyclischen carbocyclischen Ring steht, der durch eine Gruppe CO₂R^{e}, durch 0 oder 1 Fluoratome und durch 0 oder 1 (C₁-C₄)-Alkylgruppen substituiert ist, oder
Z für einen 5-, 6-, 7- oder 8-gliedrigen gesättigten oder teilweise ungesättigten bicyclischen carbocyclischen Ring steht, der durch eine Gruppe CO₂R^{e} und durch 0 oder 1 (C₁-C₄)-Alkylgruppen substituiert ist, oder
Z für einen 8-gliedrigen gesättigten polycyclischen carbocyclischen Ring steht, der durch eine Gruppe CO₂R^{e} und durch 0 oder 1 (C₁-C₄)-Alkylgruppen substituiert ist, oder
Z für einen 5- oder 6-gliedrigen gesättigten oder teilweise ungesättigten monocyclischen heterocyclischen Ring mit einem Sauerstoffatom oder einem Schwefelatom als Ringglied steht, wobei der heterocyclische Ring durch eine Gruppe CO₂R^{e} und durch 0 oder 1 (C₁-C₄)-Alkylgruppen substituiert ist, oder
Z für einen 5- oder 6-gliedrigen heteroaromatischen Ring mit 1, 2, 3 oder 4 Heteroatomen, die aus N, O und S ausgewählt sind, als Ringglieder steht, wobei der heteroaromatische Ring durch 0 oder 1 (C₁-C₄)-Alkylgruppen substituiert ist.

11. Verbindungen nach Anspruch 1, in denen die Substituenten die folgende Bedeutung haben:
R¹ steht für Wasserstoff,
R² steht für Wasserstoff,
R³ steht für Halogen, Cyano oder (C₁-C₃)-Halogenalkoxy,
R⁴ steht für Wasserstoff oder Halogen,
R⁵ steht für Wasserstoff, Halogen oder (C₁-C₃)-Alkyl, insbesondere Wasserstoff oder Halogen,
R⁶ steht für Wasserstoff,
R⁷ und R⁸ bilden zusammen mit dem Kohlenstoffatom, an das sie gebunden sind, einen gesättigten oder teilweise ungesättigten 3-, 4-, 5- oder 6-gliedrigen monocyclischen heterocyclischen Ring W oder einen 6-, 7- oder 8-gliedrigen bicyclischen heterocyclischen Ring W, wobei der Ring neben diesem Kohlenstoffatom q Kohlenstoffatome, u Sauerstoffatome, v Stickstoffatome, w Schwefelatome und x Elemente, die aus der Gruppe bestehend aus NR^{d} and NC(O)OR^{d} ausgewählt sind, enthält, wobei der Ring durch n Reste R⁹ substituiert ist,
R⁹ steht für Wasserstoff,
X steht für eine Bindung und Y steht für Z, wobei
Z für einen 4-, 5-, 6-, 7- oder 8-gliedrigen gesättigten oder teilweise ungesättigten monocyclischen carbocyclischen Ring steht, der durch eine Gruppe CO₂R^{e} und durch 0 oder 1 Fluoratome substituiert ist, oder
Z für einen 5-, 6-, 7- oder 8-gliedrigen gesättigten oder teilweise ungesättigten bicyclischen carbocyclischen Ring steht, der durch eine Gruppe CO₂R^{e} und durch 0 oder 1 (C₁-C₄)-Alkylgruppen substituiert ist, oder
Z für einen 5- oder 6-gliedrigen gesättigten oder teilweise ungesättigten monocyclischen heterocyclischen Ring mit 1 Sauerstoffatom oder einem Schwefelatom als Ringglied steht, wobei der heterocyclische Ring durch eine Gruppe CO₂R^{e} substituiert ist, oder
Z für einen 5-gliedrigen heteroaromatischen Ring mit 1, 2, 3 oder 4 Stickstoffatomen als Ringglieder steht, wobei der heteroaromatische Ring durch 0 oder 1 (C₁-C₄)-Alkylgruppen substituiert ist, oder
X steht für eine Bindung und Y steht für (C₁-C₆)-Alkyl, das durch eine Gruppe CO₂R^{e}, CONR^{b}R^{h} oder CONR^{e1}SO₂R^{a} und durch 0 oder 1 (C₁-C₄)-Alkoxygruppen substituiert ist, oder
X steht für eine Bindung und Y steht für (C₂-C₈)-Alkinyl oder
X steht für X⁶, wobei R¹⁰ bis R¹³ unabhängig für Wasserstoff stehen, und Y steht für (C₁-C₄)-Alkyl, das durch eine Gruppe CO₂R^{e} substituiert ist,
R^{a} steht für C₁-C₆-Alkyl,
R^{b} steht für Wasserstoff,
R^{d} steht jeweils unabhängig für Wasserstoff oder (C₁-C₆)-Alkyl,
R^{e1} steht für Wasserstoff oder (C₁-C₄)-Alkyl;
R^{e} steht jeweils unabhängig für Wasserstoff, (C₁-C₆)-Alkyl, das unsubstituiert oder durch 1, 2 oder 3 Fluor- oder Chloratome oder durch 1 Rest, der aus der Gruppe bestehend aus (C₁-C₂)-Alkoxy, (C₁-C₃)-Alkylsulfonyl, (C₁-C₃)-Alkylthio, Phenylsulfonyl, Phenylthio und Furanyl ausgewählt ist, substituiert ist, oder steht für (C₂-C₄)-Alkinyl oder (C₃-C₆)-Cycloalkyl-(C₁-C₃)-alkyl,
R^{g} steht für (C₁-C₃)-Alkyl oder zwei R^{g}, die an dasselbe Kohlenstoffatom gebunden sind, bilden zusammen eine Methylengruppe (=CH₂),
R^{h} steht für (C₁-C₆)-Alkyl, das durch 0 oder 1 Cyanogruppen substituiert ist, oder für (C₂-C₄)-Alkinyl,
n steht für 0, 1 oder 2,
q steht für 2, 3, 4, 5 oder 6,
u steht für 0, 1 oder 2,
v steht für 0 oder 1,
w steht für 0 oder 1,
x steht für 0 oder 1,
mit der Maßgabe, dass die Summe von u, v, w und x 1 oder 2 beträgt.

12. Zusammensetzung, umfassend mindestens eine Verbindung nach einem der Ansprüche 1 bis 11 und mindestens einen für die Formulierung von Pflanzenschutzmitteln üblichen Hilfsstoff.

13. Zusammensetzung nach Anspruch 12, umfassend ein weiteres Herbizid.

14. Verwendung einer Verbindung nach einem der Ansprüche 1 bis 11 oder einer Zusammensetzung nach einem der Ansprüche 12 oder 13 zur Bekämpfung von unerwünschtem Pflanzenwuchs.

15. Verfahren zur Bekämpfung von unerwünschtem Pflanzenwuchs, bei dem man eine herbizid wirksame Menge mindestens einer Verbindung nach einem der Ansprüche 1 bis 11 oder einer Zusammensetzung nach Anspruch 12 oder 13 auf Pflanzen, deren Saatgut und/oder deren Lebensraum einwirken lässt.

## Revendications

1. Composés de formule (I) 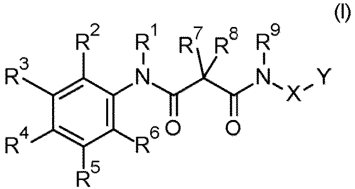 les substituants ayant les significations suivantes :
R¹ étant hydrogène ;
R² étant hydrogène ou halogène ;
R³ étant hydrogène, halogène, cyano, (C₁-C₃) -alkyle, (C₁-C₃) -halogénoalkyle, (C₁-C₃)-alcoxy ou (C₁-C₃)-halogénoalcoxy ;
R⁴ étant hydrogène ou halogène ;
R⁵ étant hydrogène, halogène, cyano, (C₁-C₃)-alkyle, (C₁-C₃)-halogénoalkyle, (C₁-C₃)-alcoxy ou (C₁-C₃)-halogénoalcoxy ;
R⁶ étant hydrogène ;
R⁷ et R⁸ formant, conjointement avec l'atome de carbone auquel ils sont liés, un cycle hétérocyclique monocyclique saturé ou partiellement insaturé à 3, 4, 5 ou 6 chaînons W ou un cycle hétérocyclique bicyclique à 6, 7 ou 8 chaînons W, le cycle contenant, en plus dudit atome de carbone, q atomes de carbone, u atomes d'oxygène, v atomes d'azote, w atomes de soufre et x éléments choisis dans le groupe constitué par NR^{d} et NC(O)OR^{d}, un atome de carbone portant p groupes oxo et le cycle étant substitué par n radicaux R^{g} ;
R⁹ étant hydrogène ou (C₁-C₄)-alkyle ;
X étant une liaison et Y étant Z,
Z étant un cycle carbocyclique monocyclique saturé ou partiellement insaturé à 3, 4, 5, 6, 7 ou 8 chaînons substitué par un groupe CO₂R^{e}, par 0 ou 1 atome de fluor et par 0 ou 1 groupe (C₁-C₄)-alkyle ; ou
Z étant un groupe carbocyclique bicyclique saturé ou partiellement insaturé à 5, 6, 7 ou 8 chaînons substitué par un groupe CO₂R^{e}, et par 0 ou 1 groupe (C₁-C₄)-alkyle ; ou
Z étant un cycle carbocyclique polycyclique saturé à 8 chaînons substitué par un groupe CO₂R^{e} et par 0 ou 1 groupe (C₁-C₄)-alkyle ; ou
Z étant cycle hétérocyclique monocyclique saturé ou partiellement insaturé à 5 ou 6 chaînons contenant un atome d'oxygène ou un atome de soufre en tant qu'élément de cycle, le cycle hétérocyclique étant substitué par un groupe CO₂R^{e} et par 0 ou 1 groupe (C₁-C₄)-alkyle ; ou
Z étant un cycle hétéroaromatique à 5 ou 6 chaînons contenant 1, 2, 3 ou 4 hétéroatomes choisis parmi N, O et S en tant qu'éléments de cycle, le cycle hétéroaromatique étant substitué par 0 ou 1 groupe (C₁-C₄)-alkyle ; ou
X étant une liaison et Y étant (C₁-C₆)-alkyle ou (C₂-C₆)-alcényle, les deux radicaux mentionnés en dernier étant substitués par un groupe CO₂R^{e}, CONR^{b}R^{h} ou CONR^{e}SO₂R^{a} et par 0 ou 1 groupe (C₁-C₄)-alcoxy ; ou
X étant une liaison et Y étant (C₁-C₆) -alkyle substitué par Z, Z étant un cycle carbocyclique monocyclique saturé à 3, 4, 5 ou 6 chaînons substitué par un groupe CO₂R^{e} et par 0 ou 1 groupe (C₁-C₄)-alkyle ou étant un cycle hétérocyclique monocyclique saturé à 5 ou 6 chaînons contenant 1 atome d'oxygène en tant qu'élément de cycle, le cycle hétérocyclique étant substitué par un groupe CO₂R^{e} et par 0 ou 1 groupe (C₁-C₄)-alkyle ; ou
X étant une liaison et Y étant (C₂-C₈)-alcynyle ; ou
X étant X⁶ 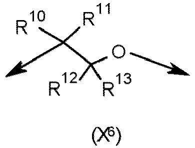
R¹⁰ à R¹³ étant indépendamment hydrogène ou méthyle, préférablement hydrogène ; et Y étant (C₁-C₆)-alkyle substitué par un groupe CO₂R^{e} et par 0 ou 1 groupe (C₁-C₄)-alcoxy ;
R^{a} étant (C₁-C₆)-alkyle ;
R^{b} étant hydrogène ou (C₁-C₆)-alkyle ;
chaque R^{d} étant indépendamment hydrogène ou (C₁-C₆)-alkyle ;
chaque R^{e} étant indépendamment hydrogène, (C₁-C₆)-alkyle qui est non substitué ou substitué par 1, 2 ou 3 atomes de fluor ou de chlore ou par 1 radical choisi dans le groupe constitué par (C₁-C₂) -alcoxy, (C₁-C₃)-alkylsulfonyle, (C₁-C₃)-alkylthio, phénylthio, phénylsulfonyle et furanyle ; ou étant (C₂-C₄)-alcynyle, (C₃-C₆)-cycloalkyle ou (C₃-C₆) -cycloalkyl-(C₁-C₃)-alkyle ; R^{g} étant (C₁-C₃)-alkyle ou (C₁-C₃)-halogénoalkyle, ou deux R^{g}, liés sur le même atome de carbone, formant ensemble un groupe méthylène (=CH₂) ;
R^{h} étant hydrogène, (C₁-C₆)-alkyle substitué par 0 ou 1 groupe cyano ; ou (C₂-C₄)-alcynyle ;
n étant 0, 1 ou 2 ;
p étant 0 ou 1 ;
q étant 1, 2, 3, 4, 5 ou 6 ;
u étant 0, 1 ou 2 ;
v étant 0 ou 1 ;
w étant 0 ou 1 ;
x étant 0 ou 1 ;
à la condition que la somme de u, v, w et x soit 1 ou 2 ; y compris leurs sels, stéréoisomères et formes tautomères acceptables sur le plan agricole.

2. Composés selon la revendication 1, R⁹ étant hydrogène.

3. Composés selon la revendication 1 ou 2, R² étant hydrogène.

4. Composés selon l'une quelconque des revendications 1 à 3, R⁵ étant hydrogène, halogène ou (C₁-C₃)-alkyle.

5. Composés selon l'une quelconque des revendications 1 à 4, les substituants ayant la signification suivante :
R³ étant halogène, cyano ou (C₁-C₃)-halogénoalcoxy ;
R⁵ étant hydrogène ou halogène.

6. Composés selon l'une quelconque des revendications 1 à 5,
u étant 1 ou 2, v étant 0, w étant 0 et x étant 0 ; ou
u étant 0 ou 1, v étant 1, w étant 0 et x étant 0 ; ou
u étant 0 ou 1, v étant 0, w étant 1 et x étant 0 ; ou
u étant 0, v étant 0, w étant 0 et x étant 1.

7. Composés selon l'une quelconque des revendications 1 à 6, les substituants ayant la signification suivante :
R⁷ et R⁸ formant, conjointement avec l'atome de carbone auquel ils sont liés, un cycle hétérocyclique monocyclique saturé ou partiellement insaturé à quatre ou cinq chaînons W, contenant, en plus de cet atome de carbone, q atomes de carbone et u atomes d'oxygène, et le cycle étant substitué par n radicaux R^{g}, u étant 1 ou 2 et q étant 1, 2 ou 3.

8. Composés selon l'une quelconque des revendications 1 à 7, chaque R^{d} dans les éléments choisis dans le groupe constitué par NR^{d} et NC(O)OR^{d} en tant qu'élément de cycle du cycle formé par R⁷ et R⁸ étant indépendamment hydrogène ou (C₁-C₃)-alkyle.

9. Composés selon l'une quelconque des revendications 1 à 8, X étant une liaison.

10. Composés selon l'une quelconque des revendications 1 à 9, X étant une liaison et Y étant Z,
Z étant un cycle carbocyclique monocyclique saturé ou partiellement insaturé à 3, 4, 5, 6, 7 ou 8 chaînons substitué par un groupe CO₂R^{e}, par 0 ou 1 atome de fluor et par 0 ou 1 groupe (C₁-C₄)-alkyle ; ou
Z étant un groupe carbocyclique bicyclique saturé ou partiellement insaturé à 5, 6, 7 ou 8 chaînons substitué par un groupe CO₂R^{e}, et par 0 ou 1 groupe (C₁-C₄)-alkyle ; ou
Z étant un cycle carbocyclique polycyclique saturé à 8 chaînons substitué par un groupe CO₂R^{e} et par 0 ou 1 groupe (C₁-C₄)-alkyle ; ou
Z étant cycle hétérocyclique monocyclique saturé ou partiellement insaturé à 5 ou 6 chaînons contenant un atome d'oxygène ou un atome de soufre en tant qu'élément de cycle, le cycle hétérocyclique étant substitué par un groupe CO₂R^{e} et par 0 ou 1 groupe (C₁-C₄)-alkyle ; ou
Z étant un cycle hétéroaromatique à 5 ou 6 chaînons contenant 1, 2, 3 ou 4 hétéroatomes choisis parmi N, O et S en tant qu'éléments de cycle, le cycle hétéroaromatique étant substitué par 0 ou 1 groupe (C₁-C₄)-alkyle.

11. Composés selon la revendication 1, les substituants ayant la signification suivante :
R¹ étant hydrogène ;
R² étant hydrogène ;
R³ étant halogène, cyano ou (C₁-C₃)-halogénoalcoxy ;
R⁴ étant hydrogène ou halogène ;
R⁵ étant hydrogène, halogène ou (C₁-C₃)-alkyle ; en particulier hydrogène ou halogène ;
R⁶ étant hydrogène ;
R⁷ et R⁸ formant, conjointement avec l'atome de carbone auquel ils sont liés, un cycle hétérocyclique monocyclique saturé ou partiellement insaturé à 3, 4, 5 ou 6 chaînons W ou un cycle hétérocyclique bicyclique à 6, 7 ou 8 chaînons W, le cycle contenant, en plus dudit atome de carbone, q atomes de carbone, u atomes d'oxygène, v atomes d'azote, w atomes de soufre et x éléments choisis dans le groupe constitué par NR^{d} et NC(O)OR^{d}, le cycle étant substitué par n radicaux R^{g} ;
R⁹ étant hydrogène ;
X étant une liaison et Y étant Z,
Z étant un cycle carbocyclique monocyclique saturé ou partiellement insaturé à 4, 5, 6, 7 ou 8 chaînons substitué par un groupe CO₂R^{e} et par 0 ou 1 atome de fluor ; ou
Z étant un groupe carbocyclique bicyclique saturé ou partiellement insaturé à 5, 6, 7 ou 8 chaînons substitué par un groupe CO₂R^{e} et par 0 ou 1 groupe (C₁-C₄)-alkyle ; ou
Z étant un cycle hétérocyclique monocyclique saturé ou partiellement insaturé à 5 ou 6 chaînons contenant 1 atome d'oxygène ou un atome de soufre en tant qu'élément de cycle, le cycle hétérocyclique étant substitué par un groupe CO₂R^{e} ; ou
Z étant un cycle hétéroaromatique à 5 chaînons contenant 1, 2, 3 ou 4 atomes d'azote en tant qu'éléments de cycle, le cycle hétéroaromatique étant substitué par 0 ou 1 groupe (C₁-C₄)-alkyle ; ou
X étant une liaison et Y étant (C₁-C₆) -alkyle substitué par un groupe CO₂R^{e}, CONR^{b}R^{h} ou CONR^{e1}SO₂R^{a} et par 0 ou 1 groupe (C₁-C₄)-alcoxy ; ou
X étant une liaison et Y étant (C₂-C₈)-alcynyle ; ou
X étant X⁶, R¹⁰ à R¹³ étant indépendamment hydrogène ; et
Y étant (C₁-C₄)-alkyle substitué par un groupe CO₂R^{e} ;
R^{a} étant (C₁-C₆)-alkyle ;
R^{b} étant hydrogène ;
chaque R^{d} étant indépendamment hydrogène ou (C₁-C₆)-alkyle ;
R^{e1} étant hydrogène ou (C₁-C₄)-alkyle ;
chaque R^{e} étant indépendamment hydrogène, (C₁-C₆)-alkyle qui est non substitué ou substitué par 1, 2 ou 3 atomes de fluor ou de chlore ou par 1 radical choisi dans le groupe constitué par (C₁-C₂) -alcoxy, (C₁-C₃)-alkylsulfonyle, (C₁-C₃)-alkylthio, phénylsulfonyle, phénylthio et furanyle ; ou étant (C₂-C₄) -alcynyle ou (C₃-C₆)-cycloalkyl-(C₁-C₃)-alkyle ;
R^{g} étant (C₁-C₃)-alkyle, ou deux R^{g}, liés sur le même atome de carbone, formant ensemble un groupe méthylène (=CH₂) ;
R^{h} étant (C₁-C₆)-alkyle substitué par 0 ou 1 groupe cyano ; ou étant (C₂-C₄)-alcynyle ;
n étant 0, 1 ou 2 ;
q étant 1, 2, 3, 4, 5 ou 6 ;
u étant 0, 1 ou 2 ;
v étant 0 ou 1 ;
w étant 0 ou 1 ;
x étant 0 ou 1 ;
à la condition que la somme de u, v, w et x soit 1 ou 2.

12. Composition comprenant au moins un composé selon l'une quelconque des revendications 1 à 11 et au moins un auxiliaire qui est usuel pour la formulation de composés de protection de cultures.

13. Composition selon la revendication 12, comprenant un autre herbicide.

14. Utilisation d'un composé selon l'une quelconque des revendications 1 à 11, ou d'une composition selon l'une quelconque des revendications 12 ou 13 pour la lutte contre de la végétation indésirable.

15. Procédé de lutte contre de la végétation indésirable, qui comprend le fait de laisser agir une quantité efficace sur le plan herbicide d'au moins un composé selon l'une quelconque des revendications 1 à 11, ou d'une composition selon la revendication 12 ou 13, sur des végétaux, leurs semences et/ou leur habitat.
